# EUROPEAN PATENT APPLICATION

(11) **EP 1 122 252 A1**
(43) Date of publication of application: **08.08.2001**
(21) Application number: 99947923.1
(22) Date of filing: 15.10.1999
(51) Int. Cl.: C07D 401/04, C07D 401/06, C07D 401/12, C07D 401/14, C07D 413/06, C07D 217/22, C07D 223/16, C07D 498/04, C07D 513/04, A61K 31/4545, A61K 31/55, A61K 31/553, A61P 3/04

(54) **NITROGENOUS FUSED HETEROCYCLE COMPOUNDS, PROCESS FOR THE PREPARATION THEREOF AND AGENTS CONTAINING THE SAME**

(30) Priority: 16.10.1998 JP 29521398; 16.10.1998 JP 29548898
(71) Applicant: Takeda Chemical Industries, Ltd., Osaka-shi, Osaka 541-0045 (JP)
(72) Inventor: ISHIHARA, Yuji, Itami-shi Hyogo 664-0874 (JP); FUJISAWA, Yukio, Tsukuba-shi, Ibaraki 305-0032 (JP); FURUYAMA, Naoki, Kobe-shi, Hyogo 657-0016 (JP); ISHICHI, Yuji, Ibaraki-shi, Osaka 567-0867 (JP); SASAKI, Mitsuru, Takatsuki-shi, Osaka 569-1041 (JP)
(74) Representative: Caffin, Lee
(86) International application number: JP9905705
(87) International publication number: WO0023437

(57) **Abstract**

A nitrogen-containing condensed heterocyclic derivative of the present invention, which is a compound represented by the formula: 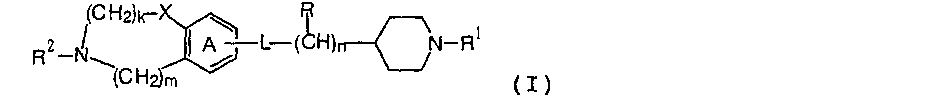 wherein ring A represents benzene ring optionally having a further substituent, -L-represents -O-, -NR^{3a}-, -S-, -SO-, -SO₂-, -SO₂NR^{3a}-, -SO₂NHCONR^{3a}-, -SO₂NHC(=NH)NR^{3a}-, -C(=S)-, 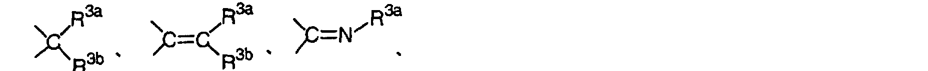 or -CONR^{3a}- (wherein R^{3a} and R^{3b} represent independently hydrogen atom, cyano group, hydroxy group, amino group, a C₁₋₆ alkyl group or a C₁₋₆ alkoxy group), n represents an integer of 0 to 6, R is hydrogen atom or a hydrocarbon group optionally having a substituent, and may be different in repetition of n, R¹ represents a hydrocarbon group optionally having a substituent or a group represented by the formula: 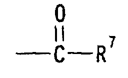 (wherein R⁷ represents a hydrocarbon group optionally having a substituent), R² represents hydrogen atom, an acyl group, a hydrocarbon group optionally having a substituent or a heterocyclic group optionally having a substituent, X represents a bond, O, S, SO, SO₂ or NR⁴ (wherein R⁴ represents hydrogen atom, an acyl group or a hydrocarbon group optionally having a substituent), k and m represent independently an integer of 0 to 5, and 1<k+m<5, or a salt thereof, and the like, has an excellent thermal production promoting activity and the like, and is useful as an agent for preventing or treating obesity and obesity-based diseases.

## Description

### TECHINICAL FIELD

The present invention relates to a pharmaceutical, more particularly, a novel nitrogen-containing condensed heterocyclic derivative which has excellent pharmaceutical activity such as thermal production promoting activity and the like, and an agent for preventing or treating obesity or obesity-based diseases containing the same.

### BACKGROUND ART

As a drug for treating obesity, central appetite inhibiting agents such as mazindol and the like are used. However, central appetite inhibiting agents have central side effects such as dependency and the like, systema digestorium side effects such as nausea, vomiting and the like and, for that reason, their application is limited only to highly obese patients.

On the other hand, a β3 adrenaline receptor agonist is proposed as an anti-obesity drug having a peripheral acting point (Nature vol. 309, p.163-165 (1984); J. Med. Chem., vol. 35, p. 3081-3084 (1992) and the like). However, it is reported that there is a mutation in a β3 adrenaline receptor gene in a considerable number of obese patients (New Engl. J. Med., vol. 333, p. 343-347 (1995); Lancet, vol. 346, p. 1433-1434 (1995); Biochem. Biophys. Res. Commun., vol. 215, p. 555-560 (1995)).

In addition, a variety of nitrogen-containing heterocyclic derivatives are proposed in JP-A 55-162783, JP-A 8-505145, JP-A 8-188564, J. Med. Chem., 40, 2085-2101 (1997), JP-A 7-500341, J. Med. Chem., 35, 4344-4361 (1992), JP-A 10-502939. However, there is no suggestion or description regarding their activity as an agent for preventing or treating obesity and diseases occurring in connection with obesity, their activity as a lipolysis promoting agent, or their activity as a thermal production promoting agent.

Particularly, JP-A 55-162783 describes an aminopropanol derivative represented by the formula: 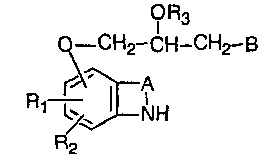 wherein R₁ and R₂ may be the same or different and each represents hydrogen atom or a lower alkyl group, or taken together to represent an alkylene group, R₃ represents hydrogen atom or an acyl group, A represents a structural element represented by the formula: 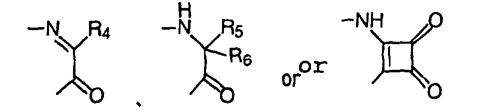 wherein R₄ represents hydrogen atom or a lower alkyl group, the alkyl group being optionally substituted with hydroxy group, halogen, phenyl group or an alkylthio group, R₅ and R₆ each represent a lower alkyl group, and may be the same or different, B represents an alkylamino group optionally having phenyl group or phenoxy group or both, the phenyl group and the phenoxy group being optionally substituted with one or more of halogen, hydroxy group, a lower alkyl group, a lower acyl group, a lower alkylthio group, an acylamino group, aminocarbonyl group, a lower alkoxy group, a lower alkenyloxy group, phenoxy group, a lower alkenyl group, a lower alkylsulfonyl group, a lower alkylsulfinyl group or a halogenoalkyl group, or B represents an aryloxymethylpiperidine group or a heteroaryloxymethylpiperidine group, these groups being optionally substituted with one or more of halogen, hydroxy group, a lower alkyl group optionally having hydroxy group or carboxyamide group, or a lower alkoxy group, a lower acyl group, amino group, carboxamide group, a lower alkylcarbonylamide group or a lower alkylsulfonylamino group, or a salt thereof to have vasodilator activity and β receptor blocking activity. Among the particular compounds, the following compound is described:

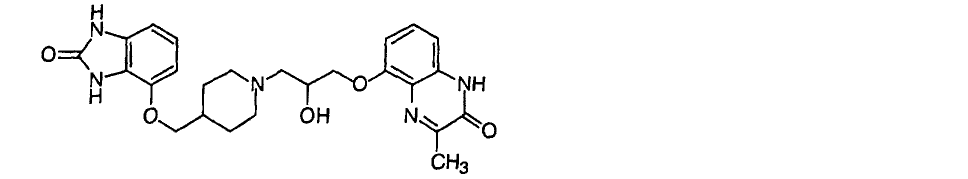

JP-A 8-505145 describes the following compound as one of the embodiments having a glycoprotein IIb/IIIa antagonistic activity and a platelet aggregation inhibiting activity.

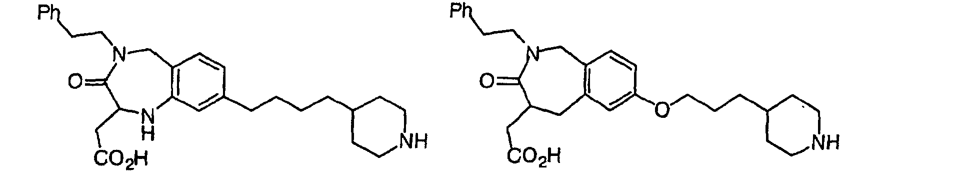

JP-A 8-188564 describes that a bicyclic compound having a skeleton formed of two 6 membered rings, A and B, and represented by the formula: 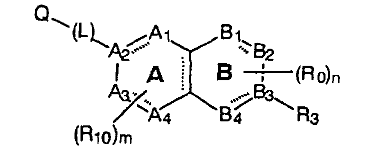 wherein B₁, B₂, B₃ and B₄ are independently selected from carbon, oxygen, sulfur and nitrogen, provided that at least two of B₁, B₂, B₃ and B₄ are carbon, R₃ is an acidic group, n is a number of 2 to 6, R₀ is the same or different and is independently selected from hydrogen, an alkyl, a halo-substituted alkyl, an alkenyl, an alkynyl, a cycloalkyl, an aryl, an arylalkyl, hydroxy, an alkoxy, an aralkoxy, amino, a substituted amino, carbamyl, carboxy, an acyl, cyano, halo, nitro, sulfo, =O and =S, provided that when R₀ is =O or =S, only one of B₁, B₂, B₃ and B₄ can be nitrogen, A₁, A₂, A₃ and A₄ are independently selected from carbon, oxygen, sulfur and nitrogen, provided that at least two of A₁, A₂, A₃ and A₄ are carbon, m is a number of 2 to 6, R₁₀ is the same or different and is independently selected from hydrogen, an alkyl, a halo-substituted alkyl, an alkenyl, an alkynyl, a cycloalkyl, an aryl, an arylalkyl, hydroxy, an alkoxy, an aralkoxy, carboxy, an acyl, cyano, halo, nitro, sulfo, =O and =S, provided that only one of R₁₀ can be =O or =S, a linking group -(L)- is a divalent substituted or unsubstituted chain of 1 to 10 atoms selected from the group consisting of carbon, oxygen, sulfur and nitrogen, and Q is an organic group containing a basic group, has a glycoprotein IIb/IIIa antagonistic activity and a platelet aggregation inhibiting activity. As one of embodiments, the following compound is described.

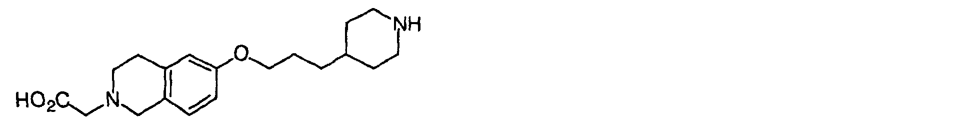

J. Med. Chem., 40, 2085-2101 (1997) describes a compound represented by the formula: 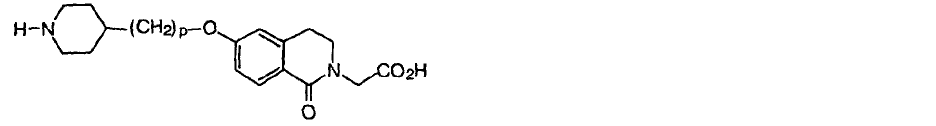 wherein p = 1-4 to have a glycoprotein IIb/IIIa antagonistic activity and a platelet aggregation inhibiting activity.

JP-A 7-500341 describes that the following compound and the like have a glycoprotein IIb/IIIa antagonistic and a platelet aggregation inhibiting activity:

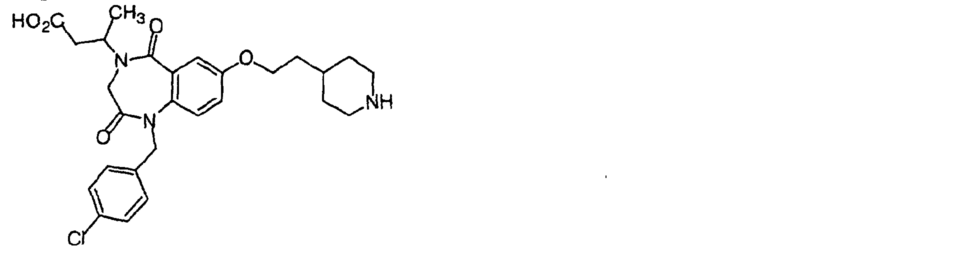

J. Med. Chem., 35, 4344-4361 (1992) describes that the following compound has a sigma receptor binding activity and an antipsychotic activity:

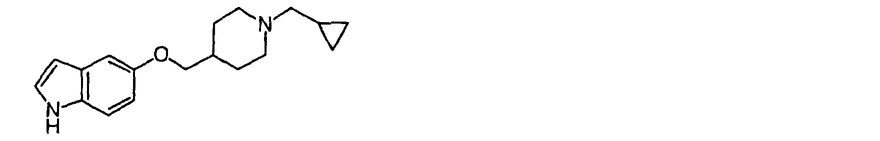

JP-A 10-502939 describes that the following compound: 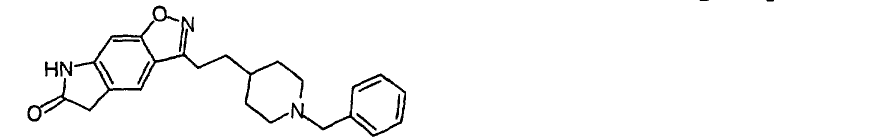 has an acetylcholinesterase inhibiting activity and the following compound: 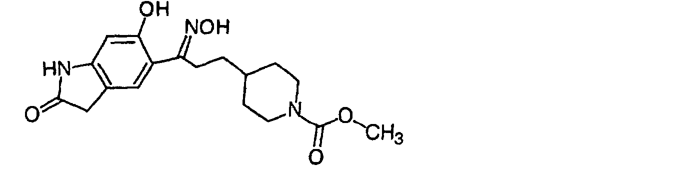 is an intermediate for synthesizing it.

Further, JP-A 6-500794 and J. Med. Chem., 38, 2802-2808 (1995) propose a nitrogen-containing heterocyclic condensed-benzisoxazole derivative. They describe activity as an acetylcholinesterase inhibiting agent but neither suggest nor disclose activity as an agent for preventing or treating obesity and diseases occurring in connection with obesity, activity as a lipolysis promoting agent and activity as a thermal production promoting agent.

More particularly, JP-A 6-500794 describes a compound represented by the formula: 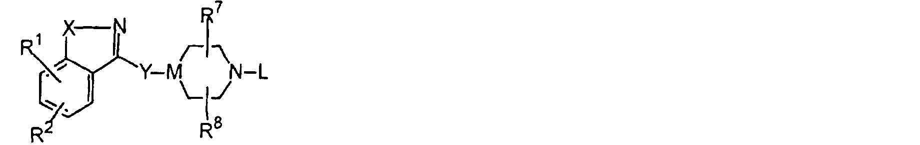 wherein R¹ and R² are independently selected from hydrogen, a (C₁-C₆)alkoxy, benzyloxy, phenoxy, hydroxy, phenyl, benzyl, halo, nitro, cyano, COR⁵, -COOR⁵, -CONHR⁵, -NR⁵R⁶, -NR⁵COR⁶, -OCONR⁵R⁶, -NHCOOR⁵, a (C₁-C₆)alkyl (this is substituted with 1 to 3 fluorine atoms); SOpCH₂-phenyl or SOp(C₁-C₆)alkyl (wherein p is 0, 1 or 2); pyridylmethyloxy or thienylmethyloxy; 2-oxazolyl, 2-thiazolyl and benzenesulfonamide, wherein a phenyl part of the above phenoxy, benzyloxy, phenyl, benzyl and benzenesulfonamide group, a pyridyl and thienyl part of the above pyridylmethyloxy or thienylmethyloxy, and an oxazolyl and thiazolyl part of the above 2-oxazolyl and 2-thiazolyl may be optionally substituted with 1 or 2 substituents selected independently from halo, a (C₁-C₆)alkyl, trifluoromethyl, a (C₁-C₆)alkoxy, cyano, nitro and hydroxy; or when R¹ and R² are bonded to the adjacent carbon atom and when X is oxygen, sulfur or NR⁴ (wherein R⁴ is hydrogen or a (C₁-C₄)alkyl), they may be taken together with a carbon atom to which they bind to form a group represented by the formula: 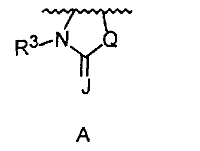 or 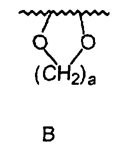 wherein J is oxygen, sulfur or NR⁴, "a" is 1 or 2, R³ is hydrogen or a (C₁-C₆)alkyl, Q is oxygen, sulfur, NH, CHCH₃, (CH₃)₂C, -CH=CH- or (CH₂)ₗ, wherein l is an integer of 1 to 3; X is oxygen, sulfur, -CH=CH-, -CH=N-, -NH=CH-, -N=N- or NR⁴, wherein R⁴ is hydrogen or a (C₁-C₄)alkyl; Y is -(CH₂)ₘ-, -CH=CH(CH₂)ₙ-, -NR⁴(CH₂)ₘ- or -O(CH₂)ₘ-, wherein R⁴ has the above meaning, n is an integer of 0 to 3, m is an integer of 1 to 3; R⁵ and R⁶ are independently selected from hydrogen, a (C₁-C₆)alkyl, phenyl or benzyl, wherein a phenyl part of the above phenyl or benzyl may be optionally substituted with 1 or 2 substituents selected independently from fluoro, chloro, bromo, iodo, a (C₁-C₄)alkyl, trifluoromethyl, a (C₁-C₄)alkoxy, cyano, nitro and hydroxy, or NR⁵R⁶ is taken together to form a 4-8 membered ring in which one atom of the ring is nitrogen and other atoms are carbon, oxygen or nitrogen, or NR⁵COR⁶ is taken together to form a 4-8 membered cyclic lactam ring; M is -CH- or nitrogen; L is phenyl, a phenyl-(C₁-C₆)alkyl, cinnamyl or pyridylmethyl, wherein a phenyl part of the above phenyl and phenyl-(C₁-C₆)alkyl may be optionally substituted with 1 to 3 substituents selected independently from a (C₁-C₆)alkyl, a (C₁-C₆)alkoxy, a (C₁-C₄)alkoxycarbonyl, a (C₁-C₄)alkylcarbonyl, -OCONR⁵R⁶, -NHCOOR⁵ or halo; or L is a group represented by the formula: 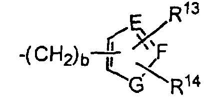 wherein b is an integer of 1 to 4, R¹³ and R¹⁴ are selected independently from hydrogen, a (C₁-C₄)alkyl, halo and phenyl, E and F are selected independently from -CH- and nitrogen, further G is oxygen, sulfur or NR⁴, wherein R⁴ has the above meaning, provided that when both E and F are nitrogen, one of R¹³ and R¹⁴ is not present; R⁷ and R⁸ are independently selected from a (C₁-C₆)alkyl, a (C₁-C₆)alkoxycarbonyl, a (C₁-C₆)alkylcarbonyl and a (C₁-C₆)alkoxy, provided that the above (C₁-C₆)alkoxy is not bonded to the adjacent carbon atom, or a salt thereof (embodiments thereof are compounds represented by the following formulae) as an acetylcholinesterase inhibitor.

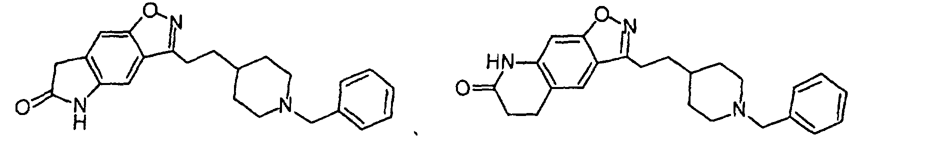

J. Med. Chem., 38, 2802-2808 (1995) describes compounds represented by the formula: 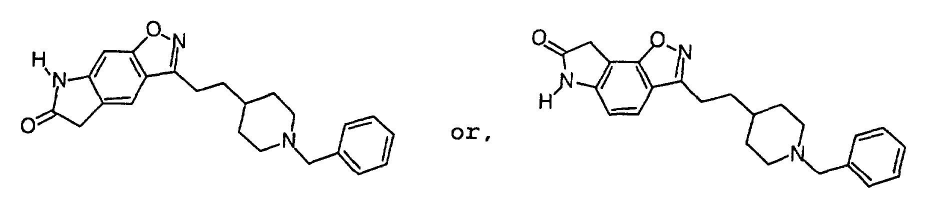 or salts thereof and the like as an acetylcholinesterase inhibitor.

Development is desired of a novel compound which has less central side effects and more general purposes as compared with known compounds known to have an anti-obesity activity, and which is useful as an agent for preventing or treating obesity and obesity-based diseases.

### DISCLOSURE OF INVENTION

The present inventors have continued to search for and examine a new thermal production promoting agent and an anti-obesity agent having no central side effects and have studied intensively and, as a result, they synthesized for the first time a novel nitrogen-containing condensed heterocyclic derivative in which a particular side chain is bonded to a condensed hetero ring via "L" having a particular chemical structure, and is represented by the formula: 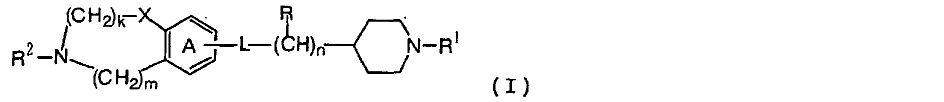 wherein ring A represents benzene ring optionally having a further substituent, -L- represents -O-, -NR^{3a}-, -S-, -SO-, -SO₂- , -SO₂NR^{3a}-, -SO₂NHCONR^{3a}-, -SO₂NHC(=NH)NR^{3a}-, -C(=S)-, 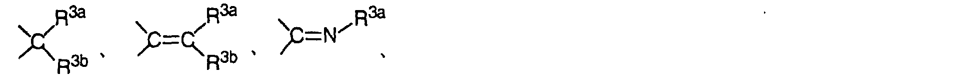 or -CONR^{3a}- (wherein R^{3a} and R^{3b} represent independently hydrogen atom, cyano group, hydroxy group, amino group, a C₁₋₆ alkyl group or a C₁₋₆ alkoxy group), n represents an integer of 0 to 6, R is hydrogen atom or a hydrocarbon group optionally having a substituent, and may be different in repetition of n, R¹ represents a hydrocarbon group optionally having a substituent or a group represented by the formula: 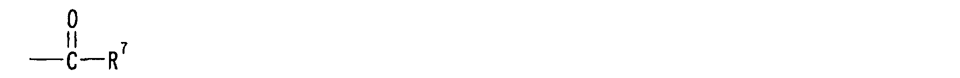 (wherein R⁷ represents a hydrocarbon group optionally having a substituent), R² represents hydrogen atom, an acyl group, a hydrocarbon group optionally having a substituent or a heterocyclic group optionally having a substituent, X represents a bond, O, S, SO, SO₂ or NR⁴ (wherein R⁴ represents hydrogen atom, an acyl group or a hydrocarbon group optionally having a substituent), k and m represent independently an integer of 0 to 5, and 1<k+m<5, (hereinafter abbreviated as Compound(I) in some cases) or a salt thereof, and a nitrogen-containing condensed heterocyclic derivative represented by the formula: 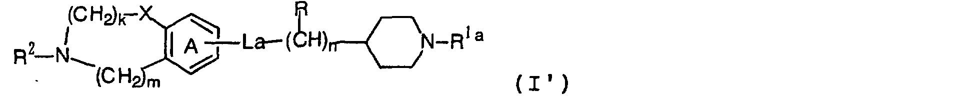 wherein ring A represents benzene ring optionally having a further substituent, -L^{a}- represents -NR^{3a}-, -S-, -SO-, -SO₂-, -SO₂NR^{3a}-, -SO₂NHCONR^{3a}-, -SO₂NHC(=NH)NR^{3a}-, -C(=S)-, 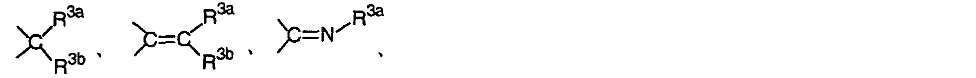 or -CONR^{3a}- (wherein R^{3a} and R^{3b} represent independently hydrogen atom, cyano group, hydroxy group, amino group, a C₁₋₆ alkyl group or a C₁₋₆ alkoxy group), n represents an integer of 0 to 6, R is hydrogen atom or a hydrocarbon group optionally having a substituent, and may be different in repetition of n, R^{1a} represents hydrogen atom or a group represented by the formula: 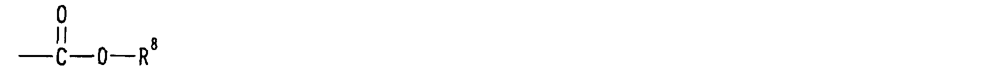 (wherein R⁸ represents a hydrocarbon group optionally having a substituent), R² represents hydrogen atom, an acyl group, a hydrocarbon group optionally having a substituent or a heterocyclic group optionally having a substituent, X represents a bond, O, S, SO, SO₂ or NR⁴ (wherein R⁴ represents hydrogen atom, an acyl group or a hydrocarbon group optionally having a substituent), k and m represent independently an integer of 0 to 5, and 1<k+m<5, (hereinafter abbreviated as Compound (I') in some cases) or a salt thereof, and found that these compounds have, based on their unique chemical structures, an unexpectedly excellent thermal production promoting activity, lipolysis promoting activity, and intraadipocellular cAMP concentration elevating activity as well as excellent pharmaceutical properties in preventing or treating obesity and obesity-based diseases.

Further, the present inventors synthesized for the first time a novel condensed benzisoazole derivative having a characteristic chemical structure in which a particular side chain is bonded to a tricyclic condensed ring formed by condensation of a benzisoazole ring with a nitrogen-containing hetero ring, and is represented by the formula: 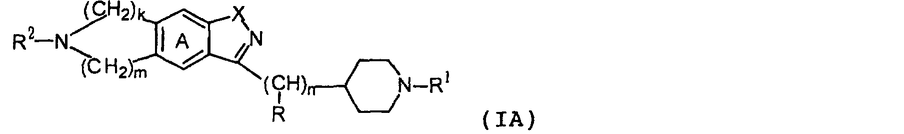 wherein ring A represents benzene ring optionally having a substituent, k and m represent independently an integer of 0 to 5, 1<k+m<5, n represents an integer of 1 to 6, R is hydrogen atom or a hydrocarbon group optionally having a substituent, and may be different in repetition of n, R¹ and R² represent independently hydrogen atom, an acyl group or a hydrocarbon group optionally having a substituent, X represents O or S, (hereinafter abbreviated as Compound (IA) in some cases) or a salt thereof, and found that these compounds have, based on their unique chemical structure, regardless of the kind of substituent on the condensed ring, an unexpectedly excellent thermal production promoting activity, lipolysis promoting activity and intraadipocellular cAMP concentration elevating activity as well as excellent pharmaceutical properties in preventing or treating obesity and obesity-based diseases.

Based on these findings, the present inventors further continued to study, which resulted in completion of the present invention.

That is, the present invention provides:
(1)a compound represented by the formula: 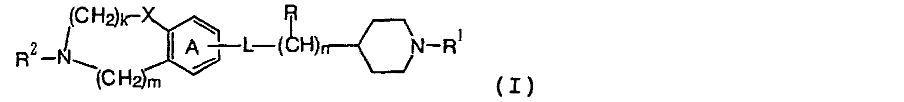 wherein ring A represents benzene ring optionally having afurthersubstituent, -L-represents-O-, -NR^{3a}- , -S-, -SO-, -SO₂-, -SO₂NR^{3a}-, -SO₂NHCONR^{3a}-, -SO₂NHC(=NH)NR^{3a}-, -C(=S)-, 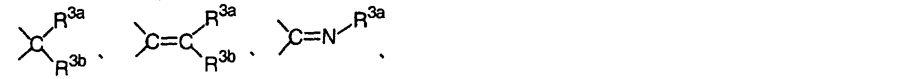 or -CONR^{3a}- (wherein R^{3a} and R^{3b} represent independently hydrogen atom, cyano group, hydroxy group, amino group, a C₁₋₆ alkyl group or a C₁₋₆ alkoxy group), n represents an integer of 0 to 6, R is hydrogen atom or a hydrocarbon group optionally having a substituent, and may be different in repetition of n, R¹ represents a hydrocarbon group optionally having a substituent or a group represented by the formula: 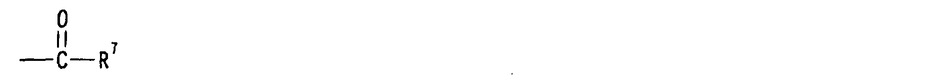 (wherein R⁷ represents a hydrocarbon group optionally having a substituent, R² represents hydrogen atom, an acyl group, a hydrocarbon group optionally having a substituent or a heterocyclic group optionally having a substituent, X represents a bond, O, S. SO, SO₂ or NR⁴ (wherein R⁴ represents hydrogen atom, an acyl group or a hydrocarbon group optionally having a substituent), k and m represent independently an integer of 0 to 5, and 1<k+m<5, or a salt thereof,
(2) the compound according to the above (1), wherein n is an integer of 1 to 6,
(3) the compound according to the above (1), wherein -L- is -O-, -S-, -SO-, -SO₂-, -CH₂-, -CHOH-, 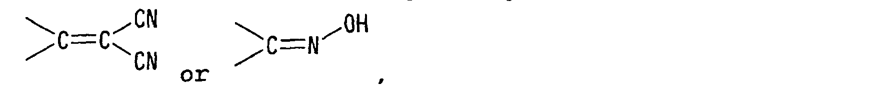
(4) the compound according to the above (1), wherein X is a bond and k=m=2,
(5) the compound according to the above (1), wherein X is a bond, k=3 and m=1,
(6) the compound according to the above (1), wherein X is O, k=2 and m=1,
(7) the compound according to the above (1), wherein R is hydrogen atom,
(8) the compound according to the above (1), wherein n is an integer of 2 to 4,
(9) the compound according to the above (1), wherein R¹ is a C₇₋₁₆ aralkyl group optionally having a substituent,
(10) the compound according to the above (1), wherein R² is a C₇₋₁₆ aralkyl group optionally having a substituent,
(11) the compound according to the above (1), wherein R is hydrogen atom, n is an integer of 2 to 4, and R¹ and R² are benzyl group optionally having a substituent,
(12) the compound according to the above (1), which is (i) 2-[(2-methylphenyl)methyl]-7-[2-[1-[[2-(trifluoromethyl)phenyl]methyl]-4-piperidinyl]ethoxy]-2,3,4,5-tetrahydro-1H-2-benzazepine, (ii) 2-[(2-methylphenyl)methyl]-8-[2-[1-[(4-chlorophenyl)methyl)-4-piperidinyl]ethoxy]-2,3,4,5-tetrahydro-1H-2-benzazepine, (iii) 1-(4-pyridyl)-5-[1-hydroxy-3-[1-(phenylmethyl)-4-piperidinyl]propyl]-2,3-dihydroindole, (iv) 3-[1-(phenylmethyl)-4-piperidinyl]-1-[3-(phenylmethyl)-2,3,4,5-tetrahydro-1H-3-benzazepin-7-yl]-1-propanone oxime, (v) 2-[1-[3-(phenylmethyl)-2,3,4,5-tetrahydro-1H-3-benzazepin-7-yl]-3-[1-(phenylmethyl)-4-piperidinyl]propylidene]malononitrile, (vi) 3-(phenylmethyl)-7-[[2-[1-(phenylmethyl)-4-piperidinyl]ethyl]sulfanyl]-2,3,4,5-tetrahydro-1H-3-benzazepine, (vii) 7-[[2-[1-[(2-chlorophenyl)methyl]-4-piperidinyl]ethyl]sulfinyl]-3-(phenylmethyl)-2,3,4,5-tetrahydro-1H-3-benzazepine, (viii) 7-[[2-[1-[(4-chlorophenyl)methyl]-4-piperidinyl]ethyl]sulfinyl]-3-(phenylmethyl)-2,3,4.5-tetrahydro-1H-3-benzazepine, (ix) 7-[[2-[1-[(3-chlorophenyl)methyl]-4-piperidinyl]ethyl]sulfonyl]-3-(phenylmethyl)-2,3,4,5-tetrahydro-1H-3-benzazepine, (x) 8-[3-[1-[[3-(4,5-dihydro-1H-2-imidazolyl)phenyl]methyl]-4-piperidinyl]propoxy]-2-[(4-fluorophenyl)methyl]-2,3,4,5-tetrahydro-1H-2-benzazepine, (xi) 4-[[4-[2-[[2-[(2-methylphenyl)methyl]-2,3,4,5-tetrahydro-1H-2-benzazepin-8-yl]oxy]ethyl]-1-piperidinyl]methyl]-1-benzenecarboxyimidamide, (xii) 8-[2-[1-[[4-(4,5-dihydro-1H-2-imidazolyl)phenyl)methyl]-4-piperidinyl]ethoxy]-2-[(2-methylphenyl)methyl]-2,3,4,5-tetrahydro-1H-2-benzazepine, (xiii) 2-(phenylmethyl)-8-[2-[1-[[4-(N,N-diethylaminomethyl)phenyl]methyl]-4-piperidinyl]ethoxy]-2,3,4,5-tetrahydro-1H-2-benzazepine, (xiv) 2-[(2-methylphenyl)methyl]-8-[2-[1-[[3-(4,5-dihydro-1H-2-imidazolyl)phenyl]methyl]-4-piperidinyl]ethoxy]-2,3,4,5-tetrahydro-1H-2-benzazepine, (xv) 2-[(2-methylphenyl)methyl]-8-[2-[1-[4-(4,5-dihydro-1H-2-imidazolyl)benzoyl]-4-piperidinyl]ethoxy]-2,3,4,5-tetrahydro-1H-2-benazepine, (xvi) 2-(phenylmethyl)-7-[[1-[[4-(4,5-dihydro-1H-2-imidazolyl)phenyl]methyl]-4-piperidinyl]methoxy]-2,3,4,5-tetrahydro-1H-2-benazepine, (xvii) 2-(phenylmethyl)-8-[[1-[[4-(4,5-dihydro-1H-2-imidazolyl)phenyl]methyl]-4-piperidinyl]methoxy]-2,3,4,5-tetrahydro-1H-2-benzazepine, (xviii) 2-(phenylmethyl)-8-[2-[1-[[4-(4,5-dihydro-1H-2-imidazolyl)phenyl]methyl]-4-piperidinyl]ethoxy]-2,3,4,5-tetrahydro-1H-2-benzazepine, or (xix) 2-(phenylmethyl)-8-[2-[1-[(4-dimethylaminophenyl)methyl]-4-piperidinyl]ethoxy]-2,3,4,5-tetrahydro-1H-2-benzazepine, or a salt thereof,
(13) a prodrug of the compound according to the above (1),
(14) a process for producing the compound according to the above (1), which comprises reacting a compound represented by the formula: 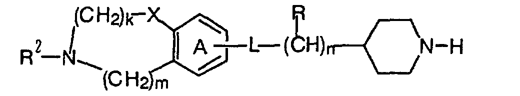 wherein respective symbols represent the same meanings as those for the above (1) or a salt thereof with a compound represented by the formula: R¹―Z¹
   wherein Z¹ represents a leaving group and R¹ represents the same meaning as that for the above (1) or a salt thereof,
(15) a compound represented by the formula: 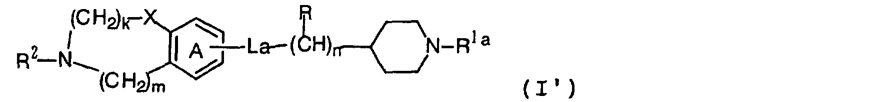 wherein ring A represents benzene ring optionally having a further substituent, -L^{a}- represents -NR^{3a}-, -S-, -SO-, -SO₂-, -SO₂NR^{3a}-, -SO₂NHCONR^{3a}-, -SO₂NHC(=NH)NR^{3a}-, -C(=S)-, 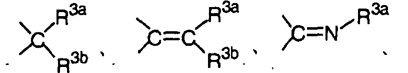 or -CONR^{3a}- (wherein R^{3a} and R^{3b} represent independently hydrogen atom, cyano group, hydroxy group, amino group, a C₁₋₆ alkyl group or a C₁₋₆ alkoxy group), n represents an integer of 0 to 6, R is hydrogen atom or a hydrocarbon group optionally having a substituent, and may be different in repetition of n, R^{1a} represents hydrogen atom or a group represented by the formula: 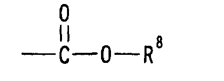 (wherein R⁸ represents a hydrocarbon group optionally having a substituent), R² represents hydrogen atom, an acyl group, a hydrocarbon group optionally having a substituent or a heterocyclic group optionally having a substituent, X represents a bond, O, S, SO, SO₂ or NR⁴ (wherein R⁴ represents hydrogen atom, an acyl group or a hydrocarbon group optionally having a substituent), k and m represent independently an integer of 0 to 5, 1<k+m<5, or a salt thereof,
(16) a compound represented by the formula: 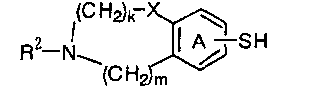 wherein ring A represents benzene ring optionally having a further substituent, R² represents hydrogen atom, an acyl group, a hydrocarbon group optionally having a substituent or a heterocyclic group optionally having a substituent, X represents a bond, O, S, SO, SO₂ or NR⁴ (wherein R⁴ represents hydrogen atom, an acyl group or a hydrocarbon group optionally having a substituent), k and m represent independently an integer of 0 to 5, 1<k+m<5, or a salt thereof,
(17) a pharmaceutical composition comprising a compound represented by the formula: 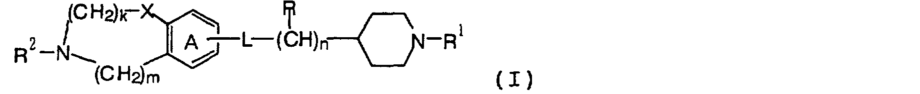 wherein ring A represents benzene ring optionally having a further substituent, -L- represents -O-, -NR^{3a}-, -S-, -SO-, -SO₂-, -SO₂NR^{3a}-, -SO₂NHCONR^{3a}-, -SO₂NHC(=NH)NR^{3a}-, -C(=S)-, 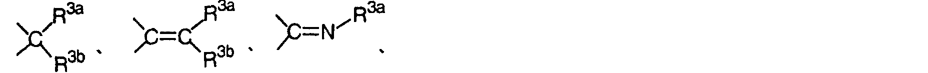 or -CONR^{3a}- (wherein R^{3a} and R^{3b} represent independently hydrogen atom, cyano group, hydroxy group, amino group, a C₁₋₆ alkyl group or a C₁₋₆ alkoxy group), n represents an integer of 0 to 6, R is hydrogen atom or a hydrocarbon group optionally having a substituent, and may be different in repetition of n, R¹ represents a hydrocarbon group optionally having a substituent or a group represented by the formula: 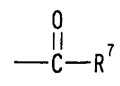 (wherein R⁷ represents a hydrocarbon group optionally having a substituent), R² represents hydrogen atom, an acyl group, a hydrocarbon group optionally having a substituent or a heterocyclic group optionally having a substituent, X represents a bond, O, S, SO, SO₂ or NR⁴ (wherein R⁴ reresents hydrogen atom, an acyl group or a hydrocarbon group optionally having a substituent), k and m represent independently an integer of 0 to 5, and 1<k+m<5, or a salt thereof or a prodrug thereof,
(18) the composition according to the above (17), which is a thermal production promoting agent,
(19) the composition according to the above (18), which is an anti-obesity agent,
(20) the composition according to the above (18), which is a lipolysis promoting agent,
(21) the composition according to the above (18), which is an agent for preventing or treating obesity-based diseases,
(22) a method for treating obesity and obesity-based diseases, which comprises administering an effective amount of the compound according to the above (1) to a mammal,
(23) use of the compound according to the above (1) for producing a thermal production promoting agent.

In addition, the present invention provides:
(24) a compound represented by the formula: 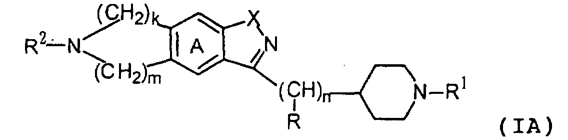 wherein ring A represents benzene ring optionally having a substituent, k and m represent independently an integer of 0 to 5, 1<k+m<5, n represents an integer of 1 to 6, R represents hydrogen atom or a hydrocarbon group optionally having a substituent, and may be different in repetition of n, R¹ and R² represent independently hydrogen atom, an acyl group or a hydrocarbon group optionally having a substituent, and X represents O or S, or a salt thereof,
(25) the compound according to the above (24), wherein k=m=2,
(26) the compound according to the above (24), wherein k=3 and m=1,
(27) the compound according to the above (24), wherein R is hydrogen atom,
(28) the compound according to the above (24), wherein n is an integer of 2 to 4,
(29) the compound according to the above (24), wherein R¹ is a C₇₋₁₆ aralkyl group optionally having a substituent,
(30) the compound according to the above (24), wherein R² is a C₇₋₁₆ aralkyl group optionally having a substituent,
(31) the compound according to the above (24), wherein X is O,
(32) the compound according to the above (24), wherein R is hydrogen atom, n is an integer of 2 to 4, R¹ and R² are benzyl group optionally having a substituent,
(33) the compound according to the above (24), which is 3-[3-[1-(phenylmethyl)-4-piperidinyl]propyl]-7-(phenylmethyl)-6,7,8,9-tetrahydro-5H-isoxazolo[4,5-h][3]benzazepine; 3-[3-[1-[(2-chlorophenyl)methyl]-4-piperidinyl]propyl]-6-(phenylmethyl)-6,7,8,9-tetrahydro-5H-isoxazolo[5,4-h][2]benzazepine; or 3-[3-[1-(phenylmethyl)-4-piperidinyl]propyl]-6,7,8,9-tetrahydro-5H-isoxazolo[5,4-h] [1]benzazepine or a salt thereof,
(34) a prodrug of the compound according to the above (24),
(35) a process for producing the compound according to the above (24), which comprises (i) ring-closing a compound represented by the formula: 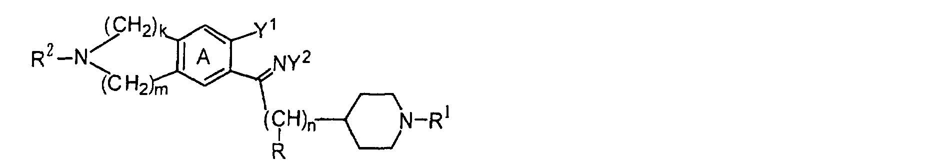 wherein Y¹ represents OZ^{a}, SZ^{a} (wherein Z^{a} represents hydrogen atom, a halogen atom, an alkyl group, or an acyl group), nitro group or a halogen atom, Y² represents hydrogen atom or OZ^{b} (wherein Z^{b} represents hydrogen atom or an acyl group) and other symbols represent the same meanings as those for the above (24) or a salt thereof, or
   (ii) reacting a compound represented by the formula: 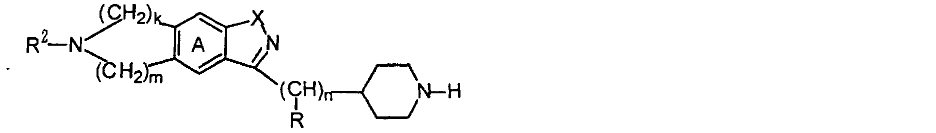 wherein respective symbols represent the same meanings as those for the above (24) or a salt thereof, with a compound represented by the formula: R¹―Z¹ wherein Z¹ represents a leaving group, and R¹ represents the same meaning as that for the above (24) or a salt thereof, or
   (iii) reacting a compound represented by the formula: 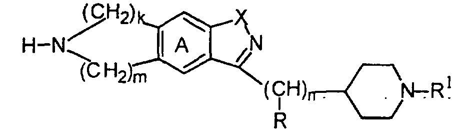 wherein respective symbols represent the same meanings as those for the above (24) or a salt thereof, with a compound represented by the formula: R²―Z¹ wherein Z¹ represents a leaving group, and R² represents the same meaning as that for the above (24) or a salt thereof, or
   (iv) reacting a compound represented by the formula: 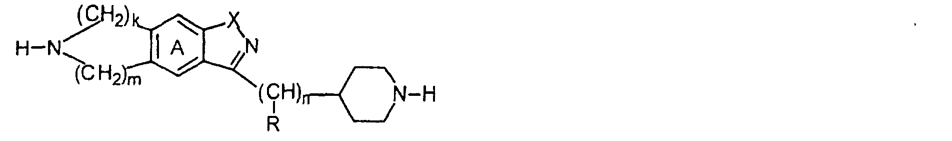 wherein respective symbols represent the same meanings as those for the above (24) or a salt thereof, with a compound represented by the formula: R¹―Z¹
      wherein Z¹ represents a leaving group, and R¹ represents the same meaning as that for the above (24) or a salt thereof,
(36) a pharmaceutical composition comprising a compound represented by the formula: 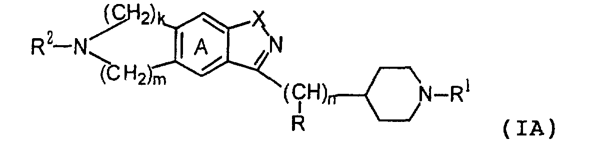 wherein ring A represents benzene ring optionally having a substituent, k and m represent independently an integer of 0 to 5, 1<k+m<5, n represents an integer of 1 to 6, R represents hydrogen atom or a hydrocarbon group optionally having a substituent, and may be different in repetition of n, R¹ and R² represent independently hydrogen, an acyl group or a hydrocarbon group optionally having a substituent, and X represents O or S, or a salt thereof or a prodrug thereof,
(37) the composition according to the above (36), which is a thermal production promoting agent,
(38) the composition according to the above (37), which is an anti-obesity agent,
(39) the composition according to the above (37), which is a lipolysis promoting agent,
(40) the composition according to the above (37), which is an agent for preventing or treating obesity-based diseases,
(41) a method for treating obesity or obesity-based diseases, which comprises administering an effective amount of the compound according to the above (24) to a mammal, and
(42) use of the compound according to the above (24) for producing a thermal production promoting agent.

[A] In this item, Compound (I) and Compound (I') will be described in detail.

As the "substituent" in the "benzene ring optionally having a substituent" for ring A in the formulae (I) and (I'), used are, for example, (i) an optionally halogenated lower alkyl group, (ii) a halogen atom (e.g., fluorine, chlorine, bromine, iodine and the like), (iii) nitro group, (iv) cyano group, (v) hydroxy group, (vi) an optionally halogenated lower alkoxy group, (vii) amino group, (viii) a mono-lower alkylamino group (e.g., a mono-C₁₋₆ alkylamino group and the like such as methylamino, ethylamino, propylamino and the like), (ix) a di-lower alkylamino group (e.g., a di-lower alkylamino group and the like such as dimethylamino, diethylamino and the like), (x) a 5 to 7 membered cyclic amino group optionally having 1 to 3 hetero atoms selected from nitrogen atom, oxygen atom and sulfur atom in addition to one nitrogen atom (e.g., pyrrolidino, piperidino, piperazino, morpholino, thiomorpholino and the like), (xi) a lower alkyl-carbonylamino group (e.g., a C₁₋₆ alkyl-carbonylamino and the like such as acetylamino, propionylamino, butyrylamino and the like), (xii) aminocarbonyloxy group, (xiii) a mono-lower alkylamino-carbonyloxy group (e.g., a mono-C₁₋₆ alkylamino-carbonyloxy group and the like such as methylaminocarbonyloxy, ethylaminocarbonyloxy group), (xiv) a di-lower alkylamino-carbonyloxy group (e.g., a di-C₁₋₆ alkylaminocarbonyloxy group and the like such as dimethylaminocarbonyloxy, diethylaminocarbonyloxy and the like), (xv) a lower alkylsulfonylamino group (e.g., a C₁₋₆ alkylsulfonylamino group and the like such as methylsulfonylamino, ethylsulfonylamino, propylsulfonylamino and the like), (xvi) a lower alkoxy-carbonyl group (e.g., a C₁₋₆ alkoxy-carbonyl group and the like such as methoxycarbonyl, ethoxycarbonyl, propoxycarbonyl, isobutoxycarbonyl and the like), (xvii) carboxy group, (xviii) a lower alkyl-carbonyl group (e.g., a C₁₋₆ alkyl-carbonyl group and the like such as methylcarbonyl, ethylcarbonyl, butylcarbonyl and the like), (xix) carbamoyl group, (xx) a mono-lower alkyl-carbamoyl group (e.g., a mono-C₁₋₆ alkyl-carbamoyl group and the like such as methylcarbamoyl, ethylcarbamoyl, propylcarbamoyl, butylcarbamoyl and the like), (xxi) a di-lower alkyl-carbamoyl group (e.g., a di-C₁₋₆ alkyl-carbamoyl group and the like such as diethylcarbamoyl, dibutylcarbamoyl and the like), (xxii) a lower alkyl-thiocarbonyl group (e.g., a C₁₋₆ alkyl-thiocarbonyl group such as methylthiocarbonyl, ethylthiocarbonyl, butylthiocarbonyl and the like), (xxiii) thiocarbamoyl group, (xxiv) a mono-lower alkyl-thiocarbamoyl group (e.g., a mono-C₁₋₆ alkyl-thiocarbamoyl group and the like such as methylthiocarbamoyl, ethylthiocarbamoyl, propylthiocarbamoyl, butylthiocarbamoyl and the like), (xxv) a di-lower alkyl-thiocarbamoyl group (e.g., a di-C₁₋₆ alkyl-thiocarbamoyl group and the like such as diethylthiocarbamoyl, dibutylthiocarbamoyl and the like), (xxvi) phenyl group [the (xxvi) phenyl group may have further 1 to 4 substituents, for example, selected from a lower alkyl (e.g., a C₁₋₆ alkyl such as methyl, ethyl, propyl, isopropyl, butyl, sec-butyl, tert-butyl, pentyl, hexyl and the like), a lower alkoxy (e.g., a C₁₋₆ alkoxy such as methoxy, ethoxy, propoxy, isopropoxy, n-butoxy, isobutoxy, sec-butoxy, tert-butoxy and the like), halogen (e.g., fluorine, chlorine, bromine, iodine and the like), hydroxy, amino, a mono-lower alkylamino (e.g., a mono-C₁₋₆ alkylamino and the like such as methylamino, ethylamino, propylamino and the like), a di-lower alkylamino (e.g., a di-C₁₋₆ alkylamino and the like such as dimethylamino, diethylamino and the like), nitro, a lower alkyl-carbonyl (e.g., a C₁₋₆ alkyl-carbonyl and the like such as methylcarbonyl, ethylcarbonyl, buthylcarbonyl and the like)].

As the above "optionally halogenated lower alkyl group", used are, for example, a lower alkyl group (e.g., a C₁₋₆ alkyl group and the like such as methyl, ethyl, propyl, isopropyl, butyl, sec-butyl, tert-butyl, pentyl, hexyl and the like) optionally having 1 to 3 halogens (e.g., fluorine, chlorine, bromine, iodine and the like). Specifically, methyl, chloromethyl, difluoromethyl, trichloromethyl, trifluoromethyl, ethyl, 2-bromoethyl, 2,2,2-trifluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl and the like are used.

As the above "optionally halogenated lower alkyl group" , for example, a lower alkoxy group (e.g., a C₁₋₆ alkoxy group and the like such as methoxy, ethoxy, propoxy, isopropoxy, n-butoxy, isobutoxy, sec-butoxy, tert-butoxy and the like) optionally having 1 to 3 halogens (e.g., fluorine, chlorine, bromine, iodine and the like) is used. Specifically, methoxy, difluoromethoxy, trifluoromethoxy, ethoxy, 2,2,2-trifluoroethoxy, n-propoxy, isopropoxy, n-butoxy, 4,4,4-trifluorobutoxy, isobutoxy, sec-butoxy, pentyloxy, hexyloxy and the like are used.

As the "substituent" in the "benzene ring optionally having a substituent", preferably used are a lower alkyl group (e.g., a C₁₋₆ alkyl group and the like such as methyl, ethyl, propyl, isopropyl, butyl, sec-butyl, tert-butyl, pentyl, hexyl and the like), a lower alkoxy group (e.g., a C₁₋₆ alkoxy group and the like such as methoxy, ethoxy, propoxy, isopropoxy, n-butoxy, isobutoxy, sec-butoxy, tert-butoxy and the like), a halogen atom (e.g., fluorine, chlorine, bromine, iodine and the like), hydroxy group, amino group, a mono-lower alkylamino group (e.g., a mono-C₁₋₆ alkylamino group and the like such as methylamino, ethylamino, propylamino and the like), a di-lower alkylamino group (e.g., a di-C₁₋₆ alkylamino group and the like such as dimethylamino, diethylamino and the like), nitro group and the like.

As the "benzene ring optionally having a substituent", benzene ring is preferably used.

The "hydrocarbon group" in the "hydrocarbon group optionally having a substituent" for R¹ and R² represents a group obtained by removing one hydrogen atom from a hydrocarbon compound, and, as an example thereof, used are the following alkyl group, alkenyl group, alkynyl group, cycloalkyl group, aryl group, aralkyl group, group comprising combination of these groups and the like.
(1) an alkyl group (e.g., a C₁₋₆ alkyl group such as methyl, ethyl, propyl, isopropyl, butyl, isobutyl, tert-butyl, sec-butyl, pentyl, hexyl and the like),
(2) an alkenyl group (e.g., a C₂₋₆ alkenyl group such as vinyl, allyl, isopropenyl, butenyl, isobutenyl, sec-butenyl and the like),
(3) an alkynyl group (e.g., a C₂₋₆ alkynyl group and the like such as propargyl, ethynyl, butynyl, 1-hexynyl and the like),
(4) a cycloalkyl group (e.g., a monocyclic C₃₋₆ cycloalkyl group and the like such as cyclopropyl, cyclobutyl, cyclopentyl, cyclohexyl and the like),
(5) a cross-linked cyclic lower saturated hydrocarbon group (e.g., a cross-linked cyclic C₈₋₁₄ saturated hydrocarbon group such as bicyclo[3.2.1]oct-2-yl, bicyclo[3.3.1]non-2-yl, adamantane-1-yl and the like),
(6) an aryl group (e.g., a C₆₋₁₄ aryl group and the like such as phenyl, 1-naphthyl, 2-naphthyl, biphenyl, 2-indenyl, 2-anthryl and the like, preferably phenyl group and the like),
(7) an aralkyl group (e.g., a phenyl-C₁₋₁₀ alkyl group such as benzyl, phenylethyl, phenylpropyl, phenylbutyl, phenylpentyl, phenylhexyl and the like; e.g., a naphthyl-C₁₋₆ alkyl group such as α-naphthylmethyl and the like; e.g., a diphenyl-C₁₋₃ alkyl group and the like such as diphenylmethyl, diphenylethyl and the like),
(8) an aryl-alkenyl group (e.g., a C₆₋₁₄ aryl-C₂₋₁₂ alkenyl group and the like such as a phenyl-C₂₋₁₂ alkenyl group and the like such as styryl, cinnamyl, 4-phenyl-2-butenyl, 4-phenyl-3-butenyl and the like),
(9) an aryl-C₂₋₁₂ alkynyl group (e.g., a C₆₋₁₄ aryl-C₂₋₁₂ alkynyl group and the like such as a phenyl-C₂₋₁₂ alkynyl group and the like such as phenylethynyl, 3-phenyl-2-propynyl, 3-phenyl-1-propynyl and the like),
(10) a cycloalkyl-lower alkyl group (e.g., a C₃₋₇ cycloalkyl-C₁₋₆ alkyl group such as cyclopropylmethyl, cyclobutylmethyl, cyclopentylmethyl, cyclohexylmethyl, cycloheptylmethyl, cyclopropylethyl, cyclobutylethyl, cyclopentylethyl, cyclohexylethyl, cycloheptylethyl, cyclopropylpropyl, cyclobutylpropyl, cyclopentylpropyl, cyclohexylpropyl, cycloheptylpropyl, cyclopropylbutyl, cyclobutylbutyl, cyclopentylbutyl, cyclohexylbutyl, cycloheptylbutyl, cyclopropylpentyl, cyclobutylpentyl, cyclopentylpentyl, cyclohexylpentyl, cycloheptylpentyl, cyclopropylhexyl, cyclobutylhexyl, cyclopentylhexyl, cyclohexylhexyl and the like),
(11) an aryl-aryl-C₁₋₁₀ alkyl group (e.g., biphenylmethyl, biphenylethyl and the like).

As the "hydrocarbon group" in the "hydrocarbon group optionally having a substituent" for R¹ and R², preferably used are, for example, a C₁₋₆ alkyl group, a C₃₋₆ cycloalkyl group, a C₇₋₁₆ aralkyl group and the like, and more preferably used are a C₇₋₁₆ aralkyl group (e.g., a phenyl-C₁₋₁₀ alkyl group and the like such as benzyl, phenylethyl, phenylpropyl and the like) and the like.

As the "substituent" in the "hydrocarbon group optionally having a substituent" for R¹ and R², used are one to five (preferably one to three) substituents selected from (i) a halogen atom (e.g., fluorine, chlorine, bromine, iodine and the like), (ii) nitro group, (iii) cyano group, (iv) oxo group, (v) hydroxy group, (vi) an optionally halogenated lower(C₁₋₆)alkyl group (e.g., methyl, ethyl, propyl, isopropyl, butyl, isobutyl, tert-butyl, sec-butyl, trifluoromethyl, trichloromethyl and the like), (vii) an optionally halogenated lower(C₁₋₆)alkoxy group (e.g., methoxy, ethoxy, n-propyloxy, i-propyloxy, n-butyloxy, trifluoromethoxy, trichloromethoxy and the like), (viii) an optionally halogenated lower(C₁₋₆)alkylthio group (e.g., methylthio, ethylthio, propylthio, trifluoromethylthio and the like), (ix) amino group, (x) a mono-lower alkylamino group (e.g., a mono-C₁₋₆ alkylamino group and the like such as methylamino, ethylamino, propylamino and the like), (xi) a di-lower alkylamino group (e.g., a di-C₁₋₆ alkylamino group and the like such as dimethylamino, diethylamino and the like), (xii) a 5 to 7 membered cyclic amino group optionally having 1 to 3 hetero atoms selected from nitrogen atom, oxygen atom and sulfur atom in addition to carbon atoms and one nitrogen atom (e.g., pyrrolidino, piperidino, piperazino, morpholino, thiomorpholino and the like), (xiii) a lower alkyl-carbonylamino group (e.g., a C₁₋₆ alkyl-carbonylamino group and the like such as acetylamino, propionylamino, butyrylamino and the like), (xiv) a lower alkylsulfonylamino group (e.g., a C₁₋₆ alkyl-carbonylamino group and the like such as methylsulfonylamino, ethylsulfonylamino and the like), (xv) a lower alkoxy-carbonyl group (e.g., a C₁₋₆ alkoxy-carbonyl group and the like such as methoxycarbonyl, ethoxycarbonyl, propoxycarbonyl and the like), (xvi) carboxyl group, (xvii) a lower alkyl-carbonyl group (e.g., a C₁₋₆ alkyl-carbonyl group and the like such as methylcarbonyl, ethylcarbonyl, propylcarbonyl and the like), (xviii) carbamoyl group, (xix) a mono-lower alkyl-carbamoyl group (e.g., a monoC₁₋₆ alkyl-carbamoyl group and the like such as methylcarbamoyl, ethylcarbamoyl and the like), (xx) a di-lower alkyl-carbamoyl group (e.g., a di-C₁₋₆ alkyl-carbamoyl group and the like such as dimethylcarbamoyl, diethylcarbamoyl and the like), (xxi) a lower alkyl sulfonyl group (e.g., a C₁₋₆ alkylsulfonyl group and the like such as methylsulfonyl, ethylsulfonyl, propylsulfonyl and the like), (xxii) a lower alkoxy-carbonyl-lower alkyl group (e.g., a C₁₋₆ alkoxy-carbonyl-C₁₋₆ alkyl group and the like such as methoxycarbonylmethyl, ethoxycarbonylmethyl, tert-butoxycarbonylmethyl, methoxycarbonylethyl, methoxycarbonyl(dimethyl)methyl, ethoxycarbonyl(dimethyl)methyl, tert-butoxycarbonyl(dimethyl)methyl and the like), (xxiii) a carboxyl-lower alkyl group (e.g., a carboxyl-C₁₋₆ alkyl group and the like such as carboxylmethyl, carboxylethyl, carboxyl(dimethyl)methyl and the like), (xxiv) a heterocyclic group optionally having a substituent, (xxv) a C₆₋₁₄ aryl group (e.g., phenyl, naphthyl and the like), (xxvi) a C₇₋₁₆ aralkyl group (e.g., benzyl and the like), ureido group optionally having a substituent (e.g., ureido, 3-methylureido, 3-ethylureido, 3-phenylureido, 3-(4-fluorophenyl)ureido, 3-(2-methylphenyl)ureido, 3-(4-methoxyphenyl)ureido, 3-(2,4-difluorophenyl)ureido, 3-[3,5-bis(trifluoromethyl)phenyl]ureido, 3-benzylureido, 3-(1-naphthyl)ureido, 3-(2-biphenylyl)ureido and the like), (xxviii) thioureido group optionally having a substituent (e.g., thioureido, 3-methylthioureido, 3-ethylthioureido, 3-phenylthioureido, 3-(4-fluorophenyl)thioureido, 3-(4-methylphenyl)thioureido, 3-(4-methoxyphenyl)thioureido, 3-(2,4-dichlorophenyl)thioureido, 3-benzylthioureido, 3-(1-naphthyl)thioureido and the like), (xxix) amidino group optionally having a substituent (e.g., amidino, N¹-methylamidiono, N¹-ethylaidino, N¹-phenylamidino, N¹,N¹-dimethylamidino, N¹,N²-dimethylamidino, N¹-methyl-N¹-ethylamidino, N¹,N¹-diethylamidino, N¹-methyl-N¹-phenylamidino, N¹,N¹-di(4-nitrophenyl)amidino and the like), (xxxx) guanidino group optionally having a substituent (e.g., guanidino, 3-methylguanidino, 3,3-dimethylguanidino, 3,3-diethylguanidino and the like), (xxxi) a cyclic aminocarbonyl group optionally having a substituent (e.g., pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino)carbonyl, [4-(4-fluorobenzoyl)piperidino]carbonyl, (4-methylpiperazino)carbonyl,(4-phenylpiperazino)carbonyl, [4-(4-nitrophenyl)piperazino]carbonyl, (4-benzylpiperazino)carbonyl, morpholinocarbonyl, thiomorpholinocarbonyl and the like), (xxxii) aminothiocarbonyl group optionally having a substituent (e.g., aminothiocarbonyl, methylaminothiocarbonyl, dimethylaminothiocarbonyl and the like), (xxxiii) aminosulfonyl group optionally having a substituent (e.g., aminosulfonyl, methylaminosulfonyl, dimethylaminosulfonyl and the like), (xxxiv) phenylsulfonylamino group optionally having a substituent (e.g., phenylsulfonylamino, (4-methylphenyl)sulfonylamino, (4-chlorophenyl)sulfonylamino, (2,5-dichlorophenyl)sulfonylamino, (4-methoxyphenyl)sulfonylamino, (4-acetylaminophenyl)sulfonylamino, (4-nitrophenyl)phenylsulfonylamino and the like), (xxxv) sulfo group, (xxxvi) sulfino group, (xxxvii) sulfeno group, (xxxviii) a C₁₋₆ alkylsulfo group (e.g., methylsulfo, ethylsulfo, propylsulfo and the like), (xxxix)a C₁₋₆ alkylsulfino group (e.g., methylsulfino, ethylsulfino, propylsulfino and the like), (xxxx) a C₁₋₆ alkylsulfeno group (e.g., methylsulfeno, ethylsulfeno, propylsulfeno and the like), (xxxxi) phosphono group, (xxxxii) a di-C₁₋₆ alkoxyphosphoryl group (e.g., dimethoxyphosphoryl, diethoxyphosphoryl, dipropoxyphosphoryl and the like), (xxxxiii) a lower alkoxy-carbonyl-lower alkoxy group (e.g., a C₁₋₆ alkoxy-carbonyl-C₁₋₆ alkoxy and the like such as methoxycarbonylmethoxy, ethoxycarbonylmethoxy, tert-butoxycarbonylmethoxy, methoxycarbonylethoxy, methoxycarbonyl(dimethyl)methoxy, ethoxycarbonyl(dimethyl)methoxy, tert-butoxycarbonyl(dimethyl)methoxy and the like), (xxxxiv) a carboxyl-lower alkoxy group (e.g., a carboxyl-C₁₋₆ alkoxy group and the like such as carboxylmethoxy, carboxylethoxy, carboxyl(dimethyl)methoxy and the like), (xxxxv) a lower alkyl-thiocarbonyl group (e.g., a C₁₋₆ alkyl-thiocarbonyl group such as methylthiocarbonyl, ethylthiocarbonyl, butylthiocarbonyl and the like), (xxxxvi) thiocarbamoyl group, (xxxxvii) a mono-lower alkyl-thiocarbamoyl group (e.g., a mono-C₁₋₆ alkyl-thiocarbamoyl group and the like such as methylthiocarbamoyl, ethylthiocarbamoyl, propylthiocarbamoyl, butylthiocarbamoyl and the like), (xxxxviii) a di-lower alkyl-thiocarbamoyl group (e.g., a di-C₁₋₆ alkyl-thiocarbamoyl group and the like such as diethylthiocarbamoyl, dibutylthiocarbamoyl and the like) and the like.

As the "heterocyclic group" in the above "heterocyclic group optionally having a substituent", for example, used is a group obtained by removing one hydrogen atom from a 5 to 14 membered (monocyclic or bi-to-tetracyclic)hetero ring containing 1 to 6 (preferably 1 to 4) hetero atoms selected from nitrogen atom, oxygen atom and sulfur atom.

As a monocyclic hetero ring, used is a group obtained by removing one hydrogen atom from a monocyclic hetero ring such as pyridine, pyrazine, pyrimidine, imidazole, furan, thiophene, dihydropyridine, diazepine, oxazepine, pyrrolidine, piperidine, hexamethyleneimine, heptamethyleneimine, tetrahydrofuran, piperazine, homopiperazine, tetrahydrooxazepine, morpholine, thiomorpholine, pyrrole, pyrazole, 1,2,3-triazole, oxazole, oxazolidine, oxadiazole, thiazole, thiazolidine, thiadiazole, oxathiadiazole, isoxazole, imidazoline, triazine, tetrazole and the like.

As a bicyclic heterocyclic group, for example, used is a group obtained by removing one hydrogen atom from a bicyclic hetero ring such as indole, dihydroindole, isoindole, dihydroisoindole, benzofuran, dihydrobenzofuran, benzimidazole, benzoxazole, benzisoxazole, benzothiazole, indazole, quinoline, tetrahydroquinoline, isoquinoline, tetrahydroisoquinoline, terahydro-1H-1-benzazepine, terahydro-1H-2-benzazepine, tetrahydro-1H-3-benzazepine, tetrahydrobenzoxazepine, quinazoline, tetrahydroquinazoline, quinoxaline, tetrahydroqunoxaline, benzodioxane, benzodioxole, benzothiazine, imidazopyridine and the like.

As a polycyclic heterocyclic group such as tricyclic or tetracyclic one, used is a group obtained by removing one hydrogen atom from a polycyclic hetero ring such as acridine, tetrahydroacridine, pyrroloquinoline, pyrroloindole, cyclopentoindole, isoindrobenzazepine, and the like.

As the "heterocyclic group", preferred is a group obtained by removing one hydrogen atom from a monocyclic hetero ring or a bicyclic hetero ring.

As the "substituent" in the "heterocyclic group optionally having a substituent" , for example, used are one to five substituents selected from (i) halogen (e.g., fluorine, chlorine, bromine, iodine and the like), (ii) nitro, (iii) cyano, (iv) oxo, (v) hydroxy, (vi) a lower alkyl (e.g., a C₁₋₆ alkyl and the like such as methyl, ethyl, propyl, isopropyl, butyl, isobutyl, tert-butyl, sec-butyl and the like), (vii) a lower alkoxy (e.g., a C₁₋₆ alkoxy and the like such as methoxy, ethoxy, n-propyloxy, i-propyloxy, n-butyloxy and the like), (viii) a lower alkylthio (e.g., a C₁₋₆ alkylthio and the like such as methylthio, ethylthio, propylthio and the like), (ix) amino, (x) a mono-lower alkylamino (e.g., a mono-C₁₋₆ alkylamino and the like such as methylamino, ethylamimo, propylamino and the like), (xi) a di-lower alkylamino (e.g., a di-C₁₋₆ alkylamino and the like such as dimethylamino, diethylamino and the like), (xii) a 5 to 7 membered cyclic amino optionally having 1 to 3 hetero atoms selected from nitrogen atom, oxygen atom and sulfur atom in addition to carbon atoms and one nitrogen atom (e.g., pyrrolidino, piperidino, piperazino, morpholino, thiomorpholino and the like), (xiii) a lower alkyl-carbonylamino (e.g., a C₁₋₆ alkyl-carbonylamino and the like such as acetylamino, propionylamino, butyrylamino and the like), (xiv) a lower alkylsulfonylamino (e.g., a C₁₋₆ alkyl-carbonylamino and the like such as methylsulfonylamino, ethylsulfonylamino and the like), (xv) a lower alkoxy-carbonyl (e.g., a C₁₋₆ alkoxy-carbonyl and the like such as methoxycarbonyl, ethoxycarbonyl, propoxycarbonyl and the like), (xvi) carboxyl, (xvii) a lower alkyl-carbonyl (e.g., a C₁₋₆ alkyl-carbonyl and the like such as methylcarbonyl, ethylcarbonyl, propylcarbonyl and the like), (xviii) carbamoyl, (xix) a mono-lower alkylcarbamoyl (e.g., a mono-C₁₋₆ alkylcarbamoyl and the like such as methylcarbamoyl, ethylcarbamoyl and the like), (xxx) a di-lower alkylcarbamoyl (e.g., a di-C₁₋₆ alkylcarbamoyl and the like such as dimethylcarbamoyl, diethylcarbamoyl and the like), (xxi) a lower alkylsulfonyl (e.g., a C₁₋₆ alkylsulfonyl and the like such as methylsulfonyl, ethylsulfonyl, propylsulfonyl and the like), (xxii) a lower alkyl-thiocarbonyl group (e.g., a C₁₋₆ alkyl-thiocarbonyl and the like such as methylthiocarbonyl, ethylthiocarbonyl, butylthiocarbonyl and the like, (xxiii) thiocarbamoyl group, (xxiv) a mono-lower alkyl-thiocarbamoyl group (e.g., a mono-C₁₋₆ alkyl-thiocarbamoyl group and the like such as methylthiocarbamonyl, ethylthiocarbamoyl, propylthiocarbamoyl, butylthiocarbamoyl and the like), (xxv) a di-lower alkyl-thiocarbamoyl group (e.g., a di-C₁₋₆ alkyl-thiocarbamoyl group and the like such as diethylthiocarbamoyl, dibutylthiocarbamoyl and the like) and the like.

As the "substituent" in the "hydrocarbon group optionally having a substituent" for R¹ and R², preferably used are a halogen atom, an optionally halogenated C₁₋₆ alkyl group, an optionally halogenated C₁₋₆ alkoxy group, hydroxy group, nitro group, cyano group, a C₁₋₆ alkoxycarbonyl group, amino group, a 5 to 7 membered cyclic amino group (e.g., pyrrolidino, piperidino, piperazino, morpholino, thiomorpholino and the like), a C₁₋₆ alkoxy-carbonyl-C₁₋₆ alkyl group, a carboxyl-C₁₋₆ alkyl group, amidino group optionally having a substituent (preferably, N¹-methylamidino and the like), a heterocyclic group (preferably, imidazolinyl group) optionally having a substituent (preferably, C₁₋₆ alkyl and the like), phenylsulfonylamino group, a C₁₋₆ alkoxy-carbonyl-C₁₋₆ alkoxy group, a carboxyl-C₁₋₆ alkoxy group, a di-C₁₋₆ alkylamino group, a C₁₋₆ alkoxy-imino group, amidino group optionally having a substituent (preferably, a C₁₋₆ alkyl and the like).

When a group represented by the formula: 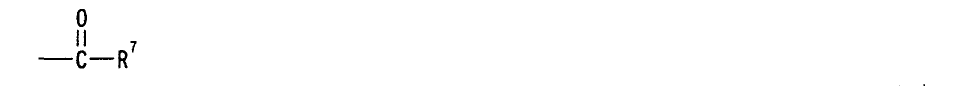 wherein R⁷ represents a hydrocarbon group optionally having a substituent, is used as R¹, as the "hydrocarbon group" and "substituent" in the "hydrocarbon group optionally having a substituent" for R⁷, the same "hydrocarbon group" and "substituent" as those for the above R¹ are used. As R⁷, a lower alkyl group (e.g., a C₁₋₆ alkyl group and the like such as methyl, ethyl, propyl, isopropyl, butyl, isobutyl, tert-butyl, sec-butyl and the like) and the like are preferably used.

As R¹, preferably used are a C₇₋₁₆ aralky group (preferably, benzyl group and the like) optionally having one to five (preferably, one to three) substituents selected from halogen (preferably, chlorine and the like), a halogenated C₁₋₆ alkyl (preferably, methyl, trifluoromethyl and the like), an optionally halogenated C₁₋₆ alkoxy (preferably, methoxy and the like), cyano, nitro, hydroxy, a C₁₋₆ alkoxy-carbonyl-C₁₋₆ alkyl group (preferably, ethoxycarbonylpropyl and the like), a carboxyl-C₁₋₆ alkyl group (preferably, carboxypropyl and the like), a carboxyl-C₁₋₆ alkoxy group (preferably, carboxymethoxy and the like), a di-C₁₋₆ alkylamino (preferably, dimethylamino and the like), a di-C₁₋₆ alkylamino-C₁₋₆ alkyl (preferably, dimethylaminomethyl and the like), a C₁₋₆ alkoxyimino (preferably, ethoxyimino and the like), amidino group optionally having a substituent (preferably, a C₁₋₆ alkyl and the like), and imidazolinyl optionally having a substituent (preferably, a C₁₋₆ alkyl and the like) and the like; a C₁₋₆ alkyl group (preferably, ethyl, propyl, isopropyl, butyl and the like) optionally having one to five substituents selected from halogen (preferably, chlorine and the like) and a C₁₋₆ alkoxy-carbonyl (preferably, ethoxycarbonyl and the like) and the like; a C₁₋₆ alkyl-carbonyl group (preferably, acetyl and the like); a C₆₋₁₄ aryl-carbonyl group (preferably, benzoyl and the like) and the like.

As R¹, more preferably used are benzyl or phenylethyl group each optionally having one to five substituents selected from halogen, an optionally halogenated C₁₋₆ alkyl, an optionally halogenated C₁₋₆ alkoxy, cyano, nitro, hydroxy and imidazolinyl optionally having a substituent (preferably, a C₁₋₆ alkyl and the like) and the like; a C₁₋₆ alkyl group optionally having one to five substituents selected from halogen and a C₁₋₆ alkoxy-carbonyl group and the like; a C₁₋₆ alkyl-carbonyl group and the like.

It is preferred that R¹ is C₇₋₁₆ aralkyl group optionally having a substituent.

When a group represented by the formula: 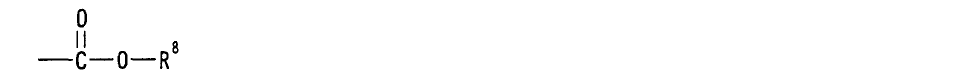 wherein R⁸ represents a hydrocarbon group optionally having a substituent, is used as R^{1a}, as the "hydrocarbon group" and "substituent" in the "hydrocarbon group optionally having a substituent" for R⁸, the same "hydrocarbon group" and "substituent" as those for the above R¹ are used.

As the "acyl group" for R², used are acyl groups represented by the formulae: -(C=O)-R⁵, -(C=S)-R⁵, -SO₂-R⁵, -SO-R⁵, -(C=O)NR⁵R⁶, -(C=S)NR⁵R⁶-(C=O)O-R⁵ or -(C=S)O-R⁵ [wherein R⁵ and R⁶ independently represent (i) hydrogen atom, (ii) a hydrocarbon group optionally having a substituent or (iii) a heterocyclic group optionally having a substituent, or R⁵ and R⁶ are taken together with the adjacent nitrogen atom to form a nitrogen-containing cyclic group optionally having a substituent, and the like.

Among these, -(C=O)-R⁵, -SO₂-R⁵, -SO-R⁵, -(C=O)NR⁵R⁶ or -(C=O)O-R⁵ (R⁵ and R⁶ represent the same meanings as above) are preferable and, inter alia, -(C=O)-R⁵ (R⁵ represents the same meaning as above) is particularly preferable.

As the "hydrocarbon group optionally having a substituent" and "heterocyclic group optionally having a substituent" for R⁵ and R⁶, the same "hydrocarbon group optionally having a substituent" and "heterocyclic group optionally having a substituent" as above are used, respectively.

As the "nitrogen-containing cyclic group optionally having a substituent" formed by R⁵ and R⁶, a 5 to 9 membered (preferably 5 to 7 membered) nitrogen-containing saturated heterocyclic group optionally containing one to three hetero atoms such as nitrogen atom, oxygen atom and sulfur atom is used. More particularly, for example, groups represented by the formulae; 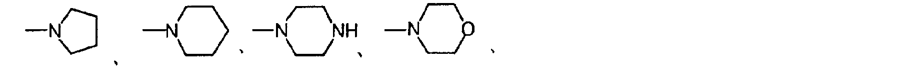 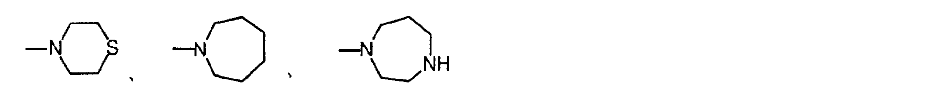 are used.

As the "substituent" in the "nitrogen-containing cyclic group optionally having a substituent", the same "substituent" in the "heterocyclic group" as above is used.

As the "acyl group" for the above R², preferably used are formyl group, an optionally halogenated C₁₋₆ alkyl-carbonyl group (e.g., acetyl, trifluoroacetyl, propionyl and the like), a 5 to 6 membered heterocyclic carbonyl group (e.g., pyridylcarbonyl, thienylcarbonyl, furylcarbonyl and the like), a C₆₋₁₄ aryl-carbonyl group (e.g., benzoyl, 1-naphthoyl, 2-naphthoyl and the like), a C₇₋₁₆ aralkyl-carbonyl group (e.g., phenylacetyl, 3-phenylpropionyl and the like), a C₆₋₁₀ aryl-sulfonyl group (e.g., benzenesulfonyl, toluenesulfonyl, naphthylsulfonyl and the like) and the like. Among these, an optionally halogenated C₁₋₆ alkyl-carbonyl group (preferably trifluoroacetyl and the like) and the like are preferable.

As the "heterocyclic group" in the "heterocyclic group optionally having a substituent" for R², the same "heterocyclic group" as that used as "substituent" in the "hydrocarbon group" for R¹ and R² is used and, as the "substituent", the same "substituent" as that for the "heterocyclic group" is used.

As the "heterocyclic group optionally having a substituent" for R², preferably used is pyridyl group optionally having a substituent (e.g., a lower alkyl such as a C₁₋₆ alkyl and the like).

As R², preferably used is hydrogen atom, a hydrocarbon group optionally having a substituent or a heterocyclic group optionally having a substituent.

As R², more preferably used are hydrogen; a C₇₋₁₆ aralkyl group (preferably benzyl, phenylethyl and the like) optionally having one to five (preferably one to three) substituents selected from halogen (preferably, fluorine and the like), an optionally halogenated C₁₋₆ alkyl (preferably methyl, trifluoromethyl and the like), an optionally halogenated C₁₋₆ alkoxy, imidazolinyl optionally having a substituent (preferably, C₁₋₆ alkyl and the like) and hydroxy and the like; an optionally halogenated C₁₋₆ alkyl-carbonyl group (preferably trifluoroacetyl and the like); pyridyl group optionally substituted by a C₁₋₆ alkyl, and the like.

As R², particularly preferably used are benzyl or phenylethyl group each optionally having one to five substituents selected from halogen, an optionally halogenated C₁₋₆ alkyl, an optionally halogenated C₁₋₆ alkoxy, imidazolinyl optionally having a substituent (preferably, a C₁₋₆ alkyl and the like) and hydroxy and the like; a C₁₋₆ alkyl group optionally having one to five substituents selected from halogen and a C₁₋₆ alkoxy-carbonyl and the like; a C₁₋₆ alkyl-carbonyl group; pyridyl group optionally substituted by a C₁₋₆ alkyl, and the like.

It is preferred that R² is a C₇₋₁₆ aralkyl group optionally having a substituent.

X represents a bond, O, S, SO, SO₂ or NR⁴, wherein R⁴ represents hydrogen atom, an acyl group or a hydrocarbon group optionally having a substituent.

As the "acyl group" and "hydrocarbon group optionally having a substituent" for R⁴, the same "acyl group" and "hydrocarbon group optionally having a substituent" as above are used, respectively.

As X, a bond or O is preferably used and, more preferably, a bond is used.

-L- represents -O-, -NR^{3a}-, -S-, -SO-, -SO₂-, -SO₂NR^{3a}-, -SO₂NHCONR^{3a}, -SO₂NHC(=NH)NR^{3a}-, -C(=S)-, 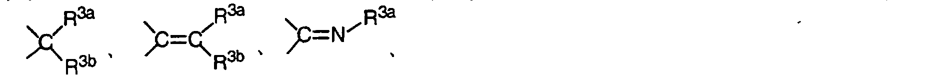 or -CONR^{3a}-, and R^{3a} and R^{3b} represent independently hydrogen atom, cyano group, hydroxy group, amino group, a C₁₋₆ alkyl group (e.g., methyl, ethyl, propyl and the like) or a C₁₋₆ alkoxy group (e.g., methoxy, ethoxy, propoxy and the like). As R^{3a} and R^{3b}, preferably used are hydrogen atom, cyano group, hydroxy group, amino group, a C₁₋₄ alkyl group (methyl, ethyl and the like) and the like.

In addition, as -L-, preferably used are -O-, -NHCO-, -S-, -SO-, -SO₂-, -SO₂NH-, -SO₂NHCONH, -SO₂NHC(=NH)NH-, -CH₂-, 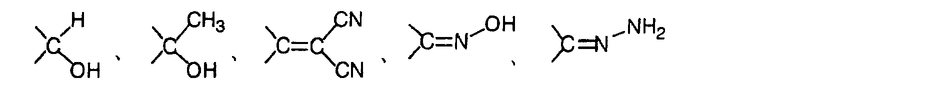 -CONH-, and, more preferably used are -O-, -S-, -SO-,-SO₂-, -CH₂-, -CHOH-,

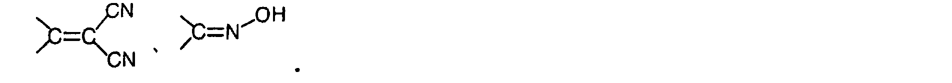

In addition, -L- may be substituted at any position on ring A, as long as it is a substitutable position.

-L^{a}- represents -NR^{3a}-, -S-, -SO-, -SO₂-, -SO₂NR^{3a}-, -SO₂NHCONR^{3a}-, -SO₂NHC(=NH)NR^{3a}- -C(=S)-, 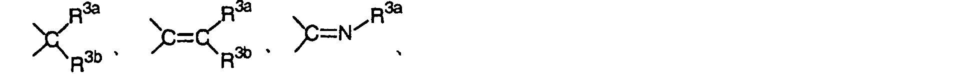 or -CONR^{3a}, and R^{3a} and R^{3b} represent independently hydrogen atom, cyano group, hydroxy group, amino group, a C₁₋₆ alkyl group (e.g., methyl group, ethyl group, propyl group and the like) or a C₁₋₆ alkoxy group (e.g., methoxy group, ethoxy group, propoxy group and the like).

In addition, -L^{a}- may be substituted at any position on ring A, as long as it is a substitutable position.

k and m represent independently an integer of 0 to 5, and 1<k+m<5. Preferably, k+m=4 and, more preferably, (1) k=m=2, (2) k=3 and m=1, (3) k=2 and m=0 or (4) k=2 and m=1. Among these, (1) k=m=2 or (2) k=3 and m=1 is preferable.

n is an integer of 0 to 6, preferably, an integer of 1 to 6, more preferably, an integer of 1 to 4, particularly preferably, an integer of 2 to 4.

R is a hydrogen atom or a hydrocarbon group optionally having a substituent, and may be different in repetition of n.

As the "hydrocarbon group optionally having a substituent" for R, the same "hydrocarbon group optionally having a substituent" as that for R¹ and R² is used.

As R, hydrogen atom is preferable.

As preferable examples of Compound (I), for example, the following compounds can be mentioned.
1) Compounds wherein R is hydrogen atom, n is an integer of 2 to 4, and R¹ and R² are benzyl group optionally having a substituent.
2) Compounds wherein ring A is benzene ring;
   -L- is -O-, -NHCO-, -S-, -SO-, -SO₂-, -SO₂NH-,-SO₂NHCONH-, SO₂NHC(=NH)NH-, -CH₂-, 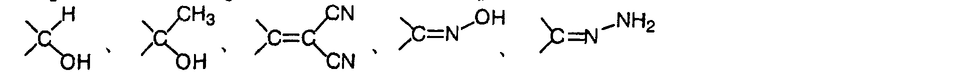 -CONH-;
   n is an integer of 0 to 3;
   R is hydrogen atom;
   (1) X is a bond and k=m=2, (2) X is a bond and k=3 and m=1, (3) X is a bond and k=2 and m=0 or (4) X is O and k=2 and m=1;
   R¹ is a C₇₋₁₆ aralkyl group (preferably, benzyl group and the like) optionally having one to five substituents selected from halogen (preferably, chlorine and the like), an optionally halogenated C₁₋₆ alkyl (preferably, methyl, trifluoromethyl and the like), an optionally halogenated C₁₋₆ alkoxy (preferably, methoxy and the like), cyano, nitro, hydroxy, a C₁₋₆ alkoxy-carbonyl-C₁₋₆ alkyl group (preferably, ethoxycarbonylpropyl and the like), a carboxyl-C₁₋₆ alkyl group (preferably, carboxylpropyl and the like), a carboxyl-C₁₋₆ alkoxy group (preferably, carboxymethoxy and the like), a di-C₁₋₆ alkylamino (preferably, dimethylamino and the like), a di-C₁₋₆ alkylamino-C₁₋₆ alkyl (preferably, dimethylaminomethyl and the like), a C₁₋₆ alkoxyimino (preferably, ethoxyimino and the like), amidino group optionally having a substituent (preferably, a C₁₋₆ alkyl and the like), and imidazolinyl optionally having a substituent (preferably, a C₁₋₆ alkyl and the like) and the like; a C₁₋₆ alkyl group (preferably, ethyl, propyl, isopropyl, butyl and the like) optionally having one to three substituents selected from halogen (preferably, chlorine and the like) and a C₁₋₆ alkoxy-carbonyl (preferably, ethoxycarbonyl and the like); a C₁₋₆ alkyl-carbonyl group (preferably, acetyl and the like); or a C₆₋₁₄ aryl-carbonyl group (preferably, benzoyl and the like);
   R² is hydrogen; a C₇₋₁₆ aralkyl group (preferably, benzyl, phenylethyl and the like) optionally having one to three substituents selected from halogen (preferably, fluorine and the like), an optionally halogenated C₁₋₆ alkyl (preferably, methyl, trifluoromethyl and the like), and an optionally halogenated C₁₋₆ alkoxy and the like; an optionally halogenated C₁₋₈ alkyl-carbonyl group (preferably, trifluoroacetyl and the like); or pyridyl group optionally substituted by a C₁₋₆ alkyl.

As Compound (I) or a salt thereof, especially preferred is (i) 2-[(2-methylphenyl)methyl]-7-[2-[1-[[2-(trifluoromethyl)phenyl]methyl)-4-piperidinyl]ethoxy]-2,3,4,5-tetrahydro-1H-2-benzazepine, (ii) 2-[(2-methylphenyl)methyl]-8-[2-[1-[(4-chlorophenyl)methyl]-4-piperidinyl]ethoxy]-2,3,4,5-tetrahydro-1H-2-benzazepine, (iii) 1-(4-pyridyl)-5-[1-hydroxy-3-[1-(phenylmethyl)-4-piperidinyl]propyl]-2,3-dihydroindole, (iv) 3-[1-(phenylmethyl)-4-piperidinyl]-1-[3-(phenylmethyl)-2,3,4,5-tetrahydro-1H-3-benzazepin-7-yl]-1-propanone oxime, (v) 2-[1-[3-(phenylmethyl)-2,3,4,5-tetrahydro-1H-3-benzazepin-7-yl]-3-[1-(phenylmethyl)-4-piperidinyl]propylidene]malononitrile, (vi) 3-(phenylmethyl)-7-[[2-[1-(phenylmethyl)-4-piperidinyl]ethyl]sulfanyl]-2,3,4,5-tetrahydro-1H-3-benzazepine, (vii) 7-[[2-[1-[(2-chlorophenyl)methyl]-4-piperidinyl]ethyl]sulfinyl]-3-(phenylmethyl)-2,3,4,5-tetrahydro-1H-3-benzazepine, (viii) 7-[[2-[1-[(4-chlorophenyl)methyl]-4-piperidinyl]ethyl]sulfinyl]-3-(phenylmethyl)-2,3,4,5-tetrahydro-1H-3-benzazepine, (ix) 7-[[2-[1-[(3-chlorophenyl)methyl]-4-piperidinyl]ethyl]sulfonyl]-3-(phenylmethyl)-2,3,4,5-tetrahydro-1H-3-benzazepine, (x) 8-[3-[1-[[3-(4,5-dihydro-1H-2-imidazolyl)phenyl]methyl]-4-piperidinyl]propoxy]-2-[(4-fluorophenyl)methyl]-2,3,4,5-tetrahydro-1H-2-benzazepine, (xi) 4-[[4-[2-[[2-[(2-mehylphenyl)methyl]-2,3,4,5-tetrahydro-1H-2-benzazepin-8-yl]oxy]ethyl]-1-piperidinyl]methyl]-1-benzenecarboxyimidamide, (xii) 8-[2-[1-[[4-(4,5-dihydro-1H-2-imidazolyl)phenyl]methyl]-4-piperidinyl]ethoxy]-2-[(2-methylphenyl)methyl]-2,3,4,5-tetrahydro-1H-2-benzazepine, (xiii) 2-(phenylmethyl)-8-[2-[1-[[4-(N,N-diethylaminomethyl)phenyl]methyl]-4-piperidinyl]ethoxy]-2,3,4,5-tetrahydro-1H-2-benzazepine, (xiv) 2-[(2-methylphenyl)methyl]-8-[2-[1-[[3-(4,5-dihydro-1H-2-imidazolyl)phenyl]methyl]-4-piperidinyl]ethoxy]-2,3,4,5-tetrahydro-1H-2-benzazepine, (xv) 2-[(2-methylphenyl)methyl]-8-[2-[1-[4-(4,5-dihydro-1H-2-imidazolyl)benzoyl]-4-piperidinyl]ethoxy]-2,3,4,5-tetrahydro-1H-2-benzazepine, (xvi) 2-(phenylmethyl)-7-[[1-[[4-(4,5-dihydro-1H-2-imidazolyl]phenyl]methyl]-4-piperidinyl]methoxy]-2,3,4,5-tetrahydro-1H-2-benzazepine, (xvii) 2-(phenylmethyl)-8-[[1-[[4-(4,5-dihydro-1H-2-imidazolyl)phenyl]methyl]-4-piperidinyl]methoxy]-2,3,4,5-tetrahydro-1H-2-benzazepine, (xviii) 2-(phenylmethyl)-8-[2- [1-f [4-(4,5-dihydro-1H-2-imidazolyl)phenyl]methyl]-4-piperidinyl]ethoxy]-2,3,4,5-tetrahydro-1H-2-benzazepine, or (xix) 2-(phenylmethyl)-8-[2-[1-[(4-dimethylaminophenyl)methyl]-4-piperidinyl]ethoxy]-2,3,4,5-tetrahydro-1H-2-benzazepine or a salt thereof. Among these, particularly preferred is 2-[(2-methylphenyl)methyl]-7-[2-[1-[[2-(trifluromethyl)phenyl]methyl]-4-piperidinyl]ethoxy]-2,3,4,5-tetrahydro-1H-2-benzazepine or a salt thereof.

As a salt of Compound (I) and Compound (I'), physiologically acceptable salts are preferable and, physiologically acceptable acid addition salts are especially preferable. As such salts, for example, used are salts with an inorganic acid (e.g., hydrochloric acid, phosphoric acid, hydrobromic acid, sulfuric acid), and salts with an organic acid (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid).

Further, when Compound (I) and Compound (I') have an acidic group such as -COOH and the like, Compound (I) and Compound (I') may form a salt with an inorganic base (e.g., sodium, potassium, calcium, magnesium, ammonia) or an organic base (e.g., triethylamine), and these salts are included in the object compound of the present invention. Further, the above Compound (I), Compound (I') or salts thereof may be a hydrate or a non-hydrate.

A prodrug of Compound (I) refers to a compound which is converted into Compound (I) by the action of an enzyme or gastric juice and the like under physiological conditions in vivo, namely a compound which is converted into compound (I) upon enzymatic oxidation, reduction, hydrolysis and the like, or a compound which is converted into compound (I) upon hydrolysis by gastric juice and the like. Examples of the prodrug of compound (I) include compounds derived by acylation, alkylation or phosphorylation of the amino group of compound (I) (e.g. compounds derived by eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation or tert-butylation of the amino group of compound (I)), compounds derived by acylation, alkylation, phosphorylation or boration of the hydroxy group of compound (I) (e.g. compounds derived by acetylation, palmitoylation, propanoylation, pivaloylation, succinylation, fumarylation, alanylation or dimethylaminomethylcarbonylation of the hydroxy group of compound (I)), and compounds derived by esterification or amidation of the carboxyl group of compound (I) (e.g. compounds derived by ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification, or methylamidation of the carboxyl group of compound (I)), and the like. These compounds can be produced from compound (I) by per se known methods.

A prodrug of compound (I) may be one that is converted into compound (I) under physiological conditions, as described in "Iyakuhin no Kaihatsu (Development of Drugs)", vol. 7, Molecular Designing, published by Hirokawa Shoten, 1990, pages 163-198.

Compound (I') may be a prodrug and, as the prodrug, the same prodrug as that for Compound (I) is used.

Compound (I) and Compound (I') may be labeled with an isotope (e.g., ³H, ¹⁴C, ³⁵S and the like).

Hereinafter, a process for producing Compound (I) or a salt thereof will be described.

Compound (I') or a salt thereof is produced in the same manner as Compound (I) or a salt thereof.

Although the following process for production is applied not only to Compound (I) itself but also to the aforementioned salt thereof, they are simply abbreviated as Compound (I) in the following explanation.

In addition, although as compounds represented by the formulae (II), (III), (IIa), (IVa), (Va), (VIa), (VIIa), (VIIIa), (IXa), (Xa), (XIa), (IIb), (IVb), (VIb), (VIIb), (VIIIb), (IXb), (Xb), (IIc), (Vc), (VIc), (IId), (IVd), (VId), (VIId), (VIIId), (IIe), (IVe), (Ve), (IIf), (Vf), (IIg), (IVg), (Vg), (IIh), (IVh), (Vh), (IIi), (Vi), (Vj), (IIj), (vk), (IIk), (IIm), (Vm), (VIm), (VIIIm), (Iee), (IVee), (Iff), (Igg), (IVgg), (Ihh), (IVhh), (Iii), (Ijj), (Ikk) and (Imm) used in respective steps, not only those compounds themselves but also salts thereof may be used, they are simply abbreviated as "compound(s)" in the following explanation in some cases. As salts of compounds used in these respective steps, salts listed for the above "salts of Compound (I)" may be used.

Further, these compounds or salts thereof may be a hydrate or a non-hydrate.

When Compound (I) and compounds (a raw material compound or an intermediate for synthesis) in respective steps for producing Compound (I) are in free form, they may be converted into a salt according to conventional methods and, when they form a salt, they may be converted into a free compound or other salt according to conventional methods.

Compounds represented by the formulae (Iee), (Iff), (Igg), (Ihh), (Iii), (Ijj), (Ikk), (Imm) are included in Compound (I).

In addition, Compound (I) and each intermediate for synthesis may be an optical isomer, a steric isomer, a positional isomer or a rotational isomer, or a mixture thereof, and these are also included in Compound (I) and a raw material compound or an intermediate for synthesis. For example, Compound (I) may be a racemate, or an optical isomer resolved from a racemate. In addition, these can be isolated and purified according to the separating method known per se.

An optical isomer can be produced according to the means known per se. Specifically, an optical isomer can be produced by using an optically active raw material compound or an intermediate for synthesis, or by optically resolving a racemate of a final compound according to the conventional method. As an optical resolving method, the method known per se, for example, a fractionating recrystallization method, an optical active column method, a diastereomer method and the like can be applied. A steric isomer, a positional isomer and a rotational isomer can be produced by applying the method known per se.

The following respective reactions may be performed without using a solvent or using a suitable solvent as necessary. As the solvent, any solvents that can be generally used for a chemical reaction may be used as long as they do not interfere with the reaction. For example, organic solvents such as hydrocarbon solvents (e.g., hexane, toluene and the like), ether solvents (e.g., ethyl ether, tetrahydrofuran, dioxane, dimethoxyethane), amide solvents (e.g., formamide, N,N-dimethylformamide, N,N-dimethylacetamide, hexamethylphosphoric triamide and the like), urea solvents (e.g., 1,3-dimethyl-2-imidazolidinone and the like), sulfoxide solvents (e.g., dimethyl sulfoxide and the like), alcohol solvents (e.g., methanol, ethanol, isopropanol, t-butanol and the like), nitrile solvents (e.g., acetonitrile, propionitrile and the like), pyridine and the like, or water and the like are used. The amount of the solvent to be used is usually about 0.5 ml to about 100 ml, preferably about 3 ml to about 30 ml relative to 1 mmol of a compound. The reaction temperature differs depending upon the kind of solvent used and is usually around about -30°C to about 180°C, preferably around about 0°C to 120°C. The reaction time differs depending upon the reaction temperature and is usually about 0.5 hours to about 72 hours, preferably about 1 hour to about 24 hours. The reaction is usually performed under normal pressure and may be performed under pressure conditions of around about 1 atm to about 100 atm as necessary.

Compounds obtained in the following respective steps are isolated and purified by the known means such as concentration, liquid nature conversion, conversional resolution, solvent extraction, fractional distillation, distillation, crystallization, recrystallization, chromatography, preparative high performance liquid chromatography or the like, and supplied as a raw material for the next reaction. These compounds as a reaction mixture may be used as a raw material without isolation or purification.

In the following explanations, "condensation reaction" may be performed in the presence of a base as necessary. As the base, for example, inorganic bases such as sodium carbonate, sodium hydrogen carbonate, potassium carbonate, lithium carbonate, sodium hydroxide, potassium hydroxide, potassium hydride, sodium hydride, sodium methoxide, potassium t-butoxide and the like, and organic bases such as pyridine, rutidine, collidine, triethylamine and the like are used. The amount of the base to be used is usually an equivalent mole amount to an excessive amount, preferably about 1 mole equivalent to about 5-fold mole equivalent relative to a compound. Further, the present reaction may be promoted in the presence of a catalytic amount of an iodide compound, for example, sodium iodide, potassium iodide, or 4-dimethylaminopyridine and the like as necessary.

In the reaction of the following respective steps, a reaction may be performed after a functional group is protected according to the conventional method as necessary, and deprotection may be optionally performed according to the conventional method after the reaction. A protecting group introducing reaction and a deprotection reaction are performed according to the means known per se or its analogous means. Specifically, employed are a method described in Protective groups in Organic Synthesis; John Wiley & Sons, Inc. and, a method, as a deprotection reaction, of treating with an acid, a base, reduction, ultraviolet-ray, hydrazine, phenylhydrazine, sodium N-methyldithiocarbamate, tetrabutylammonium fluoride, palladium acetate and the like.
1) Compound (I) can be produced by a condensation reaction of a compound represented by the formula: 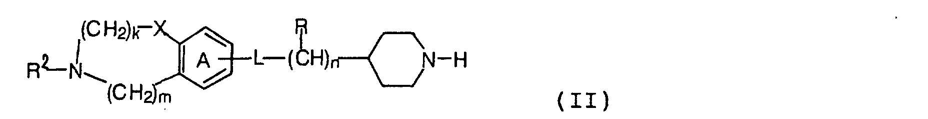 wherein respective symbols represent the same meanings as above, (hereinafter abbreviated as Compound (II) in some cases) or a salt thereof, with a compound represented by the formula: R¹-Z¹ (III) wherein Z¹ represents a leaving group, and R¹ represents the same meaning as above, (hereinafter abbreviated as Compound (III) in some cases) or a salt thereof.

As a leaving group for Z¹, for example, a halogen atom (e.g., chlorine, bromine, iodine and the like), a C₁₋₆ alkylsulfonyloxy group (e.g., methanesulfonyloxy, ethanesulfonyloxy, trifluoromethanesulfonyloxy and the like), a C₆₋₁₀ arylsulfonyloxy group (e.g., benzenesulfonyloxy, p-toluenesulfonyloxy and the like) and the like are used. In particular, for example, a halogen atom (e.g., bromine, iodine and the like) is preferably used.

As a solvent for a condensation reaction of Compound (II) and Compound (III), for example, alcohol solvents such as ethanol and the like, or nitrile solvents such as acetonitrile and the like are preferably used. The reaction temperature differs depending upon the kind of solvent used and is preferably around about 0°C to 120°C. The reaction time differs depending upon a reaction temperature and is preferably about 1 hour to 24 hours. As a base, for example, sodium carbonate, potassium carbonate, triethylamine and the like are preferably used. The amount of the base to be used is preferably about 1 equivalent to about 3 equivalents relative to Compound (III). Further, the present reaction may be promoted in the presence of a catalytic amount of an iodide compound (e.g., sodium iodide, potassium iodide and the like), or 4-dimethylaminopyridine and the like.

A raw material Compound (III) or a salt thereof can be produced by the method known per se or its analogous method.

A raw material Compound (II) or a salt thereof can be produced by the synthesis method described below.

1-1) Among raw material Compound (II), Compound (IIa) wherein -L- is -O- or a salt thereof can be produced by the following reaction formula 1-1. That is, Compound (IIa) can be produced by performing successively:
Step (aa): a condensation reaction of a compound represented by the formula (IVa) [wherein respective symbols represent the same meanings as above] (hereinafter abbreviated as Compound (IVa) in some cases) and a compound represented by the formula (Va) [wherein Z² represents a leaving group, W¹ represents a protecting group for amino group, and other symbols represent the same meanings as above] (hereinafter abbreviated as Compound (Va) in some cases), and
Step (ab): a deprotection reaction of a compound represented by the formula (VIa) [wherein W¹ represents a protecting group for amino group, and other symbols represent the same meanings as above] (hereinafter abbreviated as Compound (VIa) in some cases).

### [Reaction formula 1-1]

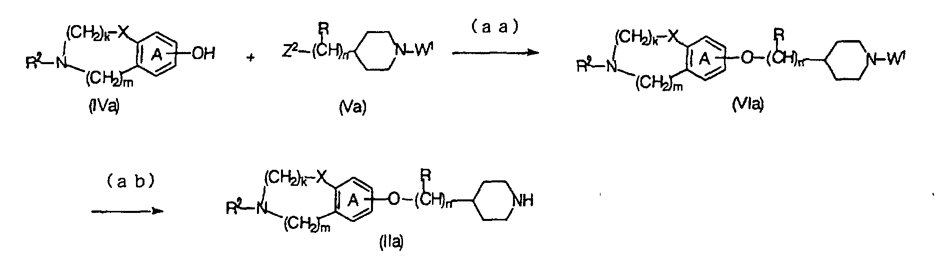

In step (aa), Compound (VIa) can be produced by a condensation reaction of Compound (IVa) and Compound (Va).

W¹ represents a general protecting group for amino group and, for example, the "hydrocarbon group optionally having a substituent" and "acyl group" for the above R² can be used. Specifically, for example, an acyl group such as formyl group, a C₁₋₆ alkyl-carbonyl group optionally having a substituent (e.g., acetyl, ethylcarbonyl and the like), benzoyl group, a C₁₋₆ alkyl-oxycarbonyl group (e.g., methoxycarbonyl, ethoxycarbonyl, t-butoxycarbonyl and the like), a C₆₋₁₄ aryloxycarbonyl group (e.g., phenoxycarbonyl and the like), a C₇₋₁₅ aralkyloxy-carbonyl group (e.g., benzyloxycarbonyl, fluorenyloxycarbonyl and the like) and the like, or a hydrocarbon group such as trityl, phthaloyl and the like is used. In particular, for example, t-butoxycarbonyl and the like are preferably used. As a "substituent" when a "hydrocarbon group optionally having a substituent" is used as W¹, halogen (e.g., fluorine, chlorine, bromine, iodine and the like), a C₁₋₆ alkyl-carbonyl (e.g., methylcarbonyl, ethylcarbonyl, butylcarbonyl and the like), nitro and the like are used. The number of substituents is preferably around 1 to 3.

As a leaving group for Z², the same group as the "leaving group for Z¹" above is used. For example, a halogen atom (preferably, bromine, iodine and the like) is preferable.

The condensation reaction of Compound (IVa) and Compound (Va) can be performed in the same manner as the condensation reaction of Compound (II) and Compound (III). Specifically, for example, the reaction can be performed in the presence of a base such as potassium carbonate, sodium hydride or the like in a solvent such as N,N-dimethylformamide or the like. The amount of the base used is preferably about 1 equivalent to about 3 equivalents relative to Compound (Va).

Compound (Va) can be produced by the method known per se or its analogous method. For example, it can be produced by a method described in J. Med. Chem., 40, 1779-1788 (1997) or JP-A 58-208289 and the like, or its analogous method.

In step (ab), Compound (IIa) can be produced by subjecting Compound (VIa) to a deprotection reaction to remove W¹.

The present deprotection reaction can be performed, for example, by the method which is generally used in peptide chemistry. For example, the present deprotection reaction can be performed by holding Compound (VIa) preferably at about 20°C to about 140°C in an aqueous solution of an acid such as a mineral acid (e.g., hydrochloric acid, sulfuric acid, hydrobromic acid, iodic acid, periodic acid and the like) or a base such as an alkali metal hydroxide (e.g., sodium hydroxide, potassium hydroxide, lithium hydroxide) and the like. The amount of the acid or the base used is usually about 1 to about 100 equivalents, preferably about 1 to about 40 equivalents relative to Compound (VIa). The strength of the acid or the base is usually about 0.1N to about 18N, preferably about 1N to about 12N. The reaction time depends upon the reaction temperature and is usually around about 1 hour to about 48 hours, preferably around about 2 hours to about 24 hours.

Alternatively, Compound (VIa) can be subjected to a catalytic reduction reaction under a normal pressure or under pressure as necessary using palladium-carbon, Raney-nickel, Raney-cobalt, platinum oxide and the like as a catalyst and an alcohol solvent or acetic acid or the like as a solvent, to deprotect W¹.

In addition, when W¹ is t-butoxycarbonyl group, deprotection can be performed using a trialkylsilyltrifluoromethanesulfonate derivative such as trimethylsilyl-trifluoromethanesulfonate, triethylsilyl-trifluoromethanesulfonate or t-butyldimethylsilyl-trifluoromethanesulfonate in the presence of an aromatic or tertiary amine such as 2,6-rutidine or triethylamine. As a solvent, preferred are a nonpolar solvent such as dichloromethane and the like, and a polar aprotic solvent such as tetrahydrofuran, diethyl ether, N,N-dimethylformamide and the like. The reaction temperature is preferably about -20°C to room temperature. In particular, the condition using trimethylsilyltrifluoromethanesulfonate and 2,6-rutidine at about 0°C to approximately room temperature in dichloromethane are preferable.

In addition, a raw material Compound (IVa) or a salt thereof in step (aa) can be produced according to the following reaction formula 1-2. That is, Compound (IVa) can be produced by performing successively the following reactions:
Step (ac): a reduction reaction of a compound represented by the formula (VIIa) [wherein W² represents a protecting group for a phenolic hydroxy group, and other symbols represent the same meanings as above] (hereinafter abbreviated as Compound (VIIa) in some cases) or a compound represented by the formula (VIIIa) [wherein respective symbols represent the same meanings as above] (hereinafter abbreviated as Compound (VIIIa) in some cases),
Step (ad): a condensation reaction of a compound represented by the formula (IXa) [wherein respective symbols represent the same meanings as above] (hereinafter abbreviated as Compound (IXa) in some cases) and a compound represented by the formula (Xa) [wherein respective symbols represent the same meanings as above] (hereinafter abbreviated as Compound (Xa) in some cases), and
Step (ae): a deprotection reaction of a compound represented by the formula (XIa) [wherein respective symbols represent the same meanings as above] (hereinafter abbreviated as Compound (XIa) in some cases).

### [Reaction formula 1-2]

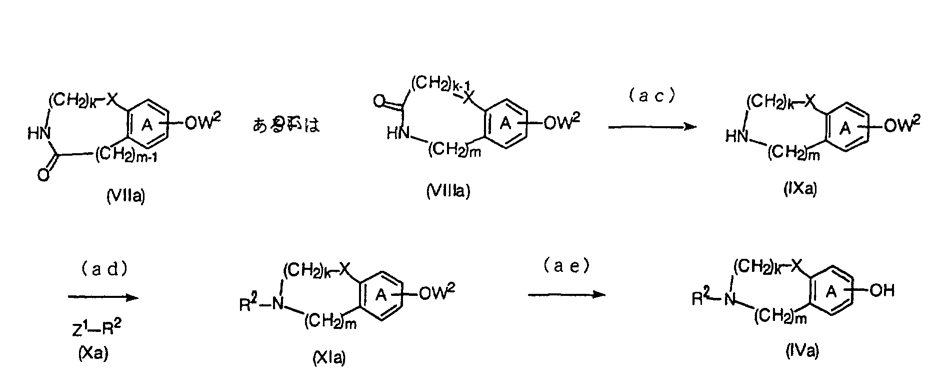

In step (ac), Compound (IXa) can be produced by a reduction reaction of Compound (VIIa) or Compound (VIIIa).

As a protecting group for a phenolic hydroxy group for W², any groups may be used as far as they are a general protecting group for a phenolic hydroxy group. Specifically, for example, protecting groups described in Protective groups in Organic Synthesis; John Wiley & Sons, Inc. and the like are used and, preferably, methyl group, benzyl group and the like are used.

The reduction reaction of Compound (VIIa) or Compound (VIIIa) can be performed, for example, using a suitable reducing agent (e.g., lithium aluminum hydride, diborane and the like) by the known method (e.g., methods described in Organic Reactions, 6, 469 (1941), Organic Synthesis, Coll. Vol. 4, 354-357 (1963), J. Am. Chem. Soc., 86, 3566 (1964), Synthesis, 752 (1978) and the like) or their analogous methods.

Compound (VIIa) or Compound (VIIIa) can be produced by the method known per se or its analogous method. For example, those compounds can be produced by methods described in J. Chem. Soc. (C), 183-188 (1969), USP 4,080,449 and the like or their analogous methods.

In step (ad), Compound (XIa) can be produced by a condensation reaction of Compound (IXa) and Compound (Xa).

The condensation reaction of Compound (IXa) and Compound (X) can be performed, for example, by the same manner as the condensation reaction of Compound (II) and Compound (III). Specifically, as a solvent, an alcohol solvent such as ethanol and the like, or a nitrile solvent such as acetonitrile and the like is preferably used. As a base, for example, sodium carbonate, potassium carbonate, triethylamine and the like are preferably used. Further, the present reaction may be promoted in the presence of a catalytic amount relative to Compound (Xa) of an iodide compound (e.g., sodium iodide, potassium iodide and the like) or 4-dimethylaminopyridine or the like.

In step (ae), Compound (IVa) can be produced by subjecting Compound (XIa) to a deprotection reaction to remove W².

The deprotection reaction can be performed by the general deprotection conditions and, for example, when W² is methyl group, methods described in Bull. Chem. Soc. Jpn., 44, 1986(1971), Tetrahedron, 42, 3259 (1986) and the like or their analogous methods can be used.

1-2) Among a raw material Compound (II), Compound (IIb) wherein -L- is -NR^{3a}- or a salt thereof can be produced by the following reaction formula 2-1. That is, Compound (IIb) can be produced by successively performing:
Step (ba): a condensation reaction of a compound represented by the formula (IVb) [wherein respective symbols represent the same meanings as above] and Compound (Va), and
Step (bb): a deprotection reaction of a compound represented by the formula (VIb) [wherein respective symbols represent the same meanings as above] (hereinafter abbreviated as Compound (VIb) in some cases).

### [Reaction formula 2-1]

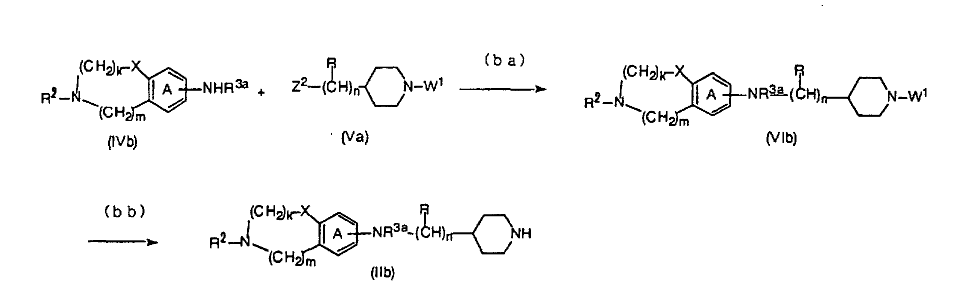

In Step (ba), Compound (VIb) can be produced by a condensation reaction of Compound (IVb) and Compound (Va).

The condensation reaction of Compound (IVb) and Compound (Va) can be performed in the same manner as the condensation reaction of Compound (II) and Compound (III). For example, the reaction can be performed in a solvent such as N,N-dimethylformamide and the like in the presence of a base such as potassium carbonate, sodium hydride and the like. The amount of the base used is preferably about 1 equivalent to about 3 equivalents relative to Compound (Va).

In Step (bb), Compound (IIb) can be produced by subjecting Compound (VIb) to a deprotection reaction to remove W¹.

The present reaction can be performed, for example, in the same manner as the deprotection reaction of Compound (VIa).

In addition, a raw material Compound (IVb) or a salt thereof in Step (ba) can be produced by the following reaction formula 2-2. That is, Compound (IVb) can be produced by performing successively:
Step (bc): a nitration reaction of a compound represented by the formula (VIIb) [wherein respective symbols represent the same meanings as above] (hereinafter abbreviated as Compound (VIIb) in some cases).
Step (bb): a reduction reaction of a compound represented by the formula (VIIIb) [wherein respective symbols represent the same meanings as above] (hereinafter abbreviated as Compound (VIIIb) in some cases), and
Step (be): a condensation reaction of a compound represented by the formula (IXb) [wherein respective symbols represent the same meanings as above] (hereinafter abbreviated as Compound (IXb) in some cases) and a compound represented by the formula (Xb) [wherein respective symbols represent the same meanings as above] (hereinafter abbreviated as Compound (Xb) in some cases).

### [Reaction formula 2-2]

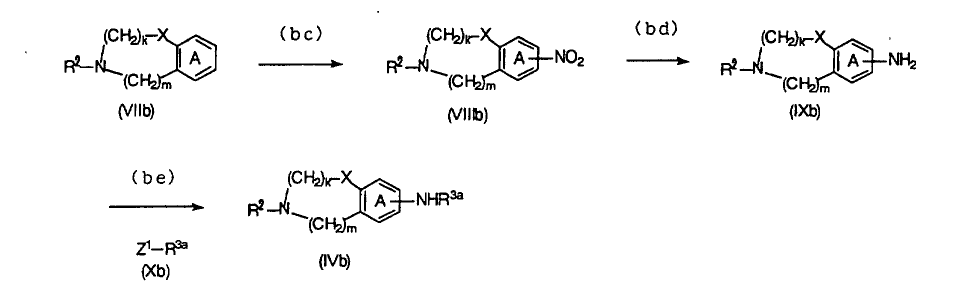

In Step (bc), Compound (VIIIb) can be produced by nitrating Compound (VIIb).

The present reaction can be performed using a suitable nitrating reagent (e.g., nitric acid, nitric acid-sulfuric acid, nitronium trifluoroborate and the like) by the known method (e.g., methods described in synthesis, 217-238 (1977), Chemistry of the Nitro and Nitroso Groups, p.1-48 Wiley (1970) and the like) or their analogous methods. Although nitro group can be introduced in any reactive position, when ring A is unsubstituted, X is a bond, and k=3 and m=1, the 8-position is mainly nitrated. However, a compound in which other position (6, 7 and 9-position) is nitrated can be also produced and separated.

Compound (VIIb) can be produced by the method known per se or its analogous method. For example, it can be produced by methods described in J. Org. Chem., 34, 2235(1969), J. Org. Chem., 54, 5574(1989), Tetrahedron Lett., 35, 3023(1977), Bull. Chem. Soc. Jpn., 56, 2300(1983), Indian. J. Chem.,, 2,211(1964), Indian. J. Chem., 12, 247(1974), Bull. Chem. Soc.,, Jpn., 43, 1824(1970), Chem. Pharm. Bull., 20, 1328 (1972), Chem. Pharm. Bull., 27, 1982 (1979), Helv. Chem. Acra, 46, 1696(1963), Synthesis, 541(1979), U.S.3,682,962, U.S. 3,911,126., Ger. Offen. 2,314,392., Ger. 1,545,805, J. Chem. Soc., 1381(1949), Can. J. Chem., 42, 2904(1964), J. Org. Chem., 28, 3058 (1963), J. Am. Chem. Soc., 76, 3194(1954), 87, 1397(1965), 88, 4061(1966), JP-A 49-21539 and the like, or their analogous methods.

In Step (bd), Compound(IXb) can be produced by a reduction reaction of Compound (VIIIb).

The present reaction can be performed using a suitable reducing reaction (e.g., a catalytic reduction reaction using a transition metal catalyst, a reduction reaction using a metal such as tin and the like in an acidic solvent). Specifically, the reaction can be performed, for example by the known method such as methods described in Organic Synthesis, Coll. Vol. 5, 839-833(1973), Organic Synthesis, Coll. Vol.1, 455(1941), J. Am. Chem. Soc., 66, 1781(1944) or their analogous methods.

In Step (be), Compound (IVb) can be produced by a condensation reaction of Compound (IXb) and Compound (Xb).

The condensation reaction of Compound (IXb) and Compound (Xb) can be performed, for example, in the same manner as the condensation reaction of Compound (II) and Compound (III).

Further, Compound (IVb) can be also produced using Compound (IXb) as a raw material, for example, by a method by reductive alkylation (e.g., methods described in J. Am. Chem. Soc., 87, 2767(1965), Organic Synthesis, Coll. Vol.4, 283-285(1963) and the like) or a method by Michael addition reaction (e.g., methods described in Helv. Chem. Acta, 43, 1898(1960), J. Org. Chem., 39, 2044(1974), Synthesis, 5, 375(1981) and the like) or their analogous methods.

1-3) Among a raw material Compound (II), Compound (IIc) wherein -L- is -NR^{3a}CO- or a salt thereof can be produced by the following Reaction formula 3. That is, Compound (IIc) can be produced by successively performing:
Step (ca): an amidation reaction of Compound (IVb) and a compound represented by the formula (Vc) [wherein Z³ represents a leaving group, other symbols represent the same meanings as above] (hereinafter abbreviated as Compound (Vc) in some cases),
Step (cb): a deprotection reaction of a compound represented by the formula (VIc) [wherein respective symbols represent the same meanings as above] (hereinafter abbreviated as Compound (VIc) in some cases).

### [Reaction formula 3]

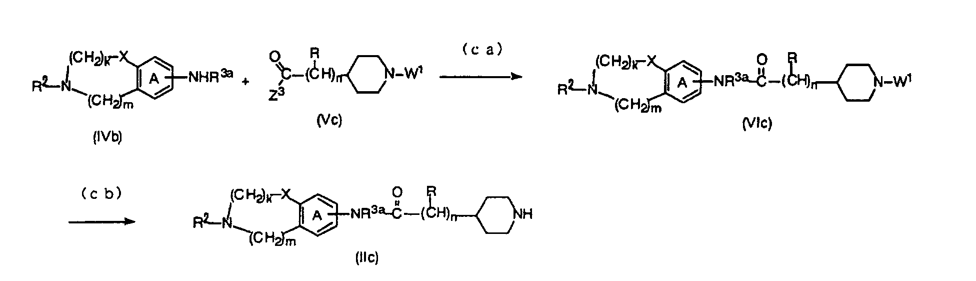

In Step (ca), Compound (VIc) can be produced by an amidation reaction of Compound (IVb) and Compound (Vc).

As a leaving group for Z³, for example, a halogen atom (e.g., chlorine, bromine, iodine), a C₁₋₆ alkyloxy group (e.g., methoxy, ethoxy, benzyloxy), a C₆₋₁₀ aryloxy group (e.g., phenoxy, p-nitrophenoxy), hydroxyl group and the like are used. In particular, for example, a halogen atom (preferably, chlorine and the like), hydroxyl group and the like are preferably used.

The amidation reaction of Compound (IVb) and Compound (Vc) can be also performed using a suitable condensing agent and a base. For example, when Z³ is hydroxyl group, the present amidation reaction can be performed using a suitable condensing agent, for example, a condensing agent which is generally used in the field of peptide chemistry, in particular, carbodiimides such as dicyclohexylcarbodiimide, 1-ethyl-3-(3-dimethylaminopropyl)carbodimide and the like, phosphonic acids such as diphenylphosphorylazide, cyanophosphonic diethyl and the like, phosgene equivalents such as 1-1'-carbonylbis-lH-imidazole and the like. The amount of the condensing agent used is usually about 1 equivalent to about 5 equivalents, preferably about 1 equivalent to about 1.5 equivalents relative to 1 mmol of Compound (IVb).

In addition, for example, when Z³ is a halogen atom, it is preferable that the reaction is performed using a suitable base, for example, sodium carbonate, potassium carbonate, triethylamine and the like. The amount of the base used is usually about 1 equivalent to about 10 equivalents, preferably about 1 equivalent to about 2 equivalents relative to Compound (IVd).

In Step (cb), a compound represented by the formula (IIc) [wherein respective symbols represent the same meanings as above] can be produced by subjecting Compound (VIc) to a deprotection reaction to remove W¹.

The deprotection reaction can be performed, for example, in the same manner as the deprotection reaction of Compound (VIa).

1-4) Among a raw material compound (II), Compound (IId) wherein -L- is -S-, -SO- or -SO₂-, or a salt thereof can be produced by the following Reaction formula 4-1. That is, Compound (IId) can be produced by successively performing:
Step (da): a condensation reaction of a compound represented by the formula (IVd) [wherein respective symbols represent the same meanings as above] (hereinafter abbreviated as Compound (IVd) in some cases) and Compound (Va),
Step (db): an oxidation reaction of a compound represented by the formula (VId) [wherein respective symbols represent the same meanings as above] (hereinafter abbreviated as Compound (VId) in some cases) as necessary, and
Step (dc): a deprotection reaction of a compound represented by the formula (VIId) [wherein -L^{b}- represents -S-, -SO- or -SO₂-, and other symbols represent the same meanings as described above] (hereinafter abbreviated as Compound (VIId) in some cases).

### [Reaction formula 4-1]

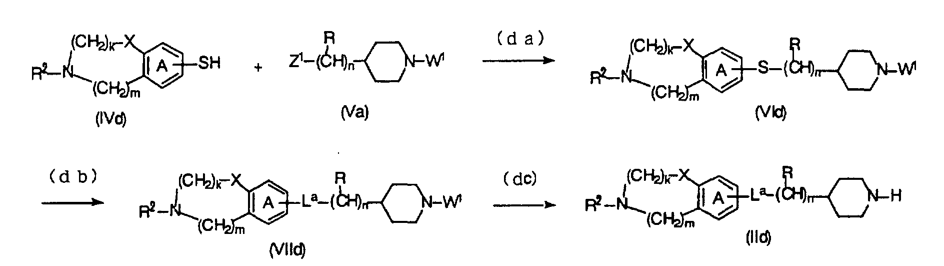

In Step (da), Compound (VId) can be produced by a condensation reaction of Compound (IVd) and Compound (Va).

The condensation reaction of Compound (IVd) and Compound (Va) can be performed, for example, in the same manner as the condensation reaction of Compound (II) and Compound (III). Specifically, for example, the reaction can be performed in a solvent of N,N-dimethylformamide and the like in the presence of a base such as potassium carbonate, sodium hydride and the like. The amount of the base used is preferably about 1 equivalent to about 3 equivalents relative to Compound (Va).

In Step (db), Compound (VIId) can be produced by performing an oxidation reaction of Compound (VId) as necessary.

Although any oxidizing agents may be used as long as they are used as an oxidizing agent for sulfides, for example, metachloroperbenzoic acid, peracetic acid, hydrogen peroxide, an alkali metal periodate and the like are preferably used. Particularly preferably, metachloroperbenzoic acid and hydrogen peroxide and the like are used. In the case of oxidation from S to SO, the amount of the oxidizing agent used is particularly preferably about 1 equivalent to about 1.1 equivalents relative to Compound (VId). In addition, in the case of oxidation from S to SO₂, the amount is particularly preferably about 2-2.5 equivalents relative to Compound (VId). As a solvent for the present reaction, for example, dichloromethane, chloroform, acetic acid, ethyl acetate and the like are preferable.

In Step (dc), Compound (IId) can be produced by subjecting Compound (VIId) to a deprotection reaction to remove W¹.

The present reaction can be performed, for example, in the same manner as the deprotection reaction of Compound (VIa)

A raw material Compound (IVd) or a salt thereof in Step (da) can be produced by the following Reaction formula 4-2. That is, Compound (IVd) can be produced by:
Step (dd): a chlorosulfonylation reaction of Compound (VIIb), and
Step (de): a reduction reaction of a compound represented by the formula (VIIId) [wherein respective symbols represent the same meanings as above] (hereinafter abbreviated as Compound (VIIId) in some cases).

### [Reaction formula 4-2]

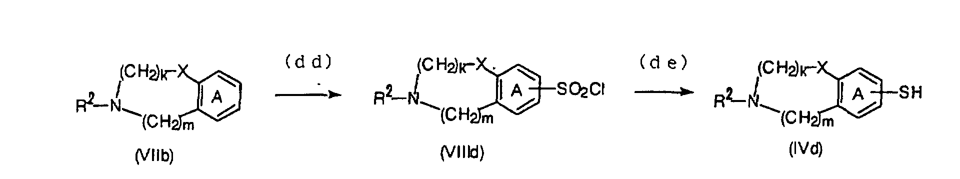

In Step (dd), Compond (VIIId) can be produced by chlorosulfonylating Compound (VIIb).

As a reagent for the present chlorosulfonylation reaction, for example, chlorosulfonic acid, sulfryl chloride, sulfur dioxide-copper chloride and the like can be used. In particular, chlorosulfonic acid and the like are preferable. The amount of the chlorosulfonylating reagent used is about 1 equivalent to large excessive amount. The present reaction may be performed without a solvent or using a solvent. As a solvent when the reaction is performed using a solvent, for example, dichloromethane, 1,2-dichloroethane, carbon disulfide and the like are preferable. A reaction without a solvent is particularly preferable. The reaction temperature is preferably about -20°C to about 100°C.

In addition, although a chlorosulfonyl group is introduced in any reactive positions, for example, when ring A is unsubstituted, X is a bond and k=m=2, the 7-position is mainly chlorosulfonylated. However, a compound in which the 6-position is chlorosulfonylated can be also produced and separated.

In Step (de), Compound (IVd) can be produced by reducing Compound (VIIId).

The present reduction reaction can be performed by suitable reduction conditions, for example, a catalytic reduction reaction using a combination of a metal and an acid such as zinc-acetic acid, tin-hydrochloric acid and the like, or with a metal hydride such as lithium aluminum hydride and the like. Particularly preferred is a reduction reaction using zinc-acetic acid.

1-5) Among a raw material compound (II), Compound (IIe) wherein -L- is -SO₂NR^{3a}- or a salt thereof can be produced by the following Reaction formula 5. That is, Compound (IIe) can be performed by successively performing:
Step (ea): a condensation reaction of Compound (VIIId) and a compound represented by the formula (IVe) [wherein respective symbols represent the same meanings as above] (hereinafter abbreviated as Compound (IVe) in some cases), and
Step (eb): a deprotection reaction of a compound represented by the formula (Ve) [wherein respective symbols represent the same meanings as above] (hereinafter abbreviated as Compound (Ve) in some cases).

### [Reaction formula 5]

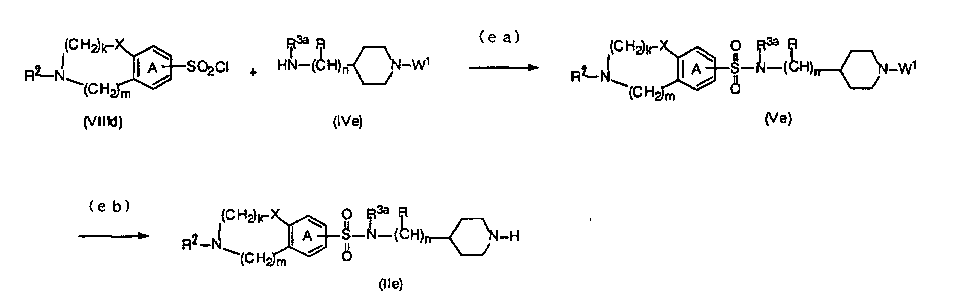

In Step (ea), Compound (Ve) can be produced by a condensation reaction of compound (VIIId) and Compound (IVe).

The condensation reaction of Compound (VIIId) and Compound (IVe) can be performed, for example, in the same manner as the amidation reaction of Compound (IVb) and Compound (Vc).

Compound (IVe) or a salt thereof can be produced by the method known per se or its analogous method. For example, the compound can be produced by a method described in J. Med. Chem., 33, 1880(1990) and the like or its analogous method.

In Step (eb), a compound represented by the formula (IIe) [wherein respective symbols represent the same meanings as above] can be produced by subjecting Compound (Ve) to a deprotection reaction to remove W¹.

The present deprotection reaction can be performed, for example, in the same manner as the deprotection reaction of Compound (VIa).

1-6) Among a raw material Compound (II), Compound (IIf) wherein -L- is -SO₂NHCONR^{3a}- or a salt thereof can be produced by the following Reaction formula 6. That is, Compound (IIf) can be produced by successively performing:
Step (fa): a condensation reaction of Compound (VIIId) and Compound (IVe), and
Step (fb): a deprotection reaction of a compound represented by the formula (Vf) [wherein respective symbols represent the same meanings as above] (hereinafter abbreviated as Compound (Vf) in some cases).

### [Reaction formula 6]

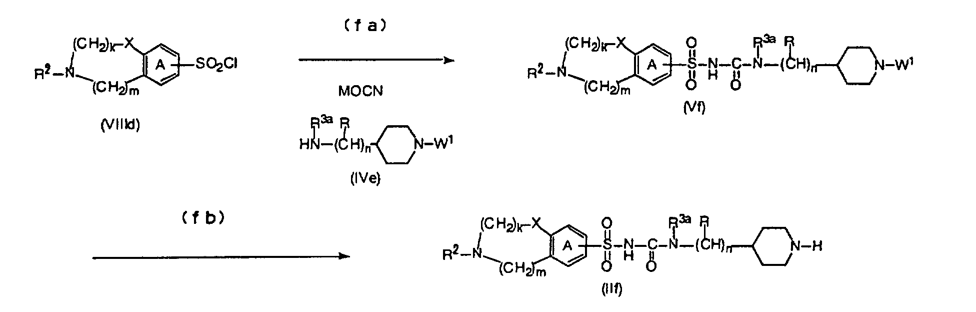

In Step (fa), Compound (Vf) can be produced by acting an alkali metal isocyanate (MOCN; wherein M represents an alkali metal) on Compound (VIIId) and, thereafter, reacting with Compound (IVe). The present reaction can be performed by methods described in EP-759431, JP-A 7-118267 and the like or their analogous methods.

The reaction of Compound (VIIId) and an alkali metal isocyanate can be performed in the presence of a base as necessary. As the base used, in particular, pyridine, triethylamine and the like are preferable. The amount of the base to be used is preferably about 1 equivalent to about 5 equivalents relative to Compound (VIIId). As the reaction solvent, in particular, acetonitrile and the like are preferably used. As the alkali metal, for example, potassium and the like are preferably used.

In Step (fb), Compound (IIf) can be produced by subjecting Compound (Vf) to a deprotection reaction to remove W¹.

The present reaction can be performed, for example, in the same manner as the deprotection reaction of Compound (VIa).

1-7) Among a raw material Compound (II), Compound (IIg) wherein -L- is -SO₂NHC(=NH)NR^{3a}- or a salt thereof can be produced by the following Reaction formula 7. That is, Compound (IIg) can be produced by successively performing:
Step (ga): a condensation reaction of Compound (VIIId) and a compound represented by the formula (IVg) [wherein respective symbols represent the same meanings as above] (hereinafter abbreviated as Compound (IVg) in some cases), and
Step (gb): a deprotection reaction of a compound represented by the formula (Vg) [wherein respective symbols represent the same meanings as above] (hereinafter abbreviated as Compound (Vg) in some cases).

### [Reaction formula 7]

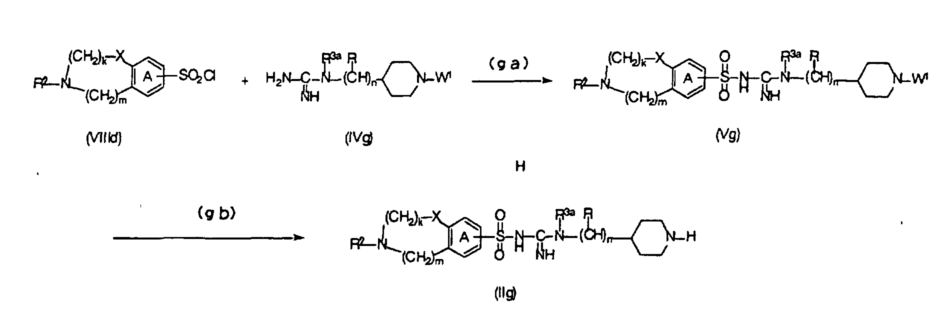

In Step (ga), Compound (Vg) can be produced by a condensation reaction of Compound (VIIId) and Compound (IVg).

The condensation reaction of Compound (VIIId) and Compound (IVd) can be performed, for example, in the same manner as the amidation reaction of Compound (IVb) and Compound (Vc).

Compound (IVg) can be produced using Compound (IVe) by the method known per se or its analogous method. For example, Compound (IVg) can be produced by a method for acting S-methylisothiourea on Compound (IVe) (e.g., a method described in J. Org. Chem., 13, 924(1948) and the like), a method for acting cyanamide thereon (e.g., a method described in Helv. Chim. Acra, 29, 324(1946) and the like), and a method for acting 1,3-bis(tert-butoxycarbonyl)-methyl-2-thiopseudourea thereon (e.g., methods described in Tetrahedron Lett., 33, 6541-6542(1992), J. Org. Chem., 52, 1700-1703 (1987)) and the like.

In Step (gb), a compound represented by the formula (IIg) [wherein respective symbols represent the same meanings as above] by subjecting Compound (Vg) to a deprotection reaction to remove W¹.

The present reaction can be performed, for example, in the same manner as the deprotection reaction of Compound (VIa).

1-8) Among a raw material Compound (II), Compound (IIh) wherein -L- is 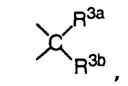 or a salt thereof can be produced by the following reaction formula 8. That is, Compound (IIh) can be produced by successively performing:
Step (ha): a carbonyl group conversion reaction of a compound represented by the formula (IVh) [wherein respective symbols represent the same meanings as above] (hereinafter abbreviated as Compound (IVh) in some cases), and
Step (hb): a deprotection reaction of a compound represented by the formula (Vh) [wherein respective symbols represent the same meanings as above] (hereinafter abbreviated as Compound (Vh) in some cases).

### [Reaction formula 8]

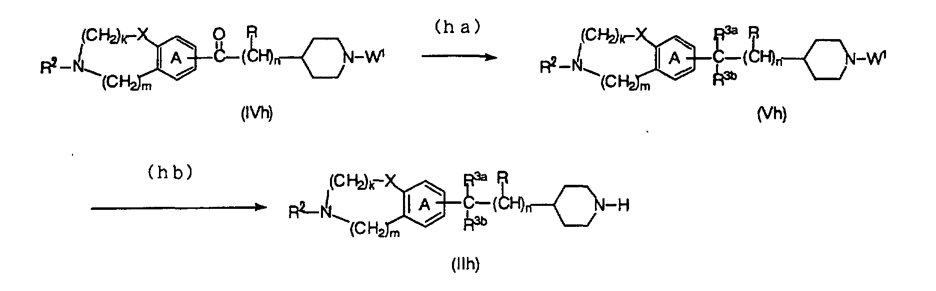

In Step (ha), Compound (Vh) can be produced by converting carbonyl group by reacting Compound (IVh) with a suitable reagent.

As a reagent used for converting carbonyl group, for example, used are a reducing agent such as sodium borohydride, lithium aluminum hydride, triethylsilane and the like, for example, an organic metal reagent such as alkyllithium, alkylmagnesium halide and the like, and others, for example, a nucleophile reacting agent such as hydrogen cyanide and the like.

Specifically, conversion of carbonyl group into-CH(OH)- or -CH₂- can be performed, for example, using a reducing agent such as sodium borohydride, lithium aluminum hydride, triethylsilane and the like, under the suitable reducing conditions (e.g., combination of triethylsilane-trifluoroacetic acid, lithium aluminum hydride-aluminum chloride, zinc-hydrochloric acid and the like).

The present reaction can be performed, for example, by methods described in Reduction with Complex Metal Hydrides Interscience, New York (1956), Chem. Soc. Rev., 5, 23(1976), Synthesis, 633(1974), J. Am. Chem. Soc. 91, 2967(1969), J. Org. Chem., 29, 121(1964), Org. Reactions, 1, 155(1942), Angew. Chem., 71, 726(1956), Synthesis, 633(1974), J. Am. Chem. Soc., 80, 2896(1958), Org. Reactions, 4, 378(1948), J. Am. Chem. Soc., 108, 3385(1986) and the like or their analogous methods.

In addition, conversion of carbonyl group into-CR^{3c}(OH)- (wherein R^{3c} represents a C₁₋₆ alkyl group) can be performed using, for example, an organic metal reagent such as alkyl lithium, alkyl magnesium halide and the like, by methods described in Grignard Reactions of Nonmetallic Substances, Prentice-Hall: Englewood Cliffs, NJ, 1954, pp. 138-528, Organolithium Methods, Academic Press: New York, 1988, pp.67-75 or their analogous methods.

In addition, conversion of carbonyl group can be performed by methods described in Advanced Organic Chemistry, 5th ed. Wiley-Interscience: New York, 1992, pp.879-981 and the like, or their analogous methods.

Compound (IVh) can be produced by the method known per se or its analogous method, for example, by methods described in JP-A 5-140149, JA-A 6-206875, J. Med. Chem., 37, 2292(1994) and the like or their analogous methods.

In Step (hb), Compound (IIh) can be produced by subjecting Compound (Vh) to a deprotection reaction to remove W¹.

The present reaction can be performed, for example, in the same manner as the deprotection reaction of Compound (VIa).

1-9) Among a raw material Compound (II), Compound (IIi) wherein -L- is 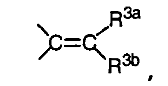 or a salt thereof can be produced by the following Reaction formula 9. That is, Compound (IIi) can be produced by successively performing:
Step (ia): a carbonyl group conversion reaction of Compound (IVh), and
Step (ib): a deprotection reaction of a compound represented by the formula (Vi) [wherein respective symbols represent the same meanings as above] (hereinafter abbreviated as Compound (Vi) in some cases).

### [Reaction formula 9]

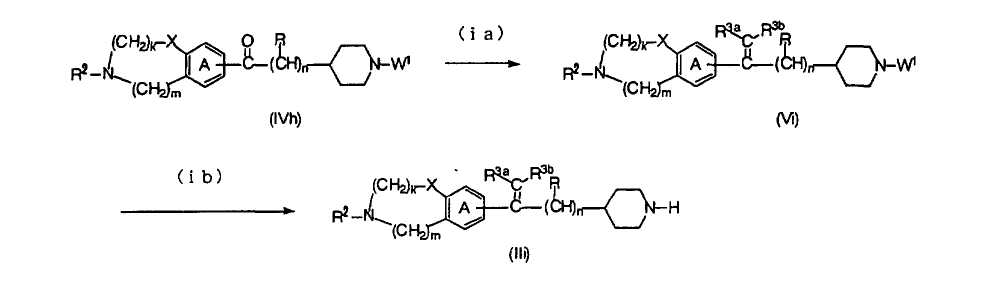

In Step (ia), Compound (Vi) can be produced by converting carbonyl group by reacting Compound (IVh) with a suitable reagent.

As a reaction for converting carbonyl group, for example, a Wittig reaction, a Horner-Wadsworth-Emmons reaction, a Petersol Olefinization reaction, a Knoevenagel reaction and the like are mentioned, and as a reagent, reagents which are generally used in those reactions are used.

The present reaction can be performed, for example, by methods described in Advanced Organic Chemistry, 5th ed. Wiley-Interscience: New York, 1992, pp.879-981, Organic Synthesis, coll. vol.5, 751(1973), Organic Synthesis, coll. vol.5, 509 (1973), Synthesis, 384(1984), Org. Reactions, 15, 204(1967) and the like or their analogous methods.

In Step (ib), Compound (IIi) can be produced by subjecting Compound (Vi) to a deprotection reaction to remove W¹.

The present reaction can be performed, for example, in the same manner as the deprotection reaction of Compound (VIa).

1-10) Among a raw material Compound (II), Compound (IIj) wherein -L- is 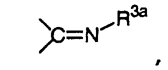 or a salt thereof can be produced by the following Reaction formula 10. That is, Compound (IIj) can be produced by successively performing:
Step (ja): a carbonyl group conversion reaction of Compound (IVh), and
Step (jb): a deprotection reaction of a compound represented by the formula (Vj) [wherein respective symbols represent the same meanings as above] (hereinafter abbreviated as Compound (Vj) in some cases).

### [Reaction formula 10]

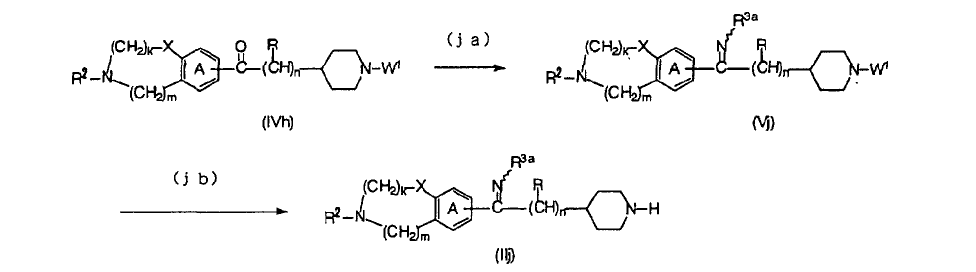

In Step (ja), Compound (Vj) can be produced by converting carbonyl group by reacting Compound (IVh) with a suitable reagent.

As a reagent used in a carbonyl group conversion reaction, for example, an optionally substituted hydrazine, an optionally substituted hydroxylamine and the like can be mentioned. As the substituent, a C₁₋₆ alkyl group and the like are used.

The present reaction can be performed by methods described in Advanced Organic Chemistry, 5th ed. Wiley-Interscience: New York, 1992, pp. 904-907, Organic Functional Group Preparations, vol. III, Academic (1983), Rodd's Chemistry of Carbon Compounds, vol.1, part C, Elsevier Publishing co. (1965) and the like or their analogous methods.

In Step (jb), Compound (IIj) can be produced by subjecting Compound (Vj) to a deprotection reaction to remove W¹.

The present reaction can be performed, for example, in the same manner as the deprotection reaction of Compound (VIa).

1-11) Among a raw material Compound (II), Compound (IIk) wherein -L- is 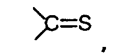 or a salt thereof can be produced by the following Reaction formula 11. That is, Compound (IIk) can be produced by successively performing:
Step (ka): a carbonyl group conversion reaction of Compound (IVh), and
Step (kb): a deprotection reaction of a compound represented by the formula (Vk) [wherein respective symbols represent the same meanings as above] (hereinafter abbreviated as Compound (Vk) in some cases].

### [Reaction formula 11]

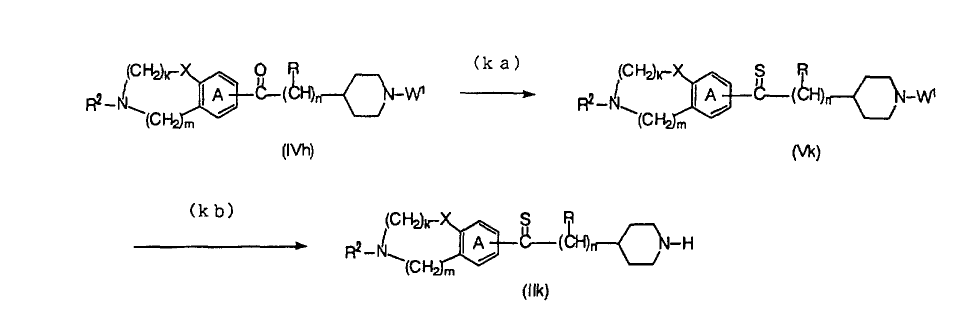

In Step (ka), Compound (vk) can be produced by converting carbonyl group into thiocarbonyl group by reacting Compound (IVh) with a suitable reagent.

As a reagent used for converting carbonyl group into thiocarbonyl group, for example, general sulfrating reagents such as a Lawesson reagent, diphosphorus pentasulfide, hydrogen sulfide-hydrochloric acid and the like are mentioned.

The present reaction can be performed by methods described in Synthesis, 7. 543(1991), J. Am. Chem. Soc., 106, 934(1984), J. Am. Chem. Soc. 68, 769(1946) and the like or their analogous methods.

In Step (kb), Compound (IIk) can be produced by subjecting Compound (Vk) to a deprotection reaction to remove W¹.

The present reaction can be performed, for example, in the same manner as the deprotection reaction of Compound (VIa).

1-12) Among a raw material Compound (II), Compound (IIm) wherein -L- is -CONR^{3a}- or a salt thereof can be produced by the following Reaction formula 12-1. That is, Compound (IIm) can be produced by successively performing:
Step (ma): a condensation reaction of a compound represented by the formula (Vm) [wherein respective symbols represent the same meanings as described above] (hereinafter abbreviated as Compound (Vm) in some cases) and Compound (IVe), and
Step (mb): a deprotection reaction of a compound represented by the formula (VIm) [wherein respective symbols represent the same meanings as described above] (hereinafter abbreviated as Compound (VIm) in some cases).

### [Reaction formula 12-1]

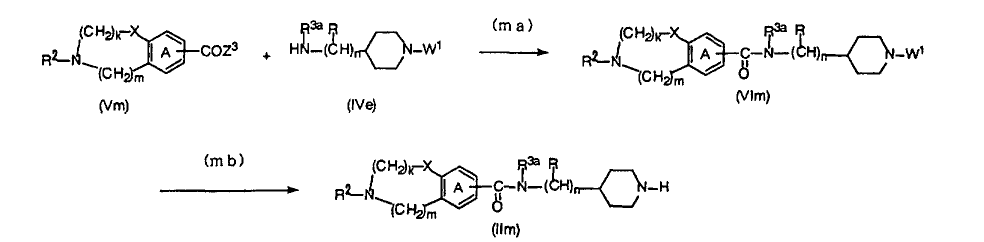

In Step (ma), Compound (VIm) can be produced by a condensation reaction of compound (Vm) and Compound (IVe).

The reaction of Compound (Vm) and Compound (IVe) can be performed, for example, in the same manner as the amidation reaction of Compound (IVb) and Compound (Vc).

In Step (mb), Compound (IIm) can be produced by subjecting Compound (VIm) to a deprotection reaction to remove W¹.

The present reaction can be performed, for example, in the same manner as the deprotection reaction of Compound (VIa).

In addition, a raw material Compound (Vm) in Step (ma) can be produced by the following Reaction formula 12-2. That is, Compound (Vm) can be produced by successively performing:
Step (mc): an acetylation reaction of Compound (VIIIb), and
Step (md): an oxidation reaction of a compound represented by the formula (VIIIm) [wherein respective symbols represent the same meanings as above] (hereinafter abbreviated as Compound (VIIIm) in some cases) and a functional group conversion as necessary.

### [Reaction formula 12-2]

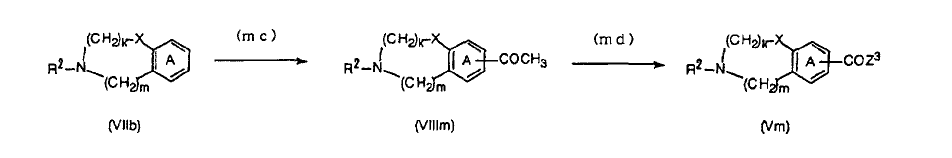

In Step (mc), Compound (VIIIm) can be produced by acetylating Compound (VIIb).

The present reaction can be performed by the general conditions for a Friedel-Crafts reaction. As a reagent for acetylation, acetyl chloride and acetic anhydride and the like are used. Specifically, the compound can be produced by methods described in JP-A 5-140149, JP-A 6-206875, J. Med. Chem., 37, 2292(1994) and the like, or their analogous methods. Although acetyl group is introduced into any reactive positions, for example, when ring A is unsubstituted benzene ring, X is a bond, and k=3 and m=1, the 8-position is mainly acetylated. However, a compound in which other position (6-, 7- and 9-positions) is acetylated can be produced and separated.

In Step (md), Compound (Vm), in particular, a compound in which Z³ is hydroxyl group can be produced by oxidizing Compound (VIIIm).

As an oxidizing agent used in the present reaction, for example, hypochlorite, hypobromite, or a halogen substance (e.g., bromine, iodine and the like) in the presence of a suitable base (e.g., sodium hydroxide and the like). Specifically, the present reaction can be performed, for example, by methods described in Org. Synthesis, Coll. Vol. 2, 428(1943), J. Am. Chem. Soc., 66, 894(1944) and the like, or their analogous methods.

In addition, if necessary, Compound (Vm) wherein Z³ is a hydroxyl group can be converted into Compound (Vm) wherein Z³ is a halogen atom (e.g., chlorine, bromine, iodine), a C₁₋₆ alkoxy group (e.g., methoxy, ethoxy and the like), a C₇₋₁₆ aralkyloxy group (e.g., benzyloxy and the like), or a C₆₋₁₀ aryloxy group (e.g., phenoxy, p-nitrophenoxy and the like) by a functional group conversion of hydroxy group thereof.

A method for the functional group conversion can be performed, for example, by methods described in Advanced Organic Chemistry, 5th ed. Wiley-Interscience: New York, 1992, pp. 393-396, 437-438, Comprehensive Organic Transformations, VCH Publishers Inc. (1989) and the like, or their analogous methods.

2) Alternatively, Compound (I) can be produced by the following method and the like.

2-1) Among Compound (I), Compound (Iee) wherein -L- is -SO₂NR^{3a}- or a salt thereof can be produced by the following Reaction formula 2-1. That is, Compound (Iee) can be produced by a condensation reaction of Compound (VIIId) and a compound represented by the formula (IVee) [wherein respective symbols represent the same meanings as above] (hereinafter abbreviated as Compound (IVee) in some cases).

### [Reaction formula 2-1]

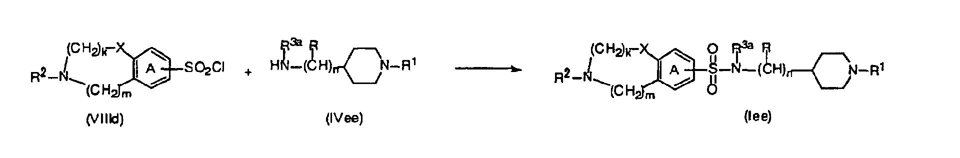

The condensation reaction of Compound (IIId) and Compound (IVee) can be performed, for example, in the same manner as the amidation reaction of Compound (IVb) and Compound (Vc).

Compound (IVee) or a salt thereof can be produced by the method known per se or its analogous method. For example, the compound can be produced by methods described in J. Med. Chem., 33, 1880(1990) and the like, or their analogous methods.

2-2) Among Compound (I), Compound (Iff) wherein -L- is -SO₂NHCONR^{3a}-, or a salt thereof can be produced by the following Reaction formula 2-2. That is, a compound represented by the formula (Iff) [wherein respective symbols represent the same meanings as above] can be produced by reacting an alkali metal isocyanate (MOCN; wherein M represents an alkali metal) on Compound (VIIId) and, thereafter, reacting with Compound (IVee).

### [Reaction formula 2-2]

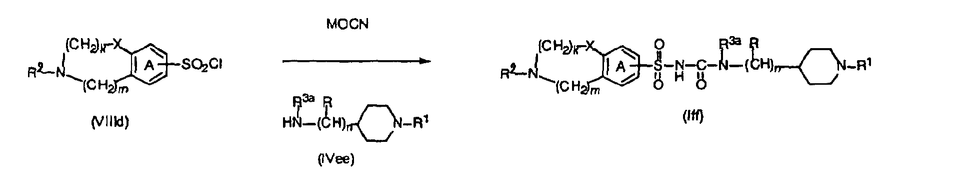

The present reaction can be performed, for example, in the same manner as the condensation reaction of Compound (VIIId) and Compound (IVe).

2-3) Among Compound (I), Compound (Igg) wherein -L- is -SO₂NHC(=NH)NR^{3a}-, or a salt thereof can be produced by the following Reaction formula 2-3. Compound (Igg) can be produced by a condensation reaction of Compound (VIIId) and a compound represented by the formula (IVgg) [wherein respective symbols represent the same meanings as above] (hereinafter abbreviated as Compound (IVgg) in some cases).

### [Reaction formula 2-3]

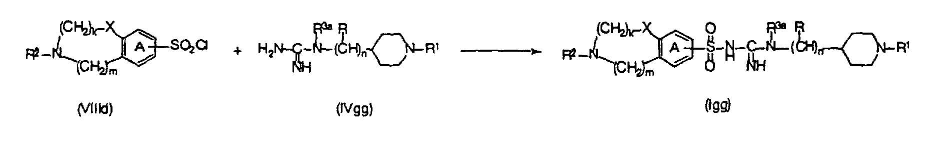

The condensation reaction of Compound (VIIIb) and Compound (IVgg) can be performed, for example, in the same manner as the amidation reaction of Compound (IVb) and Compound (Vc).

Compound (IVgg) can be produced using Compound (IVee) in the same manner as Compound (IVg).

2-4) Among Compound (I), Compound (Ihh) wherein -L- is 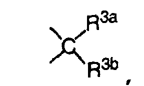 or a salt thereof can be produced by the following Reaction formula 2-4. That is, Compound (Ihh) [wherein respective symbols represent the same meanings as above] can be produced by converting its carbonyl group by reacting a compound represented by the formula (IVhh) [wherein respective symbols represent the same meanings as above] with a suitable reagent.

### [Reaction formula 2-4]

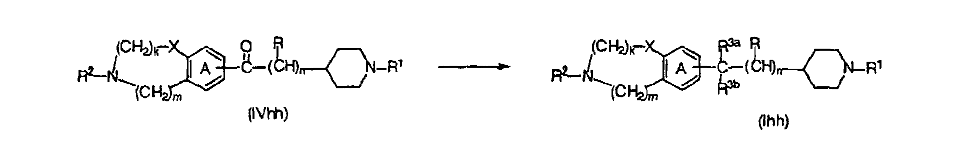

The present reaction can be performed, for example, in the same manner as the conversion reaction of Compound (IVh) into Compound (Vh) described in the above 1-8).

Compound (IVhh) can be produced by the method known per se or its analogous method, for example, methods described in JP-A 5-140149, JP-A 6-206875, J. Med. Chem., 37, 2292 (1994) and the like, or their analogous methods.

2-5) Among Compound (I), Compound (Iii) wherein -L- is 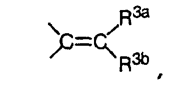 or a salt thereof can be produced by the following Reaction formula 2-5. That is, a compound represented by the formula (Iii) [wherein respective symbols represent the same meanings as above] can be produced by converting carbonyl group by reacting Compound (IVhh) with a suitable reagent.

### [Reaction formula 2-5]

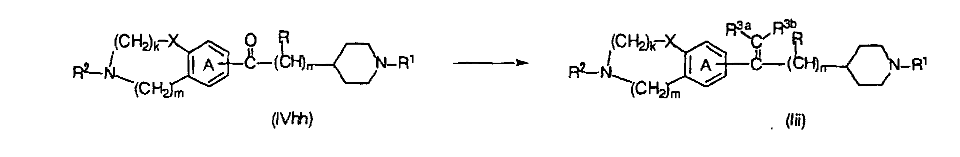

The present reaction can be performed, for example, in the same manner as the conversion reaction of Compound (IVh) into Compound (Vi).

2-6) Among Compound (I), Compound (Ijj) wherein -L- is 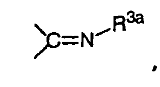 or a salt thereof can be produced by the following Reaction formula 2-6. That is, a compound represented by the formula (Ijj) [wherein respective symbols represent the same meanings as above] can be produced by converting carbonyl group by reacting Compound (IVhh) with a suitable reagent.

### [Reaction formula 2-6]

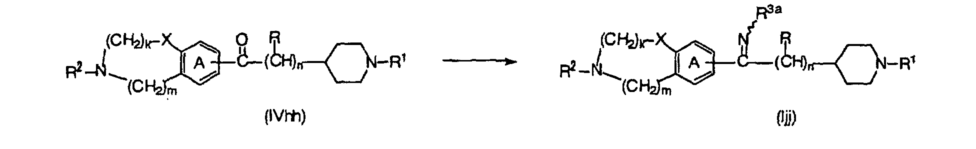

The present reaction can be performed, for example, in the same manner as the conversion reaction of Compound (IVh) into Compound (Vj) described in the above 1-10).

2-7) Among Compound (I), Compound (Ikk) wherein -L- is 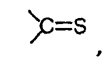 or a salt thereof can be produced by the following Reaction formula 2-7. That is, a compound represented by the formula (Ikk) [wherein respective symbols represent the same meanings as above] can be produced by converting carbonyl group into thiocarbonyl group by reacting Compound (IVhh) with a suitable reagent.

### [Reaction formula 2-7]

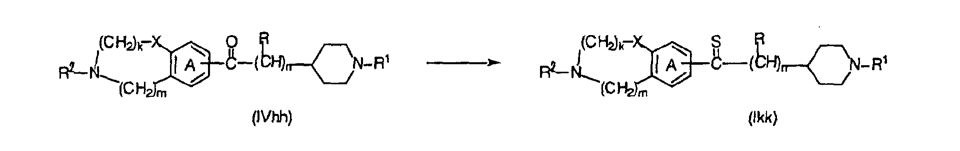

The present reaction can be performed, for example, in the same manner as the conversion reaction of Compound (IVh) into Compound (Vk) described in the above 1-11).

2-8) Among Compound (I), Compound (Imm) wherein -L- is -CONR^{3a}-, or a salt thereof can be produced by the Reaction formula 2-8. That is, a compound represented by the formula (Imm) [wherein respective symbols represent the same meanings as above] by a condensation reaction of Compound (Vm) and Compound (IVee).

### [Reaction formula 2-8]

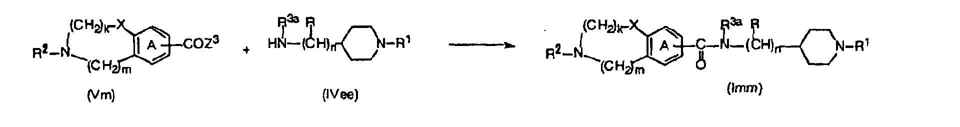

The present reaction can be performed, for example, in the same manner as the amidation reaction of Compound (IVb) and Compound (Vc).

### [B] In this item, Compound (IA) will be explained in detail.

As the "substituent" in the "benzene ring optionally having a substituent" for ring A in the formula (IA), for example, used are (i) an optionally halogenated lower alkyl group, (ii) a halogen atom (e.g., fluorine, chlorine, bromine, iodine and the like), (iii) nitro group, (iv) cyano group, (v) hydroxy group, (vi) an optionally halogenated lower alkoxy group, (vii) amino group, (viii) a mono-lower alkylamino group (e.g., a mono-C₁₋₆ alkylamino group and the like such as methylamino, ethylamino, propylamino and the like), (ix) a di-lower alkylamino group (e.g., a di-C₁₋₆ alkylamino group and the like such as dimethylamino, diethylamino and the like), (x) a 5 to 7 membered cyclic amino group (e.g., pyrrolidino, piperidino, morpholino, thiomorpholino and the like) optionally having 1 to 3 hetero atoms selected from nitrogen atom, oxygen atom and sulfur atom and the like in addition to, for example, one nitrogen atom, (xi) a lower alkyl-carbonylamino group (e.g., a C₁₋₆ alkyl-carbonylamino group and the like such as acetylamino, propionylamino, butyrylamino and the like), (xii) aminocarbonyloxy group, (xiii) a mono-lower alkylamino-carbonyloxy group (e.g., a mono-C₁₋₆ alkylamino-carbonyloxy group and the like such as methylaminocarbonyloxy, ethylaminocarbonyloxy and the like), (xiv) a di-lower alkylamino-carbonyloxy group (e.g., a di-C₁₋₆ alkylamino-carbonyloxy group and the like such as dimethylaminocarbonyloxy, diethylaminocarbonyloxy and the like), (xv) a lower alkylsulfonylamino group (e.g., a C₁₋₆ alkylsulfonylamino group and the like such as methylsulfonylamino, ethylsulfonylamino, propylsulfonylamino and the like), (xvi) a lower alkoxy-carbonyl group (e.g., a C₁₋₆ alkoxy-carbonyl group and the like such as methoxycarbonyl, ethoxycarbonyl, propoxycarbonyl, isobutoxycarbonyl and the like), (xvii) carboxyl group, (xviii) a lower alkyl-carbonyl group (e.g., a C₁₋₆ alkyl-carbonyl group and the like such as methylcarbonyl, ethylcarbonyl, butylcarbonyl and the like), (xix) carbamoyl group, (xx) a mono-lower alkyl-carbamoyl group (e.g., a mono-C₁₋₆ alkyl-carbamoyl group and the like such as methylcarbamoyl, ethylcarbamoyl, propylcarbamoyl, butylcarbamoyl and the like), (xxi) a di-lower alkyl-carbamoyl group (e.g., a di-C₁₋₆ alkyl-carbamoyl group and the like such as diethylcarbamoyl, dibutylcarbamoyl and the like), (xxii) a lower alkyl-thiocarbonyl group (e.g., a C₁₋₆ alkyl-thiocarbonyl group and the like such as methylthiocarbonyl, ethylthiocarbonyl, butylthiocarbonyl and the like), (xxiii) thiocarbamoyl group, (xxiv) a mono-lower alkyl-thiocarbamoyl group (e.g., a mono-C₁₋₆ alkyl-thiocarbamoyl and the like such as methylthiocarbamoyl, ethylthiocarbamoyl, propylthiocarbamoyl, butylthiocarbamoyl and the like), (xxv) a di-lower alkyl-thiocarbamoyl group (e.g., a di-C₁₋₆ alkyl-thiocarbamoyl group and the like such as diethylthiocarbamoyl, dibutylthiocarbamoyl and the like), (xxvi) phenyl group [the (xxvi) phenyl group may further have one to four substituents selected from, for example, a lower alkyl (a C₁₋₆ alkyl and the like such as methyl, ethyl, propyl, isopropyl, butyl, sec-butyl, tert-butyl, pentyl, hexyl and the like), a lower alkoxy (e.g., a C₁₋₆ alkoxy and the like such as methoxy, ethoxy, propoxy, isopropoxy, n-butoxy, isobutoxy, sec-butoxy, tert-butoxy and the like), halogen (e.g., fluorine, chlorine, bromine, iodine and the like), hydroxy, amino, s mono-lower alkylamino (e.g., a mono-C₁₋₆ alkylamino and the like such as methylamino, ethylamino, propylamino and the like), a di-lower alkylamino (e.g., a di-C₁₋₆ alkylamino and the like such as dimethylamino, diethylamino and the like), nitro, a lower alkyl-carbonyl (e.g., a C₁₋₆ alkyl-carbonyl and the like such as methylcarbonyl, ethylcarbonyl, butylcarbonyl and the like) and the like].

As the above "optionally halogenated lower alkyl group", for example, used are a lower alkyl group (e.g., a C₁₋₆ alkyl group and the like such as methyl, ethyl, propyl, isopropyl, butyl, sec-butyl, tert-butyl, pentyl, hexyl and the like) optionally having one to three halogens (e.g., fluorine, chlorine, bromine, iodine and the like) and, as embodiments thereof, used are methyl, chloromethyl, difluoromethyl, trichloromethyl, trifluoromethyl, ethyl, 2-bromoethyl, 2,2,2-trifluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl and the like.

As the above "optionally halogenated lower alkoxy group", for example, mentioned are a lower alkoxy group (e.g., a C₁₋₆ alkoxy group and the like such as methoxy, ethoxy, propoxy, isopropoxy, n-butoxy, isobutoxy, secbutoxy, tert-butoxy and the like) optionally having one to three halogens (e.g., fluorine, chlorine, bromine, iodine and the like) and, as embodiments thereof, for example, used are methoxy, difluoromethoxy, trifluoromethoxy, ethoxy, 2,2,2-trifluoroethoxy, n-propoxy, isopropoxy, n-butoxy, 4,4,4-trifluorobutoxy, isobutoxy, sec-butoxy, pentyloxy, hexyloxy and the like.

As the "substituent" in the "benzene optionally having a substituent", preferably used are a lower alkyl group (e.g., a C₁₋₆ alkyl group and the like such as methyl, ethyl, propyl, isopropyl, butyl, sec-butyl, tert-butyl, pentyl, hexyl and the like), a lower alkoxy group (e.g., a C₁₋₆ alkoxy group and the like such as methoxy, ethoxy, propoxy, isopropoxy, n-butoxy, isobutoxy, sec-butoxy, tert-butoxy and the like), a halogen atom (e.g., fluorine, chlorine, bromine, iodine and the like), hydroxy group, amino group, a mono-lower alkylamino group (e.g., a mono-C₁₋₆ alkylamino group and the like such as methylamino, ethylamino, propylamino and the like), a di-lower alkylamino group (e.g., a di-C₁₋₆ alkylamino group and the like such as dimethylamino, diethylamino and the like), nitro group and the like.

The "hydrocarbon group" in the "hydrocarbon group optionally having a substituent" for R¹ and R² represents a group in which a hydrogen atom is removed from a hydrocarbon compound and, as an example thereof, used are the following alkyl group, alkenyl group, alkynyl group, cycloalkyl group, aryl group, aralkyl group, a group comprising combination of these groups and the like.
(1) an alkyl group (e.g., a C₁₋₆ alkyl group and the like such as methyl, ethyl, propyl, isopropyl, butyl, isobutyl, tert-butyl, sec-butyl, pentyl, hexyl and the like),
(2) an alkenyl group (e.g., a C₂₋₆ alkenyl group and the like such as vinyl, allyl, isopropenyl, butenyl, isobutenyl, sec-butenyl and the like),
(3) an alkynyl group (e.g., a C₂₋₆ alkynyl group and the like such as propargyl, ethynyl, butynyl, 1-hexynyl and the like),
(4) a cycloalkyl group (e.g., a monocyclic C₃₋₆ cycloalkyl group and the like such as cyclopropyl, cyclobutyl, cyclopentyl, cyclohexyl and the like),
(5) a cross-linked cyclic lower saturated hydrocarbon group (e.g., a cross-linked cyclic C₈₋₁₄ saturated hydrocarbon group such as bicyclo[3.2.1]oct-2-yl, bicyclo[3.3.1]non-2-yl, adamantane-1-yl and the like),
(6) an aryl group (e.g., C₆₋₁₄ aryl group and the like such as phenyl, 1-naphthyl, 2-naphthyl, biphenyl, 2-indenyl, 2-anthryl and the like, preferably phenyl group and the like),
(7) an aralkyl group (e.g., a phenyl-C₁₋₁₀ alkyl such as benzyl, phenylethyl, phenylpropyl, phenylbutyl, phenylpentyl, phenylhexyl and the like; for example, a naphthyl-C₁₋₆ alkyl such as α-naphthylmethyl and the like; for example, a diphenyl-C₁₋₃ alkyl group and the like such as diphenylmethyl, diphenylethyl and the like),
(8) an aryl-alkenyl group (e.g., a C₆₋₁₄ aryl-C₂₋₁₂ alkenyl group and the like such as a phenyl-C₂₋₁₂ alkenyl and the like such as styryl, cinnamyl, 4-phenyl-2-butenyl, 4-phenyl-3-butenyl and the like),
(9) an aryl-C₂₋₁₂ alkynyl group (e.g., a C₆₋₁₄ aryl-C₂₋₁₂ alkynyl group and the like such as a phenyl-C₂₋₁₂ alkynyl and the like such as phenylethynyl, 3-phenyl-2-propynyl, 3-phenyl-1-propynyl and the like),
(10) a cycloalkyl-lower alkyl group (e.g., a C₃₋₇ cycloalkyl-C₁₋₆ alkyl group such as cyclopropylmethyl, cyclobutylmethyl, cyclopentylmethyl, cyclohexylmethyl, cycloheptylmethyl, cyclopropylethyl, cyclobutylethyl, cyclopentylethyl, cyclohexylethyl, cycloheptylethyl, cyclopropylpropyl, cyclobutylpropyl, cyclopentylpropyl, cyclohexylpropyl, cycloheptylpropyl, cyclopropylbutyl, cyclobutylbutyl, cyclopentylbutyl, cyclohexylbutyl, cycloheptylbutyl, cyclopropylpentyl, cyclobutylpentyl, cyclopentylpentyl, cyclohexylpentyl, cycloheptylpentyl, cyclopropylhexyl, cyclobutylhexyl, cyclopentylhexyl, cyclohexylhexyl and the like),
(11) an aryl-aryl-C₁₋₁₀ alkyl group (e.g., biphenylmethyl, biphenylethyl and the like).

As the "hydrocarbon group" in the "hydrocarbon group optionally having a substituent" for R¹ and R², preferably used are, for example, a C₁₋₆ alkyl group, a C₃₋₆ cycloalkyl group, a C₇₋₁₆ aralkyl group and the like. More preferably used are a C₇₋₁₆ aralkyl group (e.g., a phenyl-C₁₋₁₀ alkyl and the like such as benzyl, phenylethyl, phenylpropyl and the like) and the like.

As the "substituent" in the "hydrocarbon group optionally having a substituent" for R¹ and R², used are one to five (preferably one to three) substituents selected from (i) a halogen atom (e.g., fluorine, chlorine, bromine, iodine and the like), (ii) nitro group, (iii) cyano group, (iv) oxo group, (v) hydroxy group, (vi) an optionally halogenated lower (C₁₋₆)alkyl group (e.g., methyl, ethyl, propyl, isopropyl, butyl, isobutyl, tert-butyl, sec-butyl, trifluoromethyl, trichloromethyl and the like), (vii) an optionally halogenated lower (C₁₋₆)alkoxy group (e.g., methoxy, ethoxy, n-propyloxy, i-propyloxy, n-butyloxy, trifluoromethoxy, trichloromethoxy and the like), (viii) an optionally halogenated lower (C₁₋₆)alkylthio group (e.g., methylthio, ethylthio, propylthio, trifluoromethylthio and the like), (ix) amino group, (x) a mono-lower alkylamino group (e.g., a mono-C₁₋₆ alkylamino group and the like such as methylamino, ethylamino, propylamino and the like), (xi) a di-lower alkylamino group (e.g., a di-C₁₋₆ alkylamino group and the like such as dimethylamino, diethylamino and the like), (xii) a 5 to 7 membered cyclic amino group, for example, optionally having 1 to 3 hetero atoms selected from nitrogen atom, oxygen atom and sulfur atom in addition to carbon atoms and one nitrogen atom (e.g., pyrrolidino, piperidino, piperazino, morpholino, thiomorpholino and the like), (xiii) a lower alkyl-carbonylamino group (e.g., a C₁₋₆ alkyl-carbonylamino group and the like such as acetylamino, propionylamino, butyrylamino and the like), (xiv) a lower alkylsulfonylamino group (e.g., a C₁₋₆ alkyl-carbonylamino group and the like such as methylsulfonylamino, ethylsulfonylamino and the like), (xv) a lower alkoxy-carbonyl group (e.g., a C₁₋₆ alkoxy-carbonyl group and the like such as methoxycarbonyl, ethoxycarbonyl, propoxycarbonyl and the like), (xvi) carboxyl group, (xvii) a lower alkyl-carbonyl group (e.g., a C₁₋₆ alkyl-carbonyl group and the like such as methylcarbonyl, ethylcarbonyl, propylcarbonyl and the like), (xviii) carbamoyl group, (xix) a mono-lower alkyl-carbamoyl group (e.g., a mono-C₁₋₆ alkyl-carbamoyl group and the like such as methylcarbamoyl, ethylcarbamoyl and the like), (xx) a di-lower alkyl-carbamoyl group (e.g., a di-C₁₋₆ alkyl-carbamoyl group and the like such as dimethylcarbamoyl, diethylcarbamoyl and the like), (xxi) a lower alkyl sulfonyl group (e.g., a C₁₋₆ alkylsulfonyl group and the like such as methylsulfonyl, ethylsulfonyl, propylsulfonyl and the like), (xxii) a lower alkoxy-carbonyl-lower alkyl group (e.g., a C₁₋₆ alkyl-carbonyl-C₁₋₆ alkyl group and the like such as methoxycarbonylmethyl, ethoxycarbonylmethyl, tert-butoxycarbonylmethyl, methoxycarbonylethyl, methoxycarbonylmethyl, methoxycarbonyl(dimethyl)methyl, ethoxycarbonyl(dimethyl)methyl, tert-butoxycarbonyl(dimethyl)methyl and the like), (xxiii) a carboxyl-lower alkyl group (e.g., a carboxyl-C₁₋₆ alkyl group and the like such as carboxylmethyl, carboxylethyl, carboxyl(dimethyl)methyl and the like), (xxiv) a heterocyclic group optionally having a substituent, (xxv) a C₆₋₁₄ aryl group (e.g., phenyl, naphthyl and the like), (xxvi) a C₇₋₁₆ aralkyl group (e.g., benzyl and the like), (xxvii) ureido group optionally having a substituent (e.g., ureido, 3-methylureido, 3-ethylureido, 3-phenylureido, 3-(4-fluorophenyl)ureido, 3-(2-methylphenyl)ureido, 3-(4-methoxyphenyl)ureido, 3-(2,4-difluorophenyl)ureido, 3-[3,5-bis(trifluoromethyl)phenyl]ureido, 3-benzylureido, 3-(1-naphthyl)ureido, 3-(2-biphenylyl)ureido group and the like), (xxviii) thioureido group optionally having a substituent (e.g., thioureido, 3-methylthioureido, 3-ethylthioureido, 3-phenylthioureido, 3-(4-fluorophenyl)thioureido, 3-(4-methylphenyl)thioureido, 3-(4-methoxyphenyl)thioureido, 3-(2,4-dichlorophenyl)thioureido, 3-benzylthioureido, 3-(1-naphthyl)thioureido group and the like), (xxix) amidino group optionally having a substituent (e.g., amidino, N¹-methylamidiono, N¹-ethylamidino, N¹-phenylamidino, N¹,N¹-dimethylamidino, N¹,N²-dimethylamidino, N¹-methyl-N¹-ethylamidino, N¹,N¹-diethylamidino, N¹-methyl-N¹-phenylamidino, N¹,N¹-di(4-nitrophenyl)amidino group and the like), (xxx) guanidino group optionally having a substituent (e.g., guanidino, 3-methylguanidino, 3,3-dimethylguanidino, 3,3-diethylguanidino group and the like), (xxxi) a cyclic aminocarbonyl group optionally having a substituent (e.g., pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino)carbonyl, [4-(4-fluorobenzoyl)piperidino]carbonyl, (4-methylpiperazino)carbonyl, (4-phenylpiperazino)carbonyl, [4-(4-nitrophenyl)piperazino]carbonyl, (4-benzylpiperazino)carbonyl, morpholinocarbonyl, thiomorpholinocarbonyl group and the like), (xxxii) aminothiocarbonyl group optionally having a substituent (e.g., aminothiocarbonyl, methylaminothiocarbonyl, dimethylaminothiocarbonyl group and the like), (xxxiii) aminosulfonyl optionally having a substituent (e.g., aminosulfonyl, methylaminosulfonyl, dimethylaminosulfonyl group and the like), (xxxiv) phenylsulfonylamino optionally having a substituent (e.g., phenylsulfonylamino, (4-methylphenyl)sulfonylamino, (4-chlorophenyl)sulfonylamino, (2,5-dichlorophenyl)sulfonylamino, (4-methoxyphenyl)sulfonylamino, (4-acetylaminophenyl)sulfonylamino, (4-nitrophenyl)phenylsulfonylamino group and the like), (xxxv) sulfo group, (xxxvi) sulfino group, (xxxvii) sulfeno group, (xxxviii) a C₁₋₆ alkylsulfo group (e.g., methylsulfo, ethylsulfo, propylsulfo group and the like, (xxxix) a C₁₋₆ alkylsulfino group (e.g., methylsulfino, ethylsulfino, propylsulfino group and the like), (xxxx) a C₁₋₆ alkylsulfeno group (e.g., methylsulfeno, ethylsulfeno, propylsulfeno group and the like, (xxxxi) phosphono group, (xxxxii) a di-C₁₋₆ alkoxyphosphoryl group (e.g., dimethoxyphosphoryl, diethoxyphosphoryl, dipropoxyphosphoryl group and the like), (xxxxiii) a lower alkoxy-carbonyl-lower alkoxy group (e.g., a C₁₋₆ alkoxy-carbonyl-C₁₋₆ alkoxy group and the like such as methoxycarbonylmethoxy, ethoxycarbonylmethoxy, tert-butoxycarbonylmethoxy, methoxycarbonylethoxy, methoxycarbonyl (dimethyl) methoxy, ethoxycarbonyl(dimethyl)methoxy, tert-butoxycarbonyl(dimethyl)methoxy and the like), (xxxxiv) a carboxyl-lower alkoxy group (e.g., a carboxyl-C₁₋₆ alkoxy group and the like such as carboxylmethoxy, carboxylethoxy, carboxyl(dimethyl)methoxy and the like), (xxxxv) a lower alkyl-thiocarbonyl group (e.g., a C₁₋₆ alkyl-thiocarbonyl group and the like such as methylthiocarbonyl, ethylthiocarbonyl, butylthiocarbonyl and the like, (xxxxvi) thiocarbamoyl group, (xxxxvii) a mono-lower alkyl-thiocarbamoyl group (e.g., a mono-C₁₋₆ alkyl-thiocarbamoyl group and the like such as methylthiocarbamoyl, ethylthiocarbamoyl, propylthiocarbamoyl, butylthiocarbamoyl and the like), (xxxxviii) a di-lower alkyl-thiocarbamoyl group (e.g., a di-C₁₋₆ alkyl-thiocarbamoyl group and the like such as diethylthiocarbamoyl, dibutylthiocarbamoyl and the like) and the like.

As the "heterocyclic group" in the above "heterocyclic group optionally having a substituent", for example, a group obtained by removing one hydrogen atom from a 5 to 14 membered ring (monocyclic or di- to tetra-cyclic) hetero ring containing 1 to 6 (preferably 1 to 4) hetero atoms selected from nitrogen atom, oxygen atom and sulfur atom and the like are used.

As the monocyclic hetero ring, a group obtained by removing one hydrogen atom from a monocyclic hetero ring such as pyridine, pyrazine, pyrimidine, imidazole, furan, thiophene, dihydropyridine, diazepine, oxazepine, pyrrolidine, piperidine, hexamethyleneimine, heptamethyleneimine, tetrahydrofuran, piperazine, homopiperazine, tetrahydrooxazepine, morpholine, thiomorpholine, pyrrole, pyrazole, 1,2,3-triazole, oxazole, oxazolidine, oxadiazole, thiazole, thiazolidine, thiadiazole, oxathiadiazole, isoxazole, imidazoline, triazine, tetrazole and the like, and the like are used.

As the bicyclic heterocyclic group, for example, a group obtained by removing one hydrogen atom from a bicyclic hetero ring such as indole, dihydroindole, isoindole, dihydroisoindole, benzofuran, dihydrobenzofuran, benzimidazole, benzoxazole, benzisoxazole, benzothiazole, indazole, quinoline, tetrahydroquinoline, isoquinoline, tetrahydroisoquinoline, terahydro-1H-1-benzazepine, terahydro-1H-2-benzazepine, tetrahydro-1H-3-benzazepine, tetrahydrobenzoxazepine, quinazoline, tetrahydroquinazoline, quinoxaline, tetrahydroquinoxaline, benzodioxane, benzodioxole, benzothiazine, imidazopyridine and the like, and the like are used.

As the polycyclic heterocyclic group such as tricyclic or tetracyclic and the like, a group obtained by removing one hydrogen atom from a polycyclic hetero ring such as acridine, tetrahydroacridine, pyrroloquinoline, pyrroloindole, cyclopentoindole, isoindrobenzazepine and the like, and the like are used.

As the "heterocyclic group", a group obtained by removing one hydrogen atom from a monocyclic hetero ring or dicyclic hetero ring, and the like are preferable.

As the "substituent" in the "heterocyclic group optionally having a substituent", for example, used are one to five selected from (i) halogen (e.g., fluorine, chlorine, bromine, iodine and the like), (ii) nitro, (iii) cyano, (iv) oxo, (v) hydroxy, (vi) a lower alkyl (e.g., a C₁₋₆ alkyl and the like such as methyl, ethyl, propyl, isopropyl, butyl, isobutyl, tert-butyl, sec-butyl and the like), (vii) a lower alkoxy (e.g., a C₁₋₆ alkoxy and the like such as methoxy, ethoxy, n-propyloxy, i-propyloxy, n-butyloxy and the like), (viii) a lower alkylthio (e.g., a C₁₋₆ alkylthio and the like such as methylthio, ethylthio, propylthio and the like), (ix) amino, (x) a mono-lower alkylamino (e.g., a mono-C₁₋₆ alkylamino and the like such as methylamino, ethylamimo, propylamino and the like), (xi) a di-lower alkylamino (e.g., a di-C₁₋₆ alkylamino and the like such as dimethylamino, diethylamino and the like), (xii) a 5 to 7 membered cyclic amino, for example, optionally having 1 to 3 hetero atoms selected from nitrogen atom, oxygen atom and sulfur atom in addition to carbon atoms and one nitrogen atom (e.g., pyrrolidino, piperidino, piperazino, morpholino, thiomorpholino and the like), (xiii) a lower alkyl-carbonylamino (e.g., a C₁₋₆ alkyl-carbonylamino and the like such as acetylamino, propionylamino, butyrylamino and the like), (xiv) a lower alkylsulfonylamino (e.g., a C₁₋₆ alkyl-carbonylamino and the like such as methylsulfonylamino, ethylsulfonylamino and the like), (xv) a lower alkoxy-carbonyl (e.g., a C₁₋₆ alkoxy-carbonyl and the like such as methoxycarbonyl, ethoxycarbonyl, propoxycarbonyl and the like), (xvi) carboxyl, (xvii) a lower alkyl-carbonyl (e.g., a C₁₋₆ alkyl-carbonyl and the like such as methylcarbonyl, ethylcarbonyl, propylcarbonyl and the like), (xviii) carbamoyl, (xix) a mono-lower alkylcarbamoyl (e.g., a mono-C₁₋₆ alkylcarbamoyl and the like such as methylcarbamoyl, ethylcarbamoyl and the like), (xx) a di-lower alkylcarbamoyl (e.g., a di-C₁₋₆ alkylcarbamoyl and the like such as dimethylcarbamoyl, diethylcarbamoyl and the like), (xxi) a lower alkylsulfonyl (e.g., a C₁₋₆ alkylsulfonyl and the like such as methylsulfonyl, ethylsulfonyl, propylsulfonyl and the like), (xxii) a lower alkyl-thiocarbonyl group (e.g., a C₁₋₆ alkyl-thiocarbonyl group and the like such as methylthiocarbonyl, ethylthiocarbonyl, butylthiocarbonyl and the like), (xxiii) thiocarbamoyl group, (xxiv) a mono-lower alkyl-thiocarbamoyl (e.g., a mono-C₁₋₆ alkyl-thiocarbamoyl and the like such as methylthiocarbamoyl, ethylthiocarbamoyl, propylthiocarbamoyl, butylthiocarbamoyl and the like), (xxv) a di-lower alkyl-thiocarbamoyl (e.g., a di-C₁₋₆ alkyl-thiocarbamoyl and the like such as diethylthiocarbamoyl, dibutylthiocarbamoyl and the like).

As the "substituent" in the "hydrocarbon group optionally having a substituent" for R¹ and R², preferably used are a halogen atom, an optionally halogenated C₁₋₆ alkyl group, an optionally halogenated C₁₋₆ alkoxy group, hydroxy group, nitro group, cyano group, a C₁₋₆ alkoxycarbonyl, amino group, a 5 to 7 membered cyclic amino group, phenylsulfonylamino group. Especially preferred are a halogen atom (e.g., chlorine and the like) and the like.

As the "acyl group" for R¹ and R², used are acyl groups represented by the formula: -(C=O)-R³, -(C=S)-R³, -SO₂-R³, -SO-R³, -(C=O)O-R³, -(C=S)O-R³, -(C=O)NR³R⁴ or -(C=S)NR³R⁴ [wherein R³ and R⁴ represent independently (i) hydrogen atom, (ii) a hydrocarbon group optionally having a substituent or (iii) a heterocyclic group optionally having a substituent, or R³ and R⁴ may be taken together with the adjacent nitrogen atom to form a nitrogen-containing ring group optionally having a substituent].

Among these, -(C=O)-R³, -SO₂-R³, -SO-R³, -(C=O)NR³R⁴ or -(C=O)O-R³ (R³ and R⁴ represent the same meanings as above) is preferable and, -(C=O)-R³ (R³ represents the same meaning as above) is especially preferable.

As the "hydrocarbon group optionally having a substituent" and "heterocyclic group optionally having a substituent" for R³ and R⁴, the same as the above "hydrocarbon group optionally having a substituent" and "heterocyclic group optionally having a substituent" are used respectively.

As the "nitrogen-containing ring group optionally having a substituent" formed by R³ and R⁴, a 5 to 9 membered (preferably 5 to 7 membered) nitrogen-containing saturated heterocyclic group, for example, optionally containing 1 to 3 hetero atoms such as nitrogen atom, oxygen atom and sulfur atom and the like in addition to carbon atoms and one nitrogen atom, and the like are used. More particularly, for example, groups represented by the formulae; 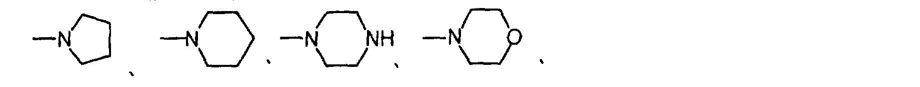 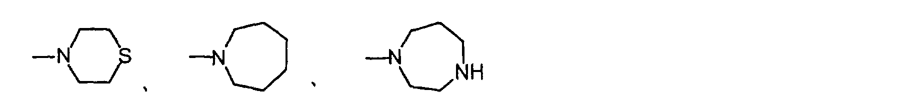 are used.

As the "substituent" in the "nitrogen-containing ring group optionally having a substituent", the same as the "substituent" in the above "heterocyclic group optionally having a substituent" is used.

As the "acyl group" for the above R¹ and R², preferably used are formyl group, an optionally halogenated C₁₋₆ alkyl-carbonyl group (e.g., acetyl, trifluoroacetyl, propionyl and the like), a 5 to 6 membered heterocyclic carbonyl group (e.g., pyridylcarbonyl, thienylcarbonyl, furylcarbonyl and the like), a C₆₋₁₄ aryl-carbonyl group (e.g., benzoyl, 1-naphthoyl, 2-naphthoyl and the like), a C₇₋₁₆ aralkyl-carbonyl group (e.g., phenylacetyl, 3-phenylpropionyl and the like), a C₆₋₁₀ aryl-sulfonyl group (e.g., benzenesulfonyl, toluenesulfonyl, naphthylsulfonyl and the like) and the like. Among these, an optionally halogenated C₁₋₆ alkyl-carbonyl group (e.g., acetyl, trifluoroacetyl and the like) and the like are preferable.

As R¹ and R², a hydrogen atom or a hydrocarbon group optionally having a substituent is preferably used and, inter alia, a C₇₋₁₆ aralkyl group optionally having a substituent is preferably used, and a C₇₋₁₆ aralkyl group (preferably benzyl group or phenylethyl group) optionally having one to five substituents selected from halogen, an optionally halogenated C₁₋₆ alkyl, an optionally halogenated C₁₋₆ alkoxy, cyano, nitro and hydroxyl and the like are more preferably used.

k and m represent independently an integer of 0 to 5, and 1<k+m<5. Preferably, k+m=4 and, more preferably, (1) k=m=2, or (2) k=3 and m=1.

n is an integer of 0 to 6, preferably, 2 to 4, more preferably, 3.

R is a hydrogen atom or a hydrocarbon group optionally having a substituent, and may be different in repetition of n.

As the "hydrocarbon group optionally having a substituent" for R, the same as the "hydrocarbon group optionally having a substituent" for R¹ and R² is used.

As R, a hydrogen atom is preferable.

X represents O or S, and O is preferable.

As a suitable example of Compound (IA), for example; the following compounds can be mentioned.
1) Compounds wherein R is hydrogen atom, n is an integer of 2 to 4, and R¹ and R² are benzyl groups optionally having a substituent.
2) Compounds wherein ring A is unsubstituted benzene ring, R is hydrogen atom, n is 3, R¹ and R² are benzyl groups optionally substituted by a halogen atom (preferably chlorine and the like), and k=m=2.
3) Compounds wherein ring A is unsubstituted benzene ring, R is hydrogen atom, n is 3, R¹ and R² are hydrogen atom or benzyl group optionally substituted by a halogen atom (preferably chlorine and the like), and k=3 and m=1.
4) Compounds wherein ring A is unsubstituted benzene ring, R is hydrogen atom, n is 3, R¹ and R² are hydrogen atom or benzyl group optionally substituted by a halogen atom (preferably chlorine and the like), and k=4 and m=0.

As Compound (IA) or a salt thereof, especially preferred are 3-[3-[1-(phenylmethyl)-4-piperidinyl]propyl]-7-(phenylmethyl)-6,7,8,9-tetrahydro-5H-isoxazolo[4,5-h][3]benzazepine; 3-[3-[1-[(2-chlorophenyl)methyl]-4-piperidinyl]propyl]-6-(phenylmethyl)-6,7,8,9-tetrahyro-5H-isoxazolo[5,4-h][2]benzazepine; or 3-[3-[1-(phenylmethyl)-4-piperidinyl]propyi]-6,7,8,9-tetrahydro-5H-isoxazolo[5,4-h][1]benzazepine or salts thereof are preferable.

As the salt of Compound (IA), physiologically acceptable salts are preferable and, physiologically acceptable acid addition salts are especially preferable. As such salts, used are, for example, salts with an inorganic acid (e.g., hydrochloric acid, phosphoric acid, hydrobromic acid, sulfuric acid), or salts with an organic acid (e.g., acetic acid, formic acid, propionic acid, fumaric acid, maleic acid, succinic acid, tartaric acid, citric acid, malic acid, oxalic acid, benzoic acid, methanesulfonic acid, bensenesulfonic acid).

Further, when Compound (IA) has an acidic group such as -COOH and the like, Compound (IA) may form a salt with an inorganic base (e.g., sodium, potassium, calcium, magnesium, ammonia) or an organic base (e.g., triethylamine), and these salts are included in the object compound of the present invention. Further, the above Compound (IA) or a salt thereof may be a hydrate or a non-hydrate.

A prodrug of Compound (IA) refers to a compound which is converted into Compound (IA) by action of an enzyme or gastric juice and the like under physiological conditions in vivo, namely a compound which is converted into compound (IA) upon enzymatic oxidation, reduction, hydrolysis and the like, or a compound which is converted into compound (IA) upon hydrolysis by gastric juice and the like. Examples of the prodrug of compound (IA) include compounds derived by acylation, alkylation or phosphorylation of the amino group of compound (IA) (e.g. compounds derived by eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation or tert-butylation of the amino group of compound (IA)), compounds derived by acylation, alkylation, phosphorylation or boration of the hydroxy group of compound (IA) (e.g. compounds derived by acetylation, palmitoylation, propanoylation, pivaloylation, succinylation, fumarylation, alanylation or dimethylaminomethylcarbonylation of the hydroxy group of compound (IA)), and compounds derived by esterification or amidation of the carboxyl group of compound (I) (e.g. compounds derived by ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification, or methylamidation of the carboxyl group of compound (IA)), and the like. These compounds can be produced from compound (IA) by per se known methods.

A prodrug of compound (IA) may be one that is converted into compound (IA) under physiological conditions, as described in "Iyakuhin no Kaihatsu (Development of Drugs)", vol. 7, Molecular Designing, published by Hirokawa Shoten, 1990, pages 163-198.

Compound (IA) may be labeled with an isotope (e.g., ³H, ¹⁴C, ³⁵S and the like).

Hereinafter, a process for producing Compound (IA) or a salt thereof will be described.

Although the following process for production is applied not only to Compound (IA) itself but also to the aforementioned salt thereof, they are simply abbreviated as Compound (IA) in the following explanation.

In addition, although as compounds represented by the formulae (II), (IIIa), (IVa), (Va), (Vb), (VIa), (Vc), (Vd), (VIIa), (Ve), (IIa), (Vf), (Vg), (Vh), (Ia), (IIb), (Id), (Ie), (If), (IIc), (VIIIa), (VIIIb), (IX), (IVb), (Ib), (Ig), (Ih), (IId) and (Ic) used in respective steps, not only those compounds themselves but also salts thereof may be used, they are simply abbreviated as compound in the following explanation in some cases. As salts of compounds used in these respective steps, salts listed for the above "salts of Compound (IA)" may be used. Further, the above compounds may be a hydrate or a non-hydrate.

When Compound (IA) and compounds (a raw material compound or an intermediate for synthesis) in respective steps for producing Compound (IA) are free, they may be converted into a salt according to the conventional method and, when they form a salt, they may be converted into a free compound or other salt according to the conventional method.

The compounds represented by the formulae (Ia), (Ib), (Ic), (Id), (Ie), (If), (Ig) and (Ih) are included in Compound (IA).

In addition, Compound (IA) and each intermediate for synthesis may be an optical isomer, a steric isomer, positional isomer or a rotational isomer, or a mixture thereof, and these are also included in Compound (IA) and a raw material compound or an intermediate for synthesis. For example, Compound (IA) may be a racemate, or an optical isomer resolved from a racemate. In addition, these can be isolated and purified according to the separating method known per se.

An optical isomer can be produced according to the means known per se. Specifically, an optical isomer can be produced by using an optically active raw material compound or an intermediate for synthesis, or by optically resolving a racemate of a final compound according to the conventional method. As an optical resolving method, the method known per se, for example, a fractionating recrystallization method, an optically active column method, a diastereomer method and the like can be applied. A steric isomer, a positional isomer and a rotational isomer can be produced by applying the method known per se.

The following respective reactions may be performed without using a solvent or using a suitable solvent as necessary. As the solvent, any solvents that can be generally used for a chemical reaction may be used as long as they do not interfere with a reaction. For example, organic solvents such as hydrocarbon solvents (e.g., hexane, toluene and the like), ether solvents (e.g., ethyl ether, tetrahydrofuran, dioxane, dimethoxyethane), amide solvents (e.g., formamide, N,N-dimethylformamide, N,N-dimethylacetamide, hexamethylphosphoric triamide and the like), urea solvents, (e.g., 1,3-dimethyl-2-imidazolidinone and the like), sulfoxide solvents (e.g., dimethyl sulfoxide and the like), alcohol solvents (e.g., methanol, ethanol, isopropanol, t-butanol and the like), nitrile solvents (e.g., acetonitrile, propionitrile and the like), pyridine and the like, or water and the like are used. The amount of the solvent to be used is usually about 0.5 ml to about 100 ml, preferably about 3 ml to about 30 ml relative to 1 mmol of a compound. The reaction temperature differs depending upon the kind of solvent used, and is usually around about -30°C to about 180°C, preferably around about 0°C to 120°C. The reaction time differs depending upon the reaction temperature, and is usually about 0.5 hours to about 72 hours, preferably about 1 hour to about 24 hours. A reaction is usually performed under a normal pressure and may be performed under pressure conditions of around about 1 atm to about 100 atm as necessary.

Compounds obtained in the following respective steps are isolated and purified by the known means such as concentration, liquid nature conversion, conversional resolution, solvent extraction, fractional distillation, distillation, crystallization, recrystallization, chromatography, preparative high performance liquid chromatography or the like, and supplied as a raw material for the next reaction. These compounds as a reaction mixture may be used as a raw material without isolation or purification.

In the following explanation, a "ring-closing reaction" and a "condensation reaction" may be performed in the presence of a base as necessary. As the base, for example, inorganic bases such as sodium carbonate, sodium hydrogen carbonate, potassium carbonate, lithium carbonate, sodium hydroxide, potassium hydroxide, potassium hydride, sodium hydride, sodium methoxide, potassium t-butoxide and the like, and organic bases such as pyridine, rutidine, collidine, triethylamine and the like are used. The amount of the base to be used is usually an equivalent mole amount to an excessive amount, preferably about 1 mole equivalent to about 5-fold mole equivalent relative to a compound. Further, a reaction may be promoted in the presence of a catalytic amount of an iodide compound, for example, sodium iodide, potassium iodide, or 4-dimethylaminopyridine, as necessary.

In the following explanations, a "ring-closing reaction" and a "Friedel-Crafts reaction" can be performed in the presence of an acid as necessary. As the acid such as inorganic acids such as hydrochloric acid, phosphoric acid, polyphosphoric acid, hydrobromic acid, sulfuric acid and the like, or organic acids such as acetic acid, trifluoroacetic acid, formic acid, propionic acid, fumaric acid, maleic acid, succinic acid, tartaric acid, citric acid, malic acid, oxalic acid, benzoic acid, methanesufonic acid, benzenesulfonic acid and the like, or Lewis acids such as aluminum chloride, aluminum bromide, zinc chloride, titanium chloride, tin (IV) chloride, iron (II) chloride, iron (III) chloride, antimony (V) pentachloride, bismuth (III) chloride, mercury (II) chloride, boron trifluoride, hydrogen fluoride, scandium (III) triluoromethanesulfonate, ytterbium (III) trifluoromethanesulfonate, hafnium (IV) trifluoromethanesulfonate and the like are used.

In a reaction of the following respective steps, a reaction may be performed after a functional group is protected according to the conventional method as necessary, and deprotection may be optionally performed according to the conventional method after the reaction. A protecting group introducing reaction and a deprotection reaction are performed according to the method known per se or an analogous reaction thereof. Specifically, a method described in Protective groups in Organic Synthesis; John Wiley & Sons, Inc. and, for example, as a deprotection reaction, a method of treating with an acid, a base, reduction, ultraviolet-ray, hydrazine, phenylhydrazine, sodium N-methyldithiocarbamate, tetrabutylammonium fluoride, palladium acetate and the like are used.

1) Compound (IA) can be produced by ring-closing a compound represented by the formula: 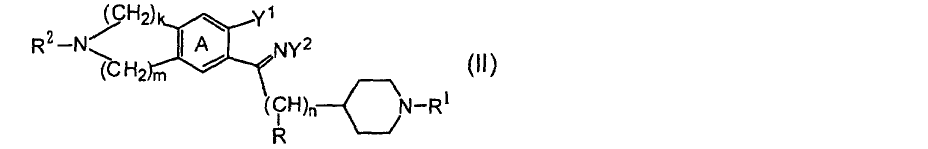 wherein Y¹ represents OZ^{a}, SZ^{a} (wherein Z^{a} represents hydrogen atom, a halogen atom, an alkyl group, an acyl group), nitro group or a halogen atom, Y² represents hydrogen atom or OZ^{b} (wherein Z^{b} represents hydrogen atom or an acyl group), and other symbols represent the same meanings as above] (hereinafter abbreviated as Compound (II) in some cases) or a salt thereof.

As the alkyl group for Z^{a}, for example, a C₁₋₆ alkyl group such as methyl, ethyl, t-butyl and the like, and the like are used. In addition, as the acyl group for Z^{a}, the same as the "acyl group" for the above R¹ and R² is used.

As the acyl group for Z^{b}, the same as the "acyl group" for the above R¹ and R² is used, and, for example, acetyl group, benzoyl group and the like are preferable.

As Y¹, OH, SH, SCH₃, SCl, SBr, nitro group and a halogen atom (e.g., fluorine, chlorine, bromine and the like) are preferable.

As Y², hydrogen atom, OH, OCOCH₃, OCOC₆H₅ and the like are preferable.

The present ring-closing reaction can be performed without using a solvent or using a suitable solvent. As the solvent, amide solvents (e.g., formamide, N,N-dimethylformamide, N,N-dimethylacetamide, hexamethylphosphoric triamide and the like), sulfoxide solvents (e.g., dimethyl sulfoxide and the like) and the like are preferably used. In particular, a reaction without a solvent, or a reaction using N,N-dimethylformamide or dimethyl sulfoxide is preferable.

The present ring-closing reaction can be performed in the presence of an acid or a base as necessary. As the acid, for example, hydrochloric acid, sulfuric acid, polyphosphoric acid and the like are used. In addition, an acid anhydride such as acetic anhydride, benzoic anhydride and the like may be used. As the base, for example, sodium carbonate, potassium carbonate, sodium hydroxide, potassium hydroxide, rutidine, collidine, triethylaminde and the like are used.

The reaction temperature differs depending upon the kind of solvent, acid or base, and is preferably about 0°C to about 200°C. The reaction time differs depending upon the reaction temperature, and is preferably about 1 hour to about 48 hours.

A raw material Compound (II) or a salt thereof can be produced by the following Reaction formula 1-1.

That is, Compound (II) can be produced by successively performing:
Step (aa): a Friedel-Crafts reaction of a compound represented by the formula (IIIa) [wherein W² represents a protecting group for amino group, and other symbols represent the same meanings as above] (hereinafter abbreviated as Compound (IIIa) in some cases) and a compound represented by the formula (IVa) [wherein Z³ represents a leaving group, W¹ represents a protecting group for amino group, and other symbols represent the same meanings as above] (hereinafter abbreviated as Compound (IVa) in some cases),
Step (ab): a deprotection reaction of a protecting group W¹ of a compound represented by the formula (Va) [wherein W¹ and W² represent a protecting group for amino group, and other symbols represent the same meanings as above] (hereinafter abbreviated as Compound (Va) in some cases),
Step (ac): a condensation reaction of a compound represented by the formula (Vb) [wherein respective symbols represent the same meanings as above] (hereinafter abbreviated as Compound (Vb) in some cases) and a compound represented by the formula:

   R¹―Z¹ (VIa)

   [wherein Z¹ represents a leaving group, and R¹ represents the same meaning as above],
Step (ad): a deprotection reaction of a protecting group W² of a compound represented by the formula (Vc) [wherein respective symbols represent the same meanings as above] (hereinafter abbreviated as Compound (Vc) in some cases),
Step (ae): a condensation reaction of a compound represented by the formula (Vd) [wherein respective symbols represent the same meanings as above] (hereinafter abbreviated as Compound (Vd) in some cases) and a compound represented by the formula:

   R²―Z¹ (VIIa)

   [wherein respective symbols represent the same meanings as above] (hereinafter abbreviated as Compound (VIIa) in some cases), and
Step (af): a carbonyl group conversion reaction of a compound represented by the formula (Ve) [wherein respective symbols represent the same meanings as above] (hereinafter abbreviated as Compound (Ve) in some cases).

### [Reaction formula 1-1]

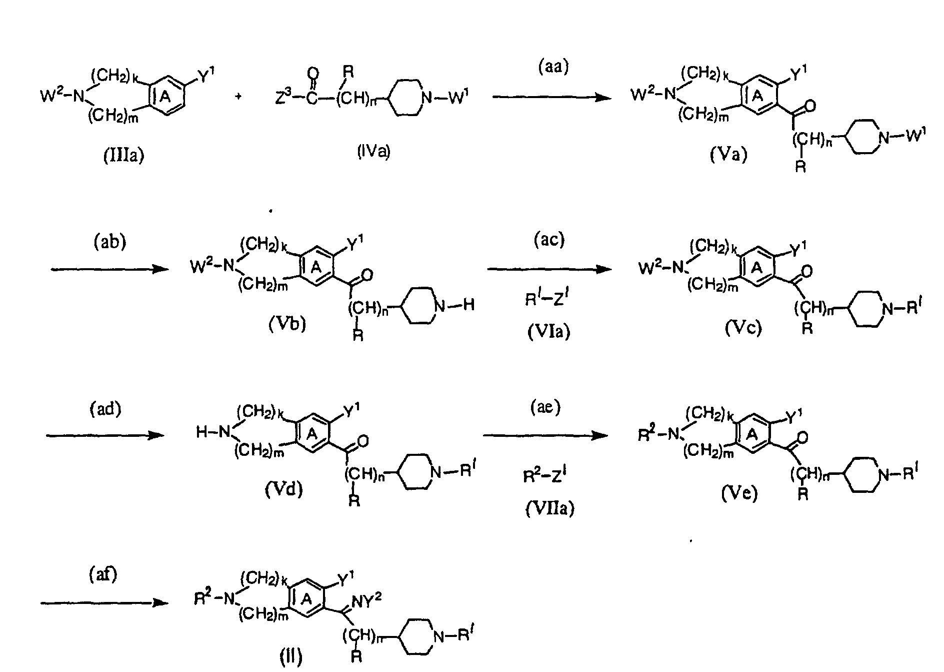

In Step (aa), Compound (Va) can be produced by a Friedel-Crafts reaction of Compound (IIIa) and Compound (IVa).

As the leaving group for Z³, for example, a halogen atom (e.g., chlorine, bromine, iodine and the like), a C₁₋₆ alkylsulfonyloxy group (e.g., methanesulfonyloxy, ethanesulfonyloxy and the like), a C₆₋₁₀ arylsulfonyloxy group (e.g., benzenesulfonyloxy, toluenesulfonyloxy and the like) and the like are used and, in particular, a halogen atom such as chlorine and the like are preferable.

W¹ and W² represent a general protecting group for amino group, for example, the same as the "hydrocarbon group optionally having a substituent" and "acyl group" for the above R¹ and R² can be used. Specifically, for example, used are acyl groups such as formyl group, a C₁₋₆ alkyl-carbonyl group optionally having a substituent (e.g., acetyl, ethylcarbonyl and the like), benzoyl group, a C₁₋₆ alkyl-oxycarbonyl group (e.g., methoxycarbonyl, ethoxycarbonyl, t-butoxycarbonyl and the like), a C₆₋₁₄ aryloxycarbonyl group (e.g., phenoxycarbonyl and the like), a C₇₋₁₅ aralkyloxy-carbonyl group (e.g., benzyloxycarbonyl, fluorenyloxycarbonyl and the like) and the like, a hydrocarbon group such as trityl, phthaloyl and the like, and the like. As these substituents, a halogen (e.g., fluorine, chlorine, bromine, iodine and the like), a C₁₋₆ alkyl-carbonyl (e.g., methylcarbonyl, ethylcarbonyl, butylcarbonyl and the like), nitro group and the like are used and, the number of the substituents is preferably around 1 to 3.

The reaction of Compound (IIIa) and Compound (IVa) can be performed by a general Friedel-Crafts reaction, methods described in Organic reaction, vol.3, pl-82, JP-A 5-140149, JP-A 6-206875, J. Med. Chem., 37, 2292 (1994) and the like or analogous methods thereof, and the like. Specifically, it is preferable that the reaction is performed using a solvent such as dichloroethane, 1,2-dichloroethane, carbon disulfide and the like in the presence of a Lewis acid such as aluminum chloride and the like. The amount of the Lewis acid used is usually about 1 equivalent to about 10 equivalents, preferably about 2 equivalents to about 5 equivalents relative to Compound (IVa). The amount of the solvent used is preferably about 5 ml to about 20 ml relative to 1 mmol of Compound (IVa). The reaction temperature is usually around about -30°C to about 150°C, preferably around about 0°C to about 100°C, and the reaction time is usually about 0.5 hours to about 72 hours, preferably about 1 hour to about 16 hours.

Compound (IIIa) can be produced by the method known per se or its analogous method. For example, the compound can be produced by methods described in Chem. Pharm. Bull., 30, 180 (1982), J. Org. Chem., 34, 235 (1969), J. Org. Chem., 54, 5574 (1989), Tetrahedron Lett., 35, 3023 (1977), Bull. Chem. Soc. Jpn., 56, 2300 (1983), J. Heterocyclic Chem., 8, 779 (1971) and the like or analogous methods thereof.

Compound (IVa) can be produced by the method known per se or its analogous method. For example, the compound can be produced by a method described in JP-A 5-140149, Chem. Pharm. Bull., 34, 3747 (1986), Chem. Pharm. Bull., 41, 529 (1993), EP-A-0,378,207 and the like or analogous methods thereof.

In Step (ab), Compound (Vb) can be produced by subjecting Compound (Va) to a deprotection reaction to remove W¹.

A deprotection reaction of a protecting group W¹ can be performed selectively using the method known per se or its analogous method without deprotecting another protecting group W² possessed by Compound (Va). As a preferable combination of W¹ and W² in this case, for example, t-butoxycarbonyl group and acetyl group, trifluoroacetyl group and acetyl group, p-nitrobenzyloxycarbonyl group and acetyl group, benzyloxycarbonyl group and t-butoxycarbonyl group, trifluoroacetyl group and benzyloxycarbonyl group and the like can be mentioned. As the present method, specifically, methods described in Protective groups in Organic Synthesis; John Wiley & Sons, Inc., for example, used are a method of treating with an acid, a base, reduction, ultraviolet-ray, hydrazine, phenylhydrazine, sodium N-methyldithiocarbamate, tetrabutylammonium fluoride, palladium acetate and the like.

For example, Compound (Va) is deprotected by holding preferably at about 20°C to about 140°C in an aqueous solution of an acid such as a mineral acid (e.g., hydrochloric acid, sulfuric acid, hydrobromic acid, trifluoroacetic acid, iodic acid, periodic acid and the like) and the like, or a base such as an alkali metal hydroxide (e.g., sodium hydroxide, potassium hydroxide, lithium hydroxide) and the like. When trifluoroacetic acid is used, a scavenger of t-butylcation such as thioanisole may be added. The amount of the acid or base to be used is usually about 1 equivalent to about 100 equivalents, preferably about 1 equivalent to about 40 equivalents relative to Compound (Va). The strength of the acid or base is usually about 0.1N to about 18N, preferably about 1N to about 12N. The reaction time depends upon the reaction temperature, and is usually around about 1 hour to about 48 hours, preferably around about 2 hours to about 24 hours.

In addition, W¹ may be deprotected by subjecting Compound (Va) to a catalytic reduction reaction under a normal pressure or under pressure as necessary using palladium , palladium-carbon, Raney-nickel, Raney-cobalt, platinum oxide and the like as a catalyst, and using an alcohol solvent such as ethanol and the like, or acetic acid and the like as a solvent.

In addition, when W¹ is t-butoxycarbonyl group, for example, deprotection can be performed using a trialkylsilyltrifluoromethanesulfonate derivative such as trimethylsilyl-trifluoromethanesulfonate, triethylsilyl-trifluoromethanesulfonate or t-butyldimethylsilyl-trifluoromethanesulfonate and the like in the presence of an aromatic or tertiary amine such as 2,6-rutidine or triethylamine. As a solvent, for example, a non-polar solvent such as dichloromethane and the like, and a polar aprotic solvent such as tetrahydrofuran, diethyl ether, N,N-dimethylformamide and the like are preferable. The reaction temperature is preferably about -20°C to room temperature. In particular, the conditions using trimethylsilyl-trifluoromethanesulfonate and 2,6-rutidine at about 0°C to approximately room temperature in dichloromethane are preferable.

In Step (ac), Compound (Vc) can be produced by subjecting Compound (Vb) and Compound (VIa) to a condensation reaction.

As the leaving group for Z¹, for example, used are a halogen atom (e.g., chlorine, bromine, iodine and the like), a C₁₋₆ alkylsulfonyloxy group (e.g., methanesulfonyloxy, ethanesulfonyloxy, trifluoromethanesulfonyloxy and the like), a C₆₋₁₀ arylsulfonyloxy group (e.g., benzenesulfonyloxy, p-toluenesulfonyloxy and the like) and the like. In particular, for example, a halogen atom (preferably, bromine, iodine and the like) and the like are preferable.

The condensation reaction of Compound (Vb) and Compound (Via) can be performed in the same manner as a general condensation reaction and, specifically, for example, the reaction can be performed using an alcohol solvent such as ethanol and the like, or a nitrile solvent such as acetonitrile and the like. The reaction temperature differs depending upon the kind of solvent, and is preferably around about 0°C to about 120°C. The reaction time differs depending upon the reaction temperature, and is preferably about 1 hour to about 24 hours. As the base, for example, sodium carbonate, potassium carbonate, triethylamine and the like are preferably used. The amount of the base used is preferably about 1 equivalent to about 3 equivalents relative to Compound (VIa). The present reaction may be promoted in the presence of a catalytic amount of an iodide compound such as sodium iodide and potassium iodide, or 4-dimethylaminopyridine or the like.

Compound (VIa) or a salt thereof can be produced by the method known per se or analogous method thereof.

In Step (ad), Compound (Vd) can be produced by subjecting Compound (Vc) to a deprotection reaction to remove W².

The deprotection reaction of a protecting group W² can be performed in the same manner as the "deprotection reaction of a protecting group W¹" described in the above Step (ab).

In Step (ae), Compound (Ve) can be produced by subjecting Compounds (Vd) and (VIIa) to a condensation reaction.

The condensation reaction of Compound (Vd) and Compound (VIIa) can be performed in the same manner as the above "condensation reaction of Compound (Vb) and Compound (VIa)".

In Step (af), Compound (II) can be produced by reacting Compound (Ve) with a suitable reagent to convert carbonyl group.

As the reagent used for a carbonyl group conversion reaction, for example, ammonia, hydroxylamine and the like can be mentioned.

The present reaction can be performed by methods described in Advanced Organic Chemistry, 5th ed., Wiley-Interseience; New York, 1992, pp. 896-907, Organic Functional Group Preparations, vol. III, Academic (1983), Rodd's Chemistry of Carbon Compounds, vol. I, partC, Elsevier Publishing co. (1965) and the like or analogous methods thereof.

In addition, a compound represented by the formula: 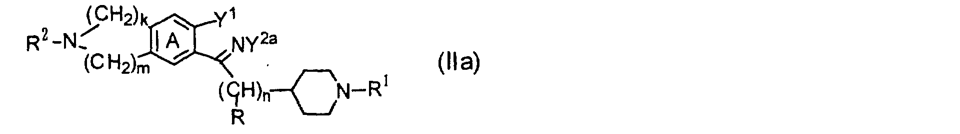 [wherein Y^{2a} represents OZ^{ba} (wherein Z^{ba} represents an acyl group), and other symbols represent the same meanings as above]
included in Compounds (II) can be produced by reacting a compound obtained by the reaction of Compound (Ve) and hydroxylamine, with an acyl halide such as acetyl chloride, benzoyl chloride and the like, or an anhydride of an organic acid such as acetic anhydride, benzoic anhydride and the like, as necessary. The present reaction can be performed in the same manner as a general condensation reaction. Specifically, the reaction can be performed, for example, in the same manner as the above "condensation reaction of Compound (Vb) and Compound (VIa)".

Alternatively, the above Compound (Ve) can be produced by the following Reaction formula 1-2. That is, Compound (Ve) can be produced by successively performing:
Step (ag): a deprotection reaction of a protecting group W² in Compound (Va),
Step (ah): a condensation reaction of a compound represented by the formula (Vf) [wherein respective symbols represent the same meanings as above] (hereinafter abbreviated as Compound (Vf) in some cases) and Compound (VIIa),
Step (ai): a deprotection reaction of a protecting group W¹ of a compound represented by the formula (Vg) [wherein respective symbols represent the same meanings as above] (hereinafter abbreviated as Compound (Vg) in some cases),
Step (aj): a condensation reaction of Compound represented by the formula (Vh) [wherein respective symbols represent the same meanings as above] (hereinafter abbreviated as Compound (Vh) in some cases) and Compound (VIa).
Step (ag) can be performed in the same manner as the above Step (ad), Step (ah) in the same manner as the above Step (ae), Step (ai) in the same manner as the above Step (ab), and Step (aj) in the same manner as the above Step (ac), respectively.

### [Reaction formula 1-2]

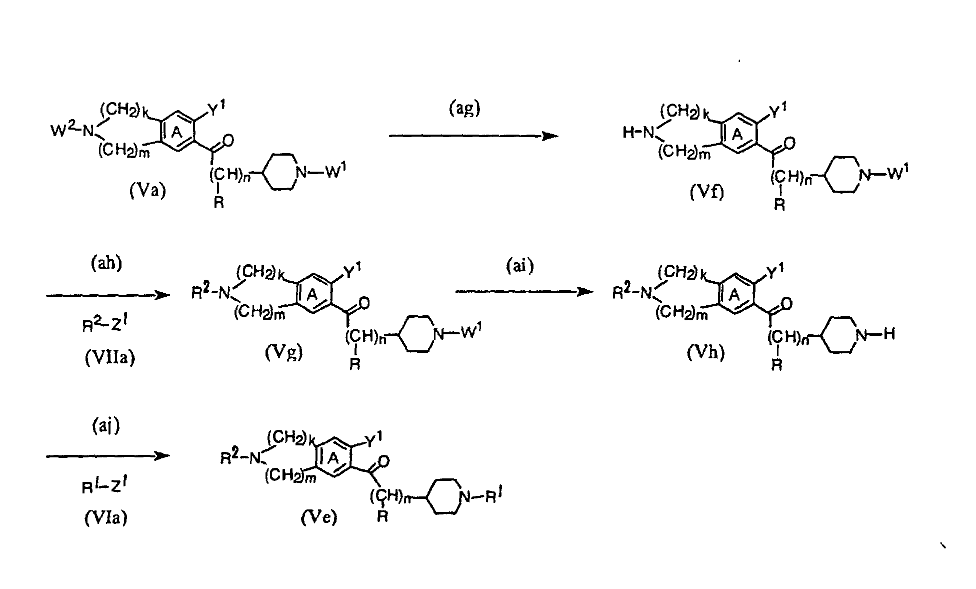

2) Compound (IA) can be produced by subjecting a compound represented by the formula: 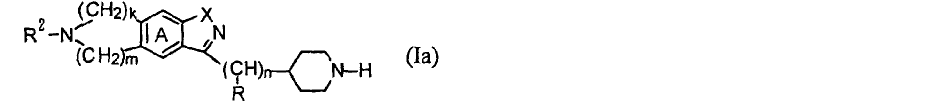 [wherein respective symbols represent the same meanings as above] (hereinafter abbreviated as Compound (Ia) in some cases) or a salt thereof and Compound (Via) to a condensation reaction.

The condensation reaction of Compound (Ia) and Compound (VIa) can be performed, for example, in the same manner as the "condensation reaction of Compound (Vb) and Compound (VIa)" described in the above 1).

A raw material Compound (Ia) or a salt thereof can be produced by the following Reaction formula 2-1.

That is, Compound (Ia) can be produced by:
Step (ba): producing a compound represented by the formula (IIb) [wherein respective symbols represent the same meanings as above] (hereinafter abbreviated as Compound (IIb) in some cases) by a carbonyl group conversion reaction of Compound (Va), then
Step (bb): producing a compound represented by the formula (Id) [wherein respective symbols represent the same meanings as above] (hereinafter abbreviated as Compound (Id) in some cases) by a ring-closing reaction of Compound (IIb),
Step (bc): producing a compound represented by the formula (Ie) [wherein respective symbols represent the same meanings as above] (hereinafter abbreviated as Compound (Ie) in some cases) by a deprotection reaction of a protecting group W² of Compound (Id), further
Step (bd): producing a compound represented by the formula (If) [wherein respective symbols represent the same meanings as above] (hereinafter abbreviated as Compound (If) in some cases) by a condensation reaction of Compound (Ie) and Compound (VIIa) and, thereafter,
Step (be): performing a deprotection reaction of a protecting group W¹ of Compound (If).
Step (ba) can be performed in the same manner as the above Step (af), Step (bb) in the same manner as the ring-closing reaction of Compound (II), Step (bc) in the same manner as the above Step (ad), Step (bd) in the same manner as the above Step (ae), and Step (be) in the same manner as the above Step (ab), respectively.

### [Reaction formula 2-1]

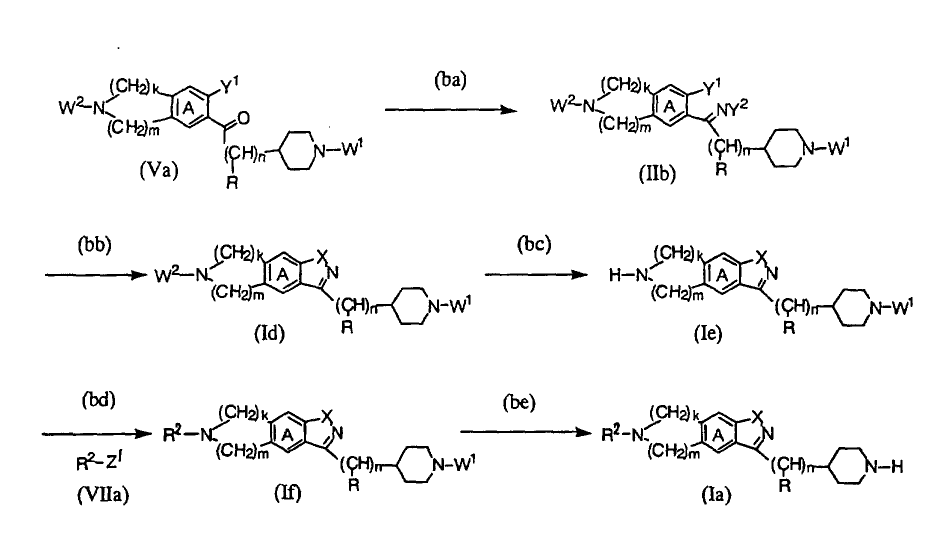

Alternatively, the above Compound (If) may be produced by the following Reaction formula 2-2. That is, Compound (If) can be produced by:
Step (bf): producing a compound represented by the formula (IIc) [wherein respective symbols represent the same meanings as above] (hereinafter abbreviated as Compound (IIc) in some cases) by a carbonyl group conversion reaction of Compound (Vg) and, thereafter,
Step (bg): performing a ring-closing reaction of Compound (IIc).
Step (bf) can be performed in the same manner as the above Step (af), and Step (bg) in the same manner as the above ring-closing reaction of Compound (II), respectively.

### [Reaction formula 2-2]

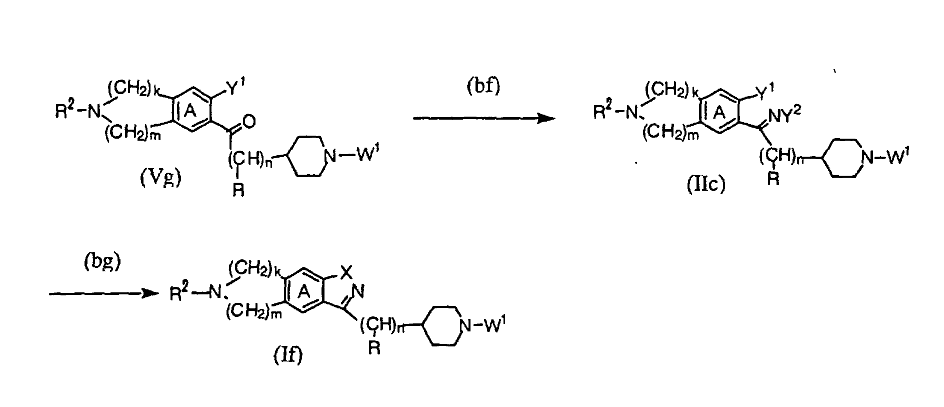

Alternatively, the above Compound (Id) can be produced by the following Reaction formula 2-3. That is, Compound (Id) can be produced by:
Step (bh): producing a compound represented by the formula (VIIIa) [wherein respective symbols represent the same meanings as above] (hereinafter abbreviated as Compound (VIIIa) in some cases) by a Friedel-Crafts reaction of Compound (IIIa) and an acetylating agent, then
Step (bi): producing a compound represented by the formula (VIIIb) [wherein respective symbols represent the same meanings as above] (hereinafter abbreviated as Compound (VIIIb) in some cases) by a carbonyl group conversion reaction of Compound (VIIIa), further
Step (bj): producing a compound represented by the formula (IX) [wherein respective symbols represent the same meanings as above] (hereinafter abbreviated as Compound (IX) in some cases) by performing a ring-closing reaction of Compound (VIIIb) and, thereafter,
Step (bk): subjecting Compound (IX) and a compound represented by the formula (IVb) [wherein respective symbols represent the same meanings as above] (hereinafter abbreviated as Compound (IVb) in some cases) to a condensation reaction.

### [Reaction formula 2-3]

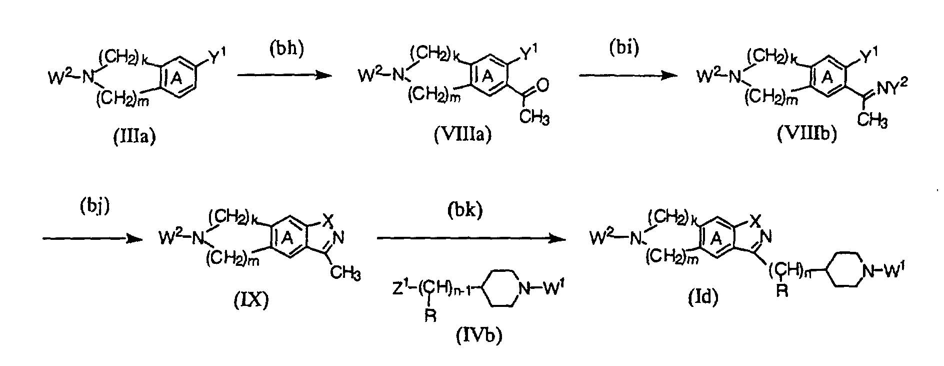

Step (bh) can be performed like the above Step (aa), Step (bi) like the above (af), and Step (bj) like the above ring-closing reaction of Compound (II), respectively.

In Step (bh), as the acetylating agent, for example, acetyl chloride, acetyl bromide, acetic anhydride and the like are used.

In Step (bk), the condensation reaction of Compound (IX) and Compound (IVb) can be performed by the method known per se or analogous method thereof, for example, methods described in J. Med. Chem., 37, 2721 (1994), J. Med. Chem., 38, 2802 (1995) and the like, or analogous methods thereof. Specifically, it is preferable that, for example, Compound (IX) and an equivalent to an excessive amount of Compound (IVb) are reacted at about -20°C to about 120°C for about 5 minutes to about 24 hours in the presence of a catalytic to excessive amount of a base.

As a solvent in the present reaction, for example, hydrocarbon solvents (e.g., hexane, toluene and the like), ether solvents (e.g., ethyl ether, tetrahydrofuran, dioxane, dimethoxyethane), amide solvents (e.g., formamide, N,N-dimethylformamide, N,N-dimethylacetamide, hexamethylphosphoric triamide and the like), sulfoxide solvents (e.g., dimethyl sulfoxide and the like) and the like are used. In particular, ether solvents such as tetrahydrofuran and the like are preferable.

As a base for the present reaction, used is a strong base such as a hydride of an alkali metal or an alkaline earth metal (e.g., lithium hydride, sodium hydride, potassium hydride, calcium hydride and the like), an amide of an alkali metal or an alkaline earth metal (e.g., lithium amide, sodium amide, lithium diisopropylamide, lithium dicyclohexylamide, lithium hexamethylsilazide, sodium hexamethylsilazide, potassium hexamethylsilazide, potassium hexamethylsilazide and the like), and a lower alkoxide of an alkali metal or an alkaline earth metal (e.g., sodium methoxide, sodium ethoxide, potassium t-butoxide and the like). In particular, lithium diisopropylamide is preferable. The amount of the amine used is preferably about 1 equivalent to about 3 equivalents relative to Compound (IX).

Compound (IVb) can be produced by the method known per se or analogous method thereof, for example, methods described in J. Med. Chem., 37, 2721 (1994), J. Med. Chem., 38, 2802 (1995), J. Med. Chem., 40, 1779 (1997) and the like, or analogous methods thereof.

In addition, as the leaving group Z¹ of Compound (IVb), a halogen atom (e.g., bromine, iodine and the like) is particularly preferable.

In addition, Compound (Va) described in the above 1) can be produced by a condensation reaction of Compound (VIIIa) and Compound (IVb). The present condensation reaction can be performed in the same manner as the above Step (bk).

3) Compound (IA) can be produced by subjecting a compound represented by the formula: 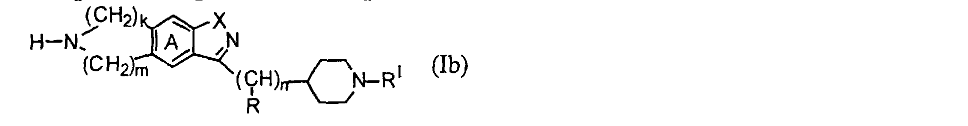 [wherein respective symbols represent the same meanings as above] (hereinafter abbreviated as Compound (Ib) in some cases) or s salt thereof included in Compound (IA) and Compound (VIIa) to a condensation reaction.

The condensation reaction of Compound (Ib) and Compound (VIIa) can be performed, for example, in the same manner as the "condensation reaction of Compound (Vb) and Compound (VIa)" described in the above 1).

A raw material Compound (Ib) or a salt thereof can be produced by the following Reaction formula 3-1.

That is, Compound (Ib) can be produced by:
Step (ca): producing a compound represented by the formula (Ig) [wherein respective symbols represent the same meanings as above] (hereinafter abbreviated as Compound (Ig) in some cases) by a deprotection reaction of a protecting group W¹ of Compound (Id),
Step (cb): producing a compound represented by the formula (Ih) [wherein respective symbols represent the same meanings as above] (hereinafter abbreviated as Compound (Ih) in some cases) by a condensation reaction of Compound (Ig) and Compound (VIa) and, thereafter,
Step (cc): performing a deprotection reaction of a protecting group W² of Compound (Ih).
Step (ca) can be performed in the same manner as the above Step (ab), Step (cb) in the same manner as the above Step (ac), and Step (cc) in the same manner as the above Step (ad), respectively.

### [Reaction formula 3-1]

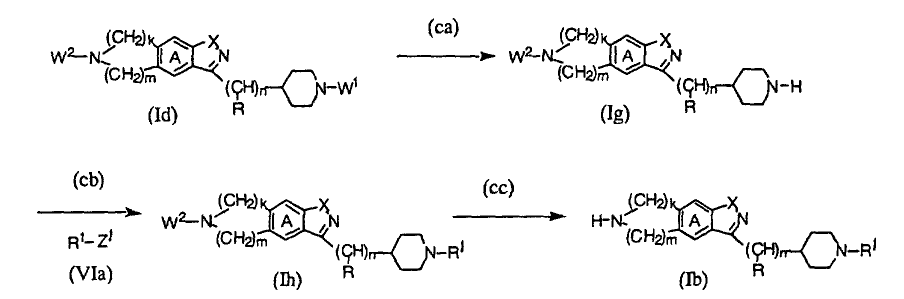

Alternatively, the above Compound (Ih) can be produced according to the following Reaction formula 3-2. That is, Compound (Ih) can be produced by:
Step (cd): producing a compound represented by the formula (IId) [wherein respective symbols as above] (hereinafter abbreviated as Compound (IId) in some cases) by a carbonyl group conversion reaction of Compound (Vc) and, thereafter,
Step (ce): performing a ring-closing reaction of Compound (IId).
Step (cd) can be performed in the same manner as the above Step (af), and Step (ce) in the same manner as the above ring-closing reaction of Compound (II).

### [Reaction formula 3-2]

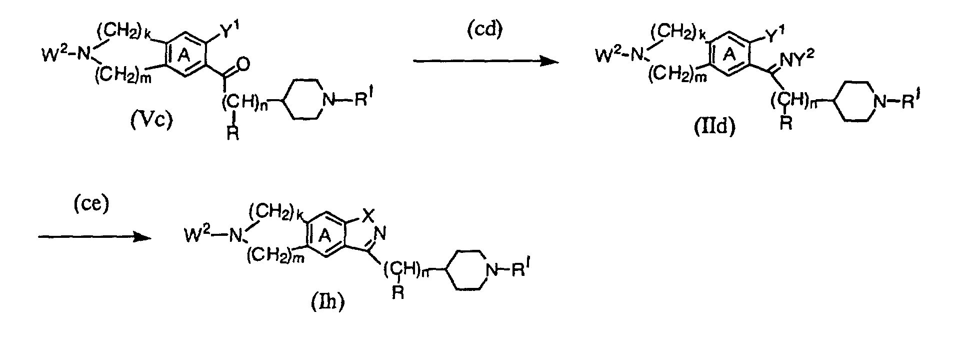

4) Compound (IA) wherein R¹=R² can be produced by subjectina a compound represented by the formula: 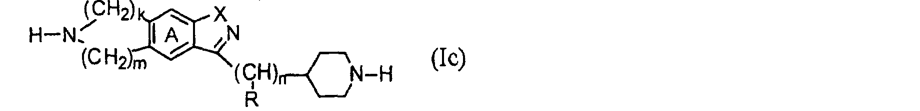 [wherein respective symbols represent the same meanings as above] (hereinafter abbreviated as Compound (Ic) in some cases) or a salt thereof included in Compound (IA) and Compound (VIa) to a condensation reaction.

The condensation reaction of Compound (Ic) and Compound (VIa) can be performed in the same manner as the "condensation reaction of Compound (Vb) and Compound (VIa)" described in the above 1). Compound (VIa) is preferably used at an amount of about 2 equivalents relative to Compound (Ic).

A raw material Compound (Ic) or a salt thereof can be produced by a deprotection reaction of a protecting group W¹ of Compound (Ie), a deprotection reaction of a protecting group W² of Compound (Ig), or a deprotection reaction of protecting groups W¹ and W² of Compound (Id). The present deprotection reaction can be performed in the same manner as the "deprotection reaction of a protecting group W¹" described in the above Step (ab).

### [C] In this item, the usefulness of Compound (I), Compound (I') and Compound (IA) will be described in detail.

Compound (I), Compound (I'), Compound (IA) or a salt thereof acts on the peripheral adipocyte of a mammal and, thus, has an activity of increasing a cAMP concentration in adipocyte, a lipolysis promoting activity, and a thermal production promoting activity, and shows an excellent body weight decreasing activity (more strictly, a body lipid rate lowering activity) and an activity of inhibiting a body weight increase in a mammal (e.g., human, monkey, mouse, rat, dog, cat, cow and the like).

Compound (I), Compound (I'), Compound (IA) or a salt thereof has the characteristics that it has the extremely excellent separation from action on the central nervous system when compared with known central anorectics such as mazindol and the like, has no or extremely low action on the central nervous system, and has low toxicity. In addition, it shows the remarkable effect by oral administration. Compound (I), Compound (I'), Compound (IA) or a salt thereof has the acute toxicity (LD₅₀) of about 100 mg/kg or more.

Therefore, Compound (I), Compound (I'), Compound (IA) or a salt thereof is useful as a safe agent for preventing or treating obesity and obesity-based diseases or diseases which develop in connection with obesity in a mammal including human being.

As the names of the diseases for which Compound (I), Compound (I'), Compound (IA) or a salt thereof is useful, for example (1) obesity, (2) as obesity-based diseases, (i) diabetes (in particular, non-insulin-dependent diabetes), (ii) hyperlipidemia, (iii) arterial sclerosis, (iv) hypertension and the like, (3) as diseases which develop in connection with obesity, (i) impaired glucose tolerance, (ii) hyperinsulinemia, (iii) hypo-HDL cholesterolemia, (iv) hyperuricemia, (v) gout, (vi) angina, (vii) cardiac infarction, (viii) cardiac function abnormality, (ix) hypercardia, (x) heart failure, (xi) chronic nephritis, (xii) Pickwick syndrome, (xiii) sleep apnea syndrome, (xiv) fatty liver, (xv) cholelithiasis, (xvi) pancreatitis, (xvii) osteoarthritis, (xviii) spondylolisthesis, (xix) ovary function abnormality, (xx) menses abnormality, (xxi) infertility, (xxii) tonsil hypertrophy, (xxiii) parotid inflation and the like can be mentioned. The above Compound (IA) or a salt thereof can be particularly used for preventing or treating obesity and non-insulin-dependent diabetes among the above diseases.

Compound (I), Compound (I'), Compound (IA) or a salt thereof can be safely administered orally or parenterally (e.g., locally, rectally, intravenously and the like) to a mammal including human being as it is or as a pharmaceutical composition in which a pharmacologically acceptable carrier is mixed according to the method known per se, for example, tablets (including sugar-coated tablets and coating tablets), powders, granules, capsules (including soft capsules), liquids, injections, suppositories, sustained-release agent and the like. The contents of Compound (I), Compound (I'), Compound (IA) or a salt thereof in the present preparations are about 0.1% by weight to about 100% by weight.

Compound (I), Compound (I'), Compound (IA) or a salt thereof can be usually formulated together with pharmaceutically acceptable carriers or excipients, and administered orally or parenterally to mammals including human beings.

The dosage differs depending upon the administration subject, the administration route, the kinds of subject diseases, and the clinical conditions and, for example, as an anti-obesity drug, is about 0.01 mg to about 10,000 mg, preferably about 0.1 mg to about 2,000 mg, more preferably about 0.5 mg to about 1,000 mg, further more preferably about 25 mg to about 500 mg in terms of an active ingredient (Compound (I), Compound (I'), Compound (IA) or a salt thereof) per day, as a single dosage or a divided dosage of 2 to 4 times per day as an oral agent, per an adult (body weight: about 70 kg).

As a pharmacologically acceptable carrier which may be used for producing the present preparations, the conventional various organic or inorganic carrier substances as preparation materials can be mentioned. For example, an excipient, a lubricant, a binder, a disintegrator in solid preparations; a solvent, a solubilizer, a suspending agent, an isotonic agent, a buffering agent, a soothing agent in liquid preparations, and the like are used. In addition, additives such as a preservative, an antioxidant, a colorant, a sweetening agent, an absorbent, a wetting agent and the like may be used as necessary.

As the excipient, for example, lactose, sucrose, D-mannitol, starch, corn starch, crystalline cellulose, light silicic acid anhydride and the like are used.

As the lubricant, for example, magnesium stearate, calcium stearate, talc, colloidal silica and the like are used.

As the binder, for example, crystalline cellulose, sucrose, D-mannitol, dextrin, hydroxypropyl cellulose, hydroxypropylmethyl cellulose, polyvinylpyrrolidone, starch, sucrose, gelatin, methyl cellulose, carboxymethylcellulose sodium and the like are used.

As the disintegrator, for example, starch, carboxymethyl cellulose, carboxymethyl cellulose calcium, carboxymethyl starch sodium, L-hydroxypropyl cellulose and the like are used.

As the solvent, for example, injectable water, an alcohol, propylene glycol, macrogol, sesame oil, corn oil and the like are used.

As the solubilizer, for example, polyethylene glycol, propylene glycol, D-mannitol, benzyl benzoate, ethanol, trisaminomethane, cholesterol, triethanolamine, sodium carbonate, sodium succinate and the like are used.

As the suspending agent, for example, surfactants such as stearyltriethanolamine, sodium laurylsulfate, laurylaminopropionic acid, lecithin, benzalkonium chloride, benzethonium chloride and monostearic glyceride; hydrophilic polymers such as polyvinyl alcohol, polyvinylpyrrolidone, sodium carboxymethyl cellulose, methyl cellulose, hydroxypropyl cellulose and the like are used.

As the isotonic agent, for example, glucose, D-sorbitol, sodium chloride, glycerin, D-mannitol and the like are used.

As the buffering agent, for example, buffers such as phosphate, acetate, carbonate, succinate and the like are used.

As the soothing agent, for example, benzyl alcohol and the like are used.

As the preservative, for example, paraoxybenzoate esters, chlorobutabol, benzyl alcohol, phenethyl alcohol, dehydroacetic acid, sorbic acid and the like are used.

As the antioxidant, for example, sulfite, ascorbic acid and the like are used.

### BEST MODE FOR CARRYING OUT THE INVENTION

The present invention will be explained in more detail by way of Reference Examples, Examples, Test Examples and Preparations examples, but these are merely exemplary and do not limit the present invention and, additionally, a variation can be made within a range without departing from the scope of the present invention.

"Room temperature" in the following Examples denotes about 0°C to about 30°C, and anhydrous magnesium sulfate or anhydrous sodium sulfate was used for drying organic solvents. Unless otherwise indicated, % means % by weight.

In addition, respective symbols represent the following meanings:
s: singlet
d: doublet
t: triplet
q: quartet
dd: double doublet
dt: double triplet
m: multiplet
br: broad
J: coupling constant
Hz: Hertz
CDCl₃: heavy chloroform
DMSO: dimethyl sulfoxide
¹H NMR: proton nuclear magnetic resonance (measured usually as a free compound in CDCl₃)

### Reference Example 1)

7-Hydroxy-2-[(2-methylphenyl)methyl]-2,3,4,5-tetrahydro-1H-2-benzazepine 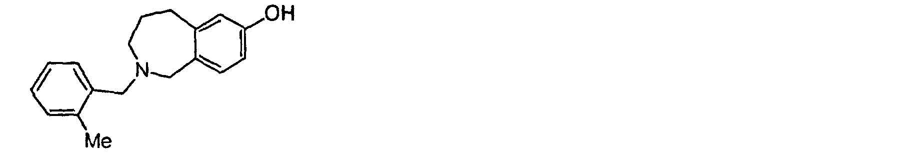
1) α-Bromo-o-xylene (3.97 ml, 29.6 mmol) was added dropwise to a suspension of 7-methoxy-2,3,4,5-tetrahydro-1H-2-benzazepine (5.00 g, 282.2 mmol), potassium carbonate (4.0 g) and potassium iodide (catalytic amount) in ethanol (150 ml) at room temperature. After the mixture was heated at reflux for 3 hours and the solvent was distilled off under reduced pressure, the residue was dissolved in water-ethyl acetate, and extracted with ethyl acetate. After the extract was washed with an aqueous saturated solution of sodium chloride and dried with anhydrous magnesium sulfate, the solvent was distilled off under reduced pressure. The resulting residue was purified by silica gel column chromatography (developing solvent; hexane-ethyl acetate=4:1) to obtain 7-methoxy-2-[(2-methylphenyl)methyl]-2,3,4,5-tetrahydro-1H-2-benzazepine (6.71 g) as a colorless oil.
   ¹H NMR (CDCl₃) δ 1.60-1.90 (2H, m), 2.28 (3H, s), 2.88 (2H, t-like, J = 5.4 Hz), 3.07 (2H, t-like, J = 5.4 Hz), 3.46 (2H, s), 3.80 (3H, s), 3.81 (2H, s), 6.62 (1H, dd, J = 8.0, 2.6 Hz), 6.73 (1H, d, J = 2.6 Hz), 6.90 (1H, d, J = 8.0Hz), 7.10-7.35 (4H, m).
2) A mixture of 7-methoxy-2-[(2-methylphenyl)methyl]-2,3,4,5-tetrahydro-1H-2-benzazepine (6.71 g, 23.8 mmol) obtained in 1) and 48% hydrobromic acid solution (80 ml) was stirred at 140°C for 2 hours. After the mixture was cooled to room temperature, the mixture was made weak alkaline (pH about 10) using 8N aqueous sodium hydroxide solution, and extracted twice with ethyl acetate. The extract was washed with an aqueous saturated solution of sodium chloride, dried with anhydrous magnesium sulfate and the solvent was distilled off to obtain the title compound (5.69 g) as colorless crystals having a melting point of 148-149°C.
   ¹H MMR (CDCl₃) δ 1.70-1.85 (2H, m), 2.27 (3H, s), 2.83 (2H, t-like, J = 5.4 Hz), 3.08 (2H, t-like, J = 5.2 Hz), 3.48 (2H, s), 3.80 (2H, s), 6.50 (1H, dd, J = 8.0, 2.6 Hz), 6.61 (1H, d, J = 2.6 Hz), 6.82 (1H, d, J = 8.0 Hz), 7.10-7.30 (4H, m).

### Reference Example 2)

8-Hydroxy-2-[(2-methylphenyl)methyl]-2,3,4,5-tetrahydro-1H-2-benzazepine 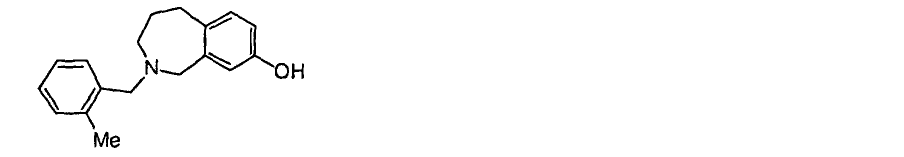
1) According to the same procedures as those of Reference Example 1)-1) using α-bromo-o-xylene (3.16 ml, 23.5 mmol) and 8-methoxy-2,3,4,5-tetrahydro-1H-2-benzazepine (3.80 g, 21.4 mmol), 8-methoxy-2-[(2-methylphenyl)methyl]-2,3,4,5-tetrahydro-lH-2-benzazepine (4.63 g) was obtained as a colorless oil. ¹H NMR (CDCl₃) δ 1.60-1.70 (2H, m), 2.19 (3H, s), 2.78 (2H, t-like, J = 5.4 Hz), 3.00 (2H, t-like, J = 5.4 Hz), 3.41 (2H, s), 3.67 (3H, s), 3.73 (2H, s), 6.46 (1H, d, J = 2.6 Hz), 6.60 (1H, dd, J = 8.0, 2.6 Hz), 6.98 (1H, d, J = 8.0 Hz), 7.00-7.20 (4H, m).
2) According to the same procedures as those of Reference Example 1)-2) using 8-methoxy-2-[(2-methylphenyl)methyl]-2,3,4,5-tetrahydro-1H-2-benzazepine (4.63 g, 16.4 mmol) obtained in 1), 8-hydroxy-2-[(2-methylphenyl)methyl]-2,3,4,5-tetrahydro-1H-2-benzazepine (4.37 g) was obtained as a colorless oil. ¹H NMR (CDCl₃) δ 1.75-1.85 (2H, m), 2.26 (3H, s), 2.80 (2H, t-like, J = 5.0 Hz), 3.03 (2H, t-like, J = 5.0 Hz), 3.48 (2H, s), 3.70 (2H, s), 6.19 (1H, d, J = 2.6 Hz), 6.46 (1H, dd, J = 8.0, 2.6 Hz), 6.88 (1H, d, J = 8.0 Hz), 7.00-7.30 (4H, m).

### Reference Example 3)

7-Methoxy-3-(phenylmethyl)-2,3,4,5-tetrahydro-1H-3-benzazepine 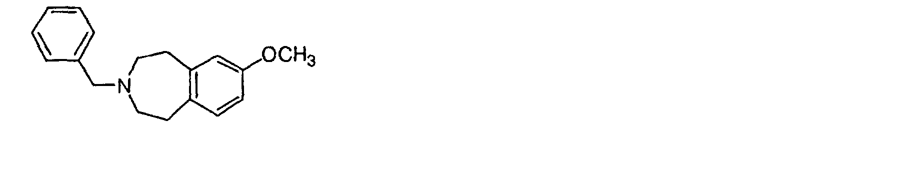
1) A solution of 8-methoxy-2,3-dihydro-1H-3-benzazepine-2-one (9.0 g, 47.5 mmol) in ethanol (200 ml) was catalytic-hydrogenated using 5% Pd/C as a catalyst to obtain 8-methoxy-2,3,4,5-tetrahydro-1H-3-benzazepine-2-one (8.3 g) as colorless needle crystals having a melting point of 162-163°C.
   ¹H NMR(CDCl₃) δ 3.06 (2H, t, J = 6.2 Hz), 3.49 - 3.60 (2H, m), 3.78 (3H, s), 3.81 (2H, s), 6.0 (1H, br, NH), 6.69 (1H, d, J = 2.6 Hz), 6.76 (1H, dd, J = 2.6, 8.4 Hz), 7.04 (1H, d, J = 8.4 Hz).
2) Lithium aluminum hydride (1.4 g, 36.8 mmol) was added to a solution of 8-methoxy-2,3,4,5-tetrahydro-1H-3-benzazepine-2-one (3.5 g, 18.5 mmol) obtained in 1) in tetrahydrofuran (300 ml) portionwise at room temperature. After the mixture was heated at reflux for 4 hours and allowed to cool, water (2.8 ml) and then a 10% aqueous solution of sodium hydroxide (2.24 ml) were added dropwise while stirring. After stirring at room temperature for 14 hours, the resulting precipitates were removed by filtration, and the solvent was distilled off under reduced pressure to obtain the crude product of 7-methoxy-2,3,4,5-tetrahydro-1H-3-benzazepine (3.0 g) as a viscous oil.
3) According to the same procedures as those of Reference Example 1)-1) using 7-methoxy-2,3,4,5-tetrahydro-1H-3-benzazepine (1.0 g) obtained in-2), the title compound (1.05 g) was obtained as an oil.
   ¹H NMR(CDCl₃) δ 2.55- 2.68 (4H, m), 2.81 - 2.91 (4H, m), 3.64 (2H, s), 3.77 (3H, s), 6.58 - 6.68 (2H, m), 6.99 (1H, d, J = 8.4 Hz), 7.18 - 7.40 (5H, m).

### Reference Example 4)

7-Hydroxy-3-(phenylmethyl)-2,3,4,5-tetrahydro-1H-3-benzazepine

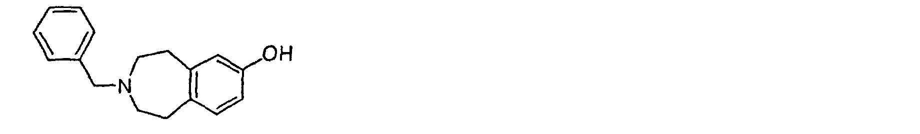

According to the same procedures as those of Reference Example 1)-2) using 7-methoxy-3-(phenylmethyl)-2,3,4,5-tetrahydro-1H-3-benzazepine (0.7 g) obtained in Reference Example 3), the title compound (0.6 g) was obtained as colorless powders having a melting point of 134-137°C. ¹H NMR(CDCl₃) δ 2.53 - 2.70 (4H, m), 2.75 - 2.92 (4H, m), ca. 3.2 (1H, br, OH), 3.65 (2H, s), 6.49 - 6.60 (2H, m), 6.92 (1H, d, J = 8.8 Hz), 7.18 - 7.40 (5H, m).

### Reference Example 5)

7-Methoxy-3-[(2-methylphenyl)methyl]-2,3,4,5-tetrahydro-1H-3-benzazepine

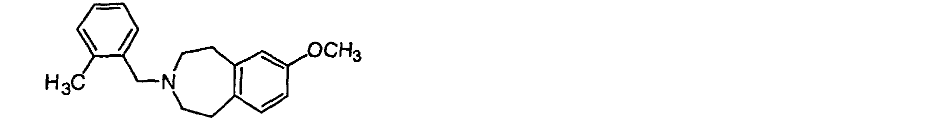

According to the same procedures as those of Reference Example 1)-1) using 7-methoxy-2,3,4,5-tetrahydro-1H-3-benzazepine (1.5 g) obtained in Reference Example 3)-2), the title compound (1.8 g) was obtained as an oil.
¹H NMR(CDCl₃) δ 52.39 (3H, s), 2.55 - 2.68 (4H, m), 2.77 - 2.89 (4H, m), 3.53 (2H, s), 3.77 (3H, s), 6.58 - 6.67 (2H, m), 6.99 (1H, d, J = 8.4 Hz), 7.10 - 7.37 (4H, m).

### Reference Example 6)

7-Hydroxy-3-[(2-methylphenyl)methyl]-2,3,4,5-tetrahydro-1H-3-benzazepine

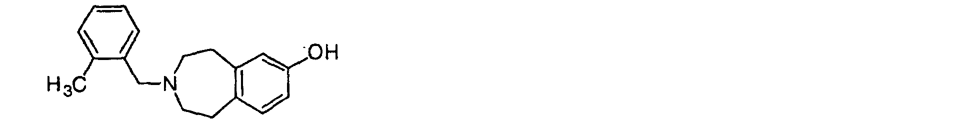

According to the same procedures as those of Reference Example 1)-2) using 7-methoxy-3-[(2-methylphenyl)methyl]-2,3,4,5-tetrahydro-1H-3-benzazepine (0.85 g) obtained in Reference Example 5), the title compound (0.7 g) was obtained as an oil.
¹H NMR(CDCl₃) δ 2.38 (3H, s), 2.53 - 2.68 (4H, m), 2.72 - 2.87 (4H, m), 3.54 (2H, s), ca. 3.7 (1H, br, OH), 6.48 - 6.58 (2H, m), 6.91 (1H, d, J = 8.8 Hz), 7.05 - 7.37 (4H, m).

### Reference Example 7)

t-Butyl 4-[2-[[2-[(2-methylphenyl)methyl]-2,3,4,5-tetrahydro-1H-2-benzazepine-7-yl]oxy]ethyl]-1-piperidinecarboxylate

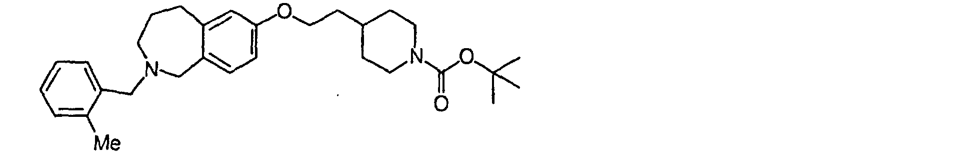

Potassium carbonate (10.0 g) was added to a solution of 7-hydroxy-2-[(2-methylphenyl)methyl]-2,3,4,5-tetrahydro-1H-2-benzazepine (5.00 g, 18.7 mmol) obtained in Reference Example 1) and t-butyl 4-(2-iodoethyl)-1-piperidinecarboxylate (6.34 g, 18.7 mmol) in N,N-dimethylformamide (80 ml), and the mixture was stirred at 80°C for 12 hours. After the solvent was distilled off under reduced pressure, the residue was dissolved in water-ethyl acetate and extracted with ethyl acetate. After the extract was washed with an aqueous saturated solution of sodium chloride and dried with anhydrous magnesium sulfate, the solvent was distilled off under reduced pressure. The resulting residue was purified by silica gel column chromatography (developing solvent; hexane-ethyl acetate=19:1) to obtain the title compound (7.46 g) as a colorless oil.
¹H NMR (CDCl₃) δ 1.00-1.30 (2H, m), 1.46 (9H, s), 1.60-1.80 (7H, m), 2.28 (3H, s), 2.60-2.80 (2H, m), 2.86 (2H, t-like, J = 5.2 Hz), 3.06 (2H, t-like, J = 5.2Hz), 3.46 (2H, s), 3.79 (2H, s), 3.98 (2H, t, J = 5.6 Hz), 4.00-4.20 (2H, m), 6.59 (1H, dd, J = 8.0, 2.4 Hz), 6.71 (1H, d, J = 2.4 Hz), 6.88 (1H, d, J = 8.0 Hz), 7.05-7.30 (4H, m).

### Reference Example 8)

2-[(2-Methylphenyl)methyl]-7-[2-(4-piperidinyl)ethoxy]-2,3,4,5-tetrahydro-1H-2-benzazepine

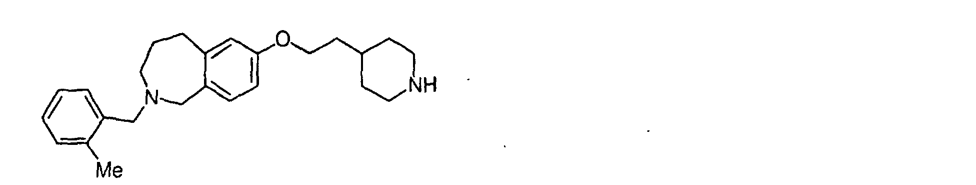

A 4N hydrogen chloride-ethyl acetate solution (100 ml) was added to a solution of t-butyl 4-[2-[[2-[(2-methylphenyl)methyl]-2,3,4,5-tetrahydro-1H-2-benzazepine-7-yl]oxy]ethyl]-1-piperidinecarboxylate (7.46 g, 15.6 mmol) in ethyl acetate (30 ml) at room temperature, and the mixture was stirred for 2 hours. After the solvent was distilled off under reduced pressure, the residue was made alkaline with an aqueous solution of potassium carbonate and extracted with ethyl acetate. After the extract was washed with an aqueous saturated solution of sodium chloride and dried with anhydrous potassium sulfate, the solvent was distilled off under reduced pressure to obtain the title compound (5.20 g) as a colorless oil. This crude product was used in the next reaction without purification.
¹H NMR (CDCl₃) δ 1.00-1.30 (2H, m), 1.60-1.80 (8H, m), 2.27 (3H, s), 2.60-2.80 (2H, m), 2.87 (2H, t-like, J = 5.2 Hz), 3.05 (2H, t-like, J = 5.2 Hz), 3.46 (2H, s), 3.79 (2H, s), 3.97 (2H, t, J = 5.6 Hz), 4.00-4.20 (2H, m), 6.58 (1H, dd, J = 8.0, 2.4 Hz), 6.72 (1H, d, J = 2.4 Hz), 6.88 (1H, d, J = 8.0 Hz), 7.05-7.30 (4H, m).

### Reference Example 9)

t-Butyl 4-[2-[[3-[(2-methylphenyl)methyl]-2,3,4,5-tetrahydro-1H-3-benzaepine-7-yl]oxy]ethyl]-1-piperidinecarboxylate

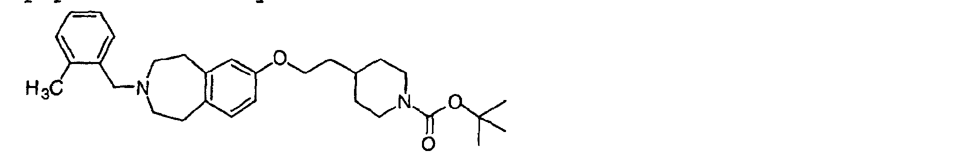

According to the same procedures as those of Reference Example 7) using 7-hydroxy-3-[(2-methylphenyl)methyl]-2,3,4,5-tetrahydro-1H-3-benzazepine (0.23 g) obtained in Reference Example 6), the title compound (0.29 g) was obtained as a viscous oil.
¹H NMR (CDCl₃) δ 1.02 - 1.27 (2H, m), 1.46 (9H, s), 1.55 - 1.79 (5H, m), 2.39 (3H, s), 2.56 - 2.89 (10H, m), 3.54 (2H, s), 3.92 - 4.17 (4H, m), 6.57 - 6.67 (2H, m), 6.98 (1H, d, J = 8.1 Hz), 7.05 - 7.37 (4H, m).

### Reference Example 10)

3-[(2-Methylphenyl)methyl]-7-[2-(4-piperidinyl)ethoxy]-2,3,4,5-tetrahydro-1H-3-benzazepine

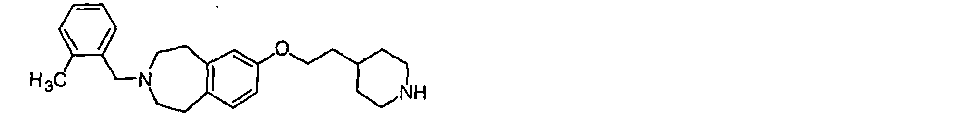

According to the same procedures as those of Reference Example 8) using t-butyl 4-[2-[[3-[(2-methylphenyl)methyl]-2,3,4,5-tetrahydro-1H-3-benzaepine-7-yl]oxy]ethyl]-1-piperidinecarboxylate (0.23 g) obtained in Reference Example 9), the title compound (0.185 g) was obtained as an oil.
¹H NMR(CDCl₃) δ 1.10 - 1.33 (2H, m), 1.60 - 1.83 (3H, m), 1.92 - 2.08 (2H, m), 2.39 (3H, s), 2.50 - 2.77 (7H, m), 2.78 - 2.90 (4H, m), 3.02 - 3.17 (2H, m), 3.53 (2H, s), 3.97 (2H, t, J = 5.9 Hz), 6.57 - 6.69 (2H, m), 6.98 (1H, d, J = 8.0 Hz), 7.11 - 7.22 (3H, m), 7.25 - 7.37 (1H, m).

### Reference Example 11)

t-Butyl 4-[3-[[3-(phenylmethyl)-2,3,4,5-tetrahydro-1H-3-benzaepine-7-yl]oxy]propyl]-1-piperidinecarboxylate

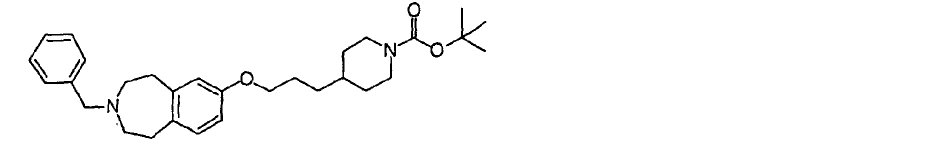

According to the same procedures as those of Reference Example 7) using 7-hydroxy-3-phenylmethyl-2,3,4,5-tetrahydro-1H-3-benzaepine (0.11 g) obtained in Reference Example 4), the title compound (0.17 g) was obtained as a viscous oil.
¹H NMR(CDCl₃) δ 0.97 - 1.23 (2H, m), 1.30 - 1.48 (12H, m), 1.58 - 1.86 (4H, m), 2.54 - 2.77 (6H, m), 2.80 - 2.92 (4H, m), 3.63 (2H, s), 3.91 (2H, t, J = 6.4 Hz), 3.98 - 4.16 (2H, m), 6.57 - 6.66 (2H, m), 6.97 (1H, d, J = 7.7 Hz), 7.21 - 7.38 (5H, m).

### Reference Example 12)

3-(Phenylmethyl)-7-[3-(4-piperidinyl)propoxy]-2,3,4,5-tetrahydro-1H-3-benzazepine

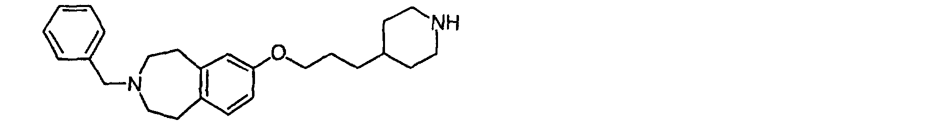

According to the same procedures as those of Reference Example 8) using t-butyl 4-[3-[[3-(phenylmethyl)-2,3,4,5-tetrahydro-1H-3-benzaepine-7-yl]oxy]propyl]-1-piperidinecarboxylate (0.15 g) obtained in Reference Example 11), the title compound (0.11 g) was obtained as an oil.
¹H NMR(CDCl₃) δ 1.03- 1.49 (5H, m), 1.63 - 1.99 (5H, m), 2.49 - 2.69 (6H, m), 2.78 - 2.93 (4H, m), 3.01 - 3.19 (2H, m), 3.63 (2H, s), 3.90 (2H, t, J = 6.2 Hz), 6.56 - 6.68 (2H, m), 6.97 (1H, d, J = 7.7 Hz), 7.20 - 7.40 (5H, m).

### Reference Example 13)

t-Butyl 4-[2-[[2-[(2-methylphenyl)methyl]-2,3,4,5-tetrahydro-1H-2-benzazepine-8-yl]oxy]ethyl]-1-piperidinecarboxylate

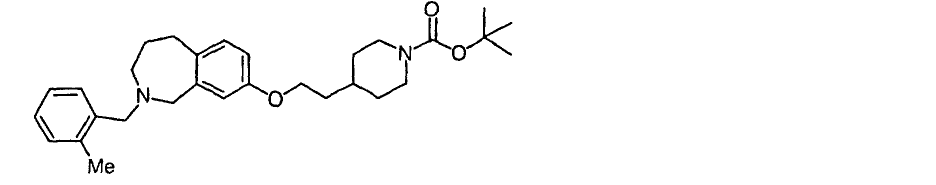

According to the same procedures as those of Reference Example 7) using 8-hydroxy-2-[(2-methylpheyl)methyl]-2,3,4,5-tetrahydro-1H-2-benzazepine (3.94 g) obtained in Reference Example 2), the title compound (4.81 g) was obtained as a viscous oil.
¹H NMR (CDCl₃) δ 1.05-1.30 (2H, m), 1.46 (9H, s), 1.60-1.80 (7H, m), 2.28 (3H, s), 2.60-2.80 (2H, m), 2.86 (2H, t-like, J = 5.4 Hz), 3.07 (2H, t-like, J = 5.2 Hz), 3.49 (2H, s), 3.81 (2H, s), 3.95 (2H, t, J = 5.8 Hz), 4.00-4.20 (2H, m), 6.54 (1H. d, J = 2.6 Hz), 6.67 (1H, dd, J = 8.0, 2.6 Hz), 7.05 (1H, d, J = 8.0 Hz), 7.10-7.30 (4H, m).

### Reference Example 14)

2-[(2-Methylphenyl)methyl]-8-[2-(4-piperidinyl)ethoxy]-2,3,4,5-tetrahydro-1H-2-benzazepine

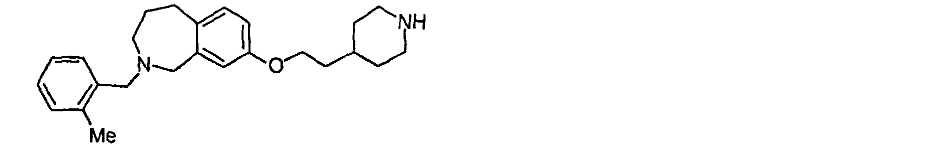

According to the same procedures as those of Reference Example 8) using t-butyl 4-[2-[[2-[(2-methylphenyl)methyl]-2,3,4,5-tetrahydro-1H-2-benzazepine-8-yl]oxy]ethyl]-1-piperidinecarboxylate (4.81 g, 10.1 mmol) obtained in Reference Example 13), the title compound (3.61 g) was obtained as colorless crystals having a melting point of 74-75°C.
¹H NMR (CDCl₃) δ 1.05-1.30 (2H, m), 1.50-1.95 (8H, m), 2.28 (3H, s), 2.50-2.70 (2H, m), 2.86 (2H, t-like, J = 5.4 Hz), 3.00-3.15 (4H, m), 3.46 (2H, s), 3.81 (2H, s), 3.94 (2H, t, J = 6.2 Hz); 6.54 (1H, d, J= 2.6 Hz), 6.67 (1H, dd, J = 8.1, 2.6 Hz), 7.04 (1H. d, J = 8.1 Hz), 7.09-7.32 (4H, m).
Elemental analysis for C₂₅H₃₄N₂O
Calcd.: C, 79.32; H, 9.05; N,7.40
Found: C, 79.00; H, 9.15; N,7.32

### Reference Example 15)

t-Butyl 4-[3-[[3-[(2-methylphenyl)methyl]-2,3,4,5-tetrahydro-1H-3-benzazepine-7-yl]oxy]propyl]-1-piperidinecarboxylate

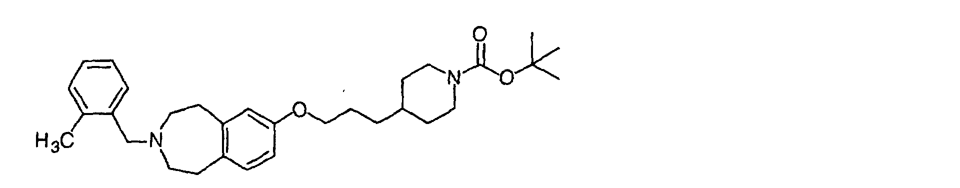

According to the same procedures as those of Reference Example 7) using 7-hydroxy-3-[(2-methylphenyl)methyl]-2,3,4,5-tetrahydro-1H-3-benzazepine (0.11 g) obtained in Reference Example 6), the title compound (0.15 g) was obtained as a viscous oil.
¹H NMR(CDCl₃) δ 0.99 - 1.22 (2H, m), 1.29 - 1.50 (12H, m), 1.53 - 1.87 (4H, m), 2.39 (3H, s), 2.54 - 2.90 (10H, m), 3.53 (2H, s), 3.91 (2H, t, J = 6.4 Hz), 4.00- 4.17 (2H, m), 6.57 - 6.67 (2H, m), 6.98 (1H, d, J = 8.1 Hz), 7.10 - 7.22 (3H, m), 7.24 - 7.36 (1H, m).

### Reference Example 16)

3-[(2-Methylphenyl)methyl]-7-[3-(4-piperdinyl)propoxy] -2,3,4,5-tetrahydro-1H-3-benzazepine

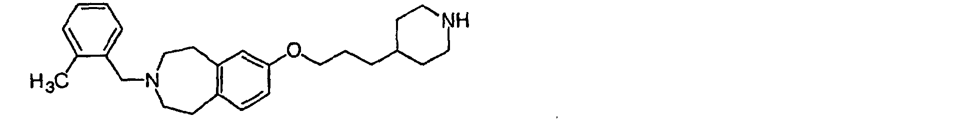

According to the same procedures as those of Reference Example 8) using t-butyl 4-[3-[[3-[(2-methylphenyl)methyl]-2,3,4,5-tetrahydro-1H-3-benzazepine-7-yl]oxy]propyl]-1-piperidinecarboxylate (0.14 g) obtained in Reference Example 15), the title compound (0.105 g) was obtained as an oil.
¹H NMR(CDCl₃) δ 1.00 - 1.46 (5H, m), 1.58 - 2.03 (5H, m), 2.39 (3H, s), 2.42 - 2.89 (10H, m), 3.02 - 3.18 (2H, m), 3.53 (2H, s), 3.91 (2H, t, J = 6.4 Hz), 6.56 - 6.67 (2H, m), 6.98 (1H, d, J = 7.7 Hz), 7.02 - 7.21 (3H, m), 7.24 - 7.37 (1H, m).

### Reference Example 17)

t-Butyl 4-[2-[[3-(phenylmethyl)-2,3,4,5-tetrahydro-1H-3-benzazepine-7-yl]oxy]ethyl]-1-piperidinecarboxylate

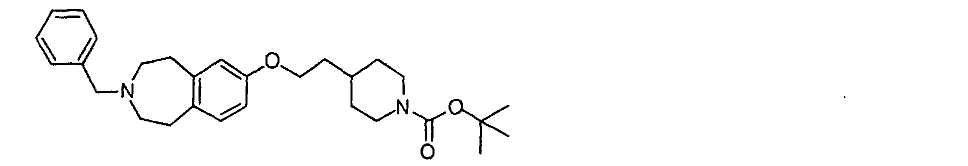

According to the same procedures as those of Reference Example 7) using 7-hydroxy-3-phenylmethyl-2,3,4,5-tetrahydro-1H-3-benzazepine (0.25 g) obtained in Reference Example 4), the title compound (0.36 g) was obtained as a viscous oil.
¹H NMR(CDCl₃) δ 1.03- 1.28 (2H, m), 1.45 (9H, s), 1.50 - 1.79 (5H, m), 2.55 - 2.92 (10H, m), 3.64 (2H, s), 3.91 - 4.16 (4H, m), 6.56 - 6.67 (2H, m), 6.97 (1H, d, J = 8.1 Hz), 7.21 - 7.38 (5H, m).

### Reference Example 18)

3-(Phenylmethyl)-7-[2-(4-piperidinyl)ethoxy]-2,3,4,5-tetrahydro-1H-3-benzazepine

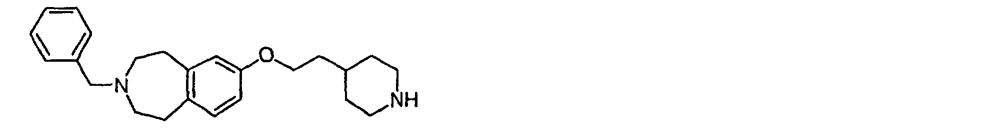

According to the same procedures as those of Reference Example 8) using t-butyl 4-[2-[[3-(phenylmethyl)-2,3,4,5-tetrahydro-1H-3-benzazepine-7-yl]oxy]ethyl]-1-piperidinecarboxylate (0.34 g) obtained in Reference Example 17), the title compound (0.265 g) was obtained as an oil.
¹H NMR(CDCl₃) δ 1.40 - 2.08 (8H, m), 2.68 - 3.00 (10H, m), 3.30 - 3.46 (2H, m), 3.81 (2H, s), 3.95 (2H, t, J = 5.7 Hz), 6.55 - 6.65 (2H, m), 6.97 (1H, d, J = 8.4 Hz), 7.28 - 7.38 (5H, m).

### Reference Example 19)

2,2,2-Trifluoro-1-(7-sulfanyl-2,3,4,5-tetrahydro-1H-3-benzazepine-3-yl)-1-ethanone

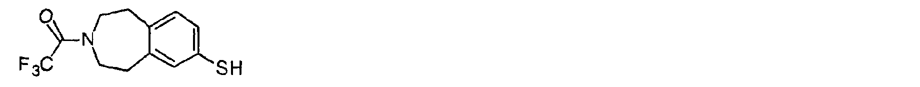

Zinc powders (10 g, 153 mmol) were added to a solution of 3-(trifluoroacetyl)-2,3,4,5-tetrahydro-1H-3-benzazepine-7-sulfonyl chloride (10.0 g, 29.3 mmol) in acetic acid (80 ml), and the mixture was heated at reflux for 10 minutes. After the solids were removed by filtration, the filtrate was concentrated under reduced pressure. The residue was dissolved in water-ethyl acetate, and extracted with ethyl acetate. The extract was washed successively with an aqueous saturated solution of sodium bicarbonate and an aqueous saturated solution of sodium chloride, dried with anhydrous magnesium sulfate, and the solvent was distilled off to obtain the title compound (6.35 g) as colorless crystals having a melting point of 81-85°C. ¹H NMR (CDCl₃) δ 2.85-3.00 (4H, m), 3.42 (1H, s), 3.60-3.80 (4H, m), 6.95-7.15 (3H, m).
Elemental analysis for C₁₂H₁₂F₃NOS·H₂O
Calcd.: C, 49.14; H, 4.81; N, 4.78
Found: C, 49.22; H, 4.06; N, 4.85

### Reference Example 20)

t-Butyl 4-[2-[[3-(trifluoroacetyl)-2,3,4,5-tetrahydro-1H-3-benzazepine-7-yl]sulfanyl]ethyl]-1-piperidinecarboxylate

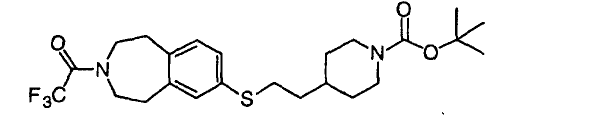

t-Butyl 4-(2-iodoethyl)-1-piperidinecarboxylate (2.97 g, 8.76 mmol) and anhydrous potassium carbonate (1 g) were added to a solution of 2,2,2-trifluoro-1-(7-sulfanyl-2,3,4,5-tetrahydro-1H-3-benzazepine-3-yl)-1-ethanone (2.41 g, 8.75 mmol) obtained in Reference Example 19) in N,N-dimethylformamide, and the mixture was stirred at room temperature for 2 hours. After the solvent was distilled off under reduced pressure, the residue was dissolved in water-ethyl acetate and extracted with ethyl acetate. After the extract was washed with an aqueous saturated solution of sodium chloride and dried with anhydrous magnesium sulfate, the solvent was distilled off under reduced pressure. The resulting residue was purified by silica gel column chromatography (developing solvent; hexane-ethyl acetate=4:1) to obtain the title compound (3.49 g) as colorless crystals having a melting point of 100-102°C.
¹H NMR (CDCl₃) δ 1.00-1.20 (2H, m), 1.45 (9H, s), 1.50-1.75 (5H, m), 2.55-2.80 (2H, m), 2.85-3.00 (6H, m), 3.60-3.80 (4H, m), 4.00-4.15 (2H, m), 7.00-7.20 (3H, m).
Elemental analysis for C₂₄H₃₃N₂O₃S
Calcd.: C, 59.24; H, 6.84; N, 5.76
Found: C, 59.21; H, 6.79; N, 5.75

### Reference Example 21)

t-Butyl 4-[2-[(2,3,4,5-tetrahydro-1H-3-benzazepine-7-yl)sulfanyl]ethyl]-1-piperidinecarboxylate

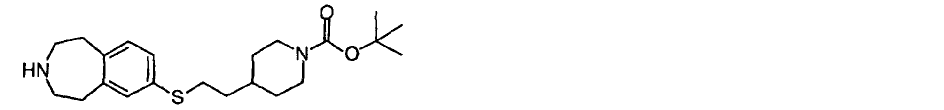

Water (10 ml) and an aqueous saturated solution of potassium carbonate (10 ml) were added to a solution of t-butyl 4-[2-[[3-(trifluoroacetyl)-2,3,4,5-tetrahydro-1H-3-benzazepine-7-yl]sulfanyl]ethyl]-1-piperidinecarboxylate (1.70 g, 3.49 mmol) obtained in Reference Example 20) in methanol (20 ml), and the mixture was stirred at room temperature for 2 hours. After methanol was distilled off under reduced pressure, the residue was dissolved in water-ethyl acetate, and extracted twice with ethyl acetate. The extract was washed with an aqueous saturated solution of sodium chloride, dried with anhydrous potassium carbonate, and the solvent was distilled off under reduced pressure to obtain the title compound (1.50 g) as a colorless oil.
¹H NMR (CDCl₃) δ 1.00-1.20 (2H, m), 1.45 (9H, s), 1.50-1.75 (5H, m), 2.00-2.20 (1H, br), 2.55-2.80 (2H, m), 2.80-3.00 (10H, m), 3.95-4.15 (2H, m), 7.00-7.10 (3H, m).

### Reference Example 22)

t-Butyl 4-[2-[[3-(phenylmethyl)-2,3,4,5-tetrahydro-1H-3-benzazepine-7-yl]sulfanyl]ethyl]-1-piperidinylcarboxylate

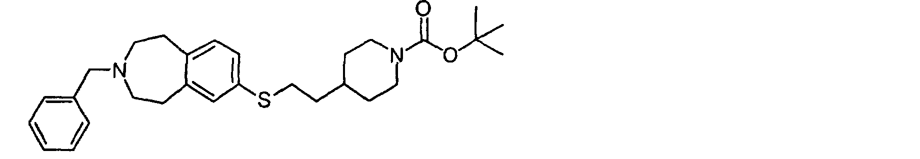

Benzyl bromide (0.503 ml, 4.23 mmol) was added dropwise to a suspension of t-butyl 4-[2-[(2,3,4,5-tetrahydro-1H-3-benzazepine-7-yl)sulfanyl]ethyl]-1-piperidinecarboxylate (1.50 g, 3.84 mmol) and potassium carbonate (2.5 g) in ethanol (50 ml) at room temperature. After the mixture was stirred at room temperature for 10 hours and the solvent was distilled off, the residue was dissolved in water-ethyl acetate and extracted with ethyl acetate. After the extract was washed with an aqueous saturated solution of sodium chloride and dried with anhydrous magnesium sulfate, the solvent was distilled off under reduced pressure. The resulting residue was purified by silica gel column chromatography (developing solvent; hexane-ethyl acetate=3:1) to obtain the title compound (1.52 g) as a colorless oil.
¹H NMR (CDCl₃) δ 1.00-1.20 (2H, m), 1.45 (9H, s), 1.50-1.80 (5H, m), 2.50-2.80 (6H, m), 2.80-3.00 (6H, m), 3.63 (2H, s), 3.95-4.15 (2H, m), 6.95-7.10 (3H, m), 7.20-7.40 (5H, m).

### Reference Example 23)

3-(Phenylmethyl)-7-[[2-(4-piperidinyl)ethyl]sulfanyl]-2,3,4,5-tetrahydro-1H-3-benzazepine dihydrochloride

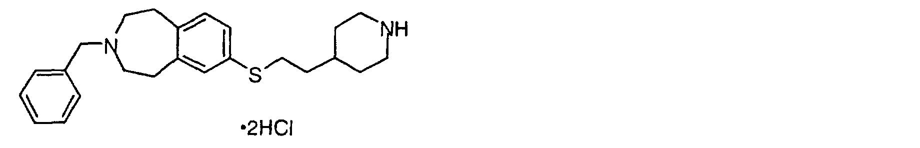

4N hydrogen chloride (ethyl acetate solution, 30 ml) was added to a solution of t-butyl 4-[2-[[3-(phenylmethyl)-2,3,4,5-tetrahydro-1H-3-benzazepine-7-yl]sulfanyl]ethyl]-1-piperidinecarboxylate (1.52 g, 3.16 mmol) obtained in Reference Example 22) in ethanol (30 ml), and the mixture was stirred at room temperature for 1 hour. After the solvent was distilled off under reduced pressure, the residue was made alkaline with an aqueous potassium carbonate, then dissolved in water-ethyl acetate and extracted twice with ethyl acetate. After the extract was washed with an aqueous saturated solution of sodium chloride and dried with anhydrous carbonate, the solvent was distilled off under reduced pressure to obtain a free base of the title compound (1.21 g) as a colorless oil. ¹H NMR (CDCl₃) δ 0.95-1.20 (2H, m), 1.40-1.75 (5H, m), 1.80-1.90 (1H, br), 2.45-2.70 (6H, m), 2.80-3.10 (8H, m), 3.62 (2H, s), 6.95-7.10 (3H, m), 7.20-7.40 (5H, m).

A solution of the above free base (240 mg) in ethanol was treated with 2 equivalents of hydrogen chloride (ethanol solution) to obtain the title compound (240 mg) as colorless crystals having a melting point of 246°C (dec.).
Elemental analysis for C₂₄H₃₂N₂S·2HCl·0.5H₂O
Calcd.: C, 62.32; H, 7.63; N, 6.06
Found: C, 62.83; H, 7.65; N, 6.44

### Reference Elemental 24)

t-Butyl 4-[2-[[3-(trifluoroacetyl)-2,3,4,5-tetrahydro-1H-3-benzazepine-7-yl]sulfinyl]ethyl]-1-piperidinecarboxylate

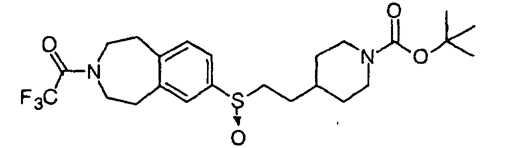

m-Chloroperbenzoic acid (683 mg, 3.95 mmol) was added portionwise to a solution of t-butyl 4-[2-[[3-(trifluoroacetyl)-2,3,4,5-tetrahydro-1H-3-benzazepine-7-yl]sulfanyl]ethyl]-1-piperidinecarboxylate (1.70 g, 3.49 mmol) in chloroform (30 ml) at room temperature. After the mixture was stirred at room temperature for 30 minutes, the mixture was dissolved in ethyl acetate and extracted with ethyl acetate. The extract was washed successively with an aqueous solution of sodium thiosulfate, an aqueous solution of sodium bicarbonate, and an aqueous saturated solution of sodium chloride, dried with anhydrous magnesium sulfate and the solvent was distilled off under reduced pressure. The resulting residue was purified by silica gel column chromatography (developing solvent; hexane-ethyl acetate=1:4) to obtain the title compound (1.71 g) as a colorless oil.
¹H NMR (CDCl₃) δ 1.00-1.20 (2H, m), 1.44 (9H, s), 1.50-1.80 (5H, m), 2.55-2.90 (4H, m), 3.00-3.15 (4H, m), 3.65-3.90 (4H, m), 4.00-4.15 (2H, m), 7.25-7.45 (3H, m).

### Reference Example 25)

t-Butyl 4-[2-[(2,3,4,5-tetrahydro-1H-3-benzazepine-7-yl)sulfinyl]ethyl]-1-piperidinecarboxylate

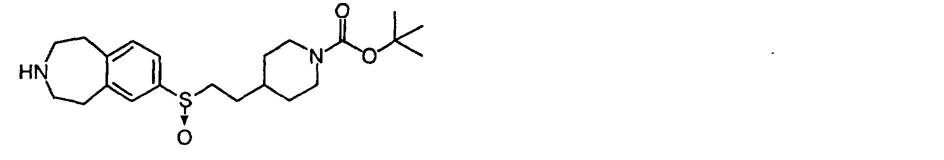

According to the same procedures as those of Reference Example 21) using t-butyl 4-[2-[[3-(trifluoroacetyl)-2,3,4,5-tetrahydro-1H-3-benzazepine-7-yl]sulfinyl]ethyl]-1-piperidinecarboxylate (1.70 g, 3.38 mmol) obtained in Reference Example 24), the title compound (1.50 g) was obtained as a colorless oil.
¹H NMR (CDCl₃) δ 1.00-1.20 (2H, m), 1.44 (9H, s), 1.50-1.80 (5H, m), 2.00-2.10 (1H, br), 2.55-3.10 (12H, m), 4.00-4.15 (2H, m), 7.20-7.40 (3H, m).

### Reference Example 26)

t-Butyl 4-[2-[[3-(phenylmethyl)-2,3,4,5-tetrahydro-1H-3-benzazepine-7-yl]sulfinyl]ethyl]-1-piperidinecarboxylate

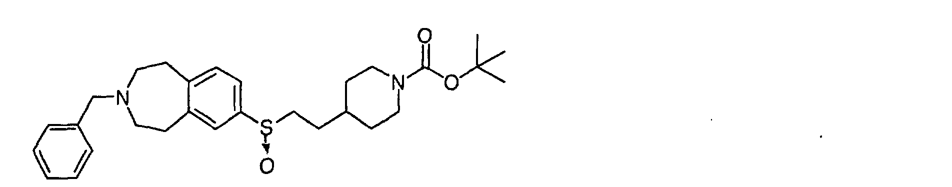

According to the same procedures as those of Reference Example 22) using t-butyl 4-[2-[(2,3,4,5-tetrahydro-1H-3-benzazepine-7-yl)sulfinyl]ethyl]-1-piperidinecarboxylate (1.50 g, 3.69 mmol) obtained in Reference Example 25) and benzyl bromide (0.483 ml, 4.06 mmol), the title compound (1.39 g) was obtained as a colorless oil.
¹H NMR (CDCl₃) δ 0.95-1.20 (2H, m), 1.44 (9H, s), 1.50-1.80 (5H, m), 2.50-2.85 (8H, m), 2.90-3.05 (4H, m), 3.63 (2H, s), 3.95-4.15 (2H, m), 7.15-7.40 (8H, m).

### Reference Example 27)

3-(Phenylmethyl)-7-[[2-(4-piperidinyl)ethyl]sulfinyl]-2,3,4,5-tetrahydro-1H-3-benzazepine dihydrochloride

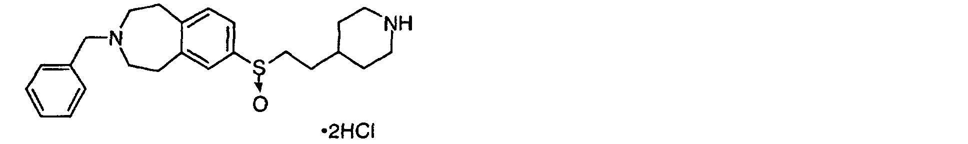

According to the same procedures as those of Reference Example 23) using t-butyl 4-[2-[[3-(phenylmethyl)-2,3,4,5-tetrahydro-1H-3-benzazepine-7-yl]sulfinyl]ethyl]-1-piperidinecarboxylate (1.39 g, 2.77 mmol) obtained in Reference Example 26), a free base of the title compound (1.03 g) was obtained as a colorless oil. ¹H NMR (CDCl₃) δ 1.00-1.20 (2H, m), 1.40-1.75 (5H, m), 1.80-2.00 (1H, br), 2.45-2.70 (6H, m), 2.75-3.20 (8H, m), 3.63 (2H, s), 6.95-7.10 (3H, m), 7.15-7.40 (5H, m).

A solution of the above free base (200 mg) in ethanol was treated with 2 equivalents of hydrogen chloride (ethanol solution) to obtain the title compound (224 mg) as colorless amorphous powders.
Elemental analysis for C₂₄H₃₂N₂OS·2HCl·H₂O
Calcd.: C, 59.13; H, 7.44; N, 5.75
Found: C, 59.05; H, 7.46; N, 5.34

### Reference Examples 28)

t-Butyl 4-[2-[[3-(trifluoroacetyl)-2,3,4,5-tetrahydro-1H-3-benzazepine-7-yl]sulfonyl]ethyl]-1-piperidinecarboxylate

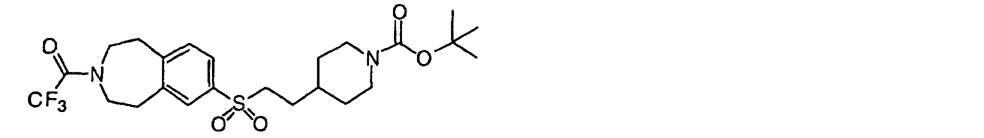

m-Chloroperbenzoic acid (1.24 g, 7.18 mmol) was added to a solution of t-butyl 4-[2-[[3-(trifluoroacetyl)-2,3,4,5-tetrahydro-1H-3-benzazepine-7-yl]sulfanyl]ethyl]-1-piperidinecarboxylate (1.40 g, 2.88 mmol) in chloroform (10 ml) portionwise at room temperature. The mixture was stirred at room temperature for 2 hours, which was dissolved in water-ethyl acetate and extracted with ethyl acetate. The extract was washed successively with an aqueous solution of sodium thiosulfate, an aqueous solution of sodium bicarbonate and an aqueous saturated solution of sodium chloride, dried with anhydrous magnesium sulfate, and the solvent was distilled off under reduced pressure. The resulting residue was purified by silica gel column chromatography (developing solvent: hexane-ethyl acetate=1:1) to obtain the title compound (1.49 g) as a colorless oil.
¹H NMR (CDCl₃) δ 1.00-1.20 (2H, m), 1.44 (9H, s), 1.50-1.80 (5H, m), 2.50-2.75 (2H, m), 3.05-3.20 (6H, m), 3.70-3.85 (4H, m), 4.00-4.15 (2H, m), 7.37 (1H, dd, J = 8.2, 5.6 Hz), 7.65-7.75 (2H, m).

### Reference Example 29)

t-Butyl 4-[2-[(2,3,4,5-tetrahydro-1H-3-benzazepine-7-yl)sulfonyl]ethyl]-1-piperidinecarboxylate

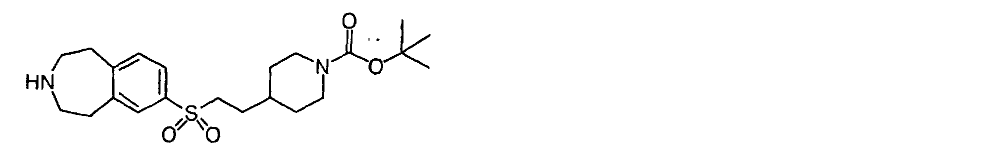

According to the same procedures as those of Reference Example 21) using t-butyl 4-[2-[[3-(trifluoroacetyl)-2,3,4,5-tetrahydro-1H-3-benzazepine-7-yl]sulfonyl]ethyl]-1-piperidinecarboxylate (1.53 g, 2.95 mmol) obtained in Reference Example 28), the title compound (1.25 g) was obtained as colorless crystals having a melting point of 102-103°C.
¹H NMR (CDCl₃) δ 0.95-1.20 (2H, m), 1.44 (9H, s), 1.50-1.75 (5H, m), 1.80-1.90 (1H, br), 2.55-2.75 (2H, m), 2.85-3.15 (10H, m), 4.00-4.15 (2H, m), 7.25-7.30 (1H, m), 7.60-7.65 (2H, m).
Elemental analysis for C₂₂H₃₄N₂O₄S
Calcd.: C, 62.53; H, 8.11; N, 6.63
Found: C, 62.63; H, 8.18; N, 6.45

### Reference Example 30)

t-Butyl 4-[2-[[3-(phenylmethyl)-2,3,4,5-tetrahydro-1H-3-benzazepine-7-yl]sulfonyl]ethyl]-1-pipridinecarboxylate

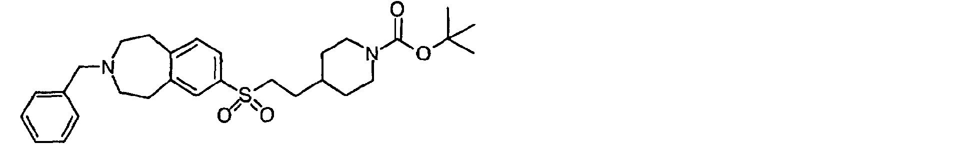

According to the same procedures as those of Reference Example 22) using t-butyl 4-[2-[(2,3,4,5-tetrahydro-1H-3-benzazepine-7-yl)sulfonyl)ethyl]-1-piperidinecarboxylate (1.43 g, 3.38 mmol) obtained in Reference Example 29) and benzyl chloride (0.443 ml, 3.72 mmol), the title compound (1.35 g) was obtained as a colorless oil.
¹H NMR (CDCl₃) δ 0.95-1.20 (2H, m), 1.44 (9H, s), 1.50-1.80 (5H, m), 2.55-2.75 (6H, m), 2.95-3.15 (6H, m), 3.64 (2H, s). 3.95-4.15 (2H, m), 7.20-7.40 (6H, m), 7.55-7.70 (2H, m).

### Reference Example 31)

3-(Phenylmethyl)-7-[[2-(4-piperidinyl)ethyl]sulfonyl]-2,3,4,5-tetrahydro-1H-3-benzazepine

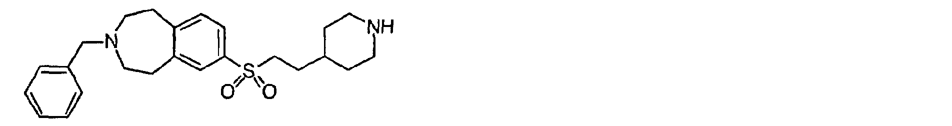

According to the same procedures as those of Reference Example 23) using t-butyl 4-[2-[[3-(phenylmethyl)-2,3,4,5-tetrahydro-1H-3-benzazepine-7-yl]sulfonyl]ethyl]-1-pipridinecarboxylate (1.35 g, 2.63 mmol) obtained in Reference Example 30), the title compound (1.08 g) was obtained as a colorless oil.
¹H NMR (CDCl₃) δ 0.95-1.20 (2H, m), 1.35-2.00 (6H, m), 2.40-2.75 (6H, m), 2.90-3.20 (8H, m), 3.64 (2H, s), 7.20-7.40 (6H, m), 7.55-7.70 (2H, m).

### Reference Example 32)

8-Nitro-1,3,4,5-tetrahydro-2H-2-benzazepine-2-carbaldehyde

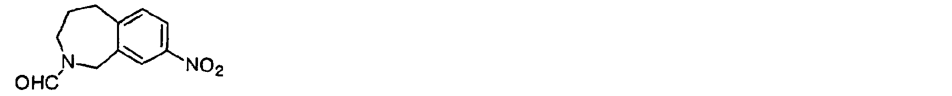

Potassium nitrate (6.4 g, 63.3 mmol) was added to a solution of 1,3,4,5-tetrahydro-2H-2-benzazepine-2-carbaldehyde (10.0 g, 57.1 mmol) in concentrated sulfuric acid (100 ml) portionwise at room temperature, and the mixture was stirred for 3 hours. The reaction mixture was added to ice-sodium bicarbonate to make the aqueous layer alkaline, which was extracted with ethyl acetate. The extract was washed successively with an aqueous solution of sodium bicarbonate and an aqueous saturated solution of sodium chloride, dried with anhydrous magnesium sulfate, and the solvent was distilled off under reduced pressure to obtain the crude product of the title compound (7.36 g) as a pale yellow solid.
¹H NMR (CDCl₃) δ 1.80-1.95 (2H, m), 3.11 (2H, t-like, J = 5.4 Hz), 3.71 (2H, t-like, J = 5.4 Hz), 4.62 (2H, s), 7.31 (1H, d, J = 8.4 Hz), 8.00-8.10 (2H, m), 8.23 (1H, s).

### Reference Example 33)

8-Nitro-2,3,4,5-tetrahydro-1H-2-benzazepine

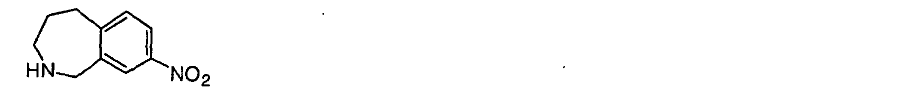

Concentrated sulfuric acid (70 ml) was added to a solution of 8-nitro-1,3,4,5-tetrahydro-2H-2-benzazepine-2-carbaldehyde (3.00 g, 13.6 mmol) obtained in Reference Example 32) in methanol (30 ml) and the mixture was heated at reflux for 2 hours. After methanol was distilled off under reduced pressure, the residue was made alkaline with sodium bicarbonate, and extracted with ethyl acetate. After the extract was washed with an aqueous saturated solution of sodium chloride and dried with magnesium sulfate, the solvent was distilled off under reduced pressure to obtain the crude product of the title compound (2.22 g) as a yellow oil. ¹H NMR (CDCl₃) δ 1.50-1.90 (3H, m), 3.04 (2H, t-like, J = 5.4 Hz), 3.24 (2H, t-like, J = 5.4 Hz), 4.02 (2H, s), 7.30 (1H, d, J = 9.2 Hz), 7.95-8.05 (2H, m).

### Reference Example 34)

8-Nitro-2-(phenylmethyl)-2,3,4,5-tetrahydro-1H-2-benzazepine

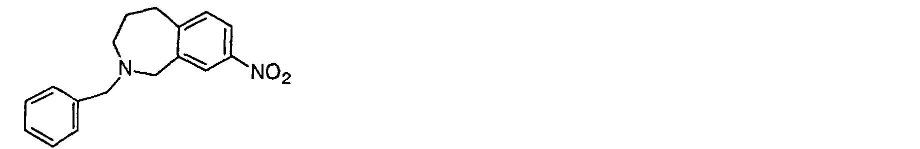

According to the same procedures as those of Reference Example 22) using the crude product of 8-nitro-2,3,4,5-tetrahydro-1H-2-benzazepine (2.22 g, 11.5 mmol) obtained in Reference Example 33) and benzyl bromide (1.51 ml, 13.9 mmol), the title compound (520 mg) was obtained as a yellow solid.
¹H NMR (CDCl₃) δ 1.70-1.90 (2H, m), 3.02 (2H, t-like, J = 5.4 Hz), 3.13 (2H, t-like, J = 5.4 Hz), 3.55 (2H, s), 3.92 (2H, s), 7.20-7.40 (6H, m), 7.79 (1H, d, J = 2.6 Hz), 8.02 (1H, dd, J = 8.0, 2.6 Hz).

### Reference Example 35)

8-Amino-2-(phenylmethyl)-2,3,4,5-tetrahydro-1H-2-benzazepine

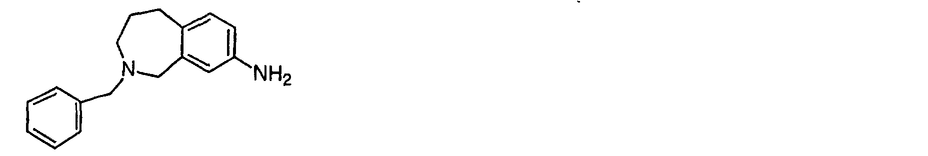

Zinc powders (3 g, 47 mmol) were added to a solution of 8-nitro-2-(phenylmethyl)-2,3,4,5-tetrahydro-1H-2-benzazepine (200 mg, 0.79 mmol) obtained in Reference Example 34) in acetic acid (10 ml), and the mixture was heated at reflux for 30 minutes. After the solids were removed by filtration and the filtrate was concentrated under reduced pressure, the residue was made alkaline by addition of an aqueous solution of potassium carbonate and extracted with ethyl acetate. The extract was washed successively with an aqueous solution of potassium carbonate and an aqueous saturated solution of sodium chloride, dried with anhydrous magnesium sulfate, and the solvent was distilled off under reduced pressure. The resulting residue was purified by silica gel column chromatography (developing solvent; ethyl acetate) to obtain the title compound (138 mg) as a colorless oil. ¹H NMR (CDCl₃) δ 1.60-1.80 (2H, m), 2.80 (2H, t-like, J = 5.4 Hz), 3.07 (2H, t-like, J = 5.4 Hz), 3.20-3.60 (2H, br), 3.53 (2H, s), 3.78 (2H, s), 6.31 (1H, d, J = 2.6 Hz), 6.47 (1H, dd, J=8.0,2.6Hz), 6.92 (1H, d, J = 8.0 Hz), 7.20-7.40 (5H, m).

### Reference Example 36)

8-Acetyl-1,3,4,5-tetrahydro-2H-2-benzazepine-2-carbaldehyde

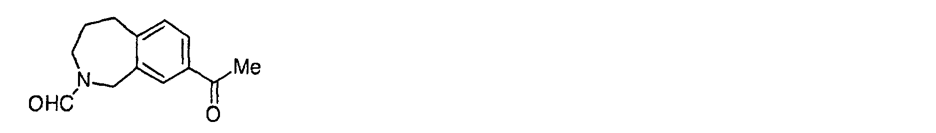

Aluminum chloride (13.3 g, 99.7 mmol) was added to a solution of 1,3,4,5-tetrahydro-2H-2-benzazepine-2-carbaldehyde (5.00 g, 28.5 mmol) and acetyl chloride (2.23 ml, 31.4 mmol) in dichloroethane (25 ml) in portionwise at room temperature, and the mixture was stirred for 12 hours. The reaction mixture was added to ice, and extracted with ethyl acetate. After the extract was washed with an aqueous saturated solution of sodium chloride and dried with anhydrous magnesium sulfate, the solvent was distilled off under reduced pressure. The resulting residue was purified by silica gel column chromatography (developing solvent; ethyl acetate) to obtain the title compound (3.0 g) as a colorless solid.
¹H NMR (CDCl₃) δ 1.80-1.95 (2H, m), 2.59 (3H, s), 3.00-3.10 (2H, m), 3.68 (2H, t-like, J = 5.6 Hz), 4.61 (2H, s), 7.20-7.30 (1H, m), 7.81 (1H, dd, J = 7.8, 2.0 Hz), 7.94 (1H, d, J = 2.0 Hz), 8.03 (1H, s).

### Reference Example 37)

2-Formyl-2,3,4,5-tetrahyro-1H-2-benzazepine-8-carboxylic acid

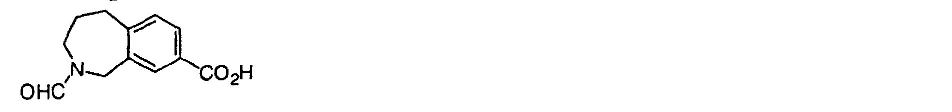

An aqueous solution of sodium hydroxide (4.8 g/ 70 ml) was added to a solution of 8-acetyl-1,3,4,5-tetrahydro-2H-2-benzazepine-2-carbaldehyde (5.00 g, 28.5 mmol) obtained in Reference Example 36) in 1,4-dioxane (50 ml). Then, bromine (2.14 ml, 41.6 mmol) was added dropwise at -15°C, and the mixture was stirred at 0°C for 30 minutes. After acetone (5 ml) was added and the mixture was stirred for 10 minutes, the mixture was concentrated under reduced pressure and washed with ethyl acetate. The aqueous layer was made acidic with 5N hydrochloric acid, the precipitated solids were filtered, then washed successively with water and ethyl ether and air-dried to obtain the title compound (1.95 g) as a colorless solid.
¹H NMR (CDCl₃) δ 1.80-1.95 (2H, m), 3.00-3.10 (2H, m), 3.69 (2H×3/5, t-like, J = 5.4 Hz), 3.85 (2H×2/5, t-like, J = 5.4 Hz), 4.53 (2H×2/5, s), 4.63 (2H×3/5, s), 7.20-7.30 (1H, m), 7.85-8.20 (3H, m).

### Reference Example 38)

2-(Phenylmethyl)-2,3,4,5-tetrahydro-1H-2-benzazepine-8-carboxylic acid 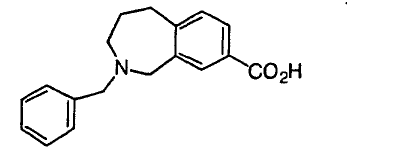
1) Concentrated hydrochloric acid (100 ml) was added to 8-acetyl-1,3,4,5-tetrahydro-2H-2-benzazepine-2-carbaldehyde (1.90 g, 8.67 mmol) obtained in Reference Example 36) and the mixture was stirred at 80°C for 2 hours. After cooling to room temperature, the mixture was concentrated under reduced pressure to obtain 2,3,4,5-tetrahydro-1H-2-benzazepine-8-carboxylic acid hydrochloride (1.81 g) as a colorless solid.
2) According to the same procedures as those of Reference Example 22) using 2,3,4,5-tetrahydro-1H-2-benzazepine-8-carboxylic acid hydrochloride (1.50 g, 6.59 mmol) obtained in 1) and benzyl bromide (0.823 ml, 6.92 mmol), 2-(phenylmethyl)-2,3,4,5-tetrahydro-1H-2-benzazepine-8-carboxylic acid benzyl ester (1.24 g) was obtained as a colorless oil.
   ¹H NMR (CDCl₃) δ 1.80-1.95 (2H, m), 2.85-3.00 (2H, m), 3.15 (2H, t-like, J = 5.4 Hz), 3.53 (2H, s), 3.93 (2H, s), 5.35 (2H, s), 7.20-7.90 (13H, m).
3) A 1N aqueous solution of sodium hydroxide (50 ml) was added to a solution of 2-(phenylmethyl)-2,3,4,5-tetrahydro-1H-2-benzazepine -8-carboxylic acid benzyl ester (1.23 g, 3.31 mmol) obtained in 2) in ethanol (50 ml), and the mixture was heated at reflux for 1 hour. After ethanol was concentrated under reduced pressure, the residue was adjusted to pH about 5 using 2N hydrochloric acid, which was extracted with ethyl acetate three times. The extract was washed with an aqueous saturated solution of sodium chloride, dried with anhydrous magnesium sulfate, the solvent was distilled off under reduced pressure to obtain the title compound (804 mg) as a colorless oil.
   ¹H NMR (CDCl₃) δ 1.80-2.10 (2H, m), 2.80-3.10 (2H, m), 3.30-3.60 (2H, m), 4.05 (2H, s), 4.70 (2H, s), 7.10-8.10 (8H, m).

### Reference Example 39)

2-[(4-Fluorophenyl)methyl]-8-[2-(4-piperidinyl)ehtoxy]-2,3,4,5-tetrahydro-1H-benzazepine dihydrochloride

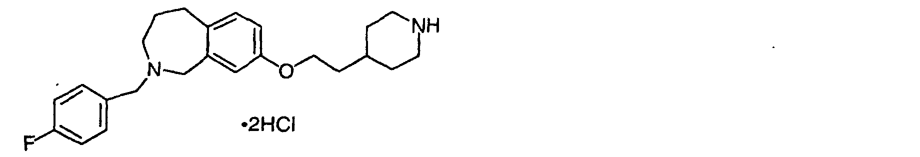

According to the same procedures as those of Reference Example 1), Reference Example 7) and Reference Example 8) successively using 4-fluorobenzyl bromide and 8-methoxy-2,3,4,5-tetrahydro-1H-2-benzazepine, the title compound was obtained as colorless amorphous powders. ¹H NMR (CDCl₃, free base) δ 1.05-1.30 (2H, m), 1.50-1.90 (6H, m), 2.50-2.70 (2H, m), 2.85 (2H, t-like, J = 5.4 Hz), 3.00-3.15 (4H, m), 3.49 (2H, s), 3.70-3.85 (4H, m), 3.94 (2H, t, J = 6.2 Hz), 6.48 (1H, d, J = 2.6 Hz), 6.67 (1H, dd, J = 8.2, 2.6 Hz), 6.90-7.10 (3H, m), 7.20-7.30 (2H, m).
Elemental analysis for C₂₄H₃₁FN₂O·2HCl·0.5H₂O
Calcd.: C, 62.07; H, 7.38;N, 6.03
Found: C, 61.99; H, 7.70; N, 5.78

### Reference Example 40)

2-[(4-Fluorophenyl)methyl]-7-[2-(4-piperidinyl)ethoxy]-2,3,4,5-tetrahydro-1H-2-benzazepine dihydrochloride

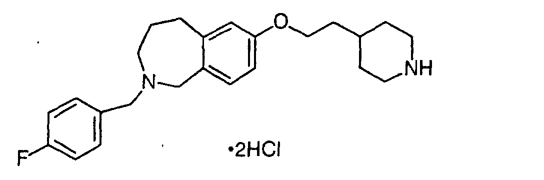

According to the same procedures as those of Reference Example 1), Reference Example 7) and Reference Example 8) successively using 4-fluorobenzyl bromide and 7-methoxy-2,3,4,5-tetrahydro-1H-2-benzazepine, the title compound was obtained as colorless amorphous powders.
¹H NMR (CDCl₃, free base) δ 1.05-1.30 (2H, m), 1.50-1.90 (6H, m), 2.50-2.70 (2H, m), 2.86 (2H, t-like, J = 5.4 Hz), 3.00-3.15 (4H, m), 3.46 (2H, s), 3.65-3.80 (4H, m), 3.99 (2H, t, J = 6.2 Hz), 6.59 (1H, dd, J = 8.0, 2.6 Hz), 6.72 (1H, d, J = 2.6 Hz), 6.81 (1H, d, J = 8.0 Hz), 6.80-7.05 (2H, m), 7.40-7.50 (2H, m).
Elemental analysis for C₂₄H₃₁FN₂O·2HCl·1.5H₂O
Calcd.: C, 59.75; H, 7.52; N, 5.81
Found: C, 59.82; H, 7.57; N, 5.32

### Reference Example 41)

2-[[2-(Trifluoromethyl)phenyl]methyl]-7-[2-(4-piperidinyl)ethoxy]-2,3,4,5-tetrahydro-1H-2-benzazepine

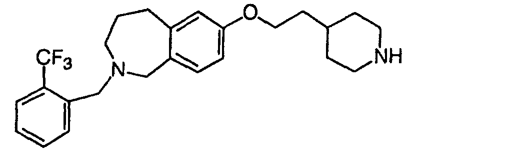

According to the same procedures as those of Reference Example 1), Reference Example 7) and Reference Example 8) successively using 2-(trifluoromethyl)benzyl bromide and 7-methoxy-2,3,4,5-tetrahydro-1H-2-benzazepine, the title compound was obtained as a colorless oil.
¹H NMR (CDCl₃) δ 1.05-1.30 (2H, m), 1.65-1.85 (6H, m), 2.50-3.20 (10H, m), 3.72 (2H, s), 3.75 (2H, s), 3.99 (2H, t, J = 6.2 Hz), 6.59 (1H, dd, J = 8.2, 2.6 Hz), 6.72 (1H, d, J = 2.6 Hz), 6.83 (1H, d, J = 8.2 Hz), 7.31 (1H, t, J = 7.6 Hz), 7.50 (1H, t, J = 7.6 Hz), 7.61 (1H, d, J = 7.8 Hz), 7.81 (1H, d, J = 7.8 Hz).

### Reference Example 42)

2-[(4-Fluorophenyl)methyl]-8-[3-(4-piperidinyl)propoxy]-2,3,4,5-tetrahydro-1H-2-benzazepine

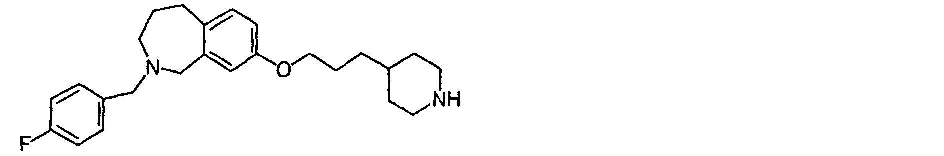

According to the same procedures as those of Reference Example 1), Reference Example 7) and Reference Example 8) successively using 4-fluorobenzyl bromide and 8-methoxy-2,3,4,5-tetrahydro-1H-2-benzazepine, the title compound was obtained as colorless crystals having a melting point of 88-89°C.
Elemental analysis for C₂₄H₃₃FN₂O·0.5H₂O
Calcd.: C, 74.04; H, 8.45; N, 6.91
Found: C, 73.82; H, 8.10; N, 6.74

### Reference Elemental 43

t-Butyl 4-[[2-(phenylmethyl)-2,3,4,5-tetrahydro-1H-2-benzazepine-7-yl]oxymethyl]-1-piperidinecarboxylate

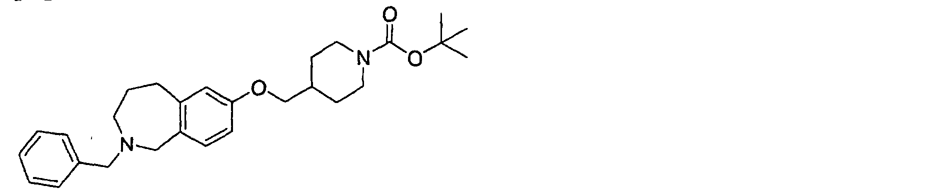

According to the same procedures as those of Reference Example 1) and Reference Example 7) successively using 7-methoxy-2,3,4,5-tetrahydro-1H-2-benzazepine, the title compound was obtained as colorless crystals having a melting point of 103-104°C.
¹H NMR (CDCl₃) δ 1.10-2.10 (7H, m), 1.47 (9H, s), 2.65-2.90 (4H, m), 3.09 (2H, t-like, J = 5.2 Hz), 3.51 (2H, s), 3.79 (2H, d, J = 7.6 Hz), 3.81 (2H, s), 4.05-4.25 (2H, br), 6.59 (1H, dd, J = 8.2, 2.6 Hz), 6.71 (1H, d, J = 2.6 Hz), 6.84 (1H, d, J = 8.2 Hz), 7.20-7.40 (5H, m).
Elemental analysis for C₂₈H₃₈N₂O₃
Calcd.: C, 74.63; H, 8.50; N, 6.22
Found: C, 74.56; H, 8.40; N, 6.43

### Reference Example 44

2-(Phenylmethyl)-7-[(4-piperidinyl)methoxy]-2,3,4,5-tetrahydro-1H-2-benzazepine dihydrochloride

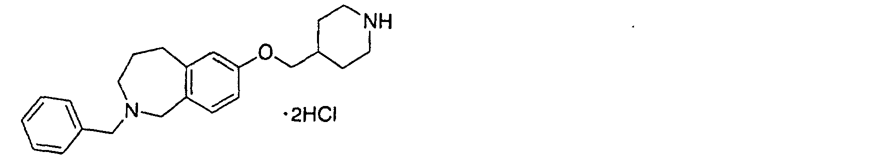

According to the same procedures as those of Reference Example 8) using t-butyl 4-[[2-(phenylmethyl)-2,3,4,5-tetrahydro-1H-2-benzazepine-7-yl]oxymethyl]-1-piperidinecarboxylate obtained in Reference Example 43), the title compound was obtained as colorless amorphous powders.
¹H NMR (CDCl₃, free base) δ 1.15-1.40 (2H, m), 1.65-2.00 (6H, m), 2.64 (2H, dt, J = 8.2, 2.6 Hz), 2.80-2.90 (2H, m), 3.00-3.20 (4H, m), 3.50 (2H, s), 3.76 (2H, d, J = 6.2 Hz), 3.81 (2H, s), 6.59 (1H, dd, J = 8.2, 2.6 Hz), 6.71 (1H, d, J = 2.6 Hz), 6.83 (1H, d, J = 8.2 Hz), 7.20-7.40 (5H, m).

### Reference Example 45

t-Butyl 4-[[2-(phenylmethyl)-2,3,4,5-tetrahydro-1H-2-benzazepine-8-yl]oxymethyl]-1-piperidinecarboxylate

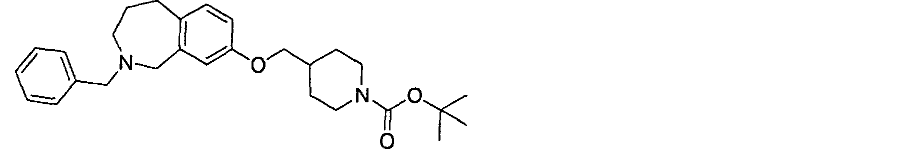

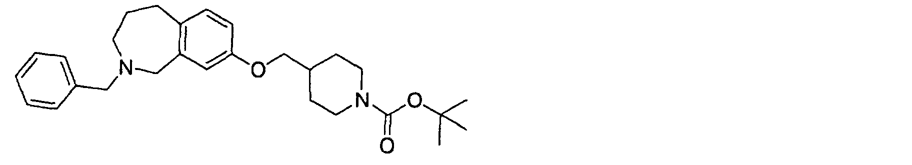

According to the same procedures as those of Reference Example 1) and Reference Example 7) successively using 8-methoxy-2,3,4,5-tetrahydro-1H-2-benzazepine, the title compound was obtained as colorless crystals having a melting point of 116-118°C.
¹H NMR (CDCl₃) δ 1.10-1.40 (2H, m), 1.47 (9H, s), 1.50-2.00 (5H, m), 2.65-2.90 (4H, m), 3.09 (2H, t-like, J = 5.2 Hz), 3.54 (2H, s), 3.73 (2H, d, J = 6.2 Hz), 3.82 (2H, s), 4.05-4.25 (2H, br), 6.49 (1H, d, J = 2.4 Hz), 6.65 (1H, dd, J = 8.2, 2.4 Hz), 7.04 (1H, d, J = 8.2 Hz), 7.20-7.40 (5H, m).
Elemental analysis for C₂₈H₃₈N₂O₃
Calcd.: C, 74.63; H, 8.50; N, 6.22
Found: C, 74.44; H, 8.55; N, 6.10

### Reference Example 46

2-(Phenylmethyl)-8-[(4-piperidinyl)methoxy]-2,3,4,5-tetrahydro-1H-2-benzazepine dihydrochloride

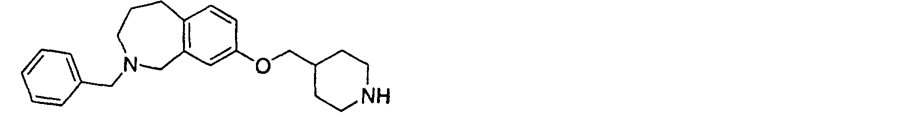

According to the same procedures as those of Reference Example 8) using t-butyl 4-[[2-(phenylmethyl)-2,3,4,5-tetrahydro-1H-2-benzazepine-8-yl]oxymethyl]-1-piperidinecarboxylate obtained in Reference Example 45), the title compound was obtained as colorless crystals having a melting point of 103-105°C.
¹H NMR (CDCl₃) δ 1.15-1.40 (2H, m), 1.65-2.00 (6H, m), 2.65 (2H, dt, J = 8.2, 2.6 Hz), 2.80-2.90 (2H, m), 3.00-3.20 (4H, m), 3.54 (2H, s), 3.72 (2H, d, J = 5.8 Hz), 3.83 (2H, s), 6.51 (1H, d, J = 2.6 Hz), 6.67 (1H, dd, J = 8.2, 2.6 Hz), 7.04 (1H, d, J = 8.2 Hz), 7.20-7.40 (5H, m).
Elemental analysis for C₂₃H₃₀N₂O·2HCl·H₂O
Calcd.: C, 62.58; H, 7.76; N, 6.35
Found: C, 62.64; H, 8.03; N, 6.07

### Reference Example 47

t-Butyl 4-[3-[[3-(trifluoroacetyl)-2,3,4,5-tetrahydro-1H-3-benzazepine-7-yl)sulfanyl]propyl]-1-piperidinecarboxylate

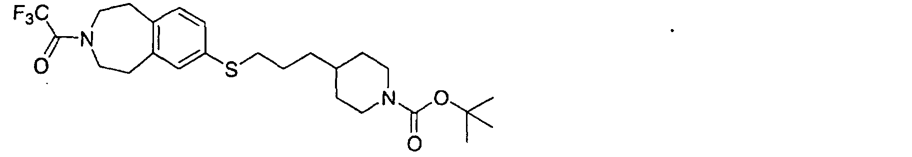

According to the same procedures as those of Reference Example 20) using 2,2,2-trifluoro-1-(7-sulfanyl-2,3,4,5-tetrahydro-1H-3-benzazepine-3-yl)-1-ethanone obtained in Reference Example 19), the title compound was obtained as colorless crystals having a melting point of 104-105°C.
¹H NMR (CDCl₃) δ 1.00-1.75 (9H, m), 1.45 (9H, s), 2.55-2.75 (2H, m), 2.85-3.00 (6H, m), 3.65-3.85 (4H, m), 4.00-4.15 (2H, m), 7.00-7.20 (2H, m), 7.27 (1H, s).

### Reference Example 48

t-Butyl 4-[3-[[3-(trifluoroacetyl)-2,3,4,5-tetrahydro-1H-3-benzazepine-7-yl]sulfonyl]propyl]-1-piperidinecarboxylate

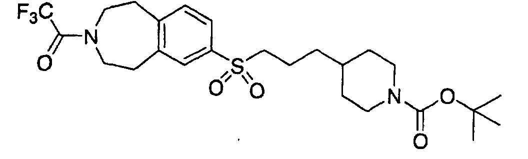

According to the same procedures as those of Reference Example 28) using t-butyl 4-[3-[[3-(trifluoroacetyl)-2,3,4,5-tetrahydro-lH-3-benzazepine-7-yl]sulfanyl]propyl]-1-piperidinecarboxylate obtained in Reference Example 47), the title compound was obtained as a colorless oil.
¹H NMR (CDCl₃) δ 0.90-1.85 (9H, m), 1.45 (9H, s), 2.55-2.75 (2H, m), 3.00-3.20 (6H, m), 3.65-3.85 (4H, m), 3.95-4.15 (2H, m), 7.30-7.45 (1H, m), 7.65-7.80 (2H, m).

### Reference Example 49

t-Butyl 4-[3-[(2,3,4,5-tetrahydro-1H-3-benzazepine-7-yl)sulfonyl]propyl]-1-piperidinecarboxylate

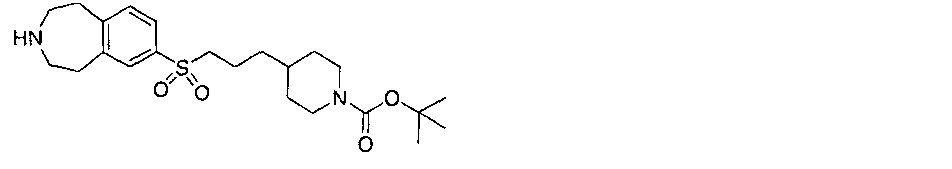

According to the same procedures as those of Reference Example 21) using t-butyl 4-[3-[[3-(trifluoroacetyl)-2,3,4,5-tetrahydro-1H-3-benzazepine-7-yl]sulfonyl]propyl]-1-piperidinecarboxylate obtained in Reference Example 48), the title compound was obtained as a colorless oil.
¹H NMR (CDCl₃) δ 0.90-1.85 (9H, m), 1.44 (9H, s), 2.10-2.30 (1H, br), 2.50-2.75 (2H, m), 2.90-3.10 (10H, m), 3.95-4.20 (2H, m), 7.20-7.35 (1H, m), 7.60-7.65 (2H, m).

### Reference Example 50

t-Butyl 4-[3-[[3-(phenylmethyl)-(2,3,4,5-tetrahydro-1H-3-benzazepine-7-yl)sulfonyl]propyl]-1-piperidinecarboxylate

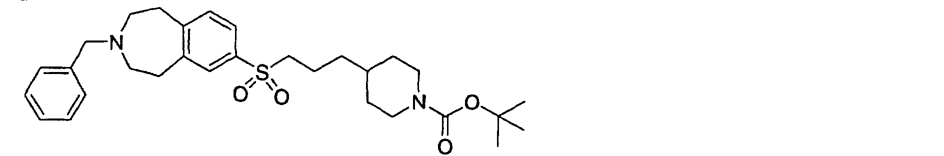

According to the same procedures as those of Reference Example 22) using t-butyl 4-[3-[(2,3,4,5-tetrahydro-1H-3-benzazepine-7-yl)sulfonyl]propyl]-1-piperidinecarboxylate obtained in Reference Example 49), the title compound was obtained as a colorless oil.
¹H NMR (CDCl₃) δ 0.90-1.85 (9H, m), 1.45 (9H, s), 2.50-2.75 (6H, m), 2.95-3.10 (6H, m), 3.64 (2H, s), 3.95-4.15 (2H, m), 7.20-7.40 (6H, m), 7.60-7.65 (2H, m).

### Reference Example 51

t-Butyl 4-[3-[[3-[(2-methylphenyl)methyl]-2,3,4,5-tetrahydro-1H-3-benzazepine-7-yl)sulfonyl]propyl]-1-piperidinecarboxylate

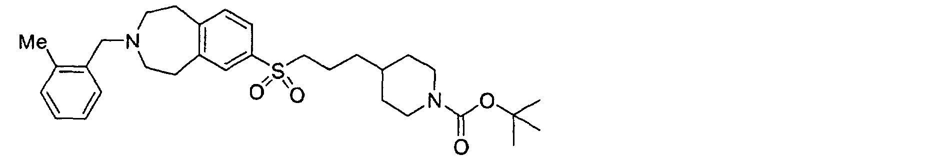

According to the same procedures as those of Reference Example 22) using t-butyl 4-[3-[(2,3,4,5-tetrahydro-1H-3-benzazepine-7-yl)sulfonyl]propyl]-1-piperidinecarboxylate obtained in Reference Example 49), the title compound was obtained as a colorless oil. ¹H NMR (CDCl₃) δ 0.90-2.00 (9H, m), 1.45 (9H, s), 2.39 (3H, s), 2.50-2.75 (6H, m), 2.90-3.10 (6H, m), 3.54 (2H, s), 3.95-4.15 (2H, m), 7.10-7.35 (5H, m), 7.60-7.65 (2H, m).

### Reference Example 52

3-(Phenylmethyl)-7-[[3-(4-piperidinyl)propyl]sulfonyl]-2,3,4,5-tetrahydro-1H-3-benzazepine

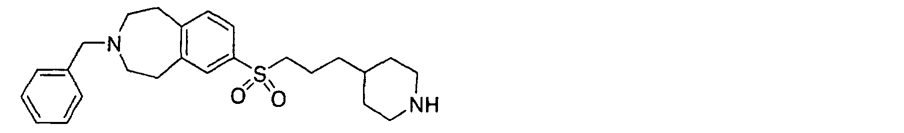

According to the same procedures as those of Reference Example 23) using t-butyl 4-[3-[[3-(phenylmethyl)-(2,3,4,5-tetrahydro-1H-3-benzazepine-7-yl)sulfonyl]propyl]-1-piperidinecarboxylate obtained in Reference Example 50), the title compound was obtained as a colorless oil.
¹H NMR (CDCl₃) δ 0.90-1.40 (5H, m), 1.50-1.95 (4H, m), 2.10-2.70 (1H, br), 2.53 (2H, dt, J = 12.2, 2.6 Hz), 2.55-2.70 (4H, m), 2.90-3.10 (8H, m), 3.64 (2H, s), 7.20-7.40 (6H, m), 7.60-7.65 (2H, m).

### Reference Example 53

3-[(2-Methylphenyl)methyl]-7-[[3-(4-piperidinyl)propyl]sulfonyl]-2,3,4,5-tetrahydro-1H-3-benzazepine

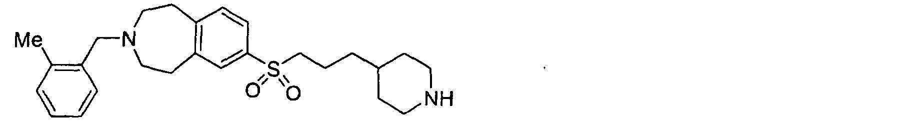

According to the same procedures as those of Reference Example 23) using t-butyl 4-[3-[[3-[(2-methylphenyl)methyl]-2,3,4,5-tetrahydro-1H-3-benzazepine-7-yl)sulfonyl]propyl]-1-piperidinecarboxylate obtained in Reference Example 51), the title compound was obtained as a colorless oil.
¹H NMR (CDCl₃) δ 0.90-1.40 (5H, m), 1.50-1.90 (4H, m), 1.90-2.05 (1H, br), 2.39 (3H, s), 2.45-2.75 (6H, m), 2.90-3.10 (8H, m), 3.54 (2H, s), 7.15-7.35 (5H, m), 7.60-7.65 (2H, m).

### Reference Example 54

t-Butyl 4-[2-[[2-(phenylmethyl)-2,3,4,5-tetrahydro-1H-2-benzazepine-8-yl]oxy]ethyl]-1-piperidinecarboxylate

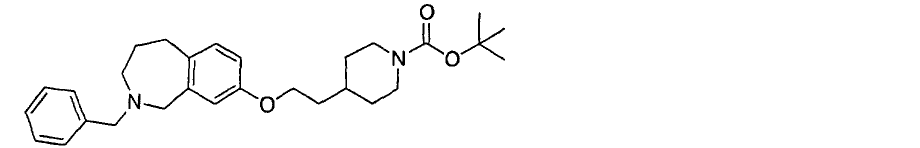

According to the same procedures as those of Reference Example 1) and Reference Example 7) successively using 8-methoxy-2,3,4,5-tetrahydro-1H-2-benzazepine, the title compound was obtained as a colorless oil.
¹H NMR (CDCl₃) δ 1.00-1.90 (9H, m), 1.46 (9H, s), 2.60-2.80 (2H, m), 2.80-2.90 (2H, m), 3.09 (2H, t-like, J = 5.2Hz). 3.54 (2H, s), 3.83 (2H, s), 3.94 (2H, t, J=5.8Hz), 4.00-4.20 (2H, m), 6.50 (1H, d, J = 2.6Hz), 6.66 (1H, dd, J = 8.0, 2.6Hz), 7.04 (1H, d, J = 8.0Hz), 7.10-7.40 (5H, m).

### Reference Example 55

2-(Phenylmethyl)-8-[[2-(4-piperidinyl)ethyl]oxy]-2,3,4,5-tetrahydro-1H-2-benzazepine

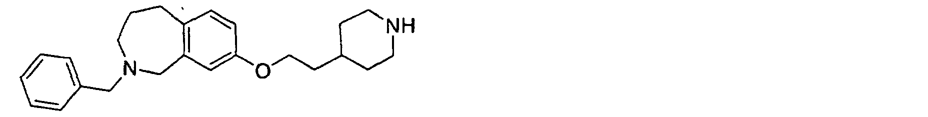

According to the same procedures as those of Reference Example 8) using t-butyl 4-[2-[[2-(phenylmethyl)-2,3,4,5-tetrahydro-1H-2-benzazepine-8-yl]oxy]ethyl]-1-piperidinecarboxylate obtained in Reference Example 54), the title compound was obtained as colorless crystals having a melting point of 43-44°C.
¹H NMR (CDCl₃) δ 1.05-1.30 (2H, m), 1.50-1.80 (7H, m), 1.85-2.05 (1H, br), 2.55-2.70 (2H, m), 2.80-2.95 (2H, m), 3.00-3.20 (4H, m), 3.54 (2H, s), 3.84 (2H, s), 3.94 (2H, t, J =6.0Hz), 6.52 (1H, d, J = 2.6Hz), 6.67 (1H, dd, J = 8.2, 2.6Hz), 7.05 (1H, d, J = 8.2Hz), 7.20-7.40 (5H, m).

### Reference Example 56

2,2,2-Trifluoro-1-(7-sulfanyl-2,3,4,5-tetrahydro-1H-2-benzazepine-2-yl)-1-ethanone

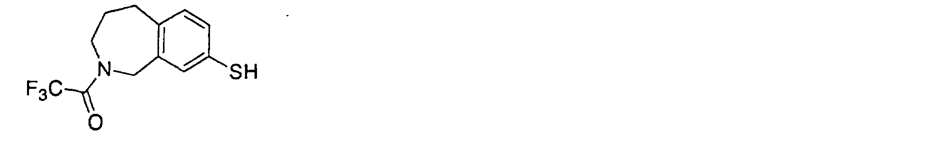

According to the same procedures as those of Reference Example 19) using 2-(trifluoroacetyl)-2,3,4,5-tetrahydro-1H-2-benzazepine-8-sulfonyl chloride, the title compound was obtained as colorless crystals having a melting point of 94-95°C.
¹H NMR (CDCl₃) δ 1.80-2.00 (2H, m), 2.41 (1H, s), 2.95-3.10 (2H, m), 3.80-4.00 (2H, m), 4.61 and 4.68 (2H, s and s), 7.15-7.45 (3H, m).
Elemental analysis for C₁₂H₁₂F₃NOS
Calcd.: C, 53.35; H, 4.39; N, 5.09
Found: C, 53.10; H, 4.47; N, 4.50

### Reference Example 57

t-Butyl 4-[2-[[2-(trifluoroaceyl)-2,3,4,5-tetrahydro-1H-2-benzazepine-8-yl]sulfanyl]ethyl]-1-piperidinecarboxylate

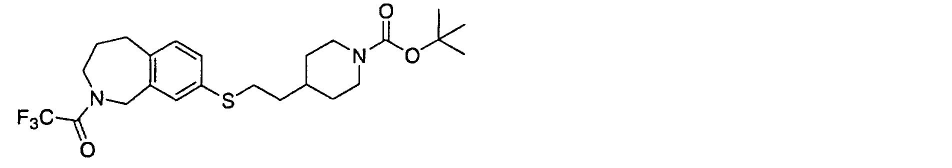

According to the same procedures as those of Reference Example 20) using 2,2,2-trifluoro-1-(7-sulfanyl-2,3,4,5-tetrahydro-1H-2-benzazepine-2-yl)-1-ethanone obtained in Reference Example 56), the title compound was obtained as a colorless oil.
¹H NMR (CDCl₃) δ 1.00-1.25 (2H, m), 1.45 (9H, s), 1.45-1.75 (9H, m), 2.55-2.80 (2H, m), 2.85-3.10 (2H, m), 3.80-4.20 (4H, m), 4.50-4.70 (2H, m), 7.00-7.25 (3H, m).

### Reference Example 58

t-Butyl 4-[2-[(2,3,4,5-tetrahydro-1H-2-benzazepine-8-yl)sulfanyl]ethyl]-1-piperidinecarboxylate

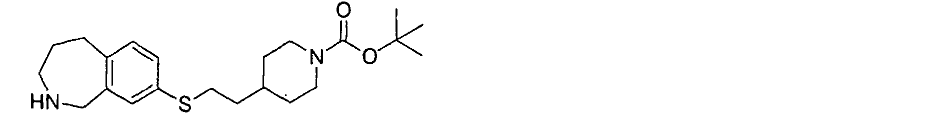

According to the same procedures as those of Reference Example 21) using t-butyl 4-[2-[[2-(trifluoroaceyl)-2,3,4,5-tetrahydro-1H-2-benzazepine-8-yl]sulfanyl]ethyl]-1-piperidinecarboxylate obtained in Reference Example 57), the title compound was obtained as a colorless oil.
¹H NMR (CDCl₃) δ 1.00-1.30 (2H, m), 1.40-1.80 (19H, m), 2.30-2.45 (1H, br), 2.55-2.80 (2H, m), 2.85-3.00 (2H, m), 3.19 (1H, t, J = 5.2Hz), 3.67 (1H, t, J = 6.4Hz), 3.90 (1H, s), 4.00-4.20 (2H, m), 7.00-7.30 (3H, m).

### Reference Example 59

t-Butyl 4-[2-[[2-(phenylmethyl)-2,3,4,5-tetrahydro-1H-2-benzazepine-8-yl)sulfanyl]ethyl]-1-piperidinecarboxylate

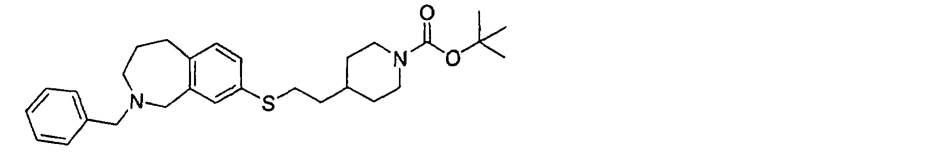

According to the same procedures as those of Reference Example 22) using t-butyl 4-[2-[(2,3,4,5-tetrahydro-1H-2-benzazepine-8-yl)sulfanyl]ethyl]-1-piperidinecarboxylate obtained in Reference Example 58), the title compound was obtained as a colorless oil.
¹H NMR (CDCl₃) δ 1.00-1.20 (2H, m), 1.45 (9H, s), 1.50-1.95 (7H, m), 2.55-2.80 (2H, m), 2.80-2.95 (4H, m), 3.11 (2H, t-like, J = 5.2Hz), 3.52 (2H, s), 3.82 (2H, s), 4.00-4.20 (2H, m), 6.85-6.90 (1H, m), 7.00-7.15 (2H, m), 7.20-7.40 (5H, m).

### Reference Example 60

2-(Phenylmethyl)-8-[[2-(4-piperidinyl)ethyl]sulfanyl]-2,3,4,5-tetrahydro-1H-2-benzazepine

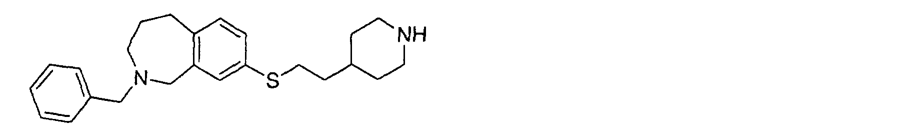

According to the same procedures as those of Reference Example 23) using t-butyl 4-[2-[[2-(phenylmethyl)-2,3,4,5-tetrahydro-1H-2-benzazepine-8-yl)sulfanyl]ethyl]-1-piperidinecarboxylate obtained in Reference Example 59), the title compound was obtained as a colorless oil.
¹H NMR (CDCl₃) δ 0.95-1.25 (2H, m), 1.40-1.80 (8H, m), 2.45-2.70 (2H, m), 2.80-2.95 (4H, m), 2.95-3.20 (4H, m), 3.53 (2H, s), 3.83 (2H, s), 6.85-6.95 (1H, m), 7.00-7.15 (2H, m), 7.20-7.40 (5H, m).

### Reference Example 61

t-Butyl 4-[2-[[2-(trifluoroacetyl)-2,3,4,5-tetrahydro-1H-2-benzazepine-8-yl)sulfinyl]ethyl]-1-piperidinecarboxylate

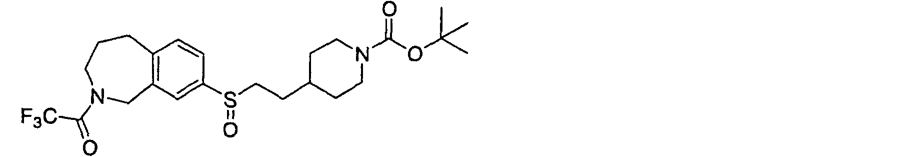

According to the same procedures as those of Reference Example 24) using t-butyl 4-[2-[[2-(trifluoroacetyl)-2,3,4,5-tetrahydro-1H-2-benzazepine-8-yl)sulfanyl]ethyl]-1-piperidinecarboxylate obtained in Reference Example 57), the title compound was obtained as a colorless oil.
¹H NMR (CDCl₃) δ 0.90-1.20 (2H, m), 1.30-1.80 (5H, m), 1.44 (9H, s), 1.85-2.00 (2H, m), 2.55-2.90 (4H, m), 3.00-3.15 (2H, m), 3.80-4.20 (4H, m), 4.55-4.80 (2H, m), 7.30-7.60 (3H, m).

### Reference Example 62

t-Butyl 4-[2-[(2,3,4,5-tetrahydro-1H-2-benzazepine-8-yl)sulfinyl]ethyl]-1-piperidinecarboxylate

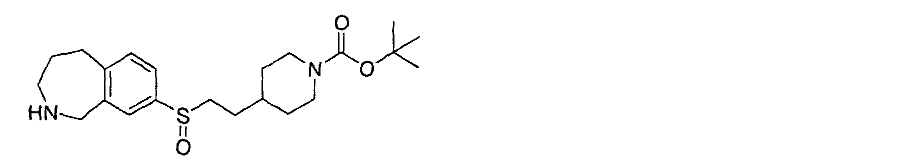

According to the same procedures as those of Reference Example 21) using t-butyl 4-[2-[[2-(trifluoroacetyl)-2,3,4,5-tetrahydro-1H-2-benzazepine-8-yl)sulfinyl]ethyl]-1-piperidinecarboxylate obtained in Reference Example 61), the title compound was obtained as a colorless oil.
¹H NMR (CDCl₃) δ 1.00-1.20 (2H, m), 1.44 (9H, s), 1.50-1.90 (8H, m), 2.55-2.85 (4H, m), 2.95-3.10 (2H, m), 3.23 (2H, t-like, J = 4.6Hz), 3.95-4.20 (4H, m), 7.25-7.40 (3H, m).

### Reference Example 63

t-Butyl 4-[2-[[2-(phenylmethyl)-2,3,4,5-tetrahydro-1H-2-benzazepine-8-yl]sulfinyl]ethyl]-1-piperidinecarboxylate

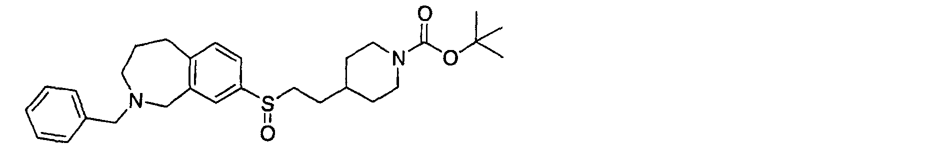

According to the same procedures as those of Reference Example 22) using t-butyl 4-[2-[(2,3,4,5-tetrahydro-1H-2-benzazepine-8-yl)sulfinyl]ethyl]-1-piperidinecarboxylate obtained in Reference Example 62), the title compound was obtained as a colorless oil.
¹H NMR (CDCl₃) δ 1.00-1.20 (2H, m), 1.44 (9H, s), 1.50-2.10 (7H, m), 2.55-2.85 (4H, m), 2.90-3.05 (2H, m), 3.12 (2H, t-like, J = 5.4Hz), 3.54 (2H, s), 3.91 (2H, s), 3.95-4.20 (2H, m), 7.13 (1H, d, J = 1.8Hz), 7.20-7.35 (6H, m), 7.42 (1H, dd, J = 7.6, 1.8Hz).

### Reference Example 64

2-(Phenylmethyl)-8-[[2-(4-piperidinyl)ethyl]sulfinyl]-2,3,4,5-tetrahydro-1H-2-benzazepine

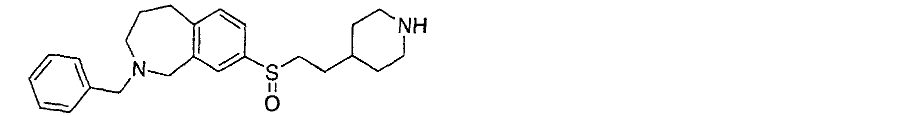

According to the same procedures as those of Reference Example 23) using t-butyl 4-[2-[[2-(phenylmethyl)-2,3,4,5-tetrahydro-1H-2-benzazepine-8-yl]sulfinyl]ethyl]-1-piperidinecarboxylate obtained in Reference Example 63), the title compound was obtained as a colorless oil.
¹H NMR (CDCl₃) δ 1.00-1.20 (2H, m), 1.45-2.10 (8H, m), 2.50-2.70 (2H, m), 2.80-2.95 (4H, m), 3.00-3.20 (4H, m), 3.52 (2H, s), 3.83 (2H, s), 6.85-7.15 (3H, m), 7.20-7.40 (5H, m).

### Reference Example 65

t-Butyl 4-[2-[[2-(trifluoroacetyl)-2,3,4,5-tetrahydro-1H-2-benzazepine-8-yl]sulfonyl]ethyl]-1-piperidinecarboxylate

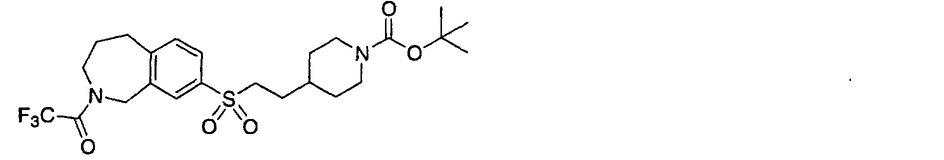

According to the same procedures as those of Reference Example 28) using t-butyl 4-[2-[[2-(trifluoroacetyl)-2,3,4,5-tetrahydro-1H-2-benzazepine-8-yl)sulfanyl]ethyl]-1-piperidinecarboxylate obtained in Reference Example 57), the title compound was obtained as colorless crystals having a melting point of 129-131°C.
¹H NMR (CDCl₃) δ 0.95-1.20 (2H, m), 1.30-1.80 (5H, m), 1.44 (9H, s), 1.85-2.05 (2H, m), 2.50-2.75 (2H, m), 3.00-3.20 (4H, m), 3.80-4.20 (4H, m), 4.67 and 4.76 (2H, s and s), 7.38 (1H, d, J = 7.38Hz), 7.76 (1H, dd, J = 8.2, 1.8 Hz), 7.91 (1H, d, J = 1.8Hz).
Elemental analysis for C₂₅H₃₃F₃N₂O₅S
Calcd.: C, 55.58; H, 6.41; N, 5.40
Found: C, 55.52; H, 6.31; N, 5.48

### Reference Example 66

t-Butyl 4-[2-[(2,3,4,5-tetrahydro-1H-2-benzazepine-8-yl]sulfonyl]ethyl]-1-piperidinecarboxylate

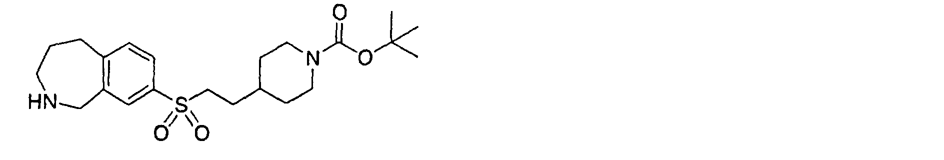

According to the same procedures as those of Reference Example 21) using t-butyl 4-[2-[[2-(trifluoroacetyl)-2,3,4,5-tetrahydro-1H-2-benzazepine-8-yl]sulfonyl]ethyl]-1-piperidinecarboxylate obtained in Reference Example 65), the title compound was obtained as a colorless oil.
¹H NMR (CDCl₃) δ 0.95-1.25 (2H, m), 1.44 (9H, s), 1.50-1.95 (8H, m), 2.55-2.75 (2H, m), 2.90-3.15 (4H, m), 3.24 (2H, t-like, J = 5.2Hz), 3.95-4.15 (2H, m), 4.00 (2H, s), 7.34 (1H, d, J = 7.4Hz), 7.60-7.75 (2H, m).

### Reference Example 67

t-Butyl 4-[2-[[2-(phenylmethyl)-2,3,4,5-tetrahydro-1H-2-benzazepine-8-yl]sulfonyl]ethyl]-1-piperidinecarboxylate

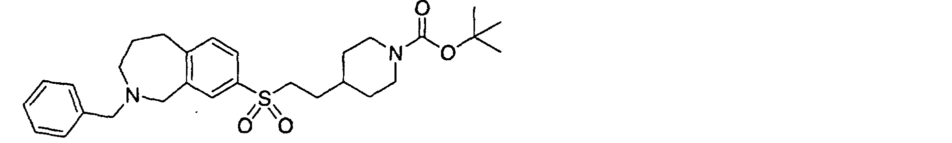

According to the same procedures as those of Reference Example 22) using t-butyl 4-[2-[(2,3,4,5-tetrahydro-1H-2-benzazepine-8-yl]sulfonyl]ethyl]-1-piperidinecarboxylate obtained in Reference Example 66), the title compound was obtained as a colorless oil.
¹H NMR (CDCl₃) δ 0.95-1.20 (2H, m), 1.44 (9H, s), 1.50-1.90 (7H, m), 2.55-2.75 (2H, m), 2.95-3.10 (4H, m), 3.13 (2H, t-like, J = 5.2Hz), 3.54 (2H, s), 3.91 (2H, s), 3.95-4.20 (2H, m), 7.20-7.40 (6H, m), 7.43 (1H, d, J = 1.8Hz), 7.68 (1H, dd, J = 7.7, 1.8Hz).

### Reference Example 68

2-(Phenylmethyl)-8-[[2-(4-piperidinyl)ethyl]sulfinyl]-2,3,4,5-tetrahydro-1H-2-benzazepine

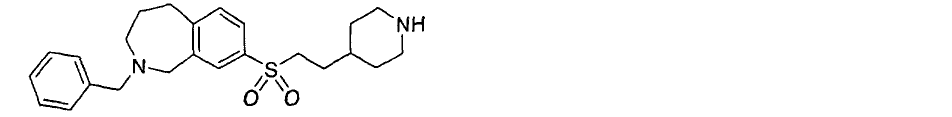

According to the same procedures as those of Reference Example 23) using t-butyl 4-[2-[[2-(phenylmethyl)-2,3,4,5-tetrahydro-1H-2-benzazepine-8-yl]sulfonyl]ethyl]-1-piperidinecarboxylate obtained in Reference Example 67), the title compound was obtained as colorless crystals having a melting point of 83-85°C.
¹H NMR (CDCl₃) δ 0.95-1.90 (12H, m), 2.45-2.65 (2H, m), 2.90-3.20 (6H, m), 3.54 (2H, s), 3.91 (2H, s), 7.20-7.40 (6H, m), 7.40-7.45 (1H, m), 7.65-7.75 (1H, m).

### Reference Example 69

N-[1-(phenylmethyl)-4-piperidinyl]guanidine 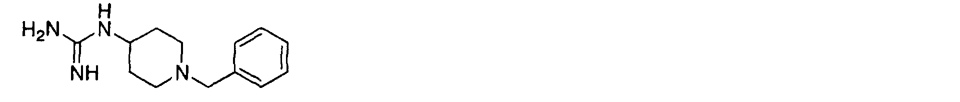
1) t-Butyl [(t-butoxycarbonyl)amino](methylthio)methylidenecarbamate (3.08 g, 10.6 mmol) was added to a solution of 1-(phenylmethyl)-4-aminopiperidine (2.0 ml, 10.6 mmol) in THF (50 ml), and the mixture was heated at reflux for 3 hours. After the solvent was distilled off under reduced pressure, the resulting residue was purified by silica gel column chromatography (developing solvent; ethyl acetate) to obtain a colorless oil (1.02 g).
   ¹H NMR (CDCl₃) δ 1.20-1.70 (2H, m), 1.45 (18H, s), 1.80-2.25 (4H, m), 2.70-2.90 (2H, m), 3.40-4.00 (1H, br), 3.45-3.55 (2H, m), 5.00-5.20 (1H, m), 7.20-7.40 (5H, m), 7.95-8.05 (1H, m).
2) A solution of 9.8N hydrogen chloride in ethanol (50 ml) was added to a solution of the oil (1.02 g, 2.36 mmol) obtained in 1) in ethanol (50 ml), and the mixture was stirred at room temperature for 24 hours. After the solvent was distilled off under reduced pressure, an aqueous solution of sodium hydroxide was added, and extracted with ethyl acetate three times. After the extract was washed with an aqueous saturated solution of sodium chloride and dried with anhydrous sodium sulfate, the solvent was distilled off under reduced pressure to obtain the title compound as a colorless oil.
   ¹H NMR (CDCl₃) δ 1.30-1.65 (2H, m), 1.80-2.00 (2H, m), 2.00-2.25 (2H, m), 2.70-2.90 (2H, m), 3.20-3.80 (2H, br), 3.40-3.55 (2H, m), 4.90-5.00 (1H, m), 7.20-7.40 (5H, m), ca.11 (2H, br).

### Example 1)

2-[(2-Methylphenyl)methyl]-7-[2-[1-[[2-(trifluoromethyl)phenyl]methyl]-4-piperidinyl]ethoxy]-2.3,4,5-tetrahydro-1H-2-benzazepine dihydrochloride

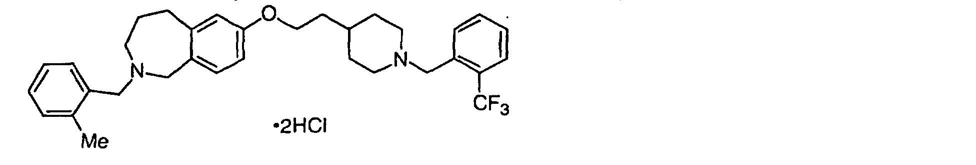

α'-Bromo-α,α,α-trifluoromethyl-o-xylene (198 mg, 0.83 mmol) was added dropwise to a suspension of 2-[(2-methylphenyl)methyl]-7-[2-(4-piperidinyl)ethoxy]-2,3,4,5-tetrahydro-1H-2-benzazepine (300 mg, 0.79 mmol) and potassium carbonate (500 mg) in acetonitrile (25 ml) at room temperature. After the mixture was stirred at room temperature for 10 hours and the solvent was distilled off under reduced pressure, the residue was dissolved in water-ethyl acetate, and extracted with ethyl acetate. After the extract was washed with an aqueous saturated solution of sodium chloride and dried with anhydrous magnesium sulfate, the solvent was distilled off under reduced pressure. The resulting residue was purified by silica gel column chromatography (developing solvent: hexane-ethyl acetate=4:1) to obtain a free base of the title compound (356 mg) as a colorless oil.
¹H NMR (CDCl₃) δ 1.20-1.80 (9H, m), 2.00-2.15 (2H, m), 2.28 (3H, s), 2.75-2.90 (4H, m), 3.06 (2H, t-like, J = 5.2 Hz), 3.46 (2H, s), 3.63 (2H, s), 3.79 (2H, s), 3.98 (2H, t, J = 6.2Hz) , 6.60 (1H, dd, J = 8.2, 2.6 Hz), 6.72 (1H, d, J = 2.6 Hz), 6.88 (1H, d, J = 8.2 Hz), 7.10-7.40 (5H, m), 7.50 (1H, t, J = 7.0 Hz), 7.60 (1H, d, J = 7.6 Hz), 7.82 (1H, d, J = 7.6 Hz).

A solution of the above free base (356 mg) in ethanol was treated with 2 equivalents of hydrogen chloride (ethanol solution) to obtain the title compound (388 mg) as colorless amorphous powders from ethyl ether.

### Example 2)

7-[2-[1-[(2-Chlorophenyl)methyl]-4-piperidinyl]ethoxy]-3-[(2-methylphenyl)methyl]-2,3,4,5-tetrahydro-1H-3-benzazepine dihydrochloride

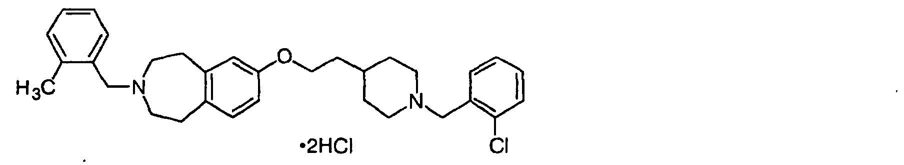

According to the same procedures as those of Example 1) using 3-[(2-methylphenyl)methyl]-7-[2-(4-piperidinyl)ethoxy]-2,3,4,5-tetrahydro-1H-3-benzazepine (0.185 g) obtained in Reference Example 10), the title compound (0.21 g) was obtained as colorless amorphous powders.
¹H NMR(CDCl₃, free base) δ 1.22 - 1.45 (2H, m), 1.48 - 1.80 (5H, m), 1.99 - 2.17 (2H, m), 2.39 (3H, s), 2.53 - 2.67 (4H, m), 2.68 - 2.97 (6H, m), 3.53 (2H, s), 3.60 (2H, s), 3.97 (2H, t, J = 6.4 Hz), 6.57 - 6.68 (2H, m), 6.98 (1H, d, J = 7.7 Hz), 7.08 - 7.37 (7H, m), 7.48 (1H, dd, J = 2.2, 7.3 Hz).
Elemental analysis for C₃₂H₃₉ClN₂O·2HCl·H₂O
Calcd.: C, 64.70; H, 7.30; N, 4.72
Found: C, 64.74; H, 7.57; N, 4.37

### Example 3)

7-[3-[1-(Phenylmethyl)-4-piperidinyl]propoxy]-3-(phenylmethyl)-2,3,4,5-tetrahydro-1H-3-benzazepine dihydrochloride

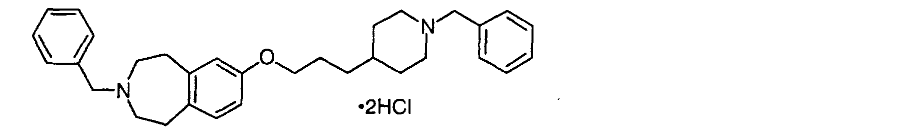

According to the same procedures as those of Example 1) using 3-(phenylmethyl)-7-[3-(4-piperidinyl)propoxy]-2,3,4,5-tetrahydro-1H-3-benzazepine (0.11 g) obtained in Reference Example 12), the title compound (0.21 g) was obtained as colorless amorphous powders.
¹H NMR(CDCl₃, free base) δ 1.15 - 1.45 (5H, m), 1.56 - 2.03 (6H, m), 2.53 - 2.67 (4H, m), 2.70 - 2.96 (6H, m), 3.49 (2H, s), 3.63 (2H, s), 3.89 (2H, t, J = 6.6 Hz), 6.55 - 6.67 (2H, m), 6.96 (1H, d, J = 7.7 Hz), 7.15 - 7.40 (10H, m).
Elemental analysis for C₃₂H₄₀N₂O·2HCl·H₂O
Calcd.: C, 67.59; H, 7.98; N, 4.93
Found: C, 67.09; H, 7.86; N, 4.92

### Example 4)

7-[(2-Methylphenyl)methyl]-8-[2-[1-[(4-chlorophenyl)methyl]-4-piperidinyl]ethoxy]-2,3,4,5-tetrahydro-1H-2-benzazepine dihydrochloride

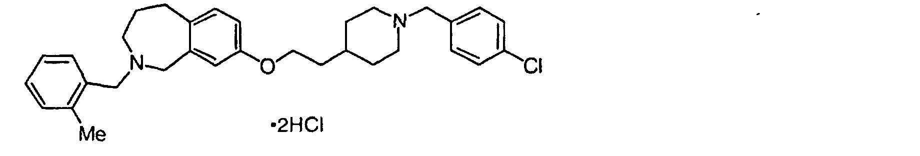

According to the same procedures as those of Example 1) using 2-[(2-methylphenyl)methyl]-8-[2-(4-piperidinyl)ethoxy]-2,3,4,5-tetrahydro-1H-2-benzazepine (250 mg) obtained in Reference Example 14), the title compound (289 mg) was obtained as colorless amorphous powders.
¹H NMR (CDCl₃, free base) δ 1.20-1.80 (9H, m), 1.80-2.05 (2H, m), 2.27 (3H, s), 2.75-2.90 (4H, m), 3.10 (2H, t-like, J = 5.2 Hz), 3.44 (2H, s), 3.48 (2H, s), 3.81 (2H, s), 3.93 (2H, t, J = 6.2 Hz), 6.53 (1H, d, J = 2.4 Hz), 6.66 (1H, dd, J = 8.0, 2.6 Hz), 7.04 (1H, d, J = 8.0 Hz), 7.10-7.30 (8H, m).
Elemental analysis for C₃₂H₃₉ClN₂O·2HCl·H₂O
Calcd.: C, 64.70; H, 7.30; N, 4.72
Found: C, 65.10; H, 7.20; N, 4.64

### Example 5)

7-[3-[1-[(2-Chlorophenyl)methyl]-4-piperidinyl]propoxy]-3-[(2-methylphenyl)methyl]-2,3,4,5-tetrahydro-1H-3-benzazepine dihydrochloride

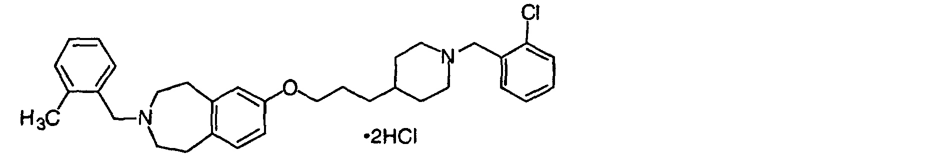

According to the same procedures as those of Example 1) using 3-[(2-methylphenyl)methyl]-7-[3-(4-piperidinyl)propoxy]-2,3,4,5-tetrahydro-1H-3-benzazepine (0.105 g) obtained in Reference Example 16), the title compound (85 mg) was obtained as colorless amorphous powders.
¹H NMR(CDCl₃, free base) δ 1.17 - 1.45 (5H, m), 1.47 - 1.87 (4H, m), 1.94 - 2.15 (2H, m), 2.39 (3H, s), 2.54 - 2.66 (4H, m), 2.75 - 2.98 (6H, m), 3.53 (2H, s), 3.61 (2H, s), 3.91 (2H, t, J = 6.6 Hz), 6.57 - 6.67 (2H, m), 6.97 (1H, d, J = 7.7 Hz), 7.09 - 7.38 (7H, m), 7.49 (1H, dd, J = 2.2, 7.3 Hz).
Elemental analysis for C₃₃H₄₁ClN₂O·2HCl·2.5H₂O
Calcd.: C, 62.41; H, 7.62; N, 4.41
Found: C, 62.58; H, 7.28; N, 4.06

### Example 6)

7-[2-[1-(Phenylmethyl)-4-piperidinyl]ethoxy]-3-(phenylmethyl)-2,3,4,5-tetrahydro-1H-3-benzazepine dihydrochloride

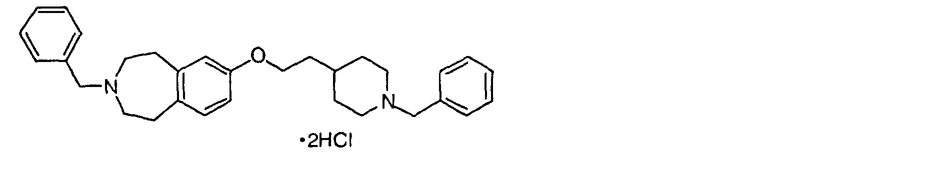

According to the same procedures as those of Example 1) using 3-(phenylmethyl)-7-[2-(4-piperidinyl)ethoxy]-2,3,4,5-tetrahydro-1H-3-benzazepine (0.265 g) obtained in Reference Example 18), the title compound (0.23 g) was obtained as colorless amorphous powders.
¹H NMR (CDCl₃, free base) δ 1.20 - 1.60 (3H, m), 1.63 - 1.79 (4H, m), 1.87 - 2.07 (2H, m), 2.54 - 2.68 (4H, m), 2.79 - 2.94 (6H, m), 3.48 (2H, s), 3.63 (2H, s), 3.95 (2H, t, J = 6.4 Hz), 6.55 - 6.67 (2H, m), 6.96 (1H, d, J = 7.7 Hz), 7.18 - 7.40 (10H, m).
Elemental analysis for C₃₁H₃₈N₂O·2HCl·H₂O
Calcd.: C, 68.24; H, 7.76; N, 5.13
Found: C, 67.98; H, 7.88; N, 5.05

### Example 7)

1-(4-Pyridyl)-5-[1-hydroxy-3-[1-(phenylmethyl)-4-piperidinyl]propyl]-2,3-dihydroindole trihydrochloride 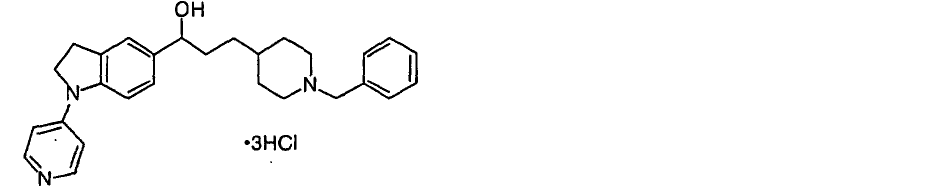
1) A solution of 3-[1-(phenylmethyl)-4-piperidinyl)-1-(2,3-dihydroindole-5-yl)-1-propanone (0.5 g, 1.43 mmol) and 4-chloropyridine hydrochloride (0.22 g, 1.37 mmol) in 1-butanol (5 ml) was stirred for 3 hours with heating. After the solvent was distilled off under reduced pressure, the residue was dissolved in a 5% aqueous solution of sodium hydroxide-ethyl acetate and extracted with ethyl acetate. After the extract was washed with an aqueous saturated solution of sodium chloride and dried with anhydrous magnesium sulfate, the solvent was distilled off under reduced pressure. The resulting residue was purified by silica gel column chromatography (developing solvent: ethyl acetate-methanol = 10:1) to obtain 3-[1-(phenylmethyl)-4-piperidinyl]-1-[1-(4-pyridyl)-2,3-dihydroindole-5-yl]-1-propanone (0.41 g) as colorless crystals having a melting point of 145-146°C
   ¹H NMR (CDCl₃) δ 1.16-1.45 (3H, m), 1.53-2.04 (6H, m), 2.80-2.97 (4H, m), 3.22 (2H, t, J = 8.5 Hz), 3.49 (2H, s), 4.09 (2H, t, J = 8.5 Hz), 7.09 (2H, d, J *=* 6.3 Hz), 7.20-7.40 (6H, m), 7.77-7.88 (2H, m), 8.48 (2H d, J = 6.3 Hz).
2) Sodium borohydride (27 mg, 0.71 mmol) was added to a solution of 3-[1-(phenylmethyl)-4-piperidinyl]-1-[1-(4-pyridyl)-2,3-dihydroindole-5-yl]-1-propanone (0.2 g, 0.47 mmol) obtained in 1) in methanol (10 ml). After the mixture was stirred at room temperature for 30 minutes, water was added. After the solvent was distilled off under reduced pressure, the residue was dissolved in water-ethyl acetate, and extracted with ethyl acetate. After the extract was washed with an aqueous saturated solution of sodium chloride and dried with anhydrous magnesium sulfate, the solvent was distilled off under reduced pressure to obtain a free base of the title compound (0.19 g) as an oil. ¹H NMR (CDCl₃) δ 1.07-1.47 (5H, m), 1.55-2.02 (7H, m), 2.79-2.93 (2H, m), 3.17 (2H, t, J = 8.4 Hz), 3.47 (2H, s), 4.00 (2H, t, J = 8.4 Hz), 4.58 (1H, t, J = 6.3Hz), 7.02 (2H, d, J = 6.6 Hz), 7.05-7.15 (1H, m), 7.17-7.35 (7H, m), 8.38 (2H d, J = 6.6 Hz).

A solution of the above free base (0.18 g) in methanol was treated with 3 equivalents of 4N hydrochloric acid (ethyl acetate solution) to obtain the title compound (0.15 g) colorless amorphous powders.
Elemental analysis for C₂₈H₃₃N₃O·2HCl·1.5H₂O
Calcd.: C, 63.75; H, 7.26; N, 7.97
Found: C, 63.79; H, 7.56; N, 7.73

### Example 8)

2-(Phenylmethyl)-8-[1-hydroxy-3-[1-(phenylmethyl)-4-piperidinyl]propyl]-2,3,4,5-tetrahydro-1H-2-benzazepine dihydrochloride

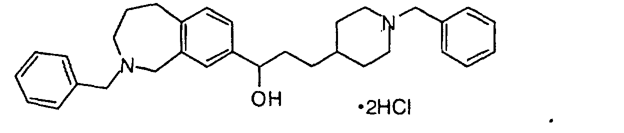

According to the same procedures as those of Example 7)-2) using 1-[2-(phenylmethyl)-2,3,4,5-tetrahydro-1H-2-benzazepine-8-yl]-3-[1-(phenylmethyl)-4-piperidinyl]-1-propanone, the title compound was obtained as colorless amorphous powders.
¹H NMR (CDCl₃, free base) δ 1.08-1.47 (4H, m), 1.56-2.00 (10H, m), 2.78-2.96 (4H, m), 3.12 (2H, t like, J = 5.3Hz), 3.47 (2H, s), 3.52 (2H, s), 3.86 (2H, s), 4.54 (1H, t, J = 7.1 Hz), 6.88 (1H, s), 7.11 (2H, s), 7.16-7.40 (10H, m).
Elemental analysis for C₃₂H₄₀N₂O·2HCl·2H₂O
Calcd.: C, 66.54; H, 8.03; N, 4.85
Found: C, 66.61; H, 7.89; N, 4.93

### Example 9)

3 -(Phenylmethyl)-7-[3-[1-[(3-chlorophenyl)methyl]-4-piperidinyl]-1-hydroxypropyl]-2,3,4,5-tetrahydro-1H-3-benzazepine

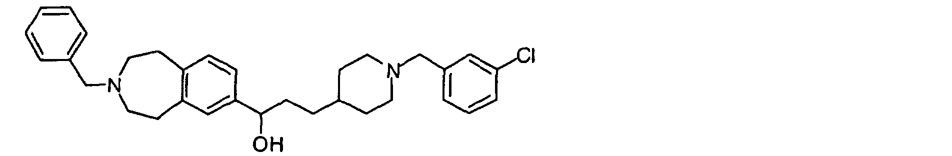

According to the same procedures as those of Example 7)-2) using 1-[3-(phenylmethyl)-2,3,4,5-tetrahydro-1H-3-benzazepine-7-yl]-3-[1-[(3-chlorophenyl)methyl]-4-piperidinyl]-1-propanone, the title compound was obtained as colorless crystals having a melting point of 126-127°C. ¹H NMR (CDCl₃) δ 1.08-1.47 (5H, m), 1.55-2.00 (7H, m), 2.57-2.68 (4H, m), 2.75-2.97 (6H, m), 3.43 (2H, s), 3.64 (2H, s), 4.57 (1H, t, J = 6.6 Hz), 7.02-7.40 (12H, m).
Elemental analysis for C₃₂H₃₉ClN₂O·0.5H₂O
Calcd.: C, 75.05; H, 7.87; N, 5.47
Found: C, 75.35; H, 7.59; N, 5.58

### Example 10)

3-(Phenylmethyl)-7-[1-hydroxy-3-[1-(phenylmethyl)-4-piperidinyl]propyl]-2,3,4,5-tetrahydro-1H-3-benzazepine dihydrochloride

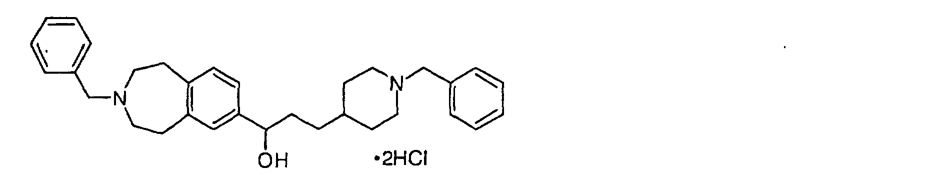

According to the same procedures as those of Example 7)-2) using 1-[3-(phenylmethyl)-2,3,4,5-tetrahydro-1H-3-benzazepine-7-yl]-3-[1-(phenylmethyl)-4-piperidinyl]-1-propanone, the title compound was obtained as colorless amorphous powders.
¹H NMR (CDCl₃, free base) δ 1.08-1.45 (5H, m), 1.54-1.99 (7H, m), 2.55-2.67 (4H, m), 2.78-2.98 (6H, m), 3.47 (2H, s), 3.64 (2H, s), 4.57 (1H, t, J = 6.6 Hz), 6.99-7.40 (13H, m).
Elemental analysis for C₃₂H₄₀N₂O·2HCl·1.5H₂O
Calcd.: C, 67.59; H, 7.98; N, 4.93
Found: C, 67.62; H, 7.97; N, 4.71

### Example 11)

3-(Phenylmethyl)-7-[3-[1-(phenylmethyl)-4-piperidinyl]propyl]-2,3,4,5-tetrahydro-1H-3-benzazepine dihydrochloride

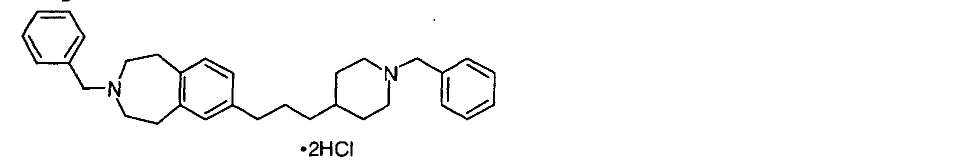

Triethylsilane (0.32 ml, 2 mmol) was added to a solution of 1-[3-(phenylmethyl)-2,3,4,5-tetrahydro-1H-3-benzazepine-7-yl]-3-[1-(phenylmethyl)-4-piperidinyl]-1-propanone (0.14 g, 0.25 mmol) in trifluoroacetic acid (5 ml) at room temperature under a nitrogen stream, and the mixture was stirred for 3 days. After trifluoroacetic acid was distilled off under reduced pressure, the residue was added to water-ice. Further, ether was added and extracted with 10% hydrochloric acid. The extract was made alkaline with a 10% aqueous solution of sodium hydroxide and extracted with ethyl acetate. After the extract was washed with an aqueous saturated solution of sodium chloride and dried with anhydrous magnesium sulfate, the solvent was distilled off under reduced pressure. The resulting residue was purified by silica gel column chromatography (developing solvent: ethyl acetate-methanol=50:1) to obtain a free base of the title compound (95 mg) as an oil. ¹H NMR (CDCl₃) δ 1.10-1.40 (5H, m), 1.48-2.00 (6H, m), 2.44-2.70 (6H, m), 2.79-2.97 (6H, m), 3.48 (2H, s), 3.63 (2H, s), 6.83-7.10 (3H, m), 7.17-7.41 (10H, m).

A solution of the above free base (90 mg) in methanol was treated with 2 equivalents of 4N hydrochloric acid (ethyl acetate solution) to obtain the title compound (90mg) as colorless amorphous powders.
Elemental analysis for C₃₂H₄₀N₂·2HCl·2.5H₂O
Calcd.: C, 67.35; H, 8.30; N, 4.91
Found: C, 67.34; H, 8.38; N, 4.45

### Example 12)

1-(4-Pyridyl)-5-[3-[1-(phenylmethyl)-4-piperidinyl]propyl]-2,3-dihydroindole dihydrochloride

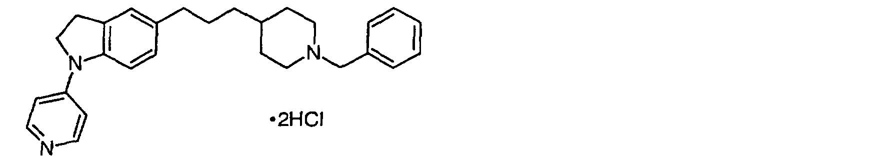

According to the same procedures as those of Example 11) using 3-[1-(phenylmethyl)-4-piperidinyl)-1-[1-(4-pyridyl)-2,3-dihydroindole-5-yl]-1-propanone, the title compound was obtained as colorless amorphous powders.
¹H NMR (CDCl₃, free base) δ 1.10-1.37 (5H, m), 1.50-2.03 (6H, m), 2.53 (2H, t, J = 7.5 Hz), 2.80-2.95 (2H, m), 3.15 (2H, t, J = 8.4 Hz), 3.48 (2H, s), 3.98 (2H, t, J = 8.4 Hz), 6.90-7.08 (4H, m), 7.20-7.39 (6H, m), 8.37 (2H d, J = 6.2 Hz).
Elemental analysis for C₂₈H₃₃N₃·2HCl·H₂O
Calcd.: C, 66.92; H, 7.42; N, 8.36
Found: C, 67.24; H, 7.71; N, 8.17

### Example 13)

4-(Phenylmethyl)-7-[3-[1-(phenylmethyl)-4-piperidinyl]propyl]-2,3,4,5-tetrahydro-1,4-benzoxazepine dihydrochloride

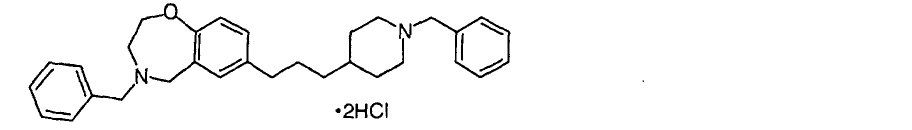

According to the same procedures as those of Example 11) using 3-[1-(phenylmethyl)-4-piperidinyl]-1-[4-(phenylmethyl)-2,3,4,5-tetrahydro-1,4-benzoxazepine-7-yl]-1-propanone, the title compound was obtained as colorless amorphous powders.
¹H NMR (CDCl₃, free base) δ 1.16-1.35 (5H, m), 1.49-1.72 (4H, m), 1.84-2.00 (2H, m), 2.50 (2H, t, J = 7.7 Hz), 2.80-2.92 (2H, m), 3.08 (2H, t, J = 4.4 Hz), 3.48 (2H, s), 3.64 (2H, s), 3.78 (2H, s), 4.06 (2H, t, J = 4.4 Hz), 6.80 (1H, d, J = 2.0 Hz), 6.88-7.03 (2H, m), 7.20-7.36 (10H, m).
Elemental analysis for C₃₁H₃₈N₂O·2HCl·1.5H₂O
Calcd.: C, 67.14; H, 7.81; N, 5.05
Found: C, 66.71; H, 7.98; N, 4.66

### Example 14)

7-[3-[1-[(3-Chlorophenyl)methyl]-4-piperidinyl]-1-hydroxypropyl]-4-(phenylmethyl)-2,3,4,5-tetrahydro-1,4-benzoxazepine dihydrochloride

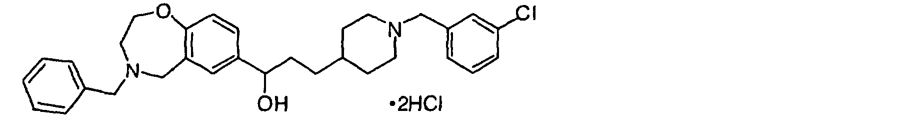

According to the same procedures as those of Example 7)-2) using 3-[1-[(3-chlorophenyl)methyl]-4-piperidinyl]-1-[4-(phenylmethyl)-2,3,4,5-tetrahydro-1,4-benzoxazepine-7-yl]-1-propanone, the title compound was obtained as colorless amorphous powders.
¹H NMR (CDCl₃, free base) δ 1.05-1.43 (5H, m), 1.51-2.00 (7H, m), 2.74-2.87 (2H, m), 3.08 (2H, t like, J = 4.2 Hz), 3.43 (2H, s), 3.64 (2H, s), 3.80 (2H, s), 4.07 (2H, t like, J = 4.2Hz), 4.55 (1H, t, J = 6.6 Hz), 6.93-7.02 (2H, m), 7.09-7.44 (10H, m).
Elemental analysis for C₃₁H₃₇ClN₂O₂·2HCl·1.5H₂O
Calcd.: C, 61.54; H, 7.00; N, 4.63
Found: C, 61.42; H, 6.94; N, 4.62

### Example 15)

3-[(4-Fluorophenyl)methyl]-7-[1-hydroxy-3-[1-[(4-methoxyphenyl)methyl]-4-piperidinyl]propyl]-2,3,4,5-tetrahydro-1H-3-benzazepine dihydrochloride

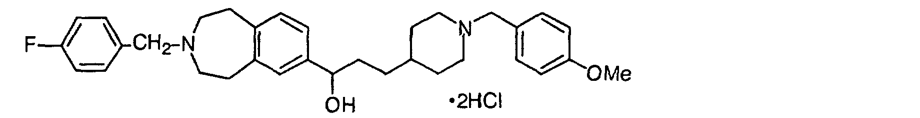

According to the same procedures as those of Example 7)-2) using 1-[3-[(4-fluorophenyl)methyl]-2,3,4,5-tetrahydro-1H-3-benzazepine-7-yl]-3-[1-[(4-methoxyphenyl)methyl]-4-piperidinyl]-1-propanone, the title compound was obtained as colorless amorphous powders. ¹H NMR (CDCl₃, free base) δ 1.07-1.47 (5H, m), 1.53-1.97 (7H, m), 2.53-2.65 (4H, m), 2.77-2.97 (6H, m), 3.40 (2H, s), 3.58 (2H, s), 3.79 (3H, s), 4.56 (1H, t like, J = 6.6 Hz), 6.83 (2H, d, J = 8.1 Hz), 6.94-7.10 (5H, m), 7.15-7.37 (4H, m).
Elemental analysis for C₃₃H₄₁FN₂O₂·2HCl·1.5H₂O
Calcd.: C, 64.28; H, 7.52; N, 4.54
Found: C, 64.46; H, 8.01; N, 4.12

### Example 16)

7-[3-[1-[(4-Chlorophenyl)methyl]-4-piperidinyl]-1-hydroxypropyl]-3-[(4-fluorophenyl)methyl]-2,3,4,5-tetrahydro-1H-3-benzazepine dihydrochloride

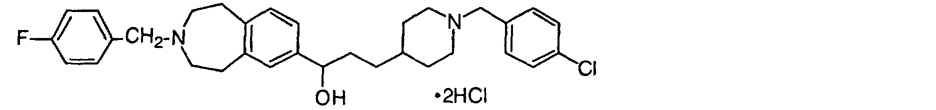

According to the same procedures as those of Example 7)-2) using 3-[1-[(4-chlorophenyl)methyl]-4-piperidinyl]-1-[3-[(4-fluorophenyl)methyl]-2,3,4,5-tetrahydro-1H-3-benzazepine-7-yl]-1-propanone, the title compound was obtained as colorless amorphous powders. ¹H NMR (CDCl₃, free base) δ 1.08-1.47 (5H, m), 1.54-1.97 (7H, m), 2.54-2.67 (4H, m), 2.75-2.97 (6H, m), 3.42 (2H, s), 3.58 (2H, s), 4.56 (1H, dd, J = 5.9, 7.3 Hz), 6.94-7.10 (5H, m), 7.13-7.37 (6H, m).
Elemental analysis for C₃₂H₃₈ClFN₂O·2HCl·1.5H₂O
Calcd.: C, 61.89; H, 6.98; N, 4.51
Found: C, 62.40; H, 7.08; N, 4.00

### Example 17)

8-[3-[1-[(3-Chlorophenyl)methyl]-4-piperidinyl]-1-hydroxypropyl]-2-(phenylmethyl)-2,3,4,5-tetrahydro-1H-2-benzazepine dihydrochloride

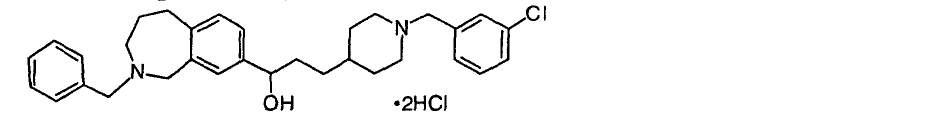

According to the same procedures as those of Example 7)-2) using 3-[1-[(3-chlorophenyl)methyl]-4-piperidinyl]-1-[2-(phenylmethyl)-2,3,4,5-tetrahydro-1H-2-benzazepine-8-yl]-1-propanone, the title compound was obtained as colorless amorphous powders.
¹H NMR (CDCl₃, free base) δ 1.10-1.50 (5H, m), 1.50-2.00 (10H, m), 2.75-2.95 (6H, m), 3.12(2H, t-like, J = 5.2 Hz), 3.43 (2H, s), 3.52 (2H, s), 3.86 (2H, s), 4.54 (1H, t, J = 7.2 Hz), 6.88 (1H, s), 7.05-7.40 (9H, m).
Elemental analysis for C₃₂H₃₉ClN₂O·2HCl·H₂O
Calcd.: C, 64.70; H, 7.30; N, 4.72
Found: C, 64.91; H, 7.47; N, 4.66

### Example 18)

7-[3-[1-[(3-Chlorophenyl)methyl]-4-piperidinyl]propyl]-3-(phenylmethyl)-2,3,4,5-tetrahydro-1H-3-benzazepine dihydrochloride

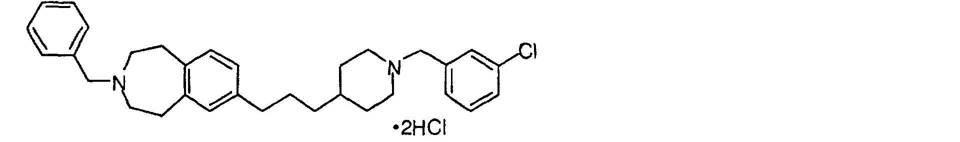

According to the same procedures as those of Example 11) using 3-[1-[(3-chlorophenyl)methyl]-4-piperidinyl]-1-[3-(phenylmethyl)-2,3,4,5-tetrahydro-1H-3-benzazepine-7-yl]-1-propanone, the title compound was obtained as colorless amorphous powders.
¹H NMR (CDCl₃, free base) δ 1.10-1.40 (5H, m), 1.50-1.76 (4H, m), 1.82-2.00 (2H, m), 2.45-2.68 (6H, m), 2.73-2.97 (6H, m), 3.43 (2H, s), 3.63 (2H, s), 6.85-7.10 (3H, m), 7.14-7.40 (9H, m).
Elemental analysis for C₃₂H₃₉N₂·2HCl·1.5H₂O
Calcd.: C, 65.47; H, 7.55; N, 4.77
Found: C, 65.17; H, 7.67; N, 4.42

### Example 19)

7-[3-[1-[(3-Chlorophenyl)methyl]-4-piperidinyl]propyl]-4-(phenylmethyl)-2,3,4,5-tetrahydro-1,4-benzoxazepine dihydrochloride

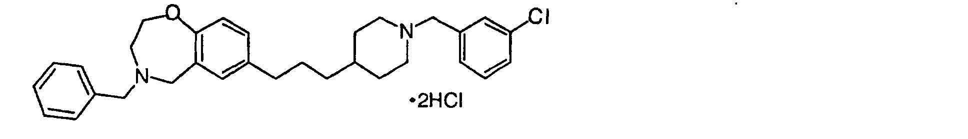

According to the same procedures as those of Example 11) using 3-[1-[(3-chlorophenyl)methyl]-4-piperidinyl]-1-[3-(phenylmethyl)-2,3,4,5-tetrahydro-1,4-benzoxazepine-7-yl]-1-propanone, the title compound was obtained as colorless amorphous powders.
¹H NMR (CDCl₃, free base) δ 1.10-1.36 (5H, m), 1.48-1.74 (4H, m), 1.84-2.00 (2H, m), 2.50 (2H, t, J = 7.7 Hz), 2.77-2.90 (2H, m), 3.08 (2H, t like, J = 4.4 Hz), 3.44 (2H, s), 3.64 (2H, s), 3.78 (2H, s), 4.06 (2H, t like, J = 4.2 Hz), 6.80 (1H, d, J = 1.8 Hz), 6.95 (1H, d, J =8.1 Hz), 6.98 (1H, dd, J = 1.8, 8.1 Hz), 7.14-7.38 (9H, m).
Elemental analysis for C₃₁H₃₇ClN₂O·2HCl·1.5H₂O
Calcd.: C, 63.21; H, 7.19; N, 4.76
Found: C, 63.30; H, 7.17; N, 4.52

### Example 20)

2-[(2-Methylphenyl)methyl]-8-[2-[1-[(3-methylphenyl)methyl]-4-piperidinyl]ethoxy]-2,3,4,5-tetrahydro-1H-2-benzazepine dihydrochloride

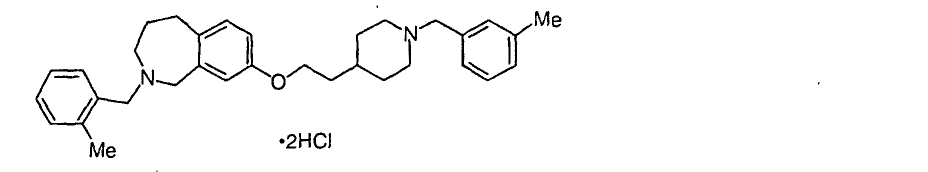

According to the same procedures as those of Example 1) using 2-[(2-methylphenyl)methyl]-8-[2-(4-piperidinyl)ethoxy]-2,3,4,5-tetrahydro-1H-2-benzazepine obtained in Reference Example 14), the title compound was obtained as colorless amorphous powders.
¹H NMR (CDCl₃, free base) δ 1.20-1.80 (9H, m), 1.85-2.05 (2H, m), 2.27 (3H, s), 2.34 (3H, s), 2.80-2.95 (4H, m), 3.07 (2H, t-like, J = 5.2 Hz), 3.46 (2H, s), 3.48 (2H, s), 3.81 (2H, s), 3.93 (2H, t, J = 6.2 Hz), 6.53 (1H, d, J = 2.6 Hz), 6.66 (1H, dd, J = 8.2, 2.6 Hz), 7.00-7.30 (9H, m).
Elemental analysis for C₃₃H₄₂N₂O·2HCl·H₂O
Calcd.: C, 69.09; H, 8.08; N, 4.88
Found: C, 69.57; H, 8.13; N, 4.77

### Example 21)

8-[2-[1-[(2-Chlorophenyl)methyl]-4-piperidinyl]ethoxy]-2-[(2-methylphenyl)methyl]-2,3,4,5-tetrahydro-1H-2-benzazepine dihvdrochloride 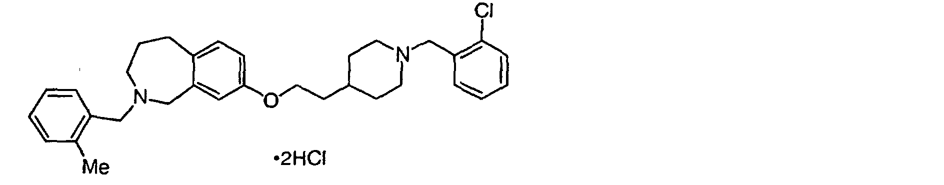 +

According to the same procedures as those of Example 1) using 2-[(2-methylphenyl)methyl]-8-[2-(4-piperidinyl)ethoxy]-2,3,4,5-tetrahydro-1H-2-benzazepine obtained in Reference Example 14), the title compound was obtained as colorless amorphous powders.
¹H NMR (CDCl₃, free base) δ 1.20-1.80 (9H, m), 2.00-2.20 (2H, m), 2.27 (3H, s), 2.80-2.95 (4H, m), 3.07 (2H, t-like, J = 5.2 Hz), 3.48 (2H, s), 3.60 (2H, s), 3.81 (2H, s), 3.94 (2H, t, J = 6.2 Hz), 6.54 (1H, d, J = 2.6 Hz), 6.66 (1H, dd, J = 8.0, 2.6 Hz), 7.04 (1H, d, J = 8.2 Hz), 7.10-7.30 (6H, m), 7.33 (1H, dd, J = 7.5, 1.8 Hz), 7.49 (1H, dd, J = 7.5, 1.8 Hz).
Elemental analysis for C₃₂H₃₉ClN₂O·2HCl·0.5H₂O
Calcd.: C, 65.69; H, 7.24; N, 4.79
Found: C, 65.23; H, 7.19; N, 4.54

### Example 22)

8-[2-[1-[(3-Chlorophenyl)methyl]-4-piperidinyl]ethoxy]-2-[(2-methylphenyl)methyl]-2,3,4,5-tetrahydro-1H-2-benzazepine dihydrochloride

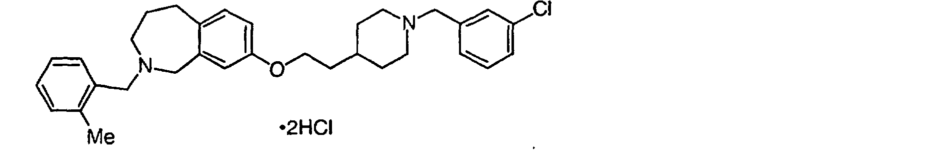

According to the same procedures as those of Example 1) using 2-[(2-methylphenyl)methyl]-8-[2-(4-piperidinyl)ethoxy]-2,3,4,5-tetrahydro-1H-2-benzazepine obtained in Reference Example 14), the title compound was obtained as colorless amorphous powders.
¹H NMR (CDCl₃, free base) δ 1.20-1.80 H, s), 3.81 (2H, s), 3.93 (2H, t, J = 6.6 Hz), 6.54 (1H, d, J = 2.6 Hz), 6.66 (1H, dd, J=8.0, 2.6 Hz), 7.04 (1H, d, J=8.0 Hz), 7.10-7.40 (8H, m).
Elemental analysis for C₃₂H₃₉ClN₂O·2HCl·0.5H₂O
Calcd.: C, 65.69; H, 7.24; N, 4.79
Found: C, 65.50; H, 7.25; N, 4.64

### Example 23)

2-[(2-Methylphenyl)methyl]-7-[2-[1-[(2-methylphenyl)methyl]-4-piperidinyl]ethoxy]-2,3,4,5-tetrahydro-1H-2-benzazepine dihydrochloride

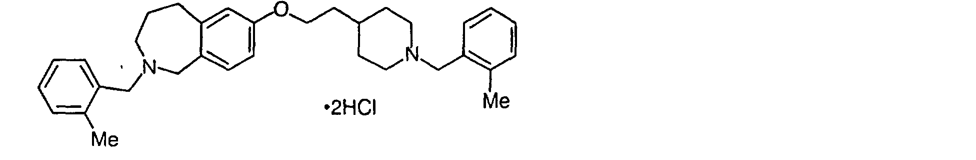

According to the same procedures as those of Example 1) using 2-[(2-methylphenyl)methyl]-7-[2-(4-piperidinyl)ethoxy]-2,3,4,5-tetrahydro-1H-2-benzazepine obtained in Reference Example 8), the title compound was obtained as colorless amorphous powders.
¹H NMR (CDCl₃, free base) δ 1.15-1.80 (9H, m), 1.85-2.05 (2H, m), 2.27 (3H, s), 2.34 (3H, s), 2.80-2.90 (4H, m), 3.05 (2H, t, J = 5.2 Hz), 3.41 (2H, s), 3.45 (2H, s), 3.79 (2H, s), 3.96 (2H, t, J = 6.6 Hz), 6.59 (1H, dd, J = 8.4, 2.2 Hz), 6.71 (1H, d, J = 2.2Hz), 6.87 (1H, d, J = 8.0Hz), 7.00-7.30 (8H, m).

### Example 24)

7-[2-[1-[(2-Chlorophenyl)methyl]-4-piperidinyl]ethoxy]-2-[(2-methylphenyl]-2,3,4,5-tetrahydro-1H-2-benzazepine dihydrochloride

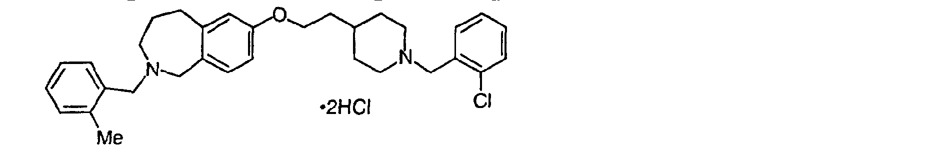

According to the same procedures as those of Example 1) using 2-[(2-methylphenyl)methyl]-7-[2-(4-piperidinyl)ethoxy]-2,3,4,5-tetrahydro-1H-2-benzazepine obtained in Reference Example 8), the title compound was obtained as colorless amorphous powders.
¹H NMR (CDCl₃, free base) δ 1.20-1.90 (9H, m), 2.00-2.20 (2H, m), 2.28 (3H, s), 2.80-3.00 (4H, m), 3.06 (2H, t, J = 5.2 Hz), 3.46 (2H, s), 3.60 (2H, s), 3.79 (2H, s), 3.98 (2H, t, J = 6.6 Hz), 6.60 (1H, dd, J = 8.0, 2.2 Hz), 6.71 (1H, d, J = 2.2Hz), 6.87 (1H, d, J = 8.0Hz), 7.10-7.25 (6H, m), 7.33 (1H, dd, J = 7.2, 2.0 Hz), 7.48 (1H, dd, J = 7.2, 2.0 Hz).

### Example 25)

2-[(2-Methylphenyl)methyl]-8-[2-[1-[(2-methylphenyl)methyl]-4-piperidinyl]ethoxy]-2,3,4,5-tetrahydro-1H-2-benzazepine dihydrochloride

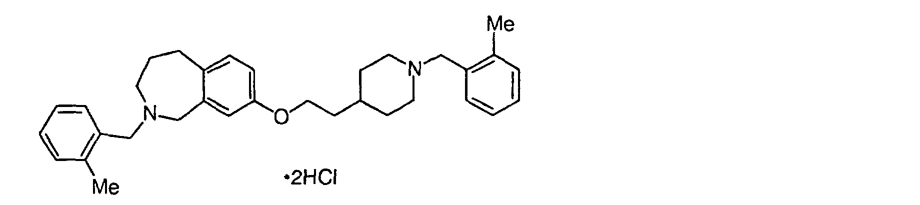

According to the same procedures as those of Example 1) using 2-[(2-methylphenyl)methyl]-8-[2-(4-piperidinyl)ethoxy]-2,3,4,5-tetrahydro-1H-2-benzazepine obtained in Reference Example 14), the title compound was obtained as colorless amorphous powders.
¹H NMR (CDCl₃, free base) δ 1.20-1.80 (9H, m), 1.90-2.05 (2H, m), 2.27 (3H, s), 2.35 (3H, s), 2.75-2.95 (4H, m), 3.07 (2H, t-like, J = 5.2 Hz), 3.44 (2H, s), 3.48 (2H, s), 3.81 (2H, s), 3.93 (2H, t, J = 6.6 Hz), 6.53 (1H, d, J = 2.4 Hz), 6.66 (1H, dd, J = 8.0. 2.4 Hz) , 7.04 (1H, d, J=8.4 Hz), 7.10-7.30 (8H, m).
Elemental analysis for C₃₃H₄₂N₂O·2HCl·H₂O
Calcd.: C, 69.09; H, 8.08; N, 4.88
Found: C, 69.30; H, 7.79; N, 4.65

### Example 26)

2-[(2-Methylphenyl)methyl]-8-[2-[1-[(4-methylphenyl)methyl]-4-piperidinyl]ethoxy]-2,3,4,5-tetrahydro-1H-2-benzazepine dihydrochloride

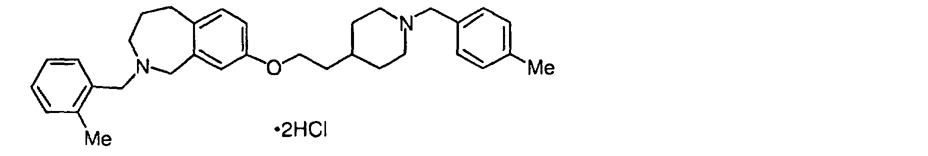

According to the same procedures as those of Example 1) using 2-[(2-methylphenyl)methyl]-8-[2-(4-piperidinyl)ethoxy]-2,3,4,5-tetrahydro-1H-2-benzazepine obtained in Reference Example 14), the title compound was obtained as colorless amorphous powders.
¹H NMR (CDCl₃, free base) δ 1.20-1.80 (9H, m), 1.85-2.05 (2H, m), 2.24 (3H, s), 2.27 (3H, s), 2.80-2.95 (4H, m), 3.07 (2H, t-like, J = 5.2 Hz), 3.46 (2H, s), 3.48 (2H, s), 3.81 (2H, 5), 3.93 (2H, t, J = 6.6 Hz), 6.53 (1H, d, J = 2.6 Hz), 6.65 (1H, dd, J=8.3, 2.6Hz), 7.04 (1H, d, J=8.0 Hz), 7.10-7.30 (8H, m).
Elemental analysis for C₃₃H₄₂N₂O·2HCl·H₂O
Calcd.: C, 69.09; H, 8.08; N, 4.88
Found: C, 69.49; H, 8.00; N, 4.74

### Example 27)

2-[(3-Methylphenyl)methyl]-8-[2-[1-[(2-methylphenyl)methyl]-4-piperidinyl]ethoxy]-2,3,4,5-tetrahydro-1H-2-benzazepine dihydrochloride 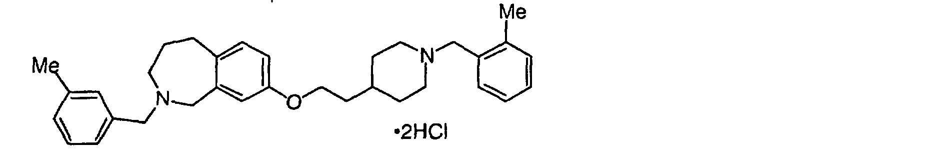
1) According to the same procedures as those of Reference Example 1), Reference Example 7) and Reference Example 8) successively using α-bromo-m-xylene and 8-methoxy-2,3,4,5-tetrahydro-1H-2-benzazepine, 2-[(3-methylphenyl)methyl]-8-[2-(4-piperidinyl)ethoxy]-2,3,4,5-tetrahydro-1H-2-benzazepine was obtained as colorless crystals having a melting point of 56-57°C.
   Elemental analysis for C₂₅H₃₄N₂O
   Calcd.: C, 79.32; H, 9.05; N, 7.40
   Found: C, 79.23; H, 8.93; N, 7.39
2) According to the same procedures as those of Example 1) using 2-[(3-methylphenyl)methyl]-8-[2-(4-piperidinyl)ethoxy]-2,3,4,5-tetrahydro-1H-2-benzazepine, the title compound was obtained as colorless amorphous powders.
   ¹H NMR (CDCl₃, free base) δ 1.15-1.80 (9H, m), 1.90-2.05 (2H, m), 2.33 (3H, s), 2.35 (3H, s), 2.75-2.90 (4H, m), 3.09 (2H, t-like, J = 5.2 Hz), 3.42 (2H, s), 3.50 (2H, s), 3.83 (2H, s), 3.92 (2H, t, J = 6.6 Hz), 6.52 (1H, d, J = 2.6 Hz), 6.66 (1H, dd, J = 8.0, 2.6 Hz), 7.00-7.30 (9H, m).
   Elemental analysis for C₃₃H₄₂N₂O·2HCl·H₂O
   Calcd.: C, 69.09; H, 8.08; N, 4.88
   Found: C, 69.16; H, 8.02; N, 4.78

### Example 28)

2-[(3-Methylphenyl)methyl]-8-[2-[1-[(3-methylphenyl)methyl]-4-piperidinyl]ethoxy]-2,3,4,5-tetrahydro-1H-2-benzazepine dihydrochloride

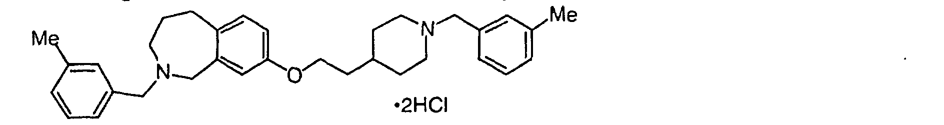

According to the same procedures as those of Example 1) using 2-[(3-methylphenyl)methyl]-8-[2-(4-piperidinyl)ethoxy]-2,3,4,5-tetrahydro-1H-2-benzazepine obtained in Example 27)-1), the title compound was obtained as colorless amorphous powders.
¹H NMR (CDCl₃, free base) δ 1.20-1.80 (9H, m), 1.85-2.05 (2H, m), 2.33 (3H, s), 2.34 (3H, s), 2.80-2.95 (4H, m), 3.10 (2H, t-like, J = 5.2 Hz), 3.45 (2H, s), 3.50 (2H, s), 3.83 (2H, s), 3.92 (2H, t, J = 6.2 Hz), 6.52 (1H, d, J = 2.6 Hz), 6.66 (1H, dd, J = 8.2, 2.6 Hz), 7.00-7.30 (9H, m).
Elemental analysis for C₃₃H₄₂N₂O·2HCl·0.5H₂O
Calcd.: C, 70.20; H, 8.03; N, 4.95
Found: C, 69.90; H, 7.83; N, 4.89

### Example 29)

2-[(3-Methylphenyl)methyl]-8-[2-[1-[(4-methylphenyl)methyl]-4-piperidinyl]ethoxy]-2,3,4,5-tetrahydro-1H-2-benzazepine dihydrochloride

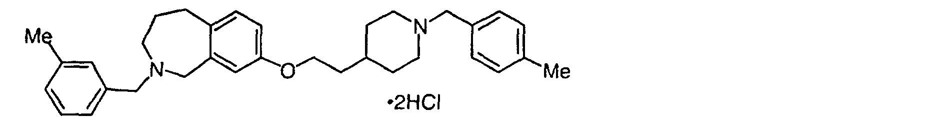

According to the same procedures as those of Example 1) using 2-[(3-methylphenyl)methyl]-8-[2-(4-piperidinyl)ethoxy]-2,3,4,5-tetrahydro-1H-2-benzazepine obtained in Example 27)-1), the title compound was obtained as colorless amorphous powders.
¹H NMR (CDCl₃, free base) δ 1.20-1.80 (9H, m), 1.85-2.00 (2H, m), 2.32 (6H, s), 2.80-2.95 (4H, m), 3.09 (2H, t-like, J = 5.2 Hz), 3.45 (2H, s), 3.49 (2H, s), 3.82 (2H, s), 3.91 (2H, t, J = 6.6 Hz), 6.51 (1H, d, J = 2.6 Hz), 6.65 (1H, dd, J = 8.3, 2.6 Hz), 7.00-7.20 (9H, m).
Elemental analysis for C₃₃H₄₂N₂O·2HCl·H₂O
Calcd.: C, 69.09; H, 8.08; N, 4.88
Found: C, 69.53; H, 7.84; N, 4.92

### Example 30)

8-[2-[1-[(2-Chlorophenyl)methyl]-4-piperidinyl]ethoxy]-2-[(3-methylphenyl)methyl]-2,3,4,5-tetrahydro-1H-2-benzazepine dihydrochloride

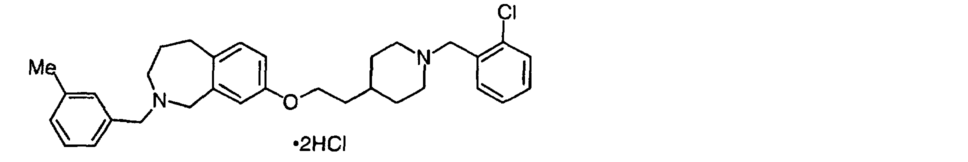

According to the same procedures as those of Example 1) using 2-[(3-methylphenyl)methyl]-8-[2-(4-piperidinyl)ethoxy]-2,3,4,5-tetrahydro-1H-2-benzazepine obtained in Example 27)-1), the title compound was obtained as colorless amorphous powders.
¹H NMR (CDCl₃, free base) δ 1.20-1.85 (9H, m), 2.00-2.20 (2H, m), 2.34 (3H, s), 2.80-3.00 (4H, m), 3.10 (2H, t-like, J = 5.2 Hz), 3.50 (2H, s), 3.60 (2H, s), 3.84 (2H, s), 3.94 (2H, t, J = 6.2 Hz), 6.53 (1H, d, J = 2.6 Hz), 6.67 (1H, dd, J = 8.2, 2.6 Hz), 7.00-7.30 (7H, m), 7.34 (1H, dd, J = 7.5, 1.6 Hz), 7.49 (1H, dd, J = 7.5, 1.6 Hz).
Elemental analysis for C₃₂H₃₉ClN₂O·2HCl·0.5H₂O
Calcd.: C, 65.69; H, 7.24; N, 4.79
Found: C, 65.41; H, 7.19; N, 4.65

### Example 31)

8-[2-[1-[(3-Chlorophenyl)methyl]-4-piperidinyl]ethoxy]-2-[(3-methylphenyl)methyl]-2,3,4,5-tetrahydro-1H-2-benzazepine dihydrochloride

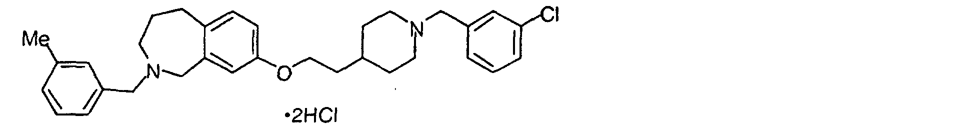

According to the same procedures as those of Example 1) using 2-[(3-methylphenyl)methyl]-8-[2-(4-piperidinyl)ethoxy]-2,3,4,5-tetrahydro-1H-2-benzazepine obtained in Example 27)-1), the title compound was obtained as colorless amorphous powders.
¹H NMR (CDCl₃, free base) δ 1.20-1.85 (9H, m), 1.90-2.05 (2H, m), 2.33 (3H, s), 2.75-2.90 (4H, m), 3.10 (2H, t-like, J = 5.2 Hz), 3.46 (2H, s), 3.51 (2H, s), 3.85 (2H, s), 3.93 (2H, t, J = 6.2 Hz), 6.52 (1H, d, J = 2.6 Hz), 6.67 (1H, dd, J = 8.3, 2.6 Hz), 7.00-7.40 (9H, m).
Elemental analysis for C₃₂H₃₉ClN₂O·2HCl·0.5H₂O
Calcd.: C, 65.69; H, 7.24; N, 4.79
Found: C, 65.50; H, 7.30; N, 4.59

### Example 32)

8-[2-[1-[(4-Chlorophenyl)methyl]-4-piperidinyl]ethoxy]-2-[(3-methylphenyl)methyl]-2,3,4,5-tetrahydro-1H-2-benzazepine dihydrochloride

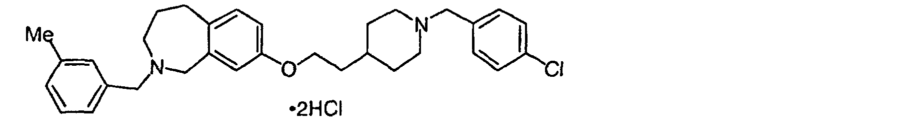

According to the same procedures as those of Example 1) using 2-[(3-methylphenyl)methyl]-8-[2-(4-piperidinyl)ethoxy]-2,3,4,5-tetrahydro-1H-2-benzazepine obtained in Example 27)-1), the title compound was obtained as colorless amorphous powders.
¹H NMR (CDCl₃, free base) δ 1.20-1.80 (9H, m), 1.90-2.10 (2H, m), 2.33 (3H, s), 2.75-2.95 (4H, m), 3.13 (2H, t-like, J = 5.2 Hz), 3.46 (2H, s), 3.50 (2H, s), 3.84 (2H, s), 3.92 (2H, t, J = 6.2 Hz), 6.52 (1H, d, J = 2.6 Hz), 6.66 (1H, dd, J = 8.2, 2.6 Hz), 7.00-7.30 (9H, m).
Elemental analysis for C₃₂H₃₉ClN₂O·2HCl·0.5H₂O
Calcd.: C, 65.69; H, 7.24; N, 4.79
Found: C, 65.46; H, 7.10; N, 4.54

### Example 33)

8-[1-Hydroxy-3-[1-(phenylmethyl)-4-piperidinyl]propyl]-2-(2-phenylethyl)-2,3,4,5-tetrahydro-1H-2-benzazepine dihydrochloride

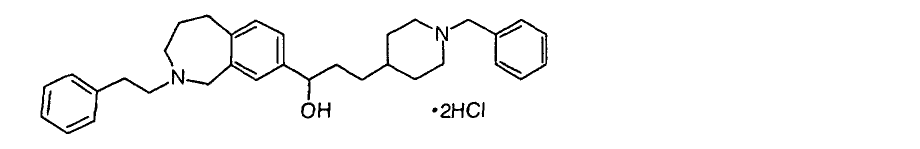

According to the same procedures as those of Example 7-2) using 1-[2-(2-phenylethyl)-2,3,4,5-tetrahydro-1H-2-benzazepine-8-yl]-3-[1-(phenylmethyl)-4-piperidinyl]-1-propanone, the title compound was obtained as colorless amorphous powders.
¹H NMR (CDCl₃, free base) δ 1.07-2.00 (14H, m), 2.54-2.66 (2H, m), 2.75-2.95 (6H, m), 3.18 (2H, t-like, J = 5.1 Hz), 3.47 (2H, s), 3.96 (2H, s), 4.58 (1H, t, J = 6.6 Hz), 7.07-7.36 (13H, m).
Elemental analysis for C₃₃H₄₂N₂O·2HCl·2H₂O
Calcd.: C, 66.99; H, 8.18; N, 4.73
Found: C, 67.01; H, 7.88; N, 4.68

### Example 34)

7-[3-(1-Ethyl-4-piperidinyl)-1-hydroxypropyl]-3-(phenylmethyl)-2,3,4,5-tetrahydro-1H-3-benzazepine

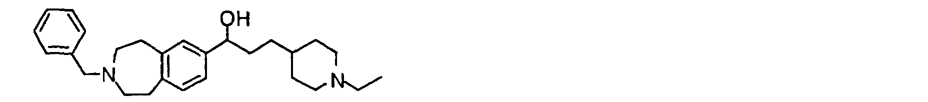

According to the same procedures as those of Example 7-2) using 3-(1-ethyl-4-piperidinyl)-1-[3-(phenylmethyl)-2,3,4,5-tetrahydro-1H-3-benzazepine-7-yl]-1-propanone, the title compound was obtained as colorless crystals having a melting point of 69-71°C.
¹H NMR (CDCl₃) δ 1.06 (3H, t, J = 7.4 Hz), 1.12-1.50 (5H, m), 1.54-2.04 (7H, m), 2.35 (2H, q, J = 7.4 Hz), 2.55-2.68 (4H, m), 2.83-2.98 (6H, m), 3.63 (2H, s), 4.51-4.62 (1H, m), 7.05 (3H, s), 7.20-7.40 (5H, m).
Elemental analysis for C₂₇H₃₈N₂O·0.5H₂O
Calcd.: C, 78.03; H, 9.46; N, 6.74
Found: C, 78.20; H, 9.41; N, 6.67

### Example 35)

3-(Phenylmethyl)-7-[3-(1-propyl-4-piperidinyl)-1-hydroxypropyl]-2,3,4,5-tetrahydro-1H-3-benzazepine

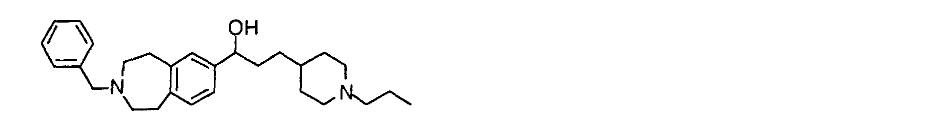

According to the same procedures as those of Example 7-2) using 1-[3-(phenylmethyl)-2,3,4,5-tetrahydro-1H-3-benzazepine-7-yl]-3-(1-propyl-4-piperidinyl)-1-propanone, the title compound was obtained as colorless crystals having a melting point of 78-80°C.
¹H NMR (CDCl₃) δ 0.87 (3H, t, J = 7.4 Hz), 1.14-1.94 (13H, m), 2.07 (1H, brs), 2.18-2.30 (2H, m), 2.56-2.67 (4H, m), 2.82-2.97 (6H, m), 3.63 (2H, s), 4.51-4.61 (1H, m), 7.05 (3H, s), 7.20-7.40 (5H, m).
Elemental analysis for C₂₈H₄₀N₂O·0.25H₂O
Calcd.: C, 79.10; H, 9.60; N, 6.59
Found: C, 79.36; H, 9.59; N, 6.68

### Example 36)

7-[3-[1-(1-Methylethyl)-4-piperidinyl]-1-hydroxypropyl]-3-(phenylmethyl)-2,3,4,5-tetrahydro-1H-3-benzazepine

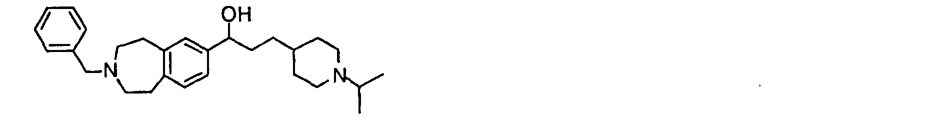

According to the same procedures as those of Example 7-2) using 3-[1-(1-methylethyl)-4-piperidinyl]-1-[3-(phenylmethyl)-2,3,4,5-tetrahydro-1H-3-benzazepine-7-yl]-1-propanone, the title compound was obtained as colorless crystals having a melting point of 97-99°C.
¹H NMR (CDCl₃) δ 1.02 (6H, d, J = 6.6 Hz), 1.13-1.84 (10H, m), 1.96-2.15 (2H, m), 2.56-2.73 (5H, m), 2.77-2.97 (6H, m), 3.64 (2H, s), 4.52-4.63 (1H, m), 7.06 (3H, s), 7.25-7.39 (5H, m).
Elemental analysis for C₂₈H₄₀N₂O·0.25H₂O
Calcd.: C, 79.10; H, 9.60; N, 6.59
Found: C, 78.05; H, 9.44; N, 6.57

### Example 37)

7-[3-(1-Butyl-4-piperidinyl)-1-hydroxypropyl]-3-(phenylmethyl)-2,3,4,5-tetrahydro-1H-3-benzazepine dihydrochloride

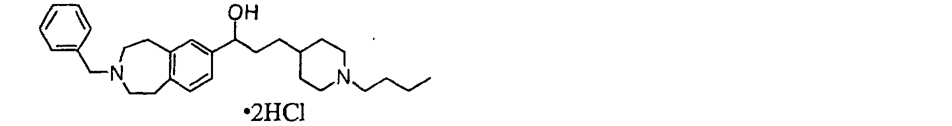

According to the same procedures as those of Example 7-2) using 3-(1-butyl-4-piperidinyl)-1-[3-(phenylmethyl)-2,3,4,5-tetrahydro-1H-3-benzazepine-7-yl]-1-propanone, the title compound was obtained as colorless amorphous powders.
¹H NMR (CDCl₃, free base) δ 0.90 (3H, t, J = 7.2 Hz), 1.10-1.56 (9H, m), 1.56-2.02 (7H, m), 2.20-2.33 (2H, m), 2.56-2.69 (4H, m), 2.82-2.97 (6H, m), 3.63 (2H, s), 4.50-4.63 (1H, m), 7.05 (3H, s), 7.20-7.40 (5H, m).

### Example 38)

7-[3-(1-Acetyl-4-piperidinyl)propyl]-3-(phenylmethyl)-2,3,4,5-tetrahydro-1H-3-benzazepine hydrochloride 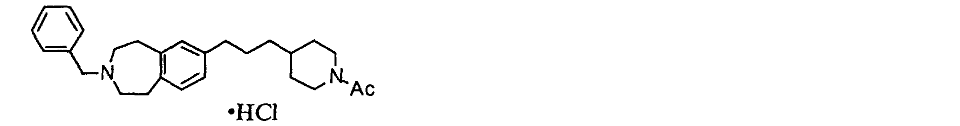
1) According to the same procedures as those of Example 11) using 3-(4-piperidinyl)-1-[3-(phenylmethyl)-2,3,4,5-tetrahydro-1H-3-benzazepine-7-yl]-1-propanone, 7-[3-(4-piperidinyl)propyl]-3-(phenylmethyl)-2,3,4,5-tetrahydro-1H-3-benzazepine (dihydrochloride) was obtained as colorless powders having a melting point of 252-274°C (dec.).
   ¹H NMR (CDCl₃, free base) δ 1.18-1.41 (4H, m), 1.41-1.69 (5H, m), 1.78-1.92 (2H, m), 2.45-2.97 (11H, m), 3.31-3.45 (2H, m), 3.68 (2H, s), 6.85-7.04 (3H, m), 7.24-7.40 (5H, m).
   Elemental analysis for C₂₅H₃₄N₂·2HCl·0.25H₂O
   Calcd.: C, 68.25; H, 8.36; N, 6.37
   Found: C, 68.17; H, 8.33; N, 6.30
2) According to the same procedures as those of Example 1) using 7-[3-(4-piperidinyl)propyl]-3-(phenylmethyl)-2,3,4,5-tetrahydro-1H-3-benzazepine (dihydrochloride) obtained in 1), the title compound was obtained as colorless amorphous powders.
   ¹H NMR (CDCl₃, free base) δ 0.95-1.36 (4H, m), 1.36-1.80 (5H, m), 2.07 (3H, s), 2.42-2.60 (3H, m), 2.62-2.76 (4H, m), 2.87-3.08 (5H, m), 3.68-3.85 (3H, m), 4.50-4.65 (1H, m), 6.86-7.03 (3H, m), 7.22-7.40 (5H, m).
   Elemental analysis for C₂₇H₃₆N₂O·HCl·3.75H₂O
   Calcd.: C, 63.76; H, 8.82; N, 5.51
   Found: C, 63.70; H, 7.81; N, 5.48

### Example 39)

Ethyl [4-[3-[3-(phenylmethyl)-2,3,4,5-tetrahydro-1H-3-benzazepine-7-yl]propyl]-1-piperidinyl]acetate dihvdrochloride

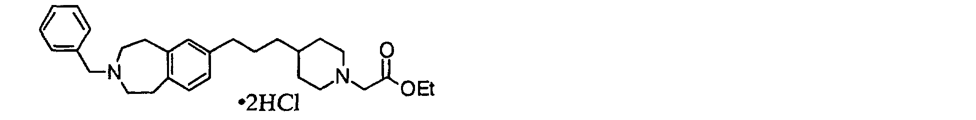

According to the same procedures as those of Example 1) using 7-[3-(4-piperidinyl)propyl]-3-(phenylmethyl)-2,3,4,5-tetrahydro-1H-3-benzazepine (dihydrochloride) obtained in Example 38)-1), the title compound was obtained as colorless amorphous powders.
¹H NMR (CDCl₃, free base) δ 1.12-1.42 (8H, m), 1.47-1.74 (4H, m), 2.00-2.20 (2H, m), 2.44-2.68 (6H, m), 2.80-2.98 (6H, m), 3.17 (2H, s), 3.64 (2H, s), 4.18 (2H, q, J = 7.2 Hz), 6.86-7.03 (3H, m), 7.20-7.40 (5H, m).
Elemental analysis for C₂₉H₄₀N₂O₂·2HCl·2.25H₂O
Calcd.: C, 61.97; H, 8.34; N, 4.98
Found: C, 61.96; H, 7.86; N, 4.71

### Example 40)

3-(Phenylmethyl)-7-[[2-[1-(phenylmethyl)-4-piperidinyl]ethyl]sulfanyl]-2,3,4,5-tetrahydro-1H-3-benzazepine dihydrochloride

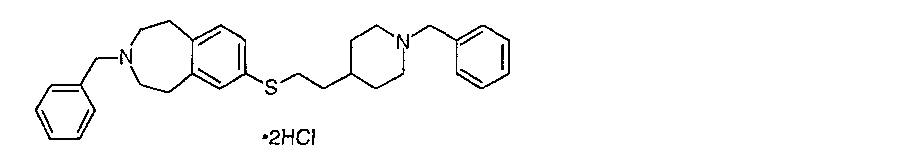

Benzyl bromide (0.082ml, 0.69 mmol) was added dropwise to a suspension of 3-(phenylmethyl)-7-[[2-(4-piperidinyl)ethyl]sulfanyl]-2,3,4,5-tetrahydro-1H-3-benzazepine (240 mg, 0.63 mmol) obtained in Reference Example 23) and potassium carbonate (400 mg) in ethanol (10 ml) at room temperature. After the mixture was stirred at room temperature for 10 hours and the solvent was distilled off under reduced pressure. The residue was dissolved in water-ethyl acetate and extracted with ethyl acetate. The extract was washed with an aqueous saturated solution of sodium chloride and dried with anhydrous magnesium sulfate, the solvent was distilled off under reduced pressure. The resulting residue was purified by silica gel column chromatography (developing solvent; hexane-ethyl acetate=1:1) to obtain a free base of the title compound (194 mg) having a melting point of 88-89°C.
¹H NMR (CDCl₃) δ 1.15-2.00 (9H, m), 2.55-2.65 (4H, m), 2.80-2.95 (8H, m), 3.48 (2H, s), 3.63 (2H, s), 6.90-7.10 (3H, m), 7.20-7.40 (10H, m).

A solution of the above free base (180 mg) in ethanol was treated with 2 equivalents of hydrogen chloride (ethanol solution) to obtain the title compound (172 mg) as colorless crystals having a melting point of 223°C (dec.).
Elemental analysis for C₃₁H₃₈N₂S·2HCl·0.5H₂O
Calcd.: C, 67.37; H, 7.48; N, 5.07
Found: C, 67.52; H, 7.35; N, 5.39

### Example 41)

7-[[2-[1-[(4-Chlorophenyl)methyl]-4-piperidinyl]ethyl]sulfanyl]-3-(phenylmethyl)-2,3,4,5-tetrahydro-1H-3-benzazepine dihydrochloride

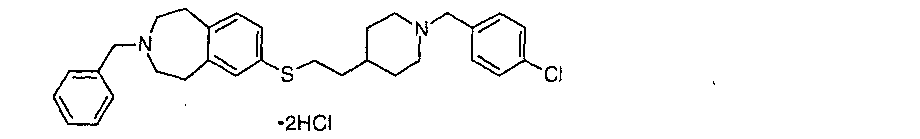

According to the same procedures as those of Example 40) using 3-(phenylmethyl)-7-[[2-(4-piperidinyl)ethyl]sulfanyl]-2,3,4,5-tetrahydro-1H-3-benzazepine (free base, 240 mg, 0.63 mmol) obtained in Reference Example 23) and 4-chlorobenzyl chloride (112 mg, 0.69 mmol), the title compound (204 mg) was obtained as colorless crystals having a melting point of 236°C (dec.). ¹H NMR (CDCl₃, free base) δ 1.10-2.00 (9H, m), 2.55-2.70 (4H, m), 2.75-2.95 (8H, m), 3.43 (2H, s), 3.63 (2H, s), 6.90-7.10 (3H, m), 7.20-7.40 (9H, m).
Elemental analysis for C₃₁H₃₇ClN₂S·2HCl
Calcd.: C, 63.42; H, 6.87; N, 4.77
Found: C, 63.82; H, 6.65; N, 5.12

### Example 42)

7-[[2-[1-[(2-Chlorophenyl)methyl]-4-piperidinyl]ethyl]sulfinyl]-3-(phenylmethyl)-2,3,4,5-tetrahydro-1H-3-benzazepine dihydrochloride

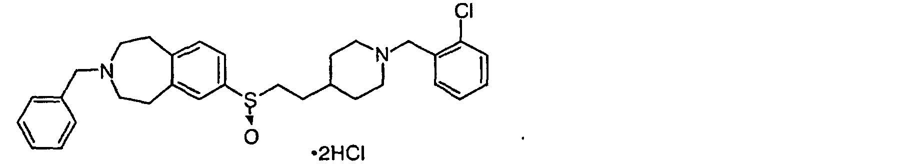

According to the same procedures as those of Example 40) using 3-(phenylmethyl)-7-[[2-(4-piperidinyl)ethyl]sulfinyl]-2,3,4,5-tetrahydro-1H-3-benzazepine (free base, 200 mg, 0.50 mmol) obtained in Reference Example 27) and 2-chlorobenzyl bromide (0.071 ml, 0.55 mmol), a free base (94 mg) of the title compound was obtained as a colorless oil.
¹H NMR (CDCl₃) δ 1.15-1.80 (7H, m), 1.95-2.15 (2H, m), 2.55-2.70 (4H, m), 2.80-3.00 (8H, m), 3.59 (2H, s), 3.63 (2H, s), 6.95-7.50 (12H, m).

A solution of the above free base (90 mg) in ethanol was treated with 2 equivalents of hydrogen chloride (ethanol solution) to obtain the title compound (90 mg) as colorless amorphous powders.
Elemental analysis for C₃₁H₃₇ClN₂OS·2HCl·0.5H₂O
Calcd.: C, 61.74; H, 6.69; N, 4.65
Found: C, 61.58; H, 6.86; N, 4.45

### Example 43)

7-[[2-[1-[(4-chlorophenyl)methyl]-4-piperidinyl]ethyl]sulfinyl]-3-(phenylmethyl)-2,3,4,5-tetrahydro-1H-3-benzazepine dihydrochloride

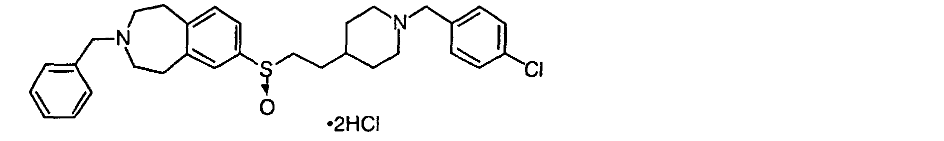

According to the same procedures as those of Example 40) using 3-(phenylmethyl)-7-[[2-(4-piperidinyl)ethyl]sulfinyl]-2,3,4,5-tetrahydro-1H-3-benzazepine (free base, 200 mg, 0.50 mmol) obtained in Reference Example 27) and 4-chlorobenzyl bromide (89 mg, 0.55 mmol), a free base (61 mg) of the title compound was obtained as a colorless oil.
¹H NMR (CDCl₃) δ 1.15-1.80 (7H, m), 1.85-2.00 (2H, m), 2.55-2.70 (4H, m), 2.75-2.95 (8H, m), 3.43 (2H, s), 3.63 (2H, s), 6.95-7.10 (3H, m), 7.20-7.40 (9H, m).

A solution of the above free base (60 mg) in ethanol was treated with 2 equivalents of hydrogen chloride (ethanol solution) to obtain the title compound (22 mg) as colorless crystals having a melting point of 225°C (dec.).

### Example 44)

7-[[2-[1-[(3-Chlorophenyl)methyl]-4-piperidinyl]ethyl]sulfonyl]-3-(phenylmethyl)-2,3,4,5-tetrahydro-1H-3-benzazepine dihydrochloride

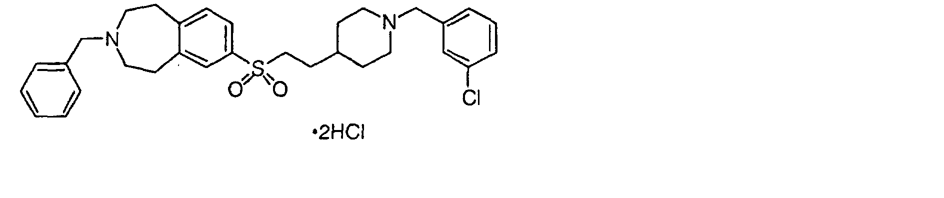

According to the same procedures as those of Example 40) using 3-(phenylmethyl)-7-[[2-(4-piperidinyl)ethyl]sulfonyl]-2,3,4,5-tetrahydro-1H-3-benzazepine (265 mg, 0.64 mmol) obtained in Reference Example 31) and 3-chlorobenzyl bromide (0.093 ml, 0.71 mmol), a free base (251 mg) of the title compound was obtained as a colorless oil.
¹H NMR (CDCl₃) δ 1.10-1.40 (2H, m), 1.50-1.75 (5H, m), 1.80-2.00 (2H, m), 2.55-2.70 (4H, m), 2.75-2.90 (2H, m), 2.95-3.15 (6H, m), 3.42 (2H, s), 3.64 (2H, s), 7.10-7.40 (10H, m), 7.55-7.65 (2H, m).

A solution of the above free base (250 mg) in ethanol was treated with 2 equivalents of hydrogen chloride (ethanol solution) to obtain the title compound (225 mg) as colorless crystals having a melting point of 249°C (dec.).
Elemental analysis for C₃₁H₃₇ClN₂O₂S·2HCl·0.5H₂O
Calcd.: C, 60.14; H, 6.51; N, 4.52
Found: C, 60.25; H, 6.27; N, 4.65

### Example 45)

3-(Phenylmethyl)-7-[[2-[1-[(4-chlorophenyl)methyl]-4-piperidinyl]ethyl]sulfonyl]-2,3,4,5-tetrahydro-1H-3-benzazepine dihydrochloride

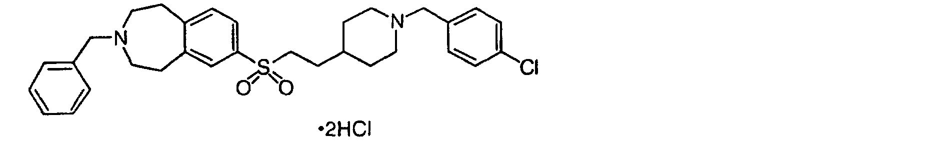

According to the same procedures as those of Example 40) using 3-(phenylmethyl)-7-[[2-(4-piperidinyl)ethyl]sulfonyl]-2,3,4,5-tetrahydro-1H-3-benzazepine (265 mg, 0.64 mmol) obtained in Reference Example 31) and 4-chlorobenzyl chloride (114 mg, 0.71 mmol), a free base (255 mg) of the title compound was obtained as a colorless oil.
¹H NMR (CDCl₃) δ 1.10-1.40 (2H, m), 1.45-1.75 (5H, m), 1.80-1.95 (2H, m), 2.55-2.70 (4H, m), 2.70-2.90 (2H, m), 2.95-3.10 (6H, m), 3.41 (2H, s), 3.63 (2H, s), 7.15-7.40 (10H, m), 7.55-7.65 (2H, m).

A solution of the above free base (250 mg) in ethanol was treated with 2 equivalents of hydrogen chloride (ethanol solution) to obtain the title compound (266 mg) as colorless crystals having a melting point of 256°C (dec.).
Elemental analysis for C₃₁H₃₇ClN₂O₂S·2HCl·0.5H₂O
Calcd.: C, 60.14; H, 6.51; N, 4.52
Found: C, 60.43; H, 6.45; N, 4.64

### Example 46)

N-[[1-(Phenylmethyl)-4-piperidinyl]methyl]-3-(trifluoroacetyl)-2,3,4,5-tetrahydro-1H-3-benzazepine-7-sulfonamide

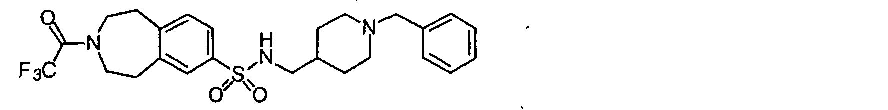

A solution of 3-(trifluoroacetyl)-2,3,4,5-tetrahydro-1H-3-benzazepine-7-sulfonyl chloride (1.50 g, 4.39 mmol) in N,N-dimethylformamide (10 ml) was added dropwise to a mixed solution of [1-(benzyl)-4-piperidinyl]methylamine dihydrochloride (1.34 g, 4.38 mmol) and triethylamine (2.02 ml, 14.5 mmol) in tetrahydrofuran (50 ml). After the mixture was stirred at room temperature for 1.5 hours and the solvent was distilled off under reduced pressure, the residue was dissolved in water-ethyl acetate and extract with ethyl acetate. After the extract was washed with an aqueous saturated solution of sodium chloride and dried with anhydrous magnesium sulfate, the solvent was distilled off under reduced pressure. The resulting residue was purified by silica gel column chromatography (developing solvent; hexane-ethyl acetate=1:1) to obtain the title compound (1.64 g) as a colorless oil.
¹H NMR (CDCl₃) δ 1.05-1.30 (2H, m), 1.30-1.70 (3H, m), 1.80-2.00 (2H, m), 2.75-2.90 (2H, m), 2.96 (2H, s), 3.00-3.10 (4H, m), 3.47 (2H, s), 3.65-3.85 (4H, m), 5.05-5.25 (1H, br), 7.20-7.35 (5H, m), 7.60-7.70 (2H, m), 8.02 (1H, s).

### Example 47)

N-[[1-(Phenylmethyl)-4-piperidinyl]methyl]-2,3,4,5-tetrahydro-1H-3-benzazepine-7-sulfonamide

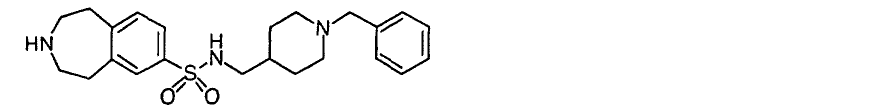

According to the same procedures as those of Reference Example 21) using N-[[1-(phenylmethyl)-4-piperidinyl]methyl]-3-(trifluoroacetyl)-2,3,4,5-tetrahydro-1H-3-benzazepine-7-sulfonamide (1.61 g, 3.16 mmol) obtained in Example 46), the title compound (1.09 g) was obtained as colorless crystals.
¹H NMR (CDCl₃) δ 1.05-1.30 (2H, m), 1.30-1.70 (3H, m), 1.80-1.95 (2H, m), 1.90-2.10 (1H, br), 2.75-3.00 (12H, m), 3.46 (2H, s), 4.60-4.75 (1H, br.), 7.15-7.35 (6H, m), 7.55-7.60 (2H, m).

### Example 48)

3-(Phenylmethyl)-N-[[1-(phenylmethyl)-4-piperidinyl]methyl]-2,3,4,5-tetrahydro-1H-3-benzazepine-7-sulfonamide dihydrochloride

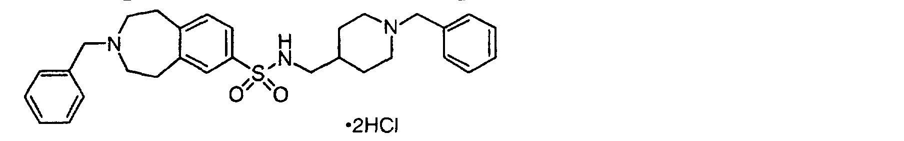

According to the same procedures as those of Example 40) using N-[[1-(phenylmethyl)-4-piperidinyl]methyl]-2,3,4,5-tetrahydro-1H-3-benzazepine-7-sulfonamide (250 mg, 0.60 mmol) obtained in Example 47) and benzyl bromide (0.071 ml, 0.60 mmol), a free base (265 mg) of the title compound (1.09 g) was obtained as a colorless oil.
¹H NMR (CDCl₃) δ 1.05-1.30 (2H, m), 1.30-1.70 (3H, m), 1.80-2.00 (2H, m), 2.55-2.70 (4H, m), 2.75-2.90 (4H, m), 2.90-3.00 (4H, m), 3.46 (2H, s), 3.63 (2H, s), 4.50-4.75 (1H, br), 7.15-7.40 (11H, m), 7.55-7.60 (2H, m).

A solution of the above free base (260 mg) in ethanol was treated with 2 equivalents of hydrogen chloride (ethanol solution) to obtain the title compound (283 mg) as colorless amorphous powders.
Elemental analysis for C₃₀H₃₇N₃O₂S·2HCl·H₂O
Calcd.: C, 60.60; H, 6.95; N, 7.07
Found: C, 60.68; H, 7.15; N, 7.00

### Example 49)

N-[2-(Phenylmethyl)-2,3,4,5-tetrahydro-1H-2-benzazepine-8-yl]-[1-(phenylmethyl)-4-piperidinyl]acetamide

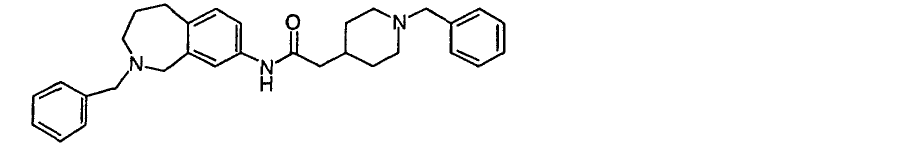

After oxalyl chloride (143 ml, 1.64 mmol) was added dropwise to a solution of [1-(benzyl)-4-piperidinyl]acetic acid (135 mg, 0.544 mmol) and N,N,-dimethylformamide (catalytic amount) in tetrahydrofuran (20 ml) at room temperature, the mixture was stirred for 15 minutes. After the solvent was distilled off under reduced pressure, the precipitated solids were washed twice with hexane (5 ml). After the solids were dissolved in tetrahydrofuran (20 ml), 8-amino-2-(phenylmethyl)-2,3,4,5-tetrahydro-1H-2-benzazepine (135 mg, 0.535 mmol) and triethylamine (0.23 ml, 1.65 mmol) were added at 0°C, the mixture was stirred for 2 hours. After the solvent was distilled off under reduced pressure, the residue was dissolved in water-ethyl acetate and extracted with ethyl acetate. After the extract was washed with an aqueous saturated solution of sodium chloride and dried with anhydrous magnesium sulfate, the solvent was distilled off under reduced pressure to obtain the title compound (170 mg) as colorless crystals having a melting point of 175-176°C.
¹H NMR (CDCl₃) δ 1.25-1.45 (2H, m), 1.60-2.10 (8H, m), 2.23 (2H, d, J = 6.6 Hz), 2.80-2.95 (3H, m), 3.07 (2H, t-like, J = 5.4 Hz), 3.48 (2H, s), 3.52 (2H, s), 3.85 (2H, s), 7.00-7.15 (3H, m), 7.20-7.40 (11H, m).
Elemental analysis for C₃₁H₃₇N₂O·0.5H₂O
Calcd.: C, 78.11; H, 8.04; N, 8.82
Found: C, 77.99; H, 7.60; N, 8.74

### Example 50)

2-(Phenylmethyl)-N-[[1-(phenylmethyl)-4-piperidinyl]methyl]-2,3,4,5-tetrahydro-1H-2-benzazepine-8-carboxamide

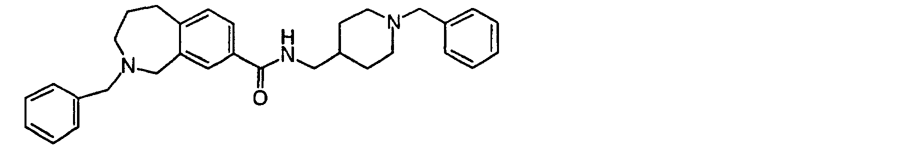

Diethylphosphoryl cyanide (0.237 ml, 1.56 mmol) was added dropwise to a solution of 2-(phenylmethyl)-2,3,4,5-tetrahydro-1H-2-benzazepine-8-carboxylic acid
(400 mg, 1.42 mmol) obtained in Reference Example 38), [1-(phenylmethyl)-4-piperidinyl]methylamine dihydrochloride (433 mg, 1.56 mmol) and triethylamine (0.652 ml, 4.68 mmol) in N,N-dimethylformamide (15 ml) at room temperature, the mixture was stirred for 12 hours. After the solvent was distilled off under reduced pressure, the residue was dissolved in water-ethyl acetate and extracted twice with ethyl acetate. After the extract was washed with an aqueous saturated solution of sodium chloride and dried with anhydrous magnesium sulfate, the solvent was distilled off under reduced pressure. The resulting residue was purified by silica gel column chromatography (developing solvent: ethyl acetate:methanol:triethylamine=45:5:1) to obtain the title compound (314 mg) as colorless crystals having a melting point of 148-149°C.
¹H NMR (CDCl₃) δ 1.20-2.10 (9H, m), 2.85-3.00 (4H, m), 3.13 (2H, t-like, J = 5.6 Hz), 3.33 (2H, t-like, J = 6.2 Hz), 3.51 (2H, s), 3.52 (2H, s), 3.90 (2H, s), 6.05-6.15 (1H, m), 7.15-7.40 (12H, m), 7.58 (1H, dd, J = 7.7, 2.0 Hz).
Elemental analysis for C₃₁H₃₇N₃O·0.5H₂O
Calcd.: C, 78.11; H, 8.04; N, 8.82
Found: C, 78.38; H, 7.88; N, 9.18

### Example 51)

4-[1-(Phenylmethyl)-4-piperidinyl]-2-[3-(phenylmethyl)-2,3,4,5-tetrahydro-1H-3-benzazepine-7-yl]-2-butanol dihydrochloride

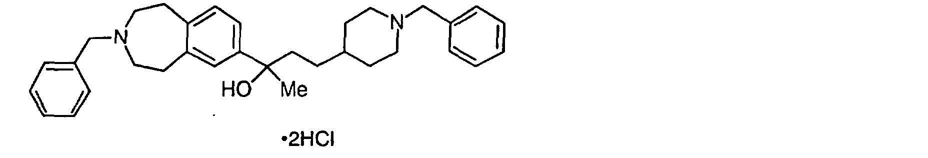

After 1M-methyllithium ethyl ether solution (1.3 ml, 1.3 mmol) was added dropwise to a solution of 3-[1-(phenylmethyl)-4-piperidinyl]-1-[3-(phenylmethyl)-2,3,4,5-tetrahydro-1H-3-benzazepine-7-yl]-1-propanone (300 mg, 0.64 mmol) in tetrahydrofuran (15 ml) at 0°C, the mixture was stirred for 30 minutes. After water (1 ml) was added and the solvent was distilled off under reduced pressure, the residue was dissolved in water-ethyl acetate and extracted with ethyl acetate. After the extract was washed with an aqueous saturated solution of sodium chloride and dried with anhydrous magnesium sulfate, the solvent was distilled off under reduced pressure. The resulting residue was purified by silica gel column chromatography (developing solvent: ethyl acetate) to obtain a free base (334 mg) of the title compound as a colorless oil.
¹H NMR (CDCl₃) δ 1.00-1.30 (4H, m), 1.51 (3H, s), 1.55-2.05 (8H, m), 2.55-2.65 (4H, m), 2.70-2.95 (6H, m), 3.45 (2H, s), 3.64 (2H, s), 6.95-7.15 (3H, m), 7.20-7.40 (10H, m).

A solution of the above free base (330 mg) in ethanol was treated with 2 equivalents of hydrogen chloride (ethanol solution) to obtain the title compound (290 mg) as colorless crystals having a melting point of 250-253°C.
Elemental analysis for C₃₃H₄₂N₂O·2HCl
Calcd.: C, 71.34; H, 7.98; N, 5.04
Found: C, 72.86; H, 7.94; N, 5.27

### Example 52)

3-[1-(Phenylmethyl)-4-piperidinyl]-1-[3-(phenylmethyl)-2,3,4,5-tetrahydro-1H-3-benzazepine-7-yl]]-1-propanone oxime

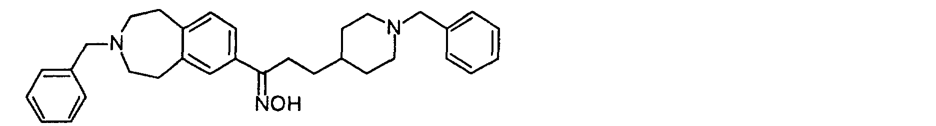

After hydroxylamine hydrochloride (180 mg, 2.6 mmol), sodium acetate (360 mg, 4.4 mmol) and water (20 ml) were added to a solution of 3-[1-(phenylmethyl)-4-piperidinyl]-1-[3-(phenylmethyl)-2,3,4,5-tetrahydro-1H-3-benzazepine-7-yl]-1-propanone (300 mg, 0.64 mmol) in methanol (180 ml), the mixture was heated at reflux for 5 hours. After methanol was distilled off under reduced pressure, the residue was dissolved in water-ethyl acetate and extracted with ethyl acetate. The extract was washed successively with an aqueous solution of potassium carbonate and an aqueous saturated solution of sodium chloride and dried with anhydrous magnesium sulfate, the solvent was distilled off under reduced pressure to obtain the title compound (271 mg) as colorless crystals having a melting point of 171-174°C.
¹H NMR (CDCl₃) δ 1.20-2.00 (10H, m), 2.55-3.00 (12H, m), 3.53 (2H, s), 3.64 (2H, s), 7.06 (1H, d, J = 7.6 Hz), 7.20-7.40 (12H, m).
Elemental analysis for C₃₂H₃₉N₃O·0.5H₂O
Calcd.: C, 78.33; H, 8.22; N, 8.56
Found: C, 78.30; H, 8.20; N, 8.29

### Example 53)

3-[1-(Phenylmethyl)-4-piperidinyl]-1-[3-(phenylmethyl)-2,3,4,5-tetrahydro-1H-3-benzazepine-7-yl]-1-propanone hydrazone

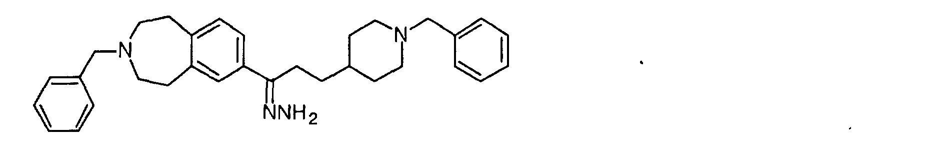

After hydrazine monohydrate (0.126 ml, 12.6 mmol) was added to a solution of 3-[1-(phenylmethyl)-4-piperidinl]-1-[3-(phenylmethyl)-2,3,4,5-tetrahydro-1H-3-benzazepine-7-yl]-1-propanone (300 mg, 0.64 mmol) in methanol (10 ml), the mixture was heated at reflux for 3 hours. After methanol was distilled off under reduced pressure, the residue was dissolved in water-ethyl acetate and extracted with ethyl acetate. After the extract was washed with an aqueous saturated solution of sodium chloride and dried with anhydrous potassium carbonate, the solvent was distilled off under reduced pressure to obtain the title compound (141 mg) as colorless crystals having a melting point of 100 to 102°C.
¹H NMR (CDCl₃) δ 1.20-2.05 (9H, m), 2.50-2.70 (6H, m), 2.80-3.00 (6H, m), 3.49 (2H, s), 3.63 (2H, s), 5.31 (2H, s), 7.04 (1H, d, J = 7.6 Hz), 7.20-7.40 (12H, m).
Elemental analysis for C₃₂H₄ON₄
Calcd.: C, 79.96; H, 8.39; N, 11.66
Found: C, 79.51; H, 8.37; N, 11.46

### Example 54)

2-[3-[1-(Phenylmethyl)-4-piperidinyl]-1-[3-(phenylmethyl)-2,3,4,5-tetrahydro-1H-3-benzazepine-7-yl]propylidene]malononitrile dihydochloride

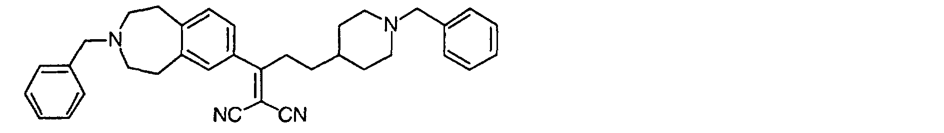

After malononitrile (163 mg, 0.64 mmol) and ammonium acetate (0.55 g, 7.1 mmol) were added to a mixed solution of 3-[1-(phenylmethyl)-4-piperidinyl]-1-[3-(phenylmethyl)-2,3,4,5-tetrahydro-1H-3-benzazepine-7-yl]-1-propanone (300 mg, 0.64 mmol) in acetic acid (5.5 ml)-toluene (5.5 ml), the mixture was heated at reflux for 12 hours. After the solvent was distilled off under reduced pressure, the residue was dissolved in an aqueous solution of potassium carbonate-ethyl acetate and extracted with ethyl acetate. After the extract was washed with an aqueous saturated solution of an aqueous saturated solution of sodium chloride and dried with anhydrous magnesium sulfate, the solvent was distilled off under reduced pressure. The resulting residue was purified by silica gel column chromatography (developing solvent: ethyl acetate:methanol=9:1) to obtain a free base (313 mg) of the title compound as a pale yellow oil.
¹H NMR (CDCl₃) δ 1.15-1.40 (5H, m), 1.55-1.70 (2H, m), 1.80-2.00 (2H, m), 2.60-2.70 (4H, m), 2.80-3.00 (8H, m), 3.47 (2H, s), 3.64 (2H, s), 7.05-7.40 (13H, m).

A solution of the above free base (310 mg) in ethanol was treated with 2 equivalents of hydrogen chloride (ethanol solution) to obtain the title compound (352 mg) as pale yellow amorphous crystals.

### Example 55)

8-[3-[1-[(3-Cyanophenyl)methyl]-4-piperidinyl]propoxy]-2-[(4-fluorophenyl)methyl]-2,3,4,5-tetrahydro-1H-2-benzazepine

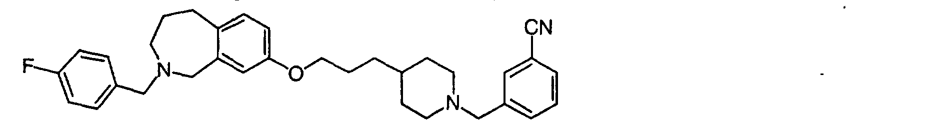

According to the same procedures as those of Example 1) using 2-[(4-fluorophenyl)methyl]-8-[3-(4-piperidinyl)propoxy]-2,3,4,5-tetrahydro-1H-2-benzazepine obtained in Reference Example 42), the title compound was obtained as colorless crystals having a melting point of 97-98°C.
¹H NMR (CDCl₃) δ 1.10-1.45 (4H, m), 1.50-1.80 (7H, m), 1.85-2.05 (2H, m), 2.75-2.90 (4H, m), 3.09 (2H, t-like, J = 5.2 Hz), 3.49 (4H, s), 3.81 (2H, s), 3.87 (2H, t, J = 6.4 Hz), 6.48 (1H, d, J = 2.6 Hz), 6.67 (1H, dd, J = 8.3, 2.6 Hz), 6.95-7.10 (3H, m), 7.20-7.30 (2H, m), 7.40 (1H, t, J = 7.4 Hz), 7.50-7.60 (2H, m), 7.65 (1H, s).
Elemental analysis for C₃₃H₃₈FN₃O
Calcd.: C, 77.46; H, 7.49; N, 8.21
Found: C, 77.30; H, 7.57; N, 8.21

### Example 56)

3-[[4-[3-[[2-[(4-Fluorophenyl)methyl]-2,3,4,5-tetrahydro-1H-2-benzazepine-8-yl]oxy]propyl]-1-piperidinyl]methyl]-1-benzenecarboxyimidamide trihvdrochloride 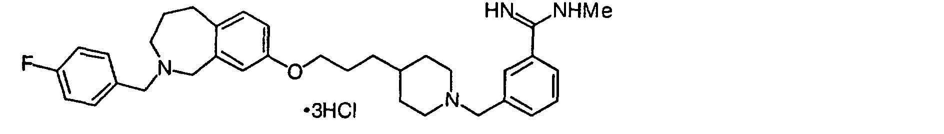
1) A mixture of 8-[3-[1-(3-cyanophenyl)methyl]-4-piperidinyl]propoxy]-2-[(4-fluorophenyl)methyl]-2,3,4,5-tetrahydro-1H-2-benzazepine (1.8 g, 3.52 mmol) obtained in Example 55) and 9.8N hydrochloric acid (ethanol solution, 80 ml) was stirred at room temperature for 16 hours. After the solvent was distilled off under reduced pressure, the residue was dissolved in water-ethyl acetate and extracted with ethyl acetate. The extract was washed with an aqueous saturated solution of sodium chloride and dried with anhydrous magnesium sulfate, the solvent was distilled off to obtain ethyl 3-[[4-[3-[[2-[(4-fluorophenyl)methyl]-2,3,4,5-tetrahydro-1H-2-benzazepine-8-yl]oxy]propyl]-1-piperidinyl]methyl]-1-benzenecarboxyimidate (2.18 g) as a colorless oil.
2) A solution of ethyl 3-[[4-[3-[[2-[(4-fluorophenyl)methyl]-2,3,4,5-tetrahydro-1H-2-benzazepine-8-yl]oxy]propyl]-1-piperidinyl]methyl]-1-benzenecarboxyimidate (500 mg, 0.9 mmol) obtained in 1) and 40% methylamine (methanol solution, 10 ml) in methanol (10 ml) was heated at 120°C for 30 minutes in a stainless pressure-resistant tube. The solvent was distilled off under reduced pressure, the residue was dissolved in ethyl acetate-a 1N aqueous solution of sodium hydroxide, and extracted with ethyl acetate. After the extract was washed with an aqueous saturated solution of sodium chloride and dried with anhydrous potassium carbonate, the solvent was distilled off under reduced pressure. The resulting residue was purified by column chromatography (developing solvent: ethyl acetate-methonal-NH4OH=1:1:0.03) using basic active alumina to obtain the title compound (512 mg) as colorless amorphous powders.
   ¹H NMR (CDCl₃, free base) δ 1.15-1.45 (5H, m), 1.55-2.05 (9H, m), 2.75-2.90 (4H, m), 2.98 (3H, s), 3.08 (2H, t-like, J = 5.2 Hz), 3.49 (4H, s), 3.80 (2H, s), 3.87 (2H, t, J = 6.4 Hz), 5.60-6.20 (1H, br), 6.47 (1H, d, J = 2.6 Hz), 6.66 (1H, dd, J = 8.0, 2.6 Hz), 6.90-7.05 (3H, m), 7.20-7.50 (5H, m), 7.53 (1H, s).
   Elemental analysis for C₃₄H₄₃FN₄O·3HCl·2H₂O
   Calcd.: C, 59.34; H, 7.32; N, 8.14
   Found: C, 59.27; H, 7.74; N, 8.41

### Example 57)

8-[3-[1-[[3-(4,5-Dihydro-1H-2-imidazolyl)phenyl]methyl]-4-piperidinyl]propoxy]-2-[(4-fluorophenyl)methyl]-2,3,4,5-tetrahydro-1H-2-benzazepine

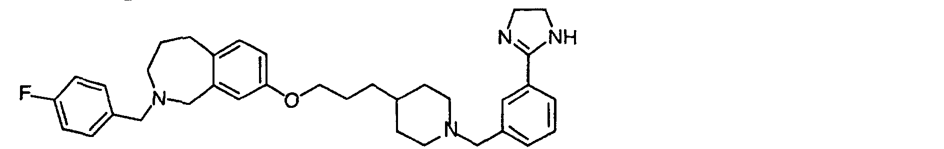

According to the same procedures as those of Example 56)-2 using ethyl 3-[[4-[3-[[2-[(4-fluorophenyl)methyl]-2,3,4,5-tetrahydro-1H-2-benzazepine-8-yl]oxy]propyl]-1-piperidinyl]methyl]-1-benzenecarboxyimidate obtained in Reference Example 56)-1) and ethylenediamine, the title compound was obtained as colorless crystals having a melting point of 119-121°C.
¹H NMR (CDCl₃) δ 1.15-1.45 (5H, m), 1.55-2.05 (9H, m), 2.80-2.90 (4H, m), 3.08 (2H, t-like, J = 5.2 Hz), 3.49 (4H, s), 3.50 (2H, s), 3.70-3.95 (6H, m), 6.48 (1H, d, J = 2.6 Hz), 6.66 (1H, dd, J = 8.0, 2.6 Hz), 6.90-7.05 (3H, m), 7.20-7.45 (4H, m), 7.66 (1H, d, J = 7.0 Hz), 7.74 (1H, s).
Elemental analysis for C₃₅H₄₃FN₄O
Calcd.: C, 75.78; H, 7.81; N, 10.10
Found: C, 75.33; H, 7.59; N, 10.05

### Example 58)

7-[2-[1-[(2-Methylphenyl)methyl]-4-piperidinyl]ethoxy]-2-[[2-(trifluoromethyl)phenyl]methyl]-2,3,4,5-tetrahydro-1H-2-bezazeDine dihydrochloride

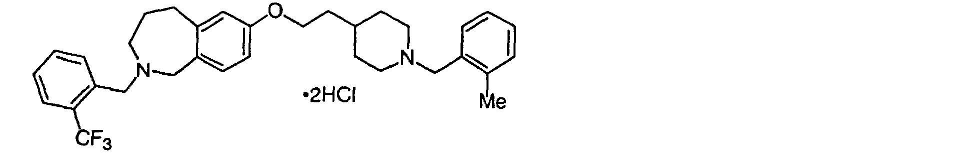

According to the same procedures as those of Example 1) using 2-[[2-(trifluoromethyl)phenyl]methyl]-7-[2-(4-piperidinyl)ethoxy]-2,3,4,5-tetrahydro-lH-2-benzazepine obtained in Reference Example 41), the title compound was obtained as colorless amorphous powders.
¹H NMR (CDCl₃, free base) δ 1.15-1.85 (9H, m), 1.90-2.05 (2H, m), 2.35 (3H, s), 2.80-2.95 (4H, m), 3.03 (2H, t-like, J = 5.2 Hz), 3.42 (2H, s), 3.71 (2H, s), 3.74 (2H, s), 3.96 (2H, t, J = 6.2 Hz), 6.58 (1H, dd, J = 8.4, 2.6 Hz), 6.71 (1H, d, J = 2.6 Hz), 6.82 (1H, d, J = 8.4 Hz), 7.05-7.35 (5H, m), 7.48 (1H, t, J = 7.4 Hz), 7.60 (1H, d, J = 7.2 Hz), 7.81 (1H, d, J = 7.2 Hz).

### Example 59)

7-[2-[1-[(2-Chlorophenyl)methyl]-4-piperidinyl]ethoxy]-2-[[2-(trifluoromethyl)phenyl]methyl]-2,3,4,5-tetrahydro-1H-2-benzazepine dihydrochloride

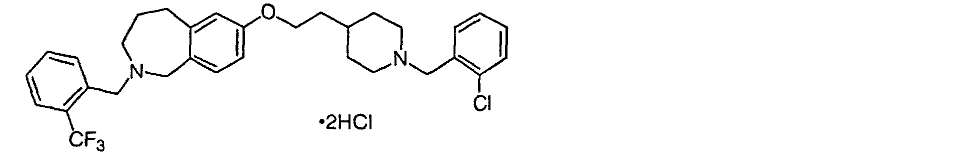

According to the same procedures as those of Example 1) using 2-[[2-(trifluoromethyl)phenyl]methyl]-7-[2-(4-piperidinyl)ethoxy]-2,3,4,5-tetrahydro-1H-2-benzazepine obtained in Reference Example 41), the title compound was obtained as colorless amorphous powders.
¹H NMR (CDCl₃, free base) *δ* 1.20-1.85 (9H, m), 2.00-2.20 (2H, m), 2.80-2.95 (4H, m), 3.04 (2H, t-like, J = 5.2 Hz), 3.50 (2H, s), 3.71 (2H, s), 3.75 (2H, s), 3.98 (2H, t, J = 6.2 Hz), 6.58 (1H, dd, J = 8.4, 2.6 Hz), 6.72 (1H, d, J = 2.6 Hz), 6.83 (1H, d, J = 8.4 Hz), 7.10-7.35 (4H, m), 7.40-7.55 (2H, m), 7.61 (1H, d, J = 7.8 Hz), 7.81 (1H, d, J = 7.2 Hz).

### Example 60)

2-[1-[2-[(4-Fluorophenyl)methyl]-2,3,4,5-tetrahydro-1H-2-bezazepine-8-yl]-3-[1-[(2-methylphenyl)methyl]-4-piperidinyl]propylidene]malononitrile dihydrochloride

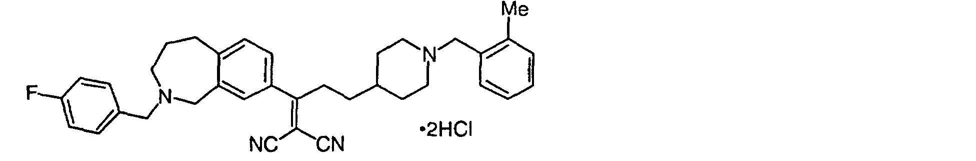

According to the same procedures as those of Example 54) using 3-[1-[(2-methylphenyl)methyl]-4-piperidinyl]-1-[3-[(4-fluorophenyl)methyl]-2,3,4,5-tetrahydro-1H-3-benzazepine-7-yl]-1-propanone, the title compound was obtained as colorless amorphous powders.
¹H NMR (CDCl₃, free base) δ 1.05-1.40 (5H, m), 1.50-1.65 (2H, m), 1.70-1.85 (2H, m), 1.85-2.00 (2H, m), 2.33 (3H, s), 2.75-3.00 (6H, m), 3.10 (2H, t-like, J = 5.2 Hz), 3.40 (2H, s), 3.50 (2H, s), 3.87 (2H, s), 6.90-7.30 (11H, m).

### Example 61)

2-[(4-Fluorophenyl)methyl]-8-[4-[1-[(2-methylphenyl)methyl]-4-piperidinyl]-2-hydroxy-2-butyl]-2,3,4,5-tetrahydro-1H-2-benzazepine dihydrochloride

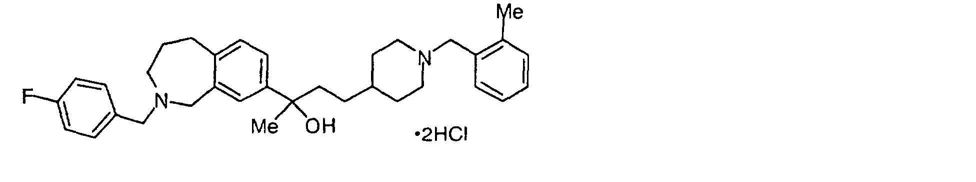

According to the same procedures as those of Example 51) using 3-[1-[(2-methylphenyl)methyl]-4-piperidinyl]-1-[3-[(4-fluorophenyl)methyl]-2,3,4,5-tetrahydro-1H-3-benzazepine-7-yl]-1-propanone, the title compound was obtained as colorless amorphous powders.
¹H NMR (CDCl₃, free base) δ 1.05-1.35 (5H, m), 1.49 (3H, s), 1.50-2.00 (9H, m), 2.32 (3H, s), 2.75-2.95 (4H, m), 3.12 (2H, t-like, J = 5.2 Hz), 3.38 (2H, s), 3.47 (2H, s), 3.84 (2H, s), 6.85-7.30 (11H, m).

### Example 62)

4-[1-(Phenylmethyl)-4-piperidinyl]-1-[3-(phenylmethyl)-2,3,4,5-tetrahydro-1H-3-benzazepine-7-yl]-1-butanol hydrazone trihydrochloride

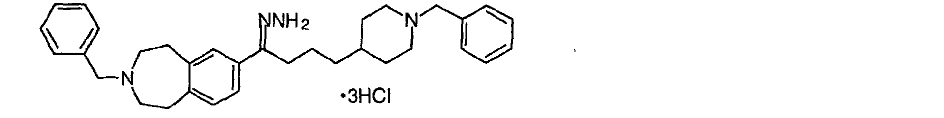

According to the same procedures as those of Example 53) using 4-[1-(phenylmethyl)-4-piperidinyl]-1-[3-(phenylmethyl)-2,3,4,5-tetrahydro-1H-3-benzazepine-7-yl]-1-butanone, the title compound was obtained as colorless powders having a melting point of 211-215°C (dec.).
¹H NMR (CDCl₃, free base) δ 1.10-1.70 (9H, m), 1.80-2.00 (2H, m), 2.35-2.70 (6H, m), 2.75-3.00 (6H, m), 3.47 (2H, s), 3.63 (2H, s), 4.95-5.05 (2H×1/6, br), 5.25-5.40 (2H ×5/6, br), 6.90-7.40 (13H, m).
Elemental analysis for C₃₃H₄₂N₄·3HCl·3H₂O
Calcd.: C, 60.22; H, 7.81; N, 8.51
Found: C, 60.23; H, 7.37; N, 8.46

### Example 63)

3-(Phenylmethyl)-7-[3-[1-(phenylmethyl)-4-piperidinyl]-1-hydroxypropyl]-2,3,4,5-tetrahydro-1H-3-benzazepine

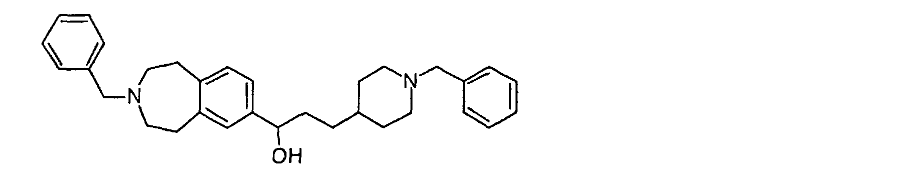

According to the same procedures as those of Example 7)-2) using 3-[1-(phenylmethyl)-4-piperidinyl]-1-[3-(phenylmethyl)-2,3,4,5-tetrahydro-1H-3-benzazepine-7-yl]-1-propanone, the title compound was obtained as colorless crystals having a melting point of 112-113°C. ¹H NMR (CDCl₃) δ 1.05-2.00 (12H, m), 2.55-2.65 (4H, m), 2.75-2.95 (6H, m), 3.47 (2H, s), 3.63 (2H, s), 4.56 (1H, t-like, J = 6.4 Hz), 7.05 (3H, s), 7.20-7.40 (10H, m).
Elemental analysis for C₃₂H₄₀N₂O
Calcd.: C, 82.01; H, 8.60; N, 5.98
Found: C, 82.18; H, 8.62; N, 5.91

### Example 64)

N-[[1-(Phenylmethyl)-4-piperidinyl]methyl]-3-[(3-methylphenyl)methyl]-2,3,4,5-tetrahydro-1H-3-benzazepine-7-sulfonamide dihydrochloride

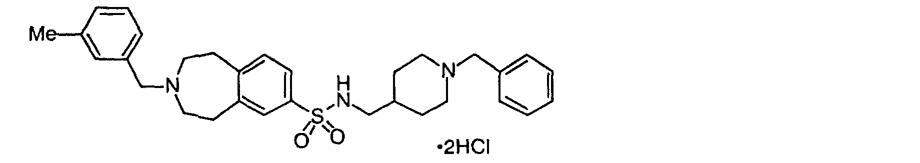

According to the same procedures as those of Example 1) using N-[[1-(phenylmethyl)-4-piperidinyl]methyl]-2,3,4,5-tetrahydro-1H-3-benzazepine-7-sulfonamide obtained in Example 47), the title compound was obtained as colorless amorphous powders.
¹H NMR (CDCl₃, free base) δ 1.05-1.70 (5H, m), 1.80-1.95 (2H, m), 2.36 (3H, s), 2.55-2.70 (4H, m), 2.75-2.90 (4H, m), 2.90-3.00 (4H, m), 3.45 (2H, s), 3.59 (2H, s), 4.60-4.80 (1H, br), 7.05-7.35 (10H, m), 7.55-7.60 (2H, m).
Elemental analysis for C₃₁H₃₉N₃O₂S·2HCl·H₂O
Calcd.: C, 61.17; H, 7.12; N, 6.90
Found: C, 61.52; H, 7.48; N, 7.15

### Example 65)

N-[[1-(Phenylmethyl)-4-piperidinyl]methyl]-3-[(4-methylphenyl)methyl]-2,3,4,5-tetrahydro-1H-3-benzazepine-7-sulfonamide

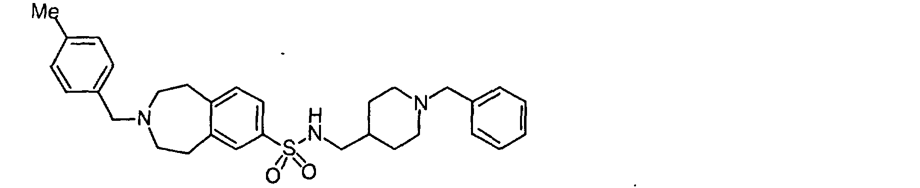

According to the same procedures as those of Example 40) using N-[[1-(phenylmethyl)-4-piperidinyl]methyl]-2,3,4,5-tetrahydro-1H-3-benzazepine-7-sulfonamide obtained in Example 47), the title compound was obtained as colorless amorphous powders.
¹H NMR (CDCl₃) δ 1.05-1.75 (5H, m), 1.80-1.95 (2H, m), 2.35 (3H, s), 2.55-2.70 (4H, m), 2.75-3.00 (8H, m), 3.46 (2H, s), 3.59 (2H, s), 4.40 (1H, t, J = 6.6 Hz), 7.10-7.35 (10H, m), 7.55-7.60 (2H, m).
Elemental analysis for C₃₁H₃₉N₃O₂S
Calcd.: C, 71.92; H, 7.59; N, 8.12
Found: C, 71.51; H, 7.37; N, 8.47

### Example 66)

7-[[2-[1-[(2-Chlorophenyl)methyl]-4-piperidinyl]ethyl]sulfanyl]-3-(phenylmethyl)-2,3,4,5-tetrahydro-1H-3-benzazepine dihydrochloride

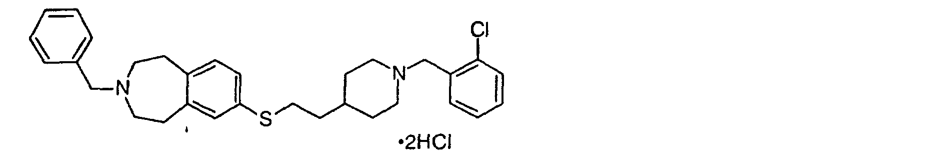

According to the same procedures as those of Example 40) using 3-(phenylmethyl)-7-[[2-(4-piperidinyl)ethyl]sulfanyl]-2,3,4,5-tetrahydro-1H-3-benzazepine (free base) obtained in Reference Example 23), the title compound was obtained as colorless amorphous powders.
¹H NMR (CDCl₃, free base) δ 1.15-1.75 (7H, m), 1.95-2.15 (2H, m), 2.55-2.70 (4H, m), 2.80-3.00 (8H, m), 3.59 (2H, s), 3.63 (2H, s), 6.95-7.50 (12H, m).
Elemental analysis for C₃₁H₃₇ClN₂S·1.5H₂O
Calcd.: C, 61.53; H, 7.00; N, 4.63
Found: C, 61.66; H, 7.03; N, 4.69

### Example 67)

7-[[2-[1-[(3-Chlorophenyl)methyl]-4-piperidinyl]ethyl]sulfanyl]-3-(phenylmethyl)-2,3,4,5-tetrahydro-1H-3-benzazepine dihydrochloride

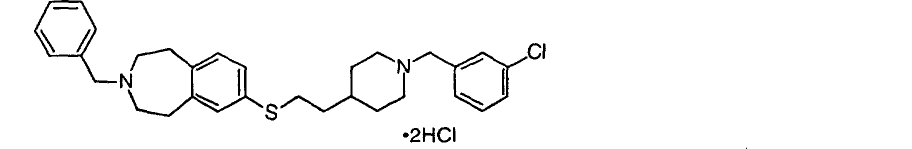

According to the same procedures as those of Example 40) using 3-(phenylmethyl)-7-[[2-(4-piperidinyl)ethyl]sulfanyl]-2,3,4,5-tetrahydro-1H-3-benzazepine (free base) obtained in Reference Example 23), the title compound was obtained as colorless crystals having a melting point of 236 (dec.).
¹H NMR (CDCl₃, free base) δ 1.15-1.75 (7H, m), 1.85-2.00 (2H, m), 2.55-2.70 (4H, m), 2.75-2.95 (8H, m), 3.43 (2H, s), 3.63 (2H, s), 6.95-7.40 (12H, m).
Elemental analysis for C₃₁H₃₇ClN₂S·2HCl·0.5H₂O
Calcd.: C, 63.42; H, 6.87; N, 4.77
Found: C, 63.84; H, 6.74; N, 5.02

### Example 68)

3-(Phenylmethyl)-7-[[2-[1-(phenylmethyl)-4-piperidinyl]ethyl]sulfinyl]-2,3,4,5-tetrahydro-1H-3-benzazepine dihydrochloride

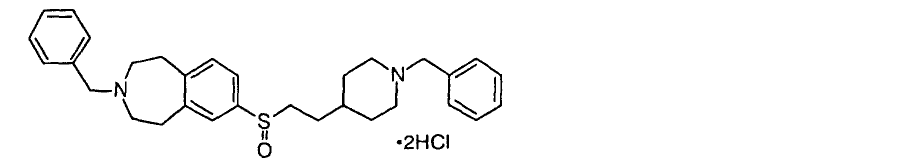

According to the same procedures as those of Example 40) using 3-(phenylmethyl)-7-[[2-(4-piperidinyl)ethyl]sulfinyl]-2,3,4,5-tetrahydro-1H-3-benzazepine (free base) obtained in Reference Example 27), the title compound was obtained as colorless amorphous powders.
¹H NMR (CDCl₃, free base) δ 1.15-2.00 (9H, m), 2.55-2.70 (4H, m), 2.80-2.95 (8H, m), 3.48 (2H, s), 3.63 (2H, s), 6.95-7.10 (3H, m), 7.20-7.40 (10H, m).
Elemental analysis for C₃₁H₃₈N₂OS·2HCl·H₂O
Calcd.: C, 64.46; H, 7.33; N, 4.85
Found: C, 64.18; H, 7.44; N, 4.95

### Example 69)

7-[[2-[1-[(3-Chlorophenyl)methyl]-4-piperidinyl]ethyl]sulfinyl]-3-(phenylmethyl)-2,3,4,5-tetrahydro-1H-3-benzazepine

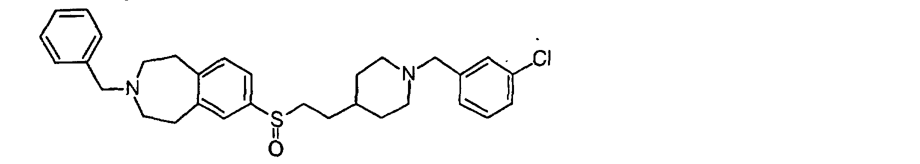

According to the same procedures as those of Example 40) using 3-(phenylmethyl)-7-[[2-(4-piperidinyl)ethyl]sulfinyl]-2,3,4,5-tetrahydro-1H-3-benzazepine (free base) obtained in Reference Example 27), the title compound was obtained as colorless amorphous powders.
¹H NMR (CDCl₃) δ 1.15-1.75 (7H, m), 1.85-2.05 (2H, m), 2.55-2.70 (4H, m), 2.75-2.95 (8H, m), 3.45 (2H, s), 3.64 (2H, s), 6.95-7.10 (3H, m), 7.20-7.40 (9H, m).

### Example 70)

3-(Phenylmethyl)-7-[[2-[1-(phenylmethyl)-4-piperidinyl]ethyl]sulfonyl]-2,3,4,5-tetrahydro-1H-3-benzazepine dihydrochloride

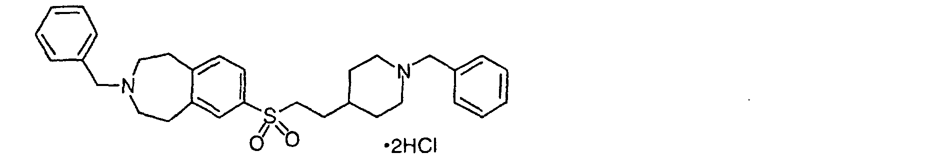

According to the same procedures as those of Example 40) using 3-(phenylmethyl)-7-[[2-(4-piperidinyl)ethyl]sulfonyl]-2,3,4,5-tetrahydro-1H-3-benzazepine obtained in Reference Example 31), the title compound was obtained as colorless amorphous powders.
¹H NMR (CDCl₃, free base) δ 1.10-1.35 (2H, m), 1.45-1.75 (5H, m), 1.80-1.95 (2H, m), 2.55-2.70 (4H, m), 2.80-2.90 (2H, m), 2.95-3.15 (6H, m), 3.45 (2H, s), 3.63 (2H, s), 7.20-7.40 (11H, m), 7.55-7.65 (2H, m).
Elemental analysis for C₃₁H₃₈N₂O₂S·2HCl·0.5H₂O
Calcd.: C, 63.69; H, 7.07; N, 4.79
Found: C, 64.03; H, 6.98; N, 4.91

### Example 71)

7-[[2-[1-[(2-Chlorophenyl)methyl]-4-piperidinyl]ethyl]sulfonyl]-3-(phenylmethyl)-2,3,4,5-tetrahydro-1H-3-benzazepine dihydrochloride

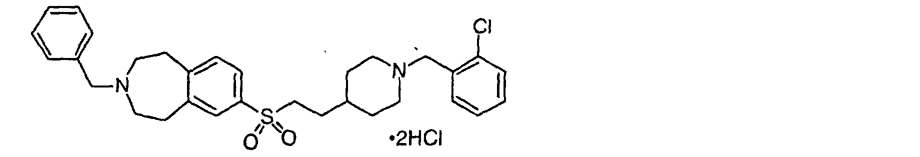

According to the same procedures as those of Example 40) using 3-(phenylmethyl)-7-[[2-(4-piperidinyl)ethyl]sulfonyl]-2,3,4,5-tetrahydro-1H-3-benzazepine obtained in Reference Example 31), the title compound was obtained as colorless amorphous powders.
¹H NMR (CDCl₃, free base) δ 1.10-1.40 (2H, m), 1.50-1.75 (5H, m), 1.85-2.10 (2H, m), 2.55-2.70 (4H, m), 2.80-2.90 (2H, m), 2.95-3.15 (6H, m), 3.56 (2H, s), 3.63 (2H, s), 7.10-7.45 (10H, m), 7.55-7.65 (2H, m).
Elemental analysis for C₃₁H₃₇N₂O₂S·2HCl·H₂O
Calcd.: C, 59.28; H, 6.58; N, 4.46
Found: C, 59.24; H, 6.76; N, 4.43

### Example 72)

2-[(4-Fluorophenyl)methyl]-7-[[2-[1-(phenylmethyl)-4-piperidinyl]ethoxy]-2,3,4,5-tetrahydro-1H-2-benzazepine dihydrochloride

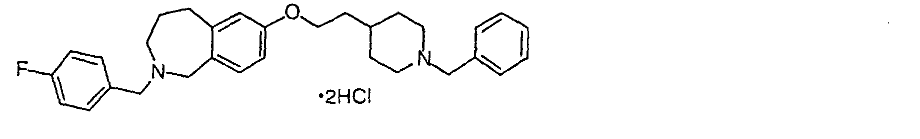

According to the same procedures as those of Example 1) using 2-[(4-fluorophenyl)methyl]-7-[2-(4-piperidinyl)ethoxy]-2,3,4,5-tetrahydro-1H-2-benzazepine (free base) obtained in Reference Example 40), the title compound was obtained as colorless amorphous powders.
¹H NMR (CDCl₃, free base) δ 1.20-1.80 (9H, m), 1.85-2.05 (2H, m), 2.80-2.95 (4H, m), 3.08 (2H, t-like, J = 5.2 Hz), 3.46 (2H, s), 3.49 (2H, s), 3.78 (2H, s), 3.97 (2H, t, J = 6.2 Hz), 6.58 (1H, dd, J = 8.4, 2.6 Hz), 6.70 (1H, d, J = 2.6 Hz), 6.80 (1H, d, J = 8.4 Hz), 6.98 (2H, t-like, J = 8.8 Hz), 7.20-7.40 (7H, m).
Elemental analysis for C₃₁H₃₇FN₂O·2HCl·1.5H₂O
Calcd.: C, 65.03; H, 7.39; N, 4.89
Found: C, 65.20; H, 7.51; N, 4.80

### Example 73)

2-[(4-Fluorophenyl)methyl]-8-[[2-[1-(phenylmethyl)-4-piperidinyl]ethoxy]-2,3,4,5-tetrahydro-1H-2-benzazepine dihydrochloride

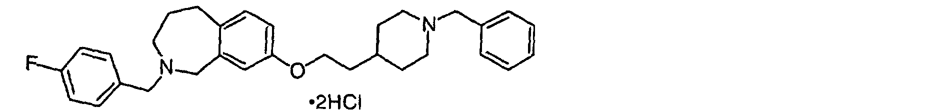

According to the same procedures as those of Example 1) using 2-[(4-fluorophenyl)methyl]-8-[2-(4-piperidinyl)ethoxy]-2,3,4,5-tetrahydro-1H-2-benzazepine (free base) obtained in Reference Example 39), the title compound was obtained as colorless amorphous powders.
¹H NMR (CDCl₃, free base) δ 1.20-1.80 (9H, m), 1.85-2.10 (2H, m), 2.80-2.95 (4H, m), 3.08 (2H, t-like, J = 5.2 Hz), 3.49 (4H, s), 3.80 (2H, s), 3.92 (2H, t, J = 6.4 Hz), 6.47 (1H, d, J = 2.6 Hz), 6.66 (1H, dd, J = 8.2, 2.6 Hz), 6.90-7.10 (3H, m), 7.20-7.35 (7H, m).
Elemental analysis for C₃₁H₃₇FN₂O·2HCl·H₂O
Calcd.: C, 66.07; H, 7.33; N, 4.97
Found: C, 66.25; H, 7.59; N, 4.84

### Example 74)

2-[(4-Fluorophenyl)methyl]-8-[2-[1-[(3-methylphenyl)methyl]-4-piperidinyl]ethoxy]-2,3,4,5-tetrahydro-1H-2-benzazepine dihydrochloride

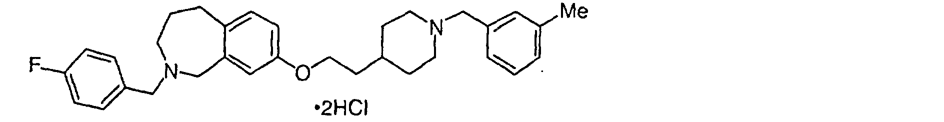

According to the same procedures as those of Example 1) using 2-[(4-fluorophenyl)methyl]-8-[2-(4-piperidinyl)ethoxy]-2,3,4,5-tetrahydro-1H-2-benzazepine (free base) obtained in Reference Example 39), the title compound was obtained as colorless amorphous powders.
¹H NMR (CDCl₃, free base) δ 1.20-1.80 (9H, m), 1.85-2.05 (2H, m), 2.34 (3H, s), 2.80-2.95 (4H, m), 3.09 (2H, t-like, J = 5.2 Hz), 3.45 (2H, s), 3.49 (2H, s), 3.80 (2H, s), 3.92 (2H, t, J = 6.4 Hz), 6.47 (1H, d, J = 2.6 Hz), 6.66 (1H, dd, J = 8.2, 2.6 Hz), 6.90-7.40 (9H, m).
Elemental analysis for C₃₂H₃₉FN₂O·2HCl·H₂O
Calcd.: C, 66.54; H, 7.50; N, 4.85
Found: C, 66.93; H, 7.85; N, 4.82

### Example 75)

8-[2-[1-[(3-Chlorophenyl)methyl]-4-piperidinyl]ethoxy]-2-[(4-fluorophenyl)methyl]-2,3,4,5-tetrahydro-1H-2-benzazepine dihvdrochloride

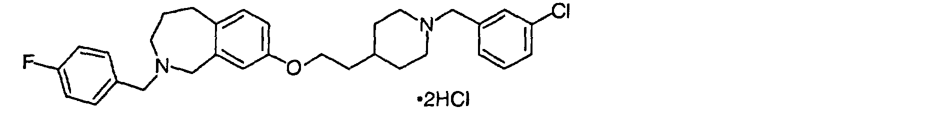

According to the same procedures as those of Example 1) using 2-[(4-fluorophenyl)methyl]-8-[2-(4-piperidinyl)ethoxy]-2,3,4,5-tetrahydro-1H-2-benzazepine (free base) obtained in Reference Example 39), the title compound was obtained as colorless amorphous powders.
¹H NMR (CDCl₃, free base) δ 1.20-1.80 (9H, m), 1.85-2.05 (2H, m), 2.75-2.95 (4H, m), 3.09 (2H, t-like, J = 5.2 Hz), 3.45 (2H, s), 3.49 (2H, s), 3.80 (2H, s), 3.93 (2H, t, J = 6.4 Hz), 6.48 (1H, d, J = 2.6 Hz), 6.67 (1H, dd, J = 8.2, 2.6 Hz), 6.90-7.10 (3H, m), 7.15-7.35 (6H, m).
Elemental analysis for C₃₁H₃₆FN₂O·2HCl·0.5H₂O
Calcd.: C, 63.21; H, 6.67; N, 4.76
Found: C, 63.02; H, 6.98; N, 4.56

### Example 76)

8-[2-(1-Acetyl-4-piperidinyl]ethoxy]-2-[(4-fluorophenyl)methyl]-2,3,4,5-tetrahydro-1H-2-benzazepine hydrochloride

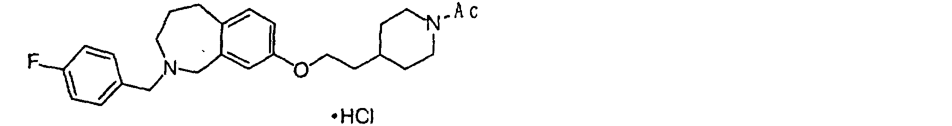

According to the same procedures as those of Example 1) using 2-[(4-fluorophenyl)methyl]-8-[2-(4-piperidinyl)ethoxy]-2,3,4,5-tetrahydro-1H-2-benzazepine (free base) obtained in Reference Example 39), the title compound was obtained as colorless amorphous powders.
¹H NMR (CDCl₃, free base) δ 1.05-1.30 (2H, m), 1.65-1.90 (8H, m), 2.09 (3H, s), 2.45-2.65 (1H, m), 2.80-2.90 (2H, m), 3.00-3.15 (2H, m), 3.50 (2H, s), 3.70-3.90 (3H, m), 3.94 (2H, t, J = 6.4 Hz), 4.50-4.70 (1H, m), 6.48 (1H, d, J = 2.6 Hz), 6.67 (1H, dd, J = 8.0, 2.6 Hz), 6.90-7.10 (3H, m), 7.20-7.30 (2H, m).
Elemental analysis for C₂₆H₃₃FN₂O₂·HCl·2H₂O
Calcd.: C, 62.83; H, 7.71; N, 5.64
Found: C, 63.03; H, 7.58; N, 5.24

### Example 77)

2-[(4-Fluorophenyl)methyl]-8-[2-[1-[(2-methylphenyl)methyl]-4-piperidinyl]ethoxy]-2,3,4,5-tetrahvdro-1H-2-benzazeDine dihvdrochloride

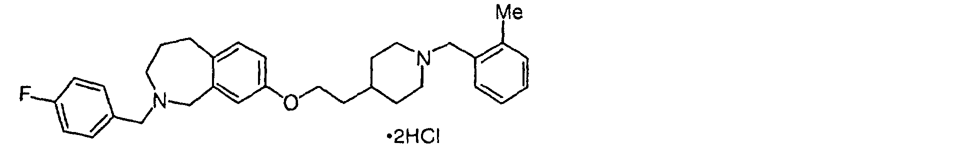

According to the same procedures as those of Example 1) using 2-[(4-fluorophenyl)methyl]-8-[2-(4-piperidinyl)ethoxy]-2,3,4,5-tetrahydro-1H-2-benzazepine (free base) obtained in Reference Example 39), the title compound was obtained as colorless amorphous powders.
¹H NMR (CDCl₃, free base) δ 1.15-1.80 (9H, m), 1.90-2.05 (2H, m), 2.35 (3H, s), 2.75-2.95 (4H, m), 3.08 (2H, t-like, J = 5.2 Hz), 3.43 (2H, s), 3.49 (2H, s), 3.80 (2H, s), 3.92 (2H, t, J = 6.4 Hz), 6.48 (1H, d, J = 2.6 Hz), 6.66 (1H, dd, J = 8.2, 2.6 Hz), 6.90-7.30 (9H, m).
Elemental analysis for C₃₂H₃₉FN₂O·2HCl·H₂O
Calcd.: C, 66.54; H, 7.50; N, 4.85
Found: C, 66.57; H, 7.34; N, 4.61

### Example 78)

8-[2-[1-[(2-Chlorophenyl)methyl]-4-piperidinyl]ethoxy]-2-[(4-fluorophenyl)methyl]-2,3,4,5-tetrahydro-1H-2-benzazepine dihydrochloride

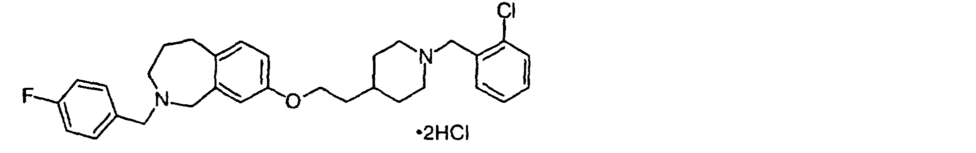

According to the same procedures as those of Example 1) using 2-[(4-fluorophenyl)methyl]-8-[2-(4-piperidinyl)ethoxy]-2,3,4,5-tetrahydro-1H-2-benzazepine (free base) obtained in Reference Example 39), the title compound was obtained as colorless amorphous powders.
¹H NMR (CDCl₃, free base) δ 1.20-1.80 (9H, m), 2.00-2.15 (2H, m), 2.75-2.95 (4H, m), 3.09 (2H, t-like, J = 5.2 Hz), 3.49 (2H, s), 3.60 (2H, s), 3.80 (2H, s), 3.93 (2H, t, J = 6.4 Hz), 6.48 (1H, d, J = 2.8 Hz), 6.66 (1H, dd, J = 8.2, 2.8 Hz), 6.90-7.30 (7H, m), 7.34 (1H, dd, J = 7.4, 1.8 Hz), 7.49 (1H, dd, J = 7.4, 1.8 Hz).
Elemental analysis for C₃₁H₃₆ClFN₂O·2HCl·H₂O
Calcd.: C, 62.26; H, 6.74; N, 4.68
Found: C, 62.16; H, 7.14; N, 4.53

### Example 79)

2-[(4-Fluorophenyl)methyl]-8-[2-[1-[(2-trifluoromethyl)phenyl]methyl]-4-piperidinyl]ethoxy]-2,3,4,5-tetrahydro-1H-2-benzazepine dihydrochloride

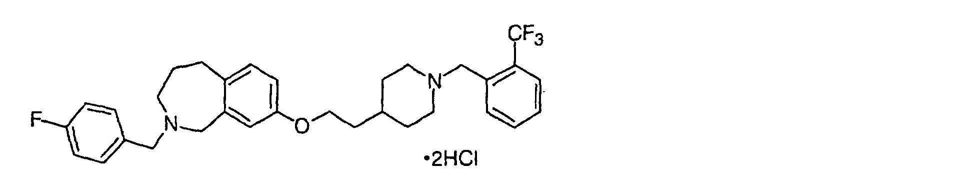

According to the same procedures as those of Example 1) using 2-[(4-fluorophenyl)methyl]-8-[2-(4-piperidinyl)ethoxy]-2,3,4,5-tetrahydro-1H-2-benzazepine (free base) obtained in Reference Example 39), the title compound was obtained as colorless amorphous powders.
¹H NMR (CDCl₃, free base) δ 1.20-1.80 (9H, m), 1.95-2.15 (2H, m), 2.75-2.95 (4H, m), 3.09 (2H, t-like, J = 5.2 Hz), 3.49 (2H, s), 3.63 (2H, s), 3.81 (2H, s), 3.93 (2H, t, J = 6.4 Hz), 6.48 (1H, d, J = 2.6 Hz), 6.67 (1H, dd, J = 8.2, 2.6 Hz), 6.90-7.10 (3H, m), 7.20-7.35 (3H, m), 7.50 (1H, t, J = 7.6 Hz), 7.60 (1H, d, J = 7.6 Hz), 7.82 (1H, d, J = 7.6 Hz).
Elemental analysis for C₃₂H₃₆F₄N₂O·2HCl·H₂O
Calcd.: C, 60.86; H, 6.38; N, 4.44
Found: C, 60.57; H, 6.37; N, 4.31

### Example 80)

2-[(4-Fluorophenyl)methyl]-8-[2-[1-[[3-(trifluoromethyl)phenyl]methyl]-4-piperidinyl]ethoxy]-2,3,4,5-tetrahydro-1H-2-benzazepine dihydrochloride

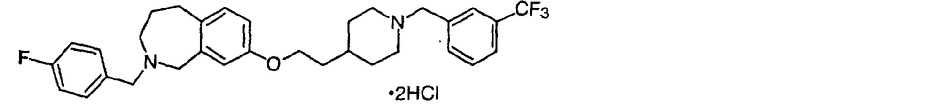

According to the same procedures as those of Example 1) using 2-[(4-fluorophenyl)methyl]-8-[2-(4-piperidinyl)ethoxy]-2,3,4,5-tetrahydro-1H-2-benzazepine (free base) obtained in Reference Example 39), the title compound was obtained as colorless amorphous powders.
¹H NMR (CDCl₃, free base) δ 1.20-1.80 (9H, m), 1.85-2.10 (2H, m), 2.75-2.90 (4H, m), 3.08 (2H, t-like, J = 5.2 Hz), 3.49 (2H, s), 3.52 (2H, s), 3.80 (2H, s), 3.93 (2H, t, J = 6.4 Hz). 6.47 (1H, d, J = 2.6 Hz), 6.66 (1H, dd, J = 8.2, 2.6 Hz), 6.90-7.10 (3H, m), 7.15-7.30 (2H, m), 7.35-7.60 (4H, m).
Elemental analysis for C₃₂H₃₆F₄N₂O·2HCl·H₂O
Calcd.: C, 60.86; H, 6.38; N, 4.44
Found: C, 61.05; H, 6.50; N, 4.20

### Example 81)

2-[[4-[2-[[2-[(4-Fluorophenyl)methyl]-2,3,4,5-tetrahydro-1H-2-benzazepine-8-yl]oxy]ethyl]-1-piperidinyl]methyl]phenoxy]acetic acid

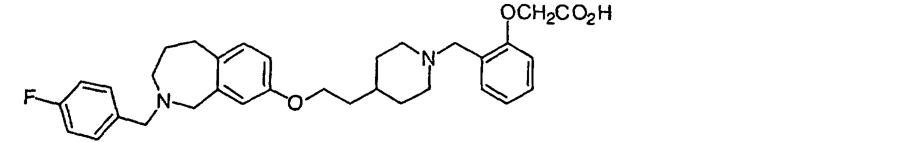

According to the same procedures as those of Example 1) and Reference Example 21) using 2-[(4-fluorophenyl)methyl]-8-[2-(4-piperidinyl)ethoxy]-2,3,4,5-tetrahydro-1H-2-benzazepine (free base) obtained in Reference Example 39), the title compound was obtained as colorless amorphous powders.
¹H NMR (CDCl₃) δ 1.60-2.10 (9H, m), 2.50-2.90 (4H, m), 3.07 (2H, t-like, J = 4.8 Hz), 3.20-3.45 (2H, m), 3.50 (2H, s), 3.80 (2H, s), 3.85-4.00 (4H, m), 4.72 (2H, s), 6.46 (1H, d, J = 2.2 Hz), 6.65 (1H, dd, J = 8.2, 2.2 Hz), 6.90-7.45 (9H, m).
Elemental analysis for C₃₃H₃₉FN₂O₄·1.5H₂O
Calcd.: C, 69.09; H, 7.38; N, 4.88
Found: C, 68.82; H, 7.22; N, 4.68

### Example 82)

2-[(4-Fluorophenyl)methyl]-8-[3-[1-(phenylmethyl)-4-piperidinyl]propoxy]-2,3,4,5-tetrahydro-1H-2-benzazepine dihydrochloride

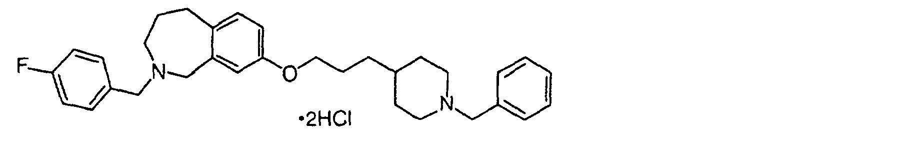

According to the same procedures as those of Example 1) using 2-[(4-fluorophenyl)methyl]-8-[3-(4-piperidinyl)propoxy]-2,3,4,5-tetrahydro-1H-2-benzazepine obtained in Reference Example 42), the title compound was,obtained as colorless amorphous powders.
¹H NMR (CDCl₃, free base) δ 1.20-1.45 (4H, m), 1.60-2.10 (9H, m), 2.80-3.00 (4H, m), 3.08 (2H, t-like, J = 5.2 Hz), 3.49 (4H, s), 3.80 (2H, s), 3.86 (2H, t, J = 6.4 Hz), 6.47 (1H, d, J = 2.6 Hz), 6.66 (1H, dd, J = 8.0, 2.6 Hz), 6.90-7.10 (3H, m), 7.20-7.40 (7H, m).
Elemental analysis for C₃₂H₃₉FN₂O·2HCl·H₂O
Calcd.: C, 66.54; H, 7.50; N, 4.85
Found: C, 66.56; H, 7.49; N, 4.78

### Example 83)

8-[3-[1-[(3-Chlorophenyl)methyl]-4-piperidinyl]propoxy]-2-[(4-fluorophenyl)methyl]-2,3,4,5-tetrahydro-1H-2-benzazepine dihydrochloride

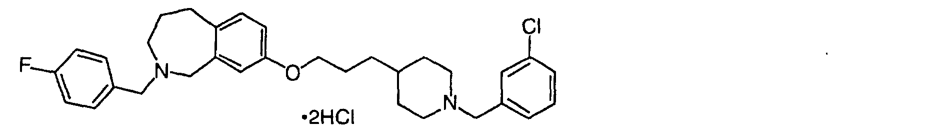

According to the same procedures as those of Example 1) using 2-[(4-fluorophenyl)methyl]-8-[3-(4-piperidinyl)propoxy]-2,3,4,5-tetrahydro-1H-2-benzazepine obtained in Reference Example 42), the title compound was obtained as colorless amorphous powders.
¹H NMR (CDCl₃, free base) δ 1.15-1.45 (4H, m), 1.55-2.05 (9H, m), 2.75-2.90 (4H, m), 3.08 (2H, t-like, J = 5.2 Hz), 3.44 (2H, s), 3.48 (2H, s), 3.80 (2H, s), 3.86 (2H, t, J = 6.6 Hz), 6.48 (1H, d, J = 2.6 Hz), 6.66 (1H, dd, J = 8.2, 2.6 Hz), 6.90-7.10 (3H, m), 7.15-7.40 (6H, m).
Elemental analysis for C₃₂H₃₈ClFN₂O·2HCl·H₂O
Calcd.: C, 62.80; H, 6.92; N, 4.58
Found: C, 62.94; H, 6.76; N, 4.33

### Example 84)

Ethyl 2-methyl-2-[3-[[4-[2-[[2-[(4-fluorophenyl)methyl]-2,3,4,5-tetrahydro-1H-2-benzazepine-8-yl]oxy]propyl]-1-piperidinyl]methyl]phenyl]propanoate dihydrochloride

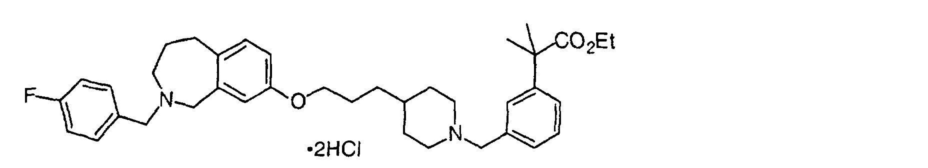

According to the same procedures as those of Example 1) using 2-[(4-fluorophenyl)methyl]-8-[3-(4-piperidinyl)propoxy]-2,3,4,5-tetrahydro-1H-2-benzazepine obtained in Reference Example 42), the title compound was obtained as colorless amorphous powders.
¹H NMR (CDCl₃, free base) δ 1.15-1.45 (4H, m), 1.17 (3H, t, J = 7.0 Hz), 1.57 (6H, s), 1.60-2.00 (9H, m), 2.75-2.90 (4H, m), 3.08 (2H, t-like, J = 5.2 Hz), 3.48 (4H, s), 3.80 (2H, s), 3.86 (2H, t, J = 6.6 Hz), 4.11 (2H, q, J = 7.0 Hz), 6.48 (1H, d, J = 2.6 Hz), 6.66 (1H, dd, J = 8.2, 2.6 Hz), 6.90-7.05 (3H, m), 7.15-7.30 (6H, m).
Elemental analysis for C₃₈H₄₉FN₂O₃·2HCl·H₂O
Calcd.: C, 65.98; H, 7.72; N, 4.05
Found: C, 66.05; H, 7.75; N, 3.97

### Example 85)

2-Methyl-2-[3-[[4-[3-[[2-[(4-fluorophenyl)methyl]-2,3,4,5-tetrahydro-1H-2-benzazepine-8-yl]oxy]propyl]-1-piperidinyl]methyl]phenyl]propionic acid dihydrochloride

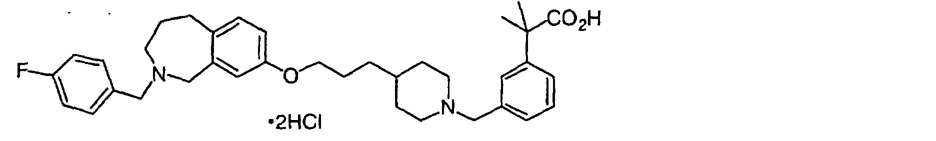

According to the same procedures as those of Reference Example 21) using ethyl 2-methyl-2-[3-[[4-[2-[[2-[(4-fluorophenyl)methyl]-2,3,4,5-tetrahydro-1H-2-benzazepine-8-yl]oxy]propyl]-1-piperidinyl]methyl]phenyl]propanoate obtained in Example 84), the title compound was obtained as colorless amorphous powders.
¹H NMR (CDCl₃, free base) δ 1.10-2.30 (19H, m), 2.80-3.30 (6H, m), 3.40-3.60 (4H, m), 3.70-4.20 (4H, m), 6.48 (1H, d, J = 2.2 Hz), 6.65 (1H, dd, J = 8.0, 2.2 Hz), 6.90-7.60 (9H, m).
Elemental analysis for C₃₆H₄₅FN₂O₃·2HCl·4H₂O
Calcd.: C, 60.24; H, 7.72; N, 3.90
Found: C, 60.22; H, 7.32; N, 3.63

### Example 86)

8-[2-[1-[(4-Cyanophenyl)methyl]-4-piperidinyl]ethoxy]-2-[(2-methylphenyl)methyl]-2,3,4,5-tetrahydro-1H-2-benzazepine dihydrochloride

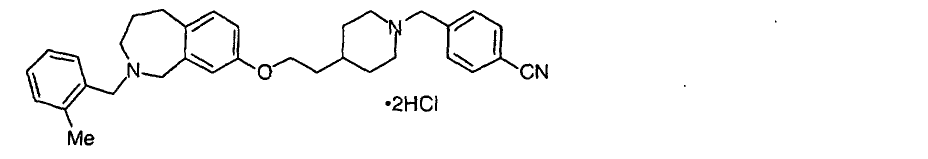

According to the same procedures as those of Reference Example 1) using 2-[(2-methylphenyl)methyl]-8-[2-(4-piperidinyl)ethoxy]-2,3,4,5-tetrahydro-1H-2-benzazepine obtained in Reference Example 14), the title compound was obtained as colorless amorphous powders.
¹H NMR (CDCl₃, free base) δ 1.20-1.80 (9H, m), 1.90-2.05 (2H, m), 2.27 (3H, s), 2.75-2.90 (4H, m), 3.07 (2H, t-like, J = 5.2 Hz), 3.48 (2H, s), 3.51 (2H, s), 3.80 (2H, s), 3.93 (2H, t, J = 6.4 Hz), 6.54 (1H, d, J = 2.6 Hz), 6.66 (1H, dd, J = 8.2, 2.6 Hz), 7.04 (1H, d, J = 8.0 Hz), 7.10-7.30 (4H, m), 7.44 (2H, d, J = 8.4 Hz), 7.59 (2H, d, J = 8.4 Hz).

### Example 87)

Ethyl 4-[[4-[2-[[2-[(2-methylphenyl)methyl]-2,3,4,5-tetrahydro-1H-2-benzazepine-8-yl]oxy]ethyl]-1-piperidinyl]methyl]-1-benzenecarboxyimidate

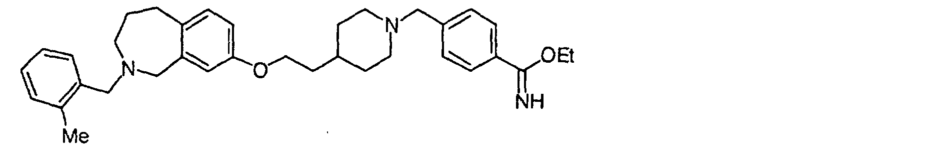

According to the same procedures as those of Reference Example 56)-1) using 8-[2-[1-[(4-cyanophenyl)methyl]-4-piperidinyl]ethoxy]-2-[(2-methylphenyl)-2,3,4,5-tetrahydro-1H-2-benzazepine (free salt) obtained in Example 86), the title compound was obtained as colorless crystals having a melting point of 103-104°C.
¹H NMR (CDCl₃) δ 1.20-1.85 (9H, m), 1.42 (3H, t, J = 7.0 Hz), 1.90-2.10 (2H, m), 2.27 (3H, s), 2.75-2.90 (4H, m), 3.07 (2H, t-like, J = 5.2 Hz), 3.48 (2H, s), 3.53 (2H, s), 3.81 (2H, s), 3.93 (2H, t, J = 6.4 Hz), 4.33 (2H, q, J = 7.0 Hz), 6.54 (1H, d, J = 2.6 Hz), 6.66 (1H, dd, J = 8.2, 2.6 Hz), 7.04 (1H, d, J = 8.0 Hz), 7.10-7.30 (4H, m), 7.38 (2H, d, J = 8.4 Hz), 7.69 (2H, d, J = 8.4 Hz).
Elemental analysis for C₃₅H₄₅N₃O₂·0.5H₂O
Calcd.: C, 76.61; H, 8.45; N, 7.29
Found: C, 76.72; H, 8.13; N, 7.61

### Example 88)

4-[[4-[2-[[2-[(2-Methylphenyl)methyl]-2,3,4,5-tetrahydro-1H-2-benzazepine-8-yl]oxy]ethyl]-1-piperidinyl]methyl]-1-benzenecarboxyimidate

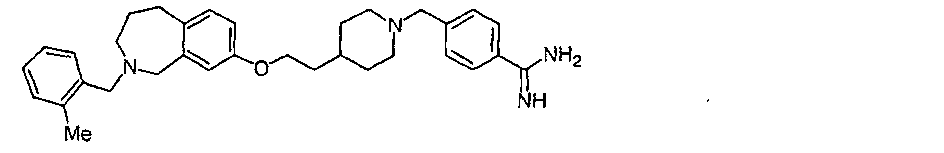

According to the same procedures as those of Example 56)-2) using ethyl 4-[[4-[2-[[2-[(2-methylphenyl)methyl]-2,3,4,5-tetrahydro-1H-2-benzazepine-8-yl]oxy]ethyl]-1-piperidinyl]methyl]-1-benzenecarboxyimidate obtained in Example 87 and ammonia (ethanol solution), the title compound was obtained as colorless crystals having a melting point of 107-108°C.
¹H NMR (CDCl₃) δ 1.20-1.80 (9H, m), 1.90-2.10 (2H, m), 2.28 (3H, s), 2.75-2.90 (4H, m), 3.07 (2H, t-like, J = 5.2 Hz), 3.48 (2H, s), 3.51 (2H, s), 3.60-4.40 (3H, br), 3.81 (2H, s), 3.93 (2H, t, J = 6.2 Hz), 6.54 (1H, d, J = 2.6 Hz), 6.66 (1H, dd, J = 8.0, 2.6 Hz), 7.04 (1H, d, J = 8.0 Hz), 7.10-7.30 (4H, m), 7.37 (2H, d, J = 8.0 Hz), 7.55 (2H, d, J = 8.0 Hz).
Elemental analysis for C₃₃H₄₂N₄O·0.5H₂O
Calcd.: C, 76.26; H, 8.34; N, 10.78
Found: C, 76.39; H, 8.13; N, 10.80

### Example 89)

8-[2-[1-[[4-(4,5-Dihydro-1H-2-imidazolyl)phenyl]methyl]-4-piperidinyl]ethoxy]-2-[(2-methylphenyl)methyl]-2,3,4,5-tetrahydro-1H-2-benzazepine

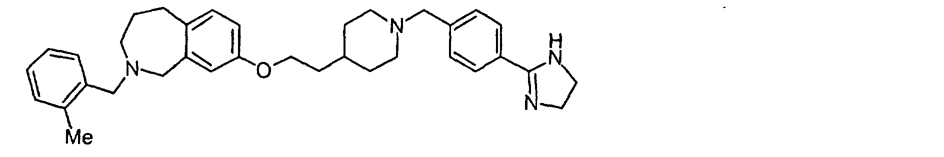

According to the same procedures as those of Example 56)-2) using 4-[[4-[2-[[2-[(2-methylphenyl)methyl]-2,3,4,5-tetrahydro-1H-2-benzazepine-8-yl]oxy]ethyl]-1-piperidinyl]methyl]-1-benzenecarboxyimidate obtained in Example 87 and ethylenediamine, the title compound was obtained as colorless crystals having a melting point of 150-151°C.
¹H NMR (CDCl₃) δ 1.20-1.80 (9H, m), 1.90-2.05 (2H, m), 2.27 (3H, s), 2.80-2.90 (4H, m), 3.07 (2H, t-like, J = 5.2 Hz), 3.48 (2H, s), 3.51 (2H, s), 3.78 (4H, s), 3.81 (2H, s), 3.93 (2H, t, J = 6.2 Hz), 6.54 (1H, d, J = 2.6 Hz), 6.66 (1H, dd, J = 8.0, 2.6 Hz), 7.04 (1H, d, J = 8.0 Hz), 7.10-7.30 (4H, m), 7.36 (2H, d, J = 8.0 Hz), 7.52 (2H, d, J = 8.0 Hz).
Elemental analysis for C₃₅H₄₄N₄O
Calcd.: C, 78.32; H, 8.26; N, 10.44
Found: C, 78.30; H, 8.12; N, 10.45

### Example 90

2-(Phenylmethyl)-8-[2-[1-[[4-(N,N-diethylaminomethyl)phenyl]methyl]-4-piperidinyl]ethoxy]-2,3,4,5-tetrahydro-1H-2-benzazepine trihydrochloride

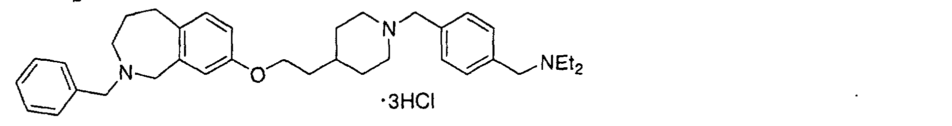

According to the same procedures as those of Reference Example 1), Reference Example 7), Reference Example 8) and Example 1) successively using 8-methoxy-2,3,4,5-tetrahydro-1H-2-benzazepine, the title compound was obtained as colorless amorphous powders.
¹H NMR (CDCl₃, free base) δ 1.04 (6H, t, J = 7.0 Hz), 1.20-1.80 (8H, m), 1.85-2.10 (3H, m), 2.51 (4H, q, J = 7.0 Hz), 2.80-2.95 (4H, m), 3.09 (2H, t-like, J = 5.2 Hz), 3.46 (2H, s), 3.53 (2H, s), 3.54 (2H, s), 3.82 (2H, s), 3.92 (2H, t, J = 6.4 Hz), 6.49 (1H, d, J = 2.6 Hz), 6.65 (1H, dd, J = 8.3, 2.6 Hz), 7.03 (1H, d, J = 8.3 Hz), 7.20-7.35 (9H, m).
Elemental analysis for C₃₆H₄₉N₃O·3HCl·H₂O
Calcd.: C, 64.81; H, 8.16; N, 6.30
Found: C, 64.44; H, 8.48; N, 6.36

### Example 91

2-[(2-Methylphenyl)methyl]-8-[2-[1-[(3-cyanophenyl)methyl]-4-piperidinyl]ethoxy]-2,3,4,5-tetrahydro-1H-2-benzazepine dihydrochloride

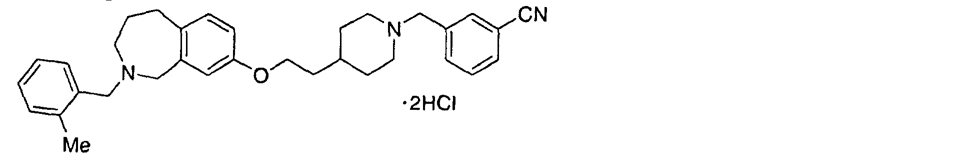

According to the same procedures as those of Example 1) using 2-[(2-methylphenyl)methyl]-8-[2-(4-piperidinyl)ethoxy]-2,3,4,5-tetrahydro-1H-2-benzazepine obtained in Reference Example 14), the title compound was obtained as colorless amorphous powders.
¹H NMR (CDCl₃, free base) δ 1.20-1.90 (9H, m), 1.90-2.10 (2H, m), 2.28 (3H, s), 2.75-2.95 (4H, m), 3.07 (2H, t-like, J = 5.2 Hz), 3.49 (4H, s), 3.81 (2H, s), 3.94 (2H, t, J = 6.2 Hz), 6.54 (1H, d, J = 2.6 Hz), 6.67 (1H, dd, J = 8.1, 2.6 Hz), 7.00-7.70 (9H, m).
Elemental analysis for C₃₃H₃₉N₃O·2HCl·H₂O
Calcd.: C, 67.80; H, 7.41; N, 7.19
Found: C, 67.95; H, 7.57; N, 7.20

### Example 92

2-[(2-Methylphenyl)methyl]-8-[2-[1-[[3-(4,5-dihydro-1H-2-imidazolyl)phenyl]methyl]-4-piperiainyl]ethoxy]-2,3,4,5-tetrahydro-1H-2-benzazepine trihydrochloride

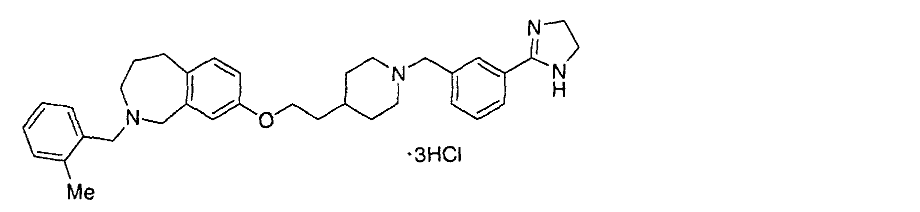

According to the same procedures as those of Example 56) using 2-[(2-methylphenyl)methyl]-8-[2-[1-[(3-cycanophenyl)methyl]-4-piperidinyl)ethoxy]-2,3,4,5-tetrahydro-1H-2-benzazepine dihydrochloride obtained in Example 91), the title compound was obtained as colorless amorphous powders.
¹H NMR (CDCl₃, free base) δ 1.20-1.80 (9H, m), 1.85-2.10 (2H, m), 2.27 (3H, s), 2.80-2.90 (4H, m), 3.06 (2H, t-like, J = 5.2 Hz), 3.48 (4H, s), 3.60-4.00 (1H, br), 3.76 (4H, s), 3.80 (2H, s), 3.92 (2H, t, J = 6.2 Hz), 6.53 (1H, d, J = 2.6 Hz), 6.65 (1H, dd, J = 8.2, 2.6 Hz), 7.03 (1H, d , J = 8.0 Hz), 7.10-7.45 (6H, m), 7.65 (1H, d, J = 7.4 Hz), 7.73 (1H, s).
Elemental analysis for C₃₅H₄₄N₄O·3HCl·1.5H₂O
Calcd.: C, 62.45; H, 7.49; N, 8.32
Found: C, 62.25; H, 7.65; N, 7.76

### Example 93

2-[(2-Methylphenyl)methyl]-8-[2-[1-(4-cyanobenzoyl)-4-piperidinyl]ethoxy]-2,3,4,5-tetrahydro-1H-2-benzazepine hydrochloride

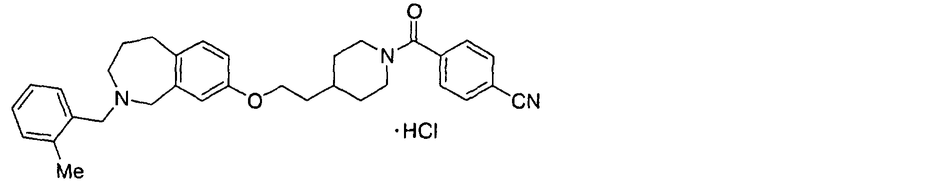

According to the same procedures as those of Example 1) using 2-[(2-methylphenyl)methyl]-8-[2-(4-piperidinyl)ethoxy]-2,3,4,5-tetrahydro-1H-2-benzazepine obtained in Reference Example 14), the title compound was obtained as colorless amorphous powders.
¹H NMR (CDCl₃, free base) δ 1.00-1.50 (2H, m), 1.60-2.00 (7H, m), 2.28 (3H, s), 2.70-3.20 (2H, br), 2.80-2.95 (2H, m), 3.07 (2H, t-like, J = 5.2 Hz), 3.49 (2H, s), 3.50-3.70 (1H, br), 3.81 (2H, s), 3.96 (2H, t, J = 6.0 Hz), 4.60-4.80 (1H, br), 6.54 (1H, d, J = 2.6 Hz), 6.66 (1H, dd, J = 8.0, 2.6 Hz), 7.00-7.30 (5H, m), 7.50 (2H, d, J = 8.0 Hz), 7.71 (2H, d, J = 8.0 Hz).
Elemental analysis for C₃₃H₃₇N₃O₂·HCl·0.5H₂O
Calcd.: C, 71.66; H, 7.11; N, 7.60
Found: C, 71.39; H, 7.16; N, 7.61

### Example 94

2-[(2-Methylphenyl)methyl]-8-[2-[1-[4-(4,5-dihydro-1H-2-imidazolyl)benzoyl]-4-piperidinyl]ethoxy]-2,3,4,5-tetrahydro-1H-2-benzazepine

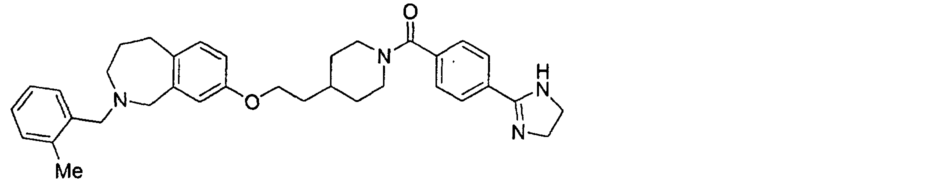

According to the same procedures as those of Example 56) using 2-[(2-methylphenyl)methyl]-8-[2-[1-(4-cyanobenzoyl)-4-piperidinyl)ethoxy]-2,3,4,5-tetrahydro-1H-2-benzazepine hydrochloride obtained in Example 93), the title compound was obtained as colorless crystals having a melting point of 145-146°C.
¹H NMR (CDCl₃) δ 1.00-1.50 (2H, m), 1.60-2.00 (8H, m), 2.28 (3H, s), 2.70-3.20 (2H, br), 2.80-2.95 (2H, m), 3.07 (2H, t-like, J = 5.2 Hz), 3.49 (2H, s), 3.60-4.00 (5H, br), 3.81 (2H, s), 3.96 (2H, t, J = 6.0 Hz), 4.60-4.80 (1H, br), 6.53 (1H, d, J = 2.6 Hz), 6.66 (1H, dd, J = 8.0, 2.6 Hz), 7.05 (1H, d, J = 8.0 Hz), 7.10-7.30 (4H, m), 7.42 (2H, d, J = 8.0 Hz), 7.81 (2H, d, J = 8.0 Hz).
Elemental analysis for C₃₅H₄₂N₄O₂
Calcd.: C, 76.33; H, 7.69; N, 10.17
Found: C, 75.97; H, 7.25; N, 10.03

### Example 95

2-(Phenylmethyl)-7-[[1-[(4-cyanophenyl)methyl]-4-piperidinyl]methoxy]-2,3,4,5-tetrahydro-1H-2-benzazepine dihydrochloride

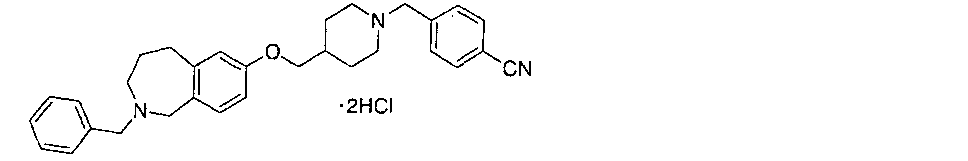

According to the same procedures as those of Example 1) using 2-(phenylmethyl)-7-[(4-piperidinyl)methoxy]-2,3,4,5-tetrahydro-1H-2-benzazepine dihydrochloride obtained in Reference Example 44), the title compound was obtained as colorless amorphous powders.
¹H NMR (CDCl₃, free base) δ 1.20-1.55 (2H, m), 1.65-1.90 (5H, m), 1.95-2.15 (2H, m), 2.80-2.95 (4H, m), 3.09 (2H, t-like, J = 5.2 Hz), 3.51 (2H, s), 3.54 (2H, s), 3.79 (2H, d, J = 6.2 Hz), 3.80 (2H, s), 6.59 (1H, dd, J = 8.2, 2.6 Hz), 6.71 (1H, d, J = 2.6 Hz), 6.83 (1H, d, J = 8.2 Hz), 7.20-7.40 (5H, m), 7.45 (2H, d, J = 8.4 Hz), 7.60 (2H, d, J = 8.4 Hz).
Elemental analysis for C₃₁H₃₅N₃O·2HCl·1.5H₂O
Calcd.: C, 65.83; H, 7.13; N, 7.43
Found: C, 65.90; H, 7.22; N, 7.37

### Example 96

2-(Phenylmethyl)-7-[[1-[[4-(4,5-dihydro-1H-2-imidazolyl)phenyl]methyl]-4-piperidinyl]methoxy]-2,3,4,5-tetrahydro-1H-2-benzazepine

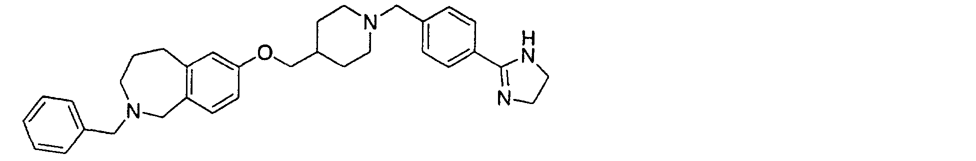

According to the same procedures as those of Example 56) using 2-(phenylmethyl)-7-[[1-[(4-cyanophenyl)methyl]-4-piperidinyl)methoxy]-2,3,4,5-tetrahydro-1H-2-benzazepine dihydrochloride obtained in Reference Example 95), the title compound was obtained as colorless crystals having a melting point of 152-154°C.
¹H NMR (CDCl₃) δ 1.30-1.95 (8H, m), 1.95-2.10 (2H, m), 2.80-3.00 (4H, m), 3.11 (2H, t-like, J = 5.2 Hz), 3.53 (2H, s), 3.56 (2H, s), 3.81 (2H, d, J = 6.2 Hz), 3.83 (6H, s), 6.60 (1H, dd, J = 8.2, 2.6 Hz), 6.73 (1H, d, J = 2.6 Hz), 6.85 (1H, d, J = 8.2 Hz), 7.20-7.35 (5H, m), 7.39 (2H, d, J = 8.0 Hz), 7.75 (2H, d, J = 8.0 Hz).
Elemental analysis for C₃₃H₄ON₄O
Calcd.: C, 77.92; H, 7.93; N, 11.01
Found: C, 77.42; H, 7.93; N, 10.93

### Example 97

2-(Phenylmethyl)-8-[[1-[(4-cyanophenyl)methyl]-4-piperidinyl]methoxy]-2,3,4,5-tetrahydro-1H-2-benzazepine dihydrochloride

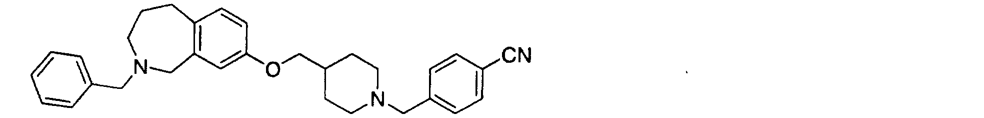

According to the same procedures as those of Example 1) using 2-(phenylmethyl)-8-[(4-piperidinyl)methoxy]-2,3,4,5-tetrahydro-1H-2-benzazepine dihydrochloride obtained in Reference Example 46), the title compound was obtained as colorless crystals having a melting point of 105-107°C.
¹H NMR (CDCl₃) δ 1.20-1.55 (2H, m), 1.60-1.90 (5H, m), 1.95-2.15 (2H, m), 2.80-2.95 (4H, m), 3.09 (2H, t-like, J = 5.2 Hz), 3.54 (4H, s), 3.74 (2H, d, J = 6.0 Hz), 3.83 (2H, s), 6.50 (1H, d, J = 2.6 Hz), 6.66 (1H, dd, J = 8.2, 2.6 Hz), 7.04 (1H, d, J = 8.2 Hz), 7.20-7.35 (5H, m), 7.45 (2H, d, J = 8.2 Hz), 7.61 (2H, d, J = 8.2 Hz).
Elemental analysis for C₃₁H₃₅N₃O·2HCl·1.5H₂O
Calcd.: C, 65.93; H, 7.13; N, 7.43
Found: C, 65.90; H, 7.22; N, 7.73

### Example 98

2-(Phenylmethyl)-8-[[1-[[4-(4,5-dihydro-1H-2-imidazolyl)phenyl]methyl]-4-piperidinyl]methoxy]-2,3,4,5-tetrahydro-1H-2-benzazepine

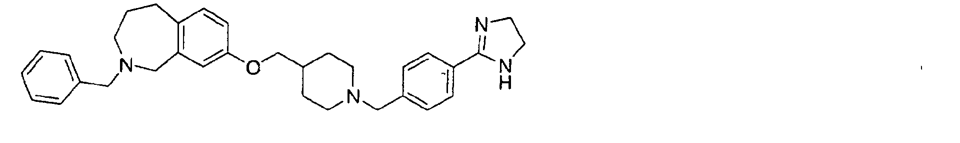

According to the same procedures as those of Example 56) using 2-(phenylmethyl)-8-[[1-[(4-cyanophenyl)methyl]-4-piperidinyl)methoxy]-2,3,4,5-tetrahydro-1H-2-benzazepine dihydrochloride obtained in Example 97), the title compound was obtained as colorless crystals having a melting point of 137-139°C.
¹H NMR (CDCl₃) δ 1.25-1.50 (2H, m), 1.60-2.10 (8H, m), 2.80-3.00 (4H, m), 3.09 (2H, t-like, J = 5.2 Hz), 3.40-4.20 (4H, br), 3.53 (4H, s), 3.73 (2H, d, J = 5.8 Hz), 3.82 (2H, s), 6.50 (1H, d, J = 2.6 Hz), 6.65 (1H, dd, J = 8.2, 2.6 Hz), 7.03 (1H, d, J = 8.2 Hz), 7.20-7.35 (5H, m), 7.37 (2H, d, J = 8.0 Hz), 7.73 (2H, d, J = 8.0 Hz).
Elemental analysis for C₃₃H₄₀N₄O
Calcd.: C, 77.92; H, 7.93; N, 11.01
Found: C, 77.87; H, 7.91; N, 10.85

### Example 99

2-(Phenylmethyl)-8-[2-[1-[(4-cyanophenyl)methyl]-4-piperidinyl]ethoxy]-2,3,4,5-tetrahydro-1H-2-benzazepine

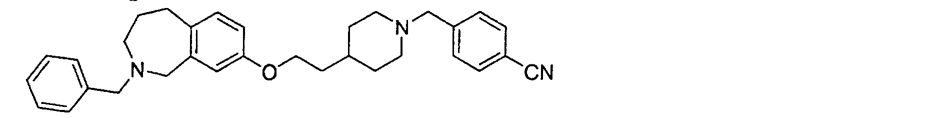

According to the same procedures as those of Reference Example 1), Reference Example 7), Reference Example 8) and Example 1) successively using 8-methoxy-2,3,4,5-tetrahydro-1H-2-benzazepine, the title compound was obtained as colorless crystals having a melting point of 83-85°C.
¹H NMR (CDCl₃) δ 1.20-1.85 (9H, m), 1.90-2.05 (2H, m), 2.75-2.90 (4H, m), 3.09 (2H, t-like, J = 5.2 Hz), 3.51 (2H, s), 3.53 (2H, s), 3.82 (2H, s), 3.93 (2H, t, J = 6.4 Hz), 6.50 (1H, d, J = 2.6 Hz), 6.66 (1H, dd, J = 8.0, 2.6 Hz), 7.04 (1H, d, J = 8.0 Hz), 7.15-7.35 (5H, m), 7.44 (2H, d, J = 8.4 Hz), 7.59 (2H, d, J = 8.4 Hz).
Elemental analysis for C₃₂H₃₇N₃O
Calcd.: C, 80.13; H, 7.28; N, 8.76
Found: C, 79.93; H, 7.95; N, 8.91

### Example 100

2-(Phenylmethyl)-8-[2-[1-[[4-(4,5-dihydro-1H-2-imidazolyl)phenyl]methyl]-4-piperidinyl]ethoxy]-2,3,4,5-tetrahydro-1H-2-benzazepine

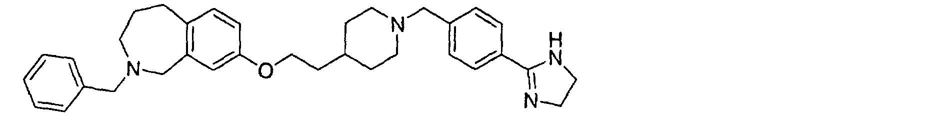

According to the same procedures as those of Example 56) using 2-(phenylmethyl)-8-[2-[1-[(4-cyanophenyl)methyl]-4-piperidinyl]ethoxy]-2,3,4,5-tetrahydro-1H-2-benzazepine obtained in Example 99), the title compound was obtained as colorless crystals having a melting point of 151-153°C.
¹H NMR (CDCl₃) δ 1.20-2.10 (12H, m), 2.80-2.90 (4H, m), 3.09 (2H, t-like, J = 5.2 Hz), 3.50 (2H, s), 3.53 (2H, s), 3.60-4.00 (4H, br), 3.82 (2H, s), 3.92 (2H, t, J = 6.4 Hz), 6.50 (1H, d, J = 2.6 Hz), 6.66 (1H, dd, J = 8.0, 2.6 Hz), 7.03 (1H, d, J = 8.0 Hz), 7.20-7.50 (5H, m), 7.35 (2H, d, J = 8.4 Hz), 7.72 (2H, d, J = 8.4 Hz).
Elemental analysis for C₃₄H₄₂N₄O
Calcd.: C, 78.12; H, 8.10; N, 10.72
Found: C, 77.64; H, 8.02; N, 10.49

### Example 101

2-(Phenylmethyl)-8-[2-[1-[(4-dimethylaminophenyl)methyl]-4-piperidinyl]ethoxy]-2,3,4,5-tetrahydro-1H-2-benzazepine trihydrochloride

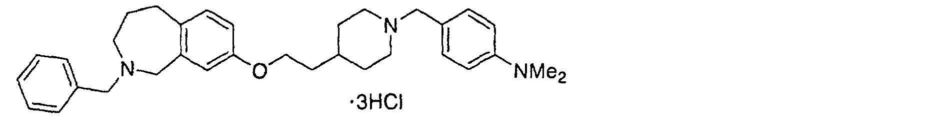

According to the same procedures as those of Reference Example 1), Reference Example 7), Reference Example 8) and Example 1) successively using 8-methoxy-2,3,4,5-tetrahydro-1H-2-benzazepine, the title compound was obtained as colorless amorphous powders.
¹H NMR (CDCl₃, free base) δ 1.20-1.80 (9H, m), 1.85-2.05 (2H, m), 2.80-2.90 (4H, m), 2.93 (6H, s), 3.10 (2H, t-like, J = 5.2 Hz), 3.43 (2H, s), 3.53 (2H, s), 3.83 (2H, s), 3.91 (2H, t, J = 6.4 Hz), 6.49 (1H, d, J = 2.6 Hz), 6.60-6.75 (2H m), 7.03 (1H, d, J = 8.0 Hz), 7.10-7.35 (8H, m).
Elemental analysis for C₃₃H₄₃N₃O·3HCl
Calcd.: C, 62.50; H, 7.79; N, 6.63
Found: C, 62.20; H, 7.97; N, 6.34

### Example 102

3-(Phenylmethyl)-7-[[3-[1-(phenylmethyl)-4-piperidinyl]propyl]sulfonyl]-2,3,4,5-tetrahydro-1H-3-benzazepine dihydrochloride

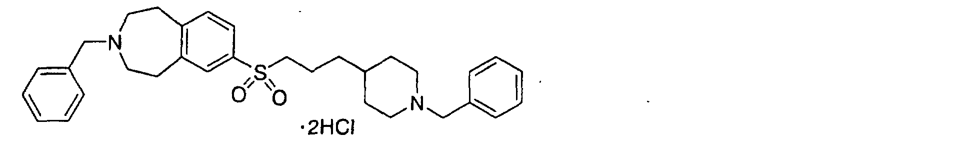

According to the same procedures as those of Example 1) using 3-(phenylmethyl)-7-[[3-(4-piperidinyl)propyl]sulfonyl]-2,3,4,5-tetrahydro-1H-3-benzazepine obtained in Reference Example 52), the title compound was obtained as colorless crystals.
¹H NMR (CDCl₃, free base) δ 1.05-1.35 (5H, m), 1.45-2.40 (6H, m), 2.55-2.70 (4H, m), 2.80-2.90 (2H, m), 2.95-3.10 (6H, m), 3.47 (2H, s), 3.63 (2H, s), 7.20-7.40 (11H, m), 7.55-7.65 (2H, m).
Elemental analysis for C₃₂H₄ON₂O₂S·2HCl·0.5H₂O
Calcd.: C, 64.20; H, 7.24; N, 4.68
Found: C, 64.07; H, 7.01; N, 4.78

### Example 103

7-[[3-[1-[(2-Chlorophenyl)methyl]-4-piperidinyl]propyl]sulfonyl]-3-(phenylmethyl)-2,3,4,5-tetrahydro-1H-3-benzazepine dihydrochloride

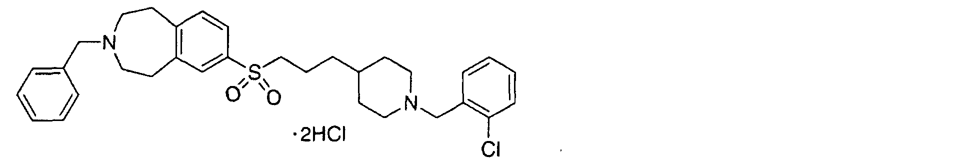

According to the same procedures as those of Example 1) using 3-(phenylmethyl)-7-[[3-(4-piperidinyl)propyl]sulfonyl]-2,3,4,5-tetrahydro-1H-3-benzazepine obtained in Reference Example 52), the title compound was obtained as colorless crystals.
¹H NMR (CDCl₃, free base) δ 1.05-1.40 (5H, m), 1.45-2.20 (6H, m), 2.55-2.75 (4H, m), 2.80-2.90 (2H, m), 2.95-3.20 (6H, m), 3.58 (2H, s), 3.63 (2H, s), 7.15-7.70 (12H, m).
Elemental analysis for C₃₂H₃₉N₂O₂S·2RCl·H₂O
Calcd.: C, 59.86; H, 6.75; N, 4.36
Found: C, 59.93; H, 6.76; N, 4.37

### Example 104

7-[[3-[1-[(3-Chlorophenyl)methyl]-4-piperidinyl]propyl]sulfonyl]-3-(phenylmethyl)-2,3,4,5-tetrahydro-1H-3-benzazepine dihydrochloride

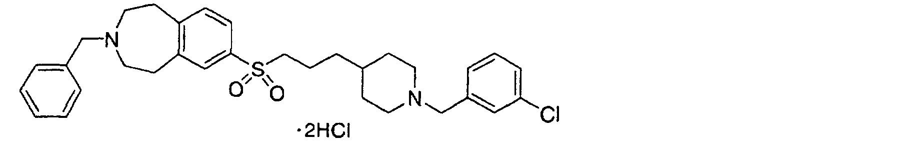

According to the same procedures as those of Example 1) using 3-(phenylmethyl)-7-[[3-(4-piperidinyl)propyl]sulfonyl]-2,3,4,5-tetrahydro-1H-3-benzazepine obtained in Reference Example 52), the title compound was obtained as colorless crystals.
¹H NMR (CDCl₃, free base) δ 1.05-1.40 (5H, m), 1.45-2.00 (6H, m), 2.55-2.75 (4H, m), 2.75-2.90 (2H, m), 2.95-3.15 (6H, m), 3.43 (2H, s), 3.63 (2H, s), 7.10-7.40 (10H, m), 7.55-7.65 (2H, m).
Elemental analysis for C₃₂H₃₉N₂O₂S·2HCl·H₂O
Calcd.: C, 59.86; H, 6.75; N, 4.36
Found: C, 60.12; H, 6.69; N, 4.23

### Example 105

7-[[3-[1-[(4-Chlorophenyl)methyl]-4-piperidinyl]propyl]sulfonyl]-3-(phenylmethyl)-2,3,4,5-tetrahydro-1H-3-benzazepine dihydrochloride

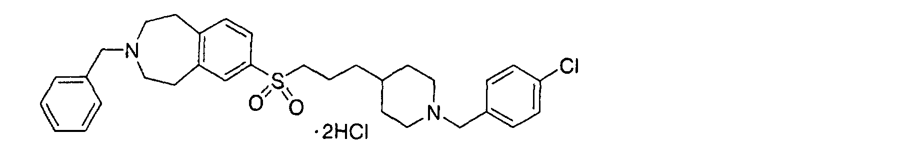

According to the same procedures as those of Example 1) using 3-(phenylmethyl)-7-[[3-(4-piperidinyl)propyl]sulfonyl]-2,3,4,5-tetrahydro-1H-3-benzazepine obtained in Reference Example 52), the title compound was obtained as colorless crystals.
¹H NMR (CDCl₃, free base) δ 1.05-1.40 (5H, m), 1.45-2.00 (6H, m), 2.55-2.75 (4H, m), 2.75-2.90 (2H, m), 2.95-3.10 (6H, m), 3.43 (2H, s), 3.63 (2H, s), 7.10-7.40 (10H, m), 7.55-7.65 (2H, m).
Elemental analysis for C₃₂H₃₉N₂O₂S·2HCl·0.5H₂O
Calcd.: C, 60.71; H, 6.69; N, 4.42
Found: C, 60.74; H, 6.59; N, 4.67

### Example 106

3-[(2-Methylphenyl)methyl]-7-[[3-[1-(phenylmethyl)-4-piperidinyl]propyl]sulfonyl]-2,3,4,5-tetrahvdro-1H-3-benzazepine

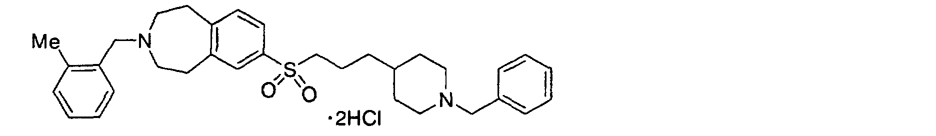

According to the same procedures as those of Example 1) using 3-[(2-methylphenyl)methyl]-7-[[3-(4-piperidinyl)propyl]sulfonyl]-2,3,4,5-tetrahydro-1H-3-benzazepine obtained in Reference Example 53), the title compound was obtained as colorless crystals.
¹H NMR (CDCl₃, free base) δ 1.05-1.35 (5H, m), 1.45-2.00 (6H, m), 2.39 (3H, s), 2.55-2.70 (4H, m), 2.80-2.90 (2H, m), 2.95-3.10 (6H, m), 3.47 (2H, s), 3.54 (2H, s), 7.15-7.35 (10H, m), 7.55-7.65 (2H, m).
Elemental analysis for C₃₃H₄₂N₂O₂S·2HCl·0.5H₂O
Calcd.: C, 64.69; H, 7.40; N, 4.57
Found: C, 64.42; H, 7.28; N, 4.33

### Example 107

7-[[3-[1-[(2-Chlorophenyl)methyl]-4-piperidinyl]propyl]sulfonyl]-3-[(2-methylphenyl)methyl]-2,3,4,5-tetrahydro-1H-3-benzazepine dihydrochloride

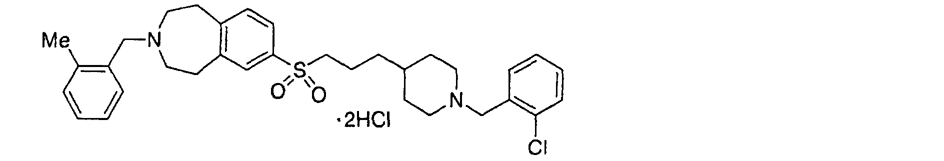

According to the same procedures as those of Example 1) using 3-[(2-methylphenyl)methyl]-7-[[3-(4-piperidinyl)propyl]sulfonyl]-2,3,4,5-tetrahydro-1H-3-benzazepine obtained in Reference Example 53), the title compound was obtained as colorless amorphous powders.
¹H NMR (CDCl₃, free base) δ 1.05-1.40 (5H, m), 1.50-2.10 (6H, m), 2.39 (3H, s), 2.55-2.70 (4H, m), 2.80-3.10 (8H, m), 3.54 (2H, s), 3.58 (2H, s), 7.10-7.40 (8H, m), 7.40-7.50 (1H, m), 7.55-7.65 (2H, m).
Elemental analysis for C₃₃H₄₁N₂O₂S·2HCl·3H₂O
Calcd.: C, 60.47; H, 7.48; N, 4.24
Found: C, 69.34; H, 7.38; N, 4.27

### Example 108

7-[[3-[1-[(3-Chlorophenyl)methyl]-4-piperidinyl]propyl]sulfonyl]-3-[(2-methylphenyl)methyl]-2,3,4,5-tetrahydro-1H-3-benzazepine dihydrochloride

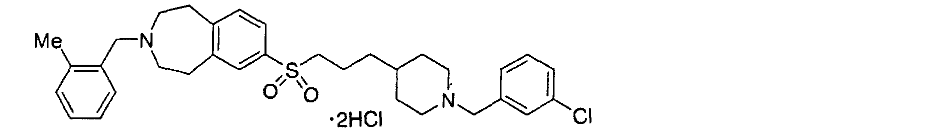

According to the same procedures as those of Example 1) using 3-[(2-methylphenyl)methyl]-7-[[3-(4-piperidinyl)propyl]sulfonyl]-2,3,4,5-tetrahydro-1H-3-benzazepine obtained in Reference Example 53), the title compound was obtained as colorless amorphous powders.
¹H NMR (CDCl₃, free base) δ 1.05-1.40 (5H, m), 1.50-2.00 (6H, m), 2.39 (3H, s), 2.55-2.70 (4H, m), 2.70-2.90 (2H, m), 2.90-3.10 (6H, m), 3.43 (2H, s), 3.54 (2H, s), 7.10-7.35 (9H, m), 7.55-7.65 (2H, m).
Elemental analysis for C₃₃H₄₁N₂O₂S·2HCl·H₂O
Calcd.: C, 60.41; H, 6.91; N, 4.27
Found: C, 60.65; H, 6.79; N, 4.41

### Example 109

7-[2-(1-Benzoyl-4-piperidinyl]ethoxy]-2-[(2-methylphenyl)methyl]-1,3,4,5-tetrahydro-1H-2-benzazepine-1-one

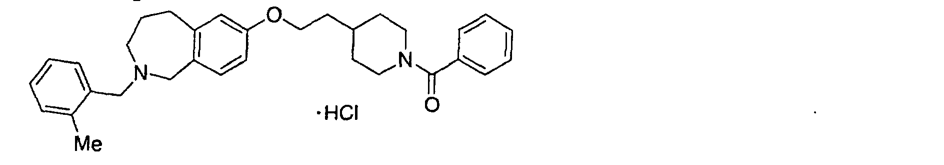

According to the same procedures as those of Example 1) using 2-[(2-methylphenyl)methyl]-7-[2-(4-piperidinyl)ethoxy]-2,3,4,5-tetrahydro-1H-2-benzazepine obtained in Reference Example 8), the title compound was obtained as colorless amorphous powders.
¹H NMR (CDCl₃, free base) δ 1.05-1.50 (2H, m), 1.65-2.00 (7H, m), 2.28 (3H, s), 2.70-3.20 (2H, br), 2.80-2.90 (2H, m), 3.06 (2H, t-like, J = 5.2 Hz), 3.46 (2H, s), 3.60-3.60 (1H, br), 3.79(2H, s), 4.00 (2H, t, J = 6.2 Hz), 4.60-4.85 (1H, br), 6.60 (1H, dd, J = 8.0, 2.6 Hz), 6.71 (1H, d, J = 2.6 Hz), 6.88 (1H, d, J = 8.0 Hz), 7.10-7.50 (9H, m).
Elemental analysis for C₃₂H₃₈N₂O₂·HCl·0.5H₂O
Calcd.: C, 72.77; H, 7.63; N, 5.30
Found: C, 72.30; H, 7.39; N, 5.40

### Example 110

N-[[1-(Phenylmethyl)-4-piperidinyl]methyl]-2-(trifluoroacetyl)-2,3,4,5-tetrahydro-1H-2-benzazepine-8-sulfonamide

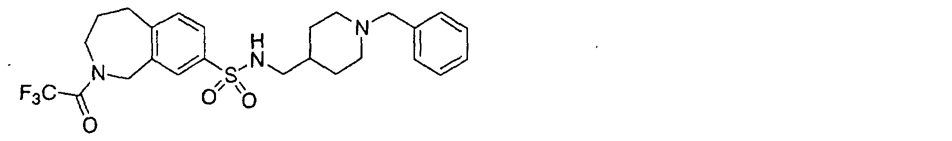

According to the same procedures as those of Example 46) using 2-(trifluoroacetyl)-2,3,4,5-terahydro-1H-2-benzazepine-8-sulfonyl chloride, the title compound was obtained as colorless crystals having a melting point of 131-132°C.
¹H NMR (CDCl₃) δ 1.20-2.20 (9H, m), 2.70-3.00 (4H, m), 3.10-3.15 (2H, m), 3.57 (2H, s), 3.80-4.00 (2H, m), 4.65 and 4.74 (2H, s and s), 4.80-4.95 (1H, br), 7.25-7.40 (6H, m), 7.72 (1H, dd, J = 8.0, 1.8Hz), 7.87 (1H, d, J = 1.8Hz).
Elemental analysis for C₂₅H₃₀F₃N₃O₃S·0.5H₂O
Calcd.: C, 57.90; H, 6.03; N, 8.10
Found: C, 57.52; H, 5.79; N, 8.39

### Example 111

N-[[1-(Phenylmethyl)-4-piperidinyl]methyl]-2,3,4,5-tetrahydro-1H-2-benzazepine-8-sulfonamide

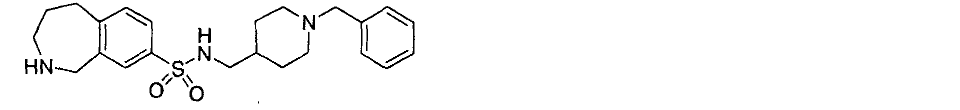

According to the same procedures as those of Reference Example 21) using N-[[1-(phenylmethyl)-4-piperidinyl]methyl]-2-(trifluoroacetyl)-2,3,4,5-terahydro-1H-2-benzazepine-8-sulfonamide obtained in Example 110), the title compound was obtained as colorless crystals having a melting point of 160-162°C.
¹H NMR (CDCl₃) δ 1.10-2.00 (10H, m), 2.75-2.95 (4H, m), 2.95-3.01 (2H, m), 3.24 (2H, t-like, J = 5.2Hz), 3.49 (2H, s), 3.99 (2H, s), 4.40-4.65 (1H, br), 7.20-7.35 (7H, m), 7.55-7.65 (1H, m).
Elemental analysis for C₂₃H₃₁N₂O₂S·0.5H₂O
Calcd.: C, 65.37; H, 7.63; N, 9.94
Found: C, 64.93; H, 7.34; N, 10.02

### Example 112

N-[[1-(Phenylmethyl)-4-piperidinyl)methyl]-2-(phenylmethyl)-2,3,4,5-tetrahydro-1H-2-benzazepine-8-sulfonamide

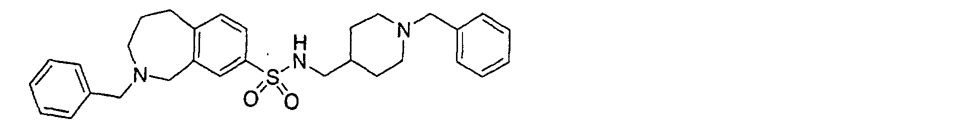

According to the same procedures as those of Example 1) using N-[[1-(phenylmethyl)-4-piperidinyl]methyl]-2,3,4,5-tetrahydro-1H-2-benzazepine-8-sulfonamide obtained in Example 111), the title compound was obtained as colorless crystals having a melting point of 122-123°C.
¹H NMR (CDCl₃) δ 1.10-2.00 (9H, m), 2.75-2.90 (4H, m), 2.95-3.01 (2H, m), 3.12 (2H, t-like, J = 5.2Hz), 3.46 (2H, s), 3.53 (2H, s), 3.89 (2H, s), 4.40 (1H, t, J = 6.4Hz), 7.20-7.35 (11H, m), 7.39 (1H, d, J = 1.8Hz), 7.63 (1H, dd, J = 8.0, 1.8HZ).
Elemental analysis for C₃₀H₃₇N₃O₂S·0.5H₂O
Calcd.: C, 70.28; H, 7.47; N, 8.20
Found: C, 70.31; H, 7.19; N, 8.27

### Example 113

2-(Phenylmethyl)-8-[[2-[1-(phenylmethyl)-4-piperidinyl]ethyl]oxy]-2,3,4,5-tetrahydro-1H-2-benzazepine

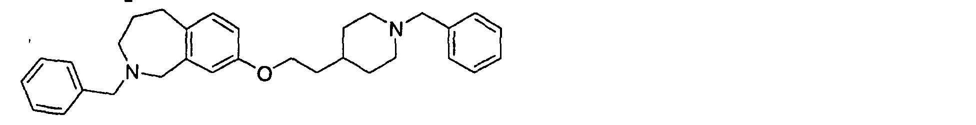

According to the same procedures as those of Example 1) using 2-(phenylmethyl)-8-[[2-(4-piperidinyl)ethyl]oxy]-2,3,4,5-tetrahydro-1H-2-benzazepine obtained in Reference Example 55), the title compound was obtained as a colorless oil.
¹H NMR (CDCl₃) δ 1.20-1.80 (9H, m), 1.85-2.10 (2H, m), 2.80-2.95 (4H, m), 3.09 (2H, t-like, J = 5.2 Hz), 3.48 (2H, s), 3.12 (2H, s), 3.82 (2H, s), 3.92 (2H, t, J = 6.4 Hz), 6.49 (1H, d, J = 2.6 Hz), 6.65 (1H, dd, J = 8.0, 2.6 Hz), 7.03 (1H, d, J = 8.0Hz), 7.20-7.40 (10H, m).

### Example 114

2-(Phenylmethyl)-8-[[2-[1-(phenylmethyl)-4-piperidinyl]ethyl]sulfanyl]-2,3,4,5-tetrahydro-1H-2-benzazepine dihydrochloride

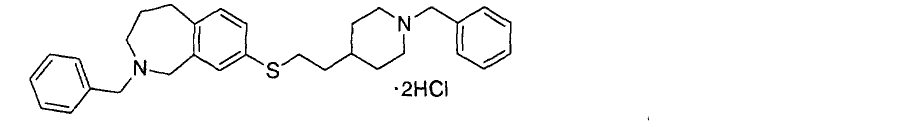

According to the same procedures as those of Example 1) using 2-(phenylmethyl)-8-[[2-(4-piperidinyl)ethyl]sulfanyl]-2,3,4,5-tetrahydro-1H-2-benzazepine obtained in Reference Example 60), the title compound was obtained as colorless amorphous powders.
¹H NMR (CDCl₃, free base) δ 1.15-1.80 (9H, m), 1.80-2.00 (2H, m), 2.80-2.95 (6H, m), 3.11 (2H, t-like, J = 5.2Hz), 3.48 (2H, s), 3.52 (2H, s), 3.82 (2H, s), 6.88 (1H, d, J = 1.6Hz), 7.05 (1H, d, J = 7.6Hz), 7.12 (1H, dd, J = 7.8, 2.0Hz), 7.20-7.40 (10H. m).
Elemental analysis for C₃₁H₃₈N₂S·2HCl·H₂O
Calcd.: C, 66.29; H, 7.54; N, 4.99
Found: C, 66.51; H, 7.67; N, 4.88

### Example 115

2-(Phenylmethyl)-8-[[2-[1-(phenylmethyl)-4-piperidinyl]ethyl]sulfinyl]-2,3,4,5-tetrahydro-1H-2-benzazepine dihydrochloride

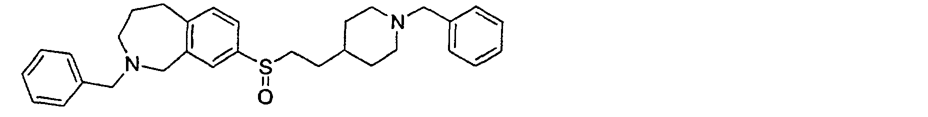

According to the same procedures as those of Example 1) using 2-(phenylmethyl)-8-[[2-(4-piperidinyl)ethyl]sulfinyl]-2,3,4,5-tetrahydro-1H-2-benzazepine obtained in Reference Example 64), the title compound was obtained as colorless amorphous powders.
¹H NMR (CDCl₃, free base) δ 1.15-2.00 (13H, m), 2.75-2.90 (4H, m), 3.11 (2H, t-like, J = 5.4Hz), 3.40-3.55 (4H, m), 3.82 (2H, s), 6.88 (1H, d, J = 1.8Hz), 7.00-7.40 (12H, m).
Elemental analysis for C₃₁H₃₈N₂OS·2HCl·1.5H₂O
Calcd.: C, 63.47; H, 7.39; N, 4.78
Found: C, 63.51; H, 7.25; N, 4.59

### Example 116

2-(Phenylmethyl)-8-[[2-[1-(phenylmethyl)-4-piperidinyl]ethyl]sulfonyl]-2,3,4,5-tetrahydro-1H-2-benzazepine

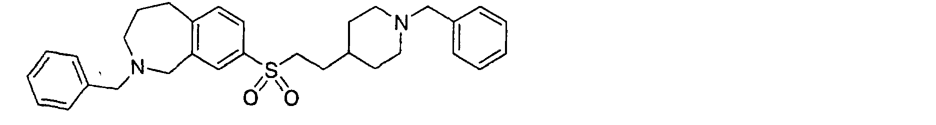

According to the same procedures as those of Example 1) using 2-(phenylmethyl)-8-[[2-(4-piperidinyl)ethyl]sulfinyl]-2,3,4,5-tetrahydro-1H-2-benzazepine obtained in Reference Example 68), the title compound was obtained as colorless crystals having a melting point of 128-129°C.
¹H NMR (CDCl₃) δ 1.10-2.00 (11H, m), 2.80-2.90 (2H, m), 2.95-3.10 (4H, m), 3.14 (2H, t-like, J = 5.4Hz), 3.46 (2H, s), 3.53 (2H, s), 3.91 (2H, s), 7.15-7.40 (11H, m), 7.43 (1H, d, J = 2.0Hz), 7.68 (1H, dd, J = 8.0, 2.0Hz).
Elemental analysis for C₃₁H₃₈N₂O₂S
Calcd.: C, 74.06; H, 7.62; N, 5.57
Found: C, 73.53; H, 7.52; N, 5.50

### Example 117

1-[2-(Phenylmethyl)-2,3,4,5-tetrahydro-1H-2-benzazepine-8-yl]-3-[1-(phenylmethyl)-4-piperidinyl]-1-propanone oxime dihydrochloride

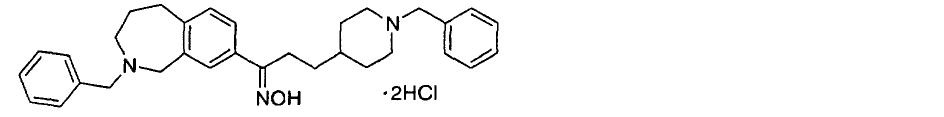

According to the same procedures as those of Example 52) using 1-[2-(phenylmethyl)-2,3,4,5-tetrahydro-1H-2-benzazepine-8-yl]-3-[1-(phenylmethyl)-4-piperidinyl]-1-propanone, the title compound was obtained as colorless amorphous powders.
¹H NMR (CDCl₃, free base) δ 1.20-2.30 (11H, m), 2.40-3.20 (8H, m), 3.54 (2H, s), 3.68 (2H, s), 3.92 (2H, s), 7.00-7.50 (13H, m).
Elemental analysis for C₃₂H₃₉N₃O·2HCl·1.5H₂O
Calcd.: C, 66.08; H, 7.63; N, 7.22
Found: C, 66.17; H, 7.53; N, 7.08

### Example 118

1-[2-(Phenylmethyl)-2,3,4,5-tetrahydro-1H-2-benzazepine-8-yl]-3-[1-(phenylmethyl)-4-piperidinyl]-1-propanone hydrazone

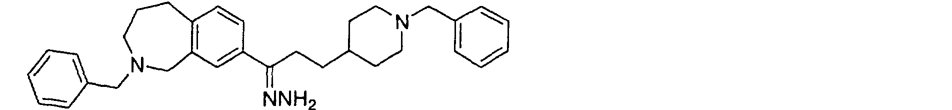

According to the same procedures as those of Example 53) using 1-[2-(phenylmethyl)-2,3,4,5-tetrahydro-1H-2-benzazepine-8-yl]-3-[1-(phenylmethyl)-4-piperidinyl]-1-propanone, the title compound was obtained as colorless crystals having a melting point of 142-143°C.
¹H NMR (CDCl₃) δ 1.20-2.05 (11H, m), 2.45-2.65 (2H, m), 2.85-2.95 (4H, m), 3.10 (2H, t-like, J = 5.2Hz), 3.49 (2H, s), 3.53 (2H, s), 3.90 (2H, s), 5.32 (2H, s), 7.12 (1H, d, J=8.0Hz), 7.20-7.35 (11H, m), 7.42(1H, dd, J=8.0,2.0Hz).
Elemental analysis for C₃₂H₄₀N₄
Calcd.: C, 79.96; H, 8.39; N, 11.66
Found: C, 79.28; H, 8.50; N, 11.26

### Example 119

2-[1-[2-(Phenylmethyl)-2,3,4,5-tetrahydro-1H-2-benzazepine-8-yl]-3-[1-(phenylmethyl)-4-piperidinyl]-1-propylidene]malononitrile dihydrochloride

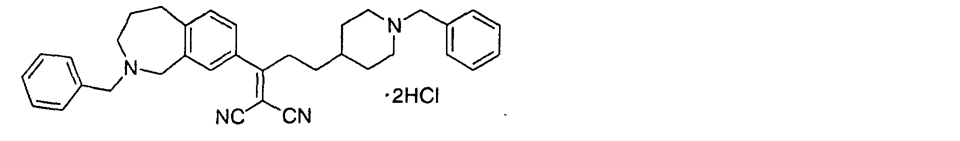

According to the same procedures as those of Example 54) using 1-[2-(phenylmethyl)-2,3,4,5-tetrahydro-1H-2-benzazepine-8-yl]-3-[1-(phenylmethyl)-4-piperidinyl]-1-propanone, the title compound was obtained as colorless amorphous powders.
¹H NMR (CDCl₃, free base) δ 1.15-2.00 (11H, m), 2.80-3.00 (6H, m), 3.13 (2H, t-like, J = 5.2Hz), 3.47 (2H, s), 3.54 (2H, s), 3.88 (2H, s), 6.97 (1H, s), 7.20-7.40 (12H, m).
Elemental analysis for C₃₅H₃₈N₄O·2HCl·1.0H₂O
Calcd.: C, 69.41; H, 6.99; N, 9.25
Found: C, 69.10; H, 6.93; N, 8.94

### Example 120

7-[[[[[1-(Phenylmethyl)-4-piperidinyl]amino]carbonyl]amino]sulfonyl]-3-(trifluoroacetyl)-2,3,4,5-tetrahvdro-1H-3-benzazeDine

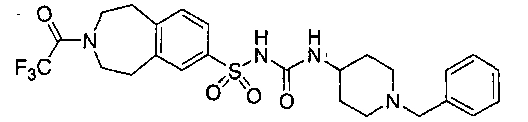

Potassium cyanate (400 mg, 4.93 mmol) and pyridine (590 mg, 7.29 mmol) were added to a solution of 3-(trifluoroacetyl)-2,3,4,5-tetrahydro-1H-3-benzazepine-7-sulfonyl chloride (1.00 g, 2.93 mmol) in acetonitrile (8 ml) at room temperature. After the mixture was stirred for 3 hours, 1-(phenylmethyl)-4-aminopiperidine (552 ml, 2.93 mmol) was added and the mixture was further stirred for 0.5 hours. After the solvent was distilled off, the residue was dissolved in ethyl acetate-water and extracted with ethyl acetate three times. After the extract was washed with an aqueous saturated solution of sodium chloride and dried with anhydrous magnesium sulfate, the solvent was distilled off under reduced pressure. The resulting residue was purified by silica gel column chromatography (developing solvent: ethyl acetate:methanol=4:1) to obtain the title compound (558 mg) as a colorless oil.
¹H NMR (DMSO―d₆) δ 1.20-1.50 (2H, m), 1.60-1.75 (2H, m), 1.90-2.15 (2H, m), 2.60-2.75 (2H, m), 2.95-3.15 (4H, m), 3.20-3.50 (2H, br), 3.46 (2H, s), 3.60-3.80 (4H, m), 6.30-6.50 (1H, br), 7.20-7.40 (6H, m), 7.60-7.70 (2H, m).

### Example 121

3-(Phenylmethyl)-7-[[[[[1-(phenylmethyl)-4-piperidinyl]amino]carbonyl]amino]sulfonyl]-2,3,4,5-tetrahydro-1H-3-benzazepine

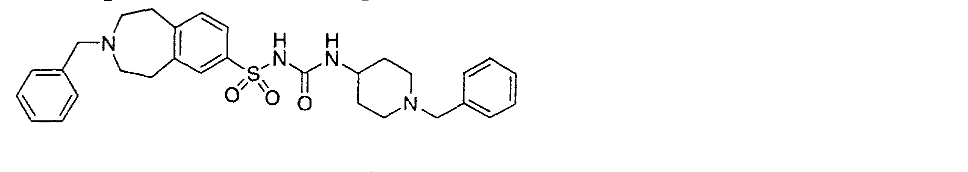

According to the same procedures as those of Reference Example 21) and Example 1) successively using 7-[[[[[1-(phenylmethyl)-4-piperidinyl]amino]carbonyl]amino]sulfonyl]-3-(trifluoroacetyl)-2,3,4,5-tetrahydro-1H-3-benzazepine obtained in Example 120), the title compound was obtained as colorless amorphous powders.
¹H NMR (CDCl₃) δ 1.20-3.00 (17H, m), 3.40-3.70 (4H, m), 4.8-5.6 (1H, br), 6.20-6.60 (1H, br), 7.00-7.40 (11H, m), 7.50-7.70 (2H, m).
MS: (FAB) m/z 533 (M+H).

### Example 122

7-[[[[[1-(Phenylmethyl)-4-piperidinyl]amino](imino)methyl]amino]sulfonyl]-3-(trifluoroacetyl)-2,3,4,5-tetrahydro-1H-3-benzazepine

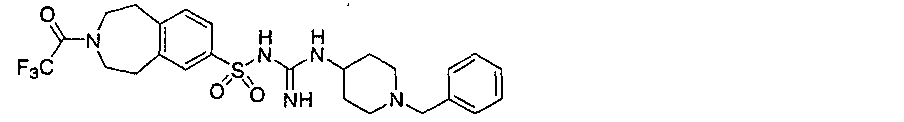

N-[1-(Phenylmethyl)-4-piperidinyl]guanidine (830 mg, 3.57 mmol) obtained in Reference Example 69) and triethylamine (600 ml, 4.30 mmol) were added to a solution of 3-(trifluoroacetyl)-2,3,4,5-tetrahydro-1H-3-benzazepine-7-sulfonyl chloride (1.22 g, 3.57 mmol) in THF (50 ml), and the mixture was stirred at room temperature for 24 hours and further heated at reflux for 3 hours. After solvent was distilled off under reduced pressure, the residue was dissolved in ethyl acetate-water and extracted with ethyl acetate twice. After the extract was washed with an aqueous saturated solution of sodium chloride and dried with anhydrous magnesium sulfate, the solvent was distilled off under reduced pressure. The resulting residue was purified by silica gel column chromatography (developing solvent; ethyl acetate) to obtain the title compound (962 mg) as a colorless oil.
¹H NMR (CDCl₃) δ 1.35-1.60 (2H, m), 1.70-2.20 (8H, m), 2.60-2.85 (2H, m), 2.90-3.05 (2H, m), 3.47 (2H, s), 3.50-3.90 (3H, m), 5.90-6.10 (1H, br), 7.15-7.40 (7H, m), 7.60-7.70 (1H, m), 9.35 (1H, d, J = 7.8 Hz), 9.50-9.65 (1H, br).

### Example 123

7-[[[[[1-(Phenylmethyl)-4-piperidinyl]amino](imino)methyl]amino]sulfonyl]-2,3,4,5-tetrahydro-1H-3-benzazepine

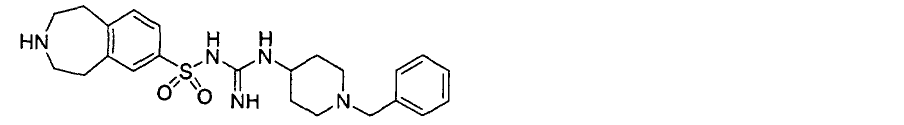

According to the same procedures as those of Reference Example 21) using 7-[[[[[1-(phenylmethyl)-4-piperidinyl]amino](imino)methyl]amino]sulfonyl]-3-(trifluoroacetyl)-2,3,4,5-tetrahydro-1H-3-benzazepine obtained in Example 122), the title compound was obtained as colorless amorphous powders.
¹H NMR (CDCl₃) δ 1.40-2.25 (9H, m), 2.60-3.00 (8H, m), 3.40-3.65 (1H, br), 3.46 (2H, s), 3.47-3.90 (1H, br), 6.10-6.70 (1H, br), 7.10-7.40 (7H, m), 7.60-7.70 (1H, m), 9.30-9.40 (1H, br).

### Example 124

3-(Phenylmethyl)-7-[[[[[1-(phenylmethyl)-4-piperidinyl]amino](imino)methyl]amino]sulfonyl]-2,3,4,5-tetrahydro-lH-3-benzazepine

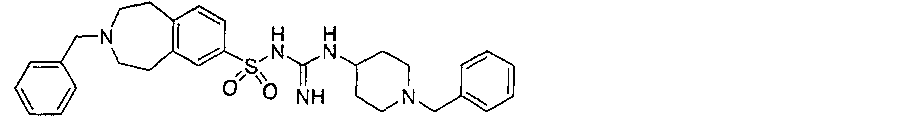

According to the same procedures as those of Example 1) using 7-[[[[[1-(phenylmethyl)-4-piperidinyl]amino](imino)methyl]amino]sulfonyl]-2,3,4,5-tetrahydro-1H-3-benzazepine obtained in Example 123), the title compound was obtained as colorless amorphous powders.
¹H NMR (CDCl₃) δ 1.35-1.60 (2H, m), 1.70-2.20 (6H, m), 2.50-3.00 (7H, m), 3.40-3.60 (1H, br), 3.47 (2H, s), 3.60 (2H, s), 3.75-3.95 (1H, br), 5.90-6.30 (1H, br), 7.05-7.40 (11H, m), 7.55-7.70 (2H, m), 9.25-9.40 (1H, br), 9.50-9.65 (1H, br).

### Example 125

3-[(2-Methylphenyl)methyl)-7-[[[[[1-(phenylmethyl)-4-piperidinyl]amino](imino)methyl]amino]sulfonyl]-2,3,4,5-tetrahydro-1H-3-benzazepine

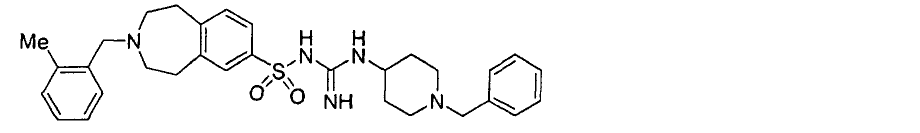

According to the same procedures as those of Example 1) using 7-[[[[[1-(phenylmethyl)-4-piperidinyl]amino](imino)methyl]amino]sulfonyl]-2,3,4,5-tetrahydro-1H-3-benzazepine obtained in Example 123), the title compound was obtained as colorless amorphous powders.
¹H NMR (CDCl₃) δ 1.35-1.65 (2H, m), 1.70-2.25 (6H, m), 2.38 (3H, s), 2.50-2.95 (7H, m), 3.40-3.60 (5H, m), 3.70-3.95 (1H, br), 6.00-6.35 (1H, br), 7.05-7.35 (10H, m), 7.55-7.70 (2H, m), 9.25-9.40 (1H, br), 9.50-9.65 (1H, br).

### Test Example 1

Measurement of the cAMP concentration increasing activity in fat cells using mouse-derived pre-fat cell strain (3T3-L1)

The cAMP concentration increasing activity of Compound (I) in fat cells was examined using a mouse-derived pre-fat cell strain (3T3-L1). That is, 3T3-L1 cells were seeded on a 96-well microtiter plate (10,000 cells/well), and cultured to confluence for 5 to 6 days. After cultured for 72 hours after confluence, the above Compound (I) (10^{-6M}, 10⁻⁷M, 10⁻⁸M and 10⁻⁹M) was added and allowed to stand at 37°C for 40 minutes (100 µl/well). After the cells were washed three times with a phosphate buffer at 4°C, 0.1N hydrochloric acid was added, which was boiled at 95°C for 10 minutes. 25 µl was taken from each well, which was dissolved in 75 µl of an assay buffer attached to Cyclic AMP Enzyme Immunoassay Kit (manufactured by Keiman Chemical Company, USA), and 50 µl of the mixture was quantitated as a sample using the above kit. That is, the above sample (50 µl), Cyclic AMP Tracer (50 µl) attached to the above kit and Cyclic AMP rabbit antibody (50 µl) attached to the above kit were added to an anti-rabbit IgG mouse antibody-solid phased 96-well microtiter plate, which was allowed to stand at room temperature for 18 hours. After each well was aspirated, the mixture was washed with a washing solution four times (400 µl/well). Then, 200 µl of a chromogenic reagent attached to the kit was added to each well, which was incubated at room temperature for 60 minutes while shaking. After completion of the reaction, the amount of cAMP was quantitated by measuring the absorbance at the wavelength of 405 nm.

The cAMP amounts at respective concentrations when a subject compound was added at 10^{-6M}, 10⁻⁷M, 10⁻⁸M and 10⁻⁹M, are shown in [Table 1]. The values are an average of four experiments. In addition, a significance test relative to the cAMP concentration value in a control experiment (without addition of the subject compound) was performed by the known ANOVA method.
(*p<0.05 vs control)

**[Table 1]**

| cAMP concentration in fat cells (pmol/ml) | | | | | |
|---|---|---|---|---|---|
| Compound No. | Compound concentration | | | | |
| (Example No.) | 10⁻⁶M | 10⁻⁷M | 10⁻⁸M | 10⁻⁹M | Control |
| 6 | 46.7* | 16.9* | 13.7* | 8.2* | 2.7 |
| 7 | 27.8* | 5.5* | 2.7* | 1.8 | 1.0 |
| 8 | 43.0* | 11.1* | 9.1 | 8.5 | 5.6 |
| 12 | 260.5* | 45.0* | 8.7* | 3.9 | 1.5 |

From [Table 1], it can be seen that Compound (I) or a salt thereof has an excellent cAMP concentration increasing activity in fat cells.

When the cAMP concentration in fat cells is increased, since the thermal production is increased (Newest Medical, vol. 52, No.6, pp.1093-1100, 1997), it can be said that Compound (I) or a salt thereof has an excellent thermal production promoting activity.

### Preparation Example 1

| | |
|---|---|
| (1) 7-[2-[1-(phenylmethyl)-4-piperidinyl]ethoxy]-3-(phenylmethyl)-2,3,4,5-tetrahyro-lH-3-benzazepine dihydrochloride (compound of Example 6) | 1 g |
| (2) Lactose | 197 g |
| (3) Corn starch | 50 g |
| (4) Magnesium stearate | 2 g |

One g of the above (1), 197 g of the above (2) and 20 g of corn starch were kneaded, which was granulated together with a paste made from 15 g of corn starch and 25 ml of water, to this were added 15 g of corn starch and 2 g of the above (4), the mixture was compressed with a compression tabletting machine to prepare 2000 tablets having a diameter of 3 mm containing 0.5 mg of the above (1) per one tablet.

### Preparation Example 2

| | |
|---|---|
| (1) 7-[2-[1-(phenylmethyl)-4-piperidinyl]ethoxy]-3-(phenylmethyl)-2,3,4,5-tetrahyro-1H-3-benzazepine dihydrochloride (compound of Example 6) | 2 g |
| (2) Lactose | 197 g |
| (3) Corn starch | 50 g |
| (4) Magnesium stearate | 2 g |

2 g of the above (1), 197 g of the above (2) and 20 g of corn starch were kneaded, which was granulated together with a paste made from 15 g of corn starch and 25 ml of water, to this were added 15 g of corn starch and 2 g of the above (4), the mixture was compressed with a compression tabletting machine to prepare 2000 tablets having a diameter of 3 mm containing 1.0 mg of the above (1) per one tablet.

### Preparation Example 3

| | |
|---|---|
| (1) 7-[2-[1-(phenylmethyl)-4-piperidinyl]ethoxy]-3-(phenylmethyl)-2,3,4,5-tetrahyro-1H-3-benzazepine dihydrochloride (compound of Example 6) | 25 g |
| (2) Lactose | 80 g |
| (3) Corn starch | 42 g |
| (4) Talc powder | 3 g |
| (5) Magnesium stearate | 0.5 g |

25 g of the above (1), 80 g of the above (2) and 21 g of corn starch were kneaded, which was granulated together with a paste made from 10 g of corn starch and 9 ml of water, to this were added 11 g of corn starch, 3 g of the above (4) and 0.5 g of the above (5), the mixture was compressed with a compression tabletting machine to prepare 1000 tablets having a diameter of 3 mm containing 25 mg of the above (1) per one tablet.

### Preparation Example 4

| | |
|---|---|
| (1) 7-[2-[1-(phenylmethyl)-4-piperidinyl]ethoxy]-3-(phenylmethyl)-2,3,4,5-tetrahyro-1H-3-benzazepine dihydrochloride (compound of Example 6) | 5.0 mg |
| (2) Lactose | 60.0 mg |
| (3) Corn starch | 35.0 mg |
| (4) Talc powder | 3.0 mg |
| (5) Magnesium stearate | 2.0 mg |

After a mixture of 5.0 mg of the above (1), 60 mg of the above (2) and 35 mg of the above (3) was granulated using 0.03 ml of a 10% aqueous gelatin solution (3.0 mg in terms of gelatin) and 1 mm mesh sieve, the granules were dried at 40°C and passed again through a sieve. The resulting granules were mixed with 2.0 mg of the above (5), and compressed. The resulting core tablets were coated with a sugar-coating by an aqueous suspension of sucrose, titanium dioxide, talc and gum arabic. The coated tablets were polished with a beewax to obtain coated tablets.

### Reference Example 1A

7-Methoxy-3-(trifluoroacetyl)-2,3,4,5-tetrahydro-1H-3-benzazepine 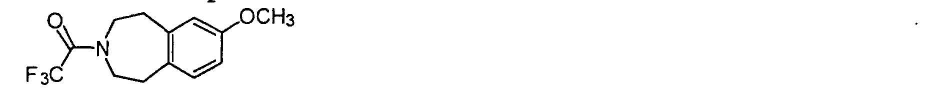
1) A solution of 8-methoxy-2,3-dihydro-1H-3-benzazepine-2-one (9.0 g, 47.5 mmol) in ethanol (200 ml) was subjected to a catalytic hydrogenation reaction at room temperature using 5% Pd/C as a catalyst to obtain 8-methoxy-2,3,4,5-tetrahydro-1H-3-benzazepine-2-one (8.3 g) as colorless needle crystals having a melting point of 162-163°C.
   ¹H NMR(CDCl₃) δ 3.06(2H, t, J=6.2Hz), 3.49-3.60(2H, m), 3.78(3H, s), 3.81(2H, s), 6.0(1H, br, NH), 6.69(1H, d, J=2.6 Hz), 6.76(1H, dd, J=2.6, 8.4Hz), 7.04(1H, d, J=8.4Hz).
2) Lithium aluminum hydride (1.4 g, 36.8 mmol) was added to a solution of 8-methoxy-2,3,4,5-tetrahydro-1H-3-benzazepine-2-one (3.5 g, 18.5 mmol) obtained in 1) in tetrahydrofuran (300 ml) portionwise at room temperature. After the mixture was heated at reflux for 4 hours and allowed to cool, water (2.8 ml) then a 10% aqueous solution of sodium hydroxide (2.24 ml) were added dropwise. After stirring at room temperature for 14 hours, the resulting precipitates were removed by filtration, and the solvent was distilled off under reduced pressure to obtain the crude product of 7-methoxy-2,3,4,5-terahydro-1H-3-benzazepine (3.0 g) as a viscous oil.
3) Trifluoroacetic acid anhydride (3.3 g, 15.7 mmol) was added dropwise to a solution of 7-methoxy-2,3,4,5-terahydro-1H-3-benzazepine (2.5 g, 14.1 mmol) obtained in 2) in tetrahydrofuran (10 ml). After the mixture was heated to 70-75°C for 1 hour, the solvent was distilled off under reduced pressure. The residue was dissolved in water-ethyl acetate and extracted with ethyl acetate. The extract was washed with an aqueous saturated solution of sodium chloride and dried with anhydrous magnesium sulfate. The solvent was distilled off under reduced pressure to obtain the residue, which was purified by silica gel column chromatography (developing solvent: hexane-ethyl acetate=5:1) to obtain the title compound (2.2 g) as an oil.
   ¹H NMR(CDCl₃) δ 2.87-2.99(4H, m), 3.62-3.84(7H, m), 6.66-6.76(2H, m), 7.02-7.13(1H, m).

### Reference Example 2A

4-(1-Acetyl-4-piperidinyl)-1-[7-hydroxy-3-(trifluoroacetyl)-2,3,4,5-tetrahydro-1H-3-benzazepine-8-yl]-1-butanone

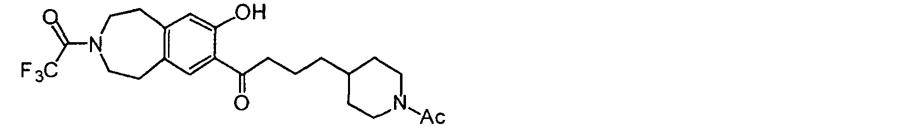

4-(1-Acetyl-4-piperidinyl)butyric acid (0.375 g, 1.76 mmol) was added to thionyl chloride (2.6 ml) under ice-cooling. After stirring for 10 minutes, excess thionyl chloride was distilled off under reduced pressure under ice-cooling. The residue was washed with hexane and dried under reduced pressure. To a solution of the resulting solid and 7-methoxy-3-(trifluoroacetyl)-2,3,4,5-tetrahydro-1H-3-benzazepine (0.4 g, 1.46 mmol) in 1,2-dichloroethane (10 ml) was added aluminum chloride powder (0.68 , 5.1 mmol) portionwise at room temperature. The mixture was stirred for 16 hours, which was poured into ice-water and extracted with ethyl acetate. The extract was washed with an aqueous saturated solution of sodium chloride and dried with anhydrous magnesium sulfate. The solvent was distilled off under reduced pressure to obtain the residue, which was purified by silica gel column chromatography (developing solvent: ethyl acetate) to obtain the title compound (0.5 g) as a viscous oil. This oil was solidified upon allowing to stand at room temperature.
¹H NMR(CDCl₃) δ 1.00-1.87(9H, m), 2.09(3H, s), 2.45-2.64(1H. m), 2.89-3.12(7H, m), 3.62-3.93(5H, m), 4.53-4.68(1H, m), 6.80 and 6.82(1H, each s), 7.49 and 7.52(1H, each s).

### Reference Example 3A

4-(1-Acetyl-4-piperidinyl)-1-[7-hydroxy-2-(trifluoroacetyl)-2,3,4,5-tetrahydro-1H-2-benzazepine-8-yl]-1-butanone

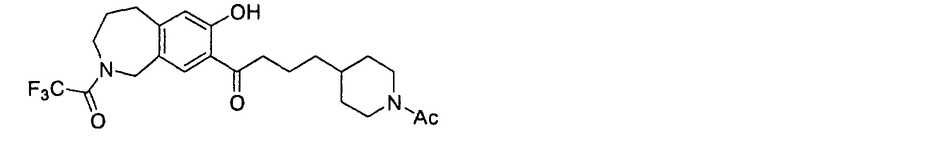

According to the same procedures as those of Reference Example 2A) using 7-methoxy-2-(trifluoroacetyl)-2,3,4,5-tetrahydro-1H-2-benzazepine, the title compound was obtained as a colorless amorphous powder.
¹H NMR (CDCl₃) δ 1.00-2.00 (14H, m), 2.08 (3H, s), 2.45-2.65 (1H, m), 2.90-3.10 (4H, m), 3.70-4.00 (2H, m), 4.50-4.65 (2H, m), 6.81 (1H, s), 7.74 (1H, s), 12.42 (1H, s).

### Reference Example 4A

4-(1-Acetyl-4-piperidinyl)-1-[7-hydroxy-1-(trifluoroacetyl)-2,3,4,5-tetrahydro-1H-2-benzazepine-8-yl]-1-butanone oxime

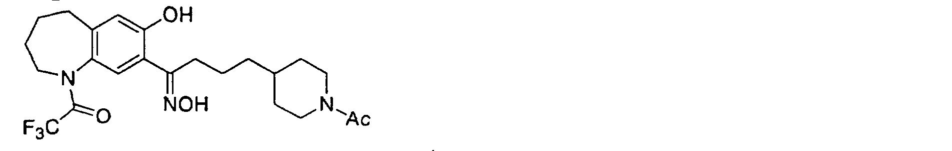

According to the same procedures as those of Reference Example 2A) and Example 1A)-1) in this order using 7-methoxy-1-(trifluoroacetyl)-2,3,4,5-tetrahydro-1H-1-benzazepine, the title compound was obtained as colorless crystals having a melting point of 196-197°C.
¹H NMR (CDCl₃) δ 0.97-2.16 (16H, m), 2.42-3.13 (7H, m), 3.70-3.88 (1H, m), 4.49-4.75 (2H, m), 6.83 (1H, s), 7.17 (1H, s), 9.15 (1H, br), 11.68 (1H, br) .
Elemental analysis for C₂₃H₃₀F₃N₃O₄
Calcd.: C, 58.84; H, 6.44; N, 8.05
Found: C, 58.97; H, 6.44; N, 8.69

### Example 1A

3-[3-(1-Acetyl-4-piperidinyl)propyl]-7-(trifluoroacetyl)-6,7,8,9-tetrahydro-5H-isoxazolo[4,5-h][3]benzazepine 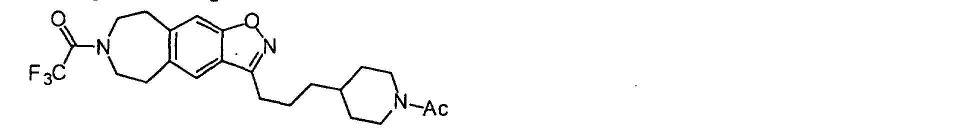
1) A mixture of 4-(1-acetyl-4-piperidinyl)-1-[7-hydroxy-3-(trifluoroacetyl)-2,3,4,5-tetrahydro-1H-3-benzazepine-8-yl]-1-butanone (0.35 g, 0.77 mmol), hydroxyamine hydrochloride (0.16 g, 2.3 mmol) and sodium acetate (0.19 g, 2.31 mmol) was heated in a mixed solution of water-ethanol (2/8 ml) at 80°C for 4 hours. The solvent was distilled off under reduced pressure to obtain the residue, which was dissolved in water-ethyl acetate and extracted with ethyl acetate. The extract was washed with an aqueous saturated solution of sodium chloride and dried with anhydrous magnesium sulfate. The solvent was distilled off under reduced pressure to obtain the residue, which was purified by silica gel column chromatography (developing solvent: ethyl acetate) to obtain a solid (about 0.36 g) having a melting point of 183-189°C.
2) A mixture of the solid (about 0.36 g) obtained in 1), acetic acid anhydride (80 mg, 0.78 mmol) and sodium acetate (70 mg, 0.85 mmol) was stirred in tetrahydrofuran solution (10 ml) at room temperature for 16 hours. The solvent was distilled off under reduced pressure to obtain the residue, which was dissolved in water-ethyl acetate and extracted with ethyl acetate. The extract was washed with an aqueous saturated solution of sodium chloride and dried with anhydrous magnesium sulfate. The solvent was distilled off to obtain the residue, which was purified by silica gel column chromatography (developing solvent: ethyl acetate) to obtain an oil (about 0.3 g).
3) A mixture of the oil (about 0.3 g) obtained in 2) and 2,6-rutidine was heated at 120°C for 16 hours. After allowing to cool, the mixture was dissolved in ethyl acetate, which was washed with 2N hydrochloric acid and then an aqueous saturated solution of sodium chloride, and dried with anhydrous magnesium sulfate. The solvent was distilled off under reduced pressure to obtain the residue, which was purified by silica gel column chromatography (developing solvent: ethyl acetate) to obtain the title compound (85 mg) as an oil.
   ¹H NMR(CDCl₃) δ 0.96-1.23(2H, m), 1.30-1.99(7H, m), 2.08(3H, s), 2.43-2.60(1H, m), 2.88-3.17(7H, m), 3.65-3.93(5H, m), 4.51-4.66(1H, m), 7.34-7.46(2H, m).

### Example 2A

3-[3-[1-(Phenylmethyl)-4-piperidinyl]propyl]-7-(phenylmethyl)-6,7,8,9-tetrahydro-5H-isoxazolo[4,5-h][3]benzazepine dihydrochloride 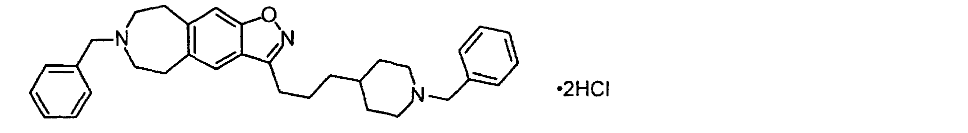
1) An aqueous solution (2 ml) of potassium carbonate (50 mg) was added to a solution of 3-[3-(1-acetyl-4-piperidinyl]propyl]-7-(trifluoroacetyl)-6,7,8,9-tetrahydro-5H-isoxazolo[4,5-h][3]benzazepine (70 mg, 0.155 mmol) obtained in Example 1A) in methanol (10 ml). After the mixture was stirred at room temperature for 2 hours, the solvent was distilled off under reduced pressure. The resulting residue was dissolved in water-ethyl acetate and extracted with ethyl acetate. The extract was washed with an aqueous saturated solution of sodium chloride and dried with anhydrous magnesium sulfate. The solvent was distilled off under reduced pressure to obtain 3-[3-(1-acetyl-4-piperidinyl)propyl]-6,7,8,9-tetrahyro-5H-isoxazolo[4,5-h][3]benzazepine (52 mg) as an oil.
   ¹H NMR(CDCl₃) δ 0.97-1.98(9H, m), 2.07(3H, s), 2.42-2.60(2H, m), 2.83-3.13(11H, m), 3.72-3.83(1H, m), 4.51-4.65(1H, m). 7.30(1H, s), 7.33(1H,s).
2) A mixture of 3-[3-(1-acetyl-4-piperidinyl)propyl]-6,7,8,9-tetrahyro-5H-isoxazolo[4,5-h][3]benzazepine (52 mg) obtained in 1) and concentrated hydrochloric acid (4 ml) was heated at reflux for 5 hours. After allowing to cool, the solution was made alkaline by addition of a 8N aqueous solution of sodium hydroxide and extracted with ethyl acetate. The extract was washed with an aqueous saturated solution of sodium chloride and dried with anhydrous magnesium sulfate. The solvent was distilled off under reduced pressure to obtain 3-[3-(4-piperidinyl)propyl]-6,7,8,9-tetrahydro-5H-isoxazolo[4,5-h][3]benzazepine (40 mg) as an oil. This oil became solid having a melting point of 186-190°C upon allowing to stand at room temperature.
   ¹H NMR(CDCl₃) δ 0.97-1.53(7H, m), 1.62-1.96(4H, m), 2.12-2.42(2H, br), 2.48-2.67(2H, m), 2.82-3.15(10H, m), 7.29(1H, s), 7.33(1H,s).
3) A solution of benzyl bromide (43 mg, 0.25 mmol) in ethanol (1 ml) was added to a suspension of 3-[3-(4-piperidinyl)propyl]-6,7,8,9-tetrahydro-5H-isoxazolo[4,5-h][3]benzazepine (40 mg, 0.127 mmol) and potassium carbonate (40 mg, 0.289 mmol) in ethanol (5 ml) under ice-cooling. After the mixture was stirred at room temperature for 4 hours, the solvent was distilled off under reduced pressure. The residue was dissolved in water-ethyl acetate and extracted with ethyl acetate. The extract was washed with an aqueous saturated solution of sodium chloride and dried with anhydrous magnesium sulfate. The solvent was distilled off under reduced pressure to obtain the residue, which was purified by silica gel column chromatography (developing solvent: ethyl acetate) to obtain a free base (45 mg) of the title compound as an oil.
   ¹H NMR(CDCl₃) δ 1.09-1.43(5H, m), 1.55-2.02(6H, m), 2.57-2.72(4H, m), 2.81-2.95(4H, m), 2.98-3.10(4H, m), 3.49(2H, s), 3.63(2H, s), 7.18-7.41(12H, m).

A solution of the resulting oil (40 mg) in ethyl acetate-ethanol was treated with 2 equivalents of 4N hydrochloric acid (ethyl acetate solution ) to obtain the title compound (30 mg) as a colorless powder having a melting point of 222-255°C (dec.).
Elemental analysis for C₃₃H₃₉N₂O·2HCl·0.5H₂O
Calcd.: C, 68.86; H, 7.35; N, 7.30
Found: C, 68.88; H, 7.04; N, 7.08

### Example 3A

3-[3-(1-Acetyl-4-piperidinyl)propyl]-6-(trifluoroacetyl)-6,7,8,9-tetrahydro-5H-isoxazolo[5,4-h][2]benzazepine

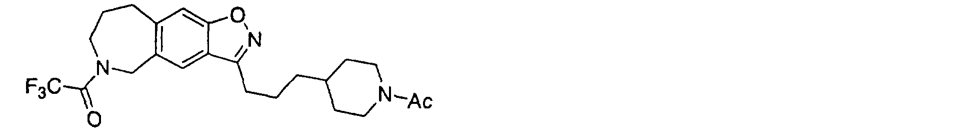

According to the same procedures as those of Example 1A) using 4-(1-acetyl-4-piperidinyl)-1-[7-hydroxy-2-(trifluoroacetyl)-2,3,4,5-tetrahydro-1H-2-benzazepine-8-yl]-1-butanone obtained in Reference Example 3A), the title compound was obtained as a colorless oil.
¹H NMR (CDCl₃) δ 0.95-2.00 (14H, m), 2.07 (3H, s), 2.40-2.60 (1H, m), 2.80-3.20 (4H, m), 3.70-4.00 (2H, m), 4.50-4.80 (2H, m), 7.25-7.70 (2H, m).

### Example 4A

3-[3-(1-Acetyl-4-piperidinyl)propyl]-6,7,8,9-tetrahydro-5H-isoxazolo[5,4,-h][2]benzazepine

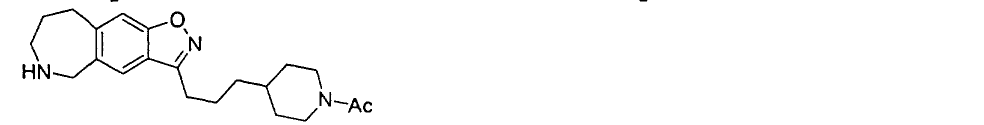

According to the same procedures as those of Example 2A)-1) using 3-[3-(1-acetyl-4-piperidinyl)propyl]-6-(tifluoroacetyl)-6,7,8,9-tetrahydro-5H-isoxazolo[5,4-h][2]benzazepine obtained in Example 3A), the title compound was obtained as a colorless oil.
¹H NMR (CDCl₃) δ 1.00-2.00 (13H, m), 2.07 (3H, s), 2.40-2.60 (1H, m), 2.85-3.15 (4H, m), 3.23 (2H, t-like, J=5.2Hz), 3.70-3.90 (1H, m), 4.04 (2H, s), 4.50-4.70 (1H, m), 7.25-7.45 (2H, m).

### Example 5A

3-[3-(1-Acetyl-4-piperidinyl)propyl]-6-(phenylmethyl)-6,7,8,9-tetrahydro-5H-isoxazolo[5,4-h][2]benzazepine hydrochloride

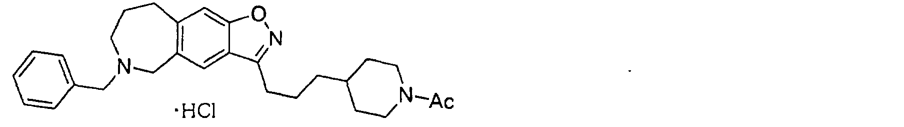

According to the same procedures as those of Example 2A)-3) using 3-[3-(1-acetyl-4-piperidinyl)propyl]-6,7,8,9-tetrahydro-5H-isoxazolo[5,4-h][2]benzazepine obtained in Example 4A), the title compound was obtained as a colorless amorphous powder.
¹H NMR (CDCl₃, free base) δ 0.95-2.00 (7H, m), 2.07 (3H, s), 2.40-2.60 (2H, m), 2.90 (2H, t, J = 7.6 Hz), 2.95-3.10 (4H, m), 3.14 (2H, t-like, J = 5.2 Hz), 3.51 (2H, s), 3.70-3.90 (2H, m), 3.96 (2H, s), 4.50-4.70 (2H, m), 7.13 (1H, s), 7.10-7.40 (6H, m).
Elemental analysis for C₂₈H₃₅N₃O₂·HCl·2H₂O
Calcd.: C, 64.91; H, 7.78; N, 8.11
Found: C, 65.30; H, 7.61; N, 8.23

### Example 6A

6-(Phenylmethyl)-3-[3-(4-piperidinyl)propyl]-6,7,8,9-tetrahydro-5H-isoxazolo[5,4,-h][2]benzazepine dihydrochloride

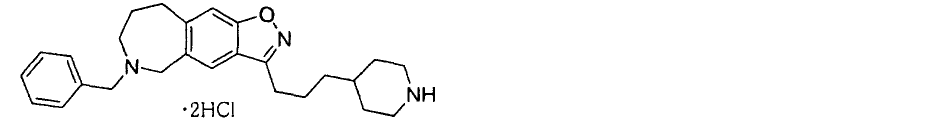

According to the same procedures as those of Example 2A)-2) using 3-[3-(1-acetyl-4-piperidinyl)propyl]-6-(phenylmethyl)-6,7,8,9-tetrahydro-5H-isoxazolo[5,4-h][2]benzazepine obtained in Example 5A), the title compound was obtained as a colorless amorphous powder.
¹H NMR (CDCl₃, free base) δ 0.95-2.10 (12H, m), 2.45-2.65 (2H, m), 2.90 (2H, t, J = 7.6 Hz), 2.95-3.10 (4H, m), 3.15 (2H, t-like, J = 5.2 Hz), 3.54 (2H, s), 3.97 (2H, s), 7.14 (1H, s), 7.20-7.40 (6H, m).
Elemental analysis for C₂₆H₃₃N₃O·2HCl·0.5H₂O
Calcd.: C, 64.32; H, 7.47; N, 8.66
Found: C, 64.07; H, 7.62; N, 8.23

### Example 7A

6-(Phenylmethyl)-3-[3-[1-(phenylmethyl)-4-piperidinyl]propyl]-6,7,8,9-tetrahydro-5H-isoxazolo[5,4-h][2]benzazepine dihydrochloride

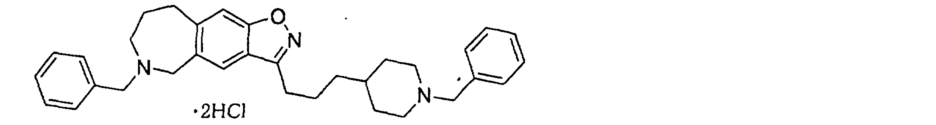

According to the same procedures as those of Example 2A)-3) using 6-(phenylmethyl)-3-[3-(4-piperidinyl)propyl]-6,7,8,9-tetrahydro-5H-isoxazolo[5,4-h][2]benzazepine dihydrochloride obtained in Example 6A), the title compound was obtained as a colorless amorphous powder.
¹H NMR (CDCl₃, free base) δ 1.10-1.45 (4H, m), 1.55-2.00 (9H, m), 2.80-2.95 (4H, m), 3.00-3.10 (2H, m), 3.14 (2H, t-like, J = 5.2 Hz), 3.47 (2H, s), 3.52 (2H, s), 3.95 (2H, s), 7.12 (1H, s), 7.20-7.40 (11H, m).
Elemental analysis for C₃₃H₃₉N₃O·2HCl·H₂O
Calcd.: C, 67.80; H, 7.41; N,7.19
Found: C, 67.86; H, 7.43; N,7.23

### Example 8A

3-[3-[1-[(2-Chlorophenyl)methyl]-4-piperidinyl]propyl]-6-(phenylmethyl)-6,7,8,9-tetrahydro-5H-isoxazolo[5,4-h][2]benzazepine dihydrochloride

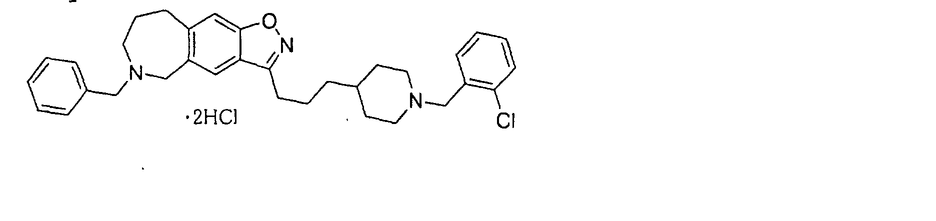

According to the same procedures as those of Example 2A)-3) using 6-(phenylmethyl)-3-[3-(4-piperidinyl)propyl]-6,7,8,9-tetrahydro-5H-isoxazolo[5,4-h][2]benzazepine dihydrochloride obtained in Example 6A), the title compound was obtained as a colorless amorphous powder.
¹H NMR (CDCl₃, free base) δ 1.15-1.45(4H, m), 1.55-2.20(9H, m), 2.80-2.95(4H, m), 3.00-3.10(2H, m), 3.16(2H, t-like, J = 5.2 Hz), 3.53(2H, s), 3.60(2H, s), 3.96(2H, s), 7.10-7.40(10H, m), 7.48(1H, dd, J = 8.0, 2.0 Hz).
Elemental analysis for C₃₃H₃₈ClN₃O·2HCl·H₂O
Calcd.: C, 64.02; H, 6.84; N,6.79
Found: C, 64.29; H, 6.84; N,6.67

### Example 9A

3-[3-[1-[(2-Chlorophenyl)methyl]-4-piperidinyl]propyl]-6-(phenylmethyl)-6,7,8,9-tetrahydro-5H-isoxazolo[5,4-h][2]benzazepine dihydrochloride

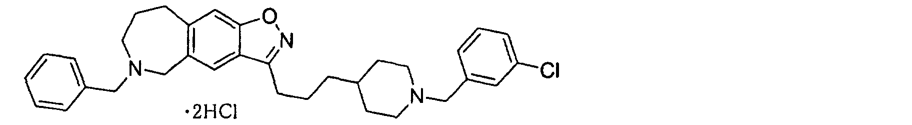

According to the same procedures as those of Example 2A)-3) using 6-(phenylmethyl)-3-[3-(4-piperidinyl)propyl]-6,7,8,9-tetrahydro-5H-isoxazolo[5,4-h][2]benzazepine dihydrochloride obtained in Example 6A), the title compound was obtained as a colorless amorphous powder.
¹H NMR (CDCl₃, free base) δ 1.10-1.45 (4H, m), 1.55-2.00 (9H, m), 2.75-2.95 (4H, m), 3.00-3.10 (2H, m), 3.14 (2H, t-like, J = 5.2 Hz), 3.43 (2H, s), 3.53 (2H, s), 3.96 (2H, s), 7.13 (1H, s), 7.15-7.40 (10H, m).
Elemental analysis for C₃₃H₃₈ClN₃O·2HCl·H₂O
Calcd.: C, 64.02; H, 6.84; N,6.79
Found: C, 64.01; H, 7.02; N,6.58

### Example 10A

3-[3-[1-[(4-Chlorophenyl)methyl]-4-piperidinyl]propyl]-6-(phenylmethyl)-6,7,8,9-tetrahydro-5H-isoxazolo[5,4-h][2]benzazepine dihydrochloride

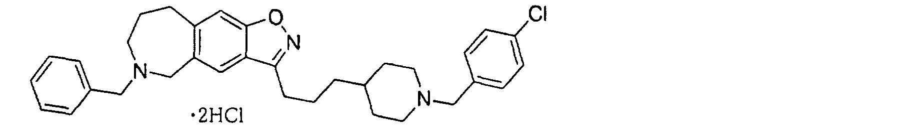

According to the same procedures as those of Example 2A)-3) using 6-(phenylmethyl)-3-[3-(4-piperidinyl)propyl]-6,7,8,9-tetrahydro-5H-isoxazolo[5,4-h][2]benzazepine dihydrochloride obtained in Example 6A), the title compound was obtained as a colorless amorphous powder.
¹H NMR (CDCl₃, free base) δ 1.10-1.45 (4H, m), 1.55-2.00 (9H, m), 2.75-2.95 (4H, m), 3.00-3.10 (2H, m), 3.14 (2H, t-like, J = 5.2 Hz), 3.43 (2H, s), 3.53 (2H, s), 3.96 (2H, s), 7.13 (1H, s), 7.20-7.40 (10H. m).
Elemental analysis for C₃₃H₃₈ClN₃O·2HCl·H₂O
Calcd.: C, 64.02; H, 6.84; N,6.79
Found: C, 63.71; H, 6.93; N,6.48

### Example 11A

3-[3-(1-Acetyl-4-piperidinyl)propyl]-5-(trifluoroacetyl)-6,7,8,9-tetrahydro-5H-isoxazolo[5,4-h][1]benzazepine

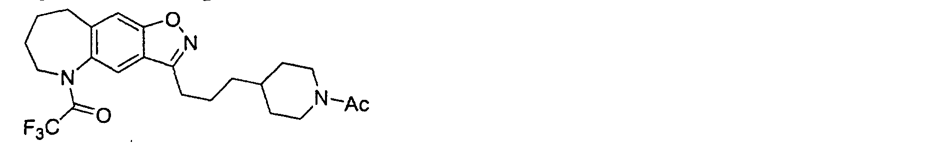

According to the same procedures as those of Example 1A)-3) using 4-(1-acetyl-4-piperidinyl)-1-[7-hydroxy-1-(trifluoroacetyl)-2,3,4,5-tetrahydro-1H-1-benzazepine-8-yl]-1-butanone oxime obtained in Reference Example 4A), the title compound was obtained as colorless crystals having a melting point of 108-110°C.
¹H NMR (CDCl₃) δ 0.97-1.63 (6H, m), 1.63-2.12 (10H, m), 2.42-2.61 (1H, m), 2.75-3.11 (6H, m), 3.70-3.86 (1H, m), 4.52-4.80 (2H, m), 7.46 (1H, s), 7.47 (1H, s).
Elemental analysis for C₂₃H₂₈F₃N₃O₃
Calcd.: C, 61.19; H, 6.25; N,9.31
Found: C, 60.92; H, 6.24; N,9.24

### Example 12A

3-[3-[1-(Phenylmethyl)-4-piperidinyl)propyl]-6,7,8,9-tetrahydro-5H-isoxazolo[5,4-h][1]benzazepine dihydrochloride

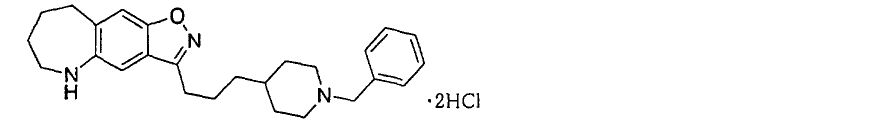

According to the same procedures as those of Example 2A) using 3-[3-(1-acetyl-4-piperidinyl)propyl]-5-(trifluoroacetyl)-6,7,8,9-tetrahydro-5H-isoxazolo[5,4-h][1]benzazepine obtained in Example 11A, the title compound was obtained as a colorless crystal having a melting point of 124-127°C.
¹H NMR (CDCl₃, free base) 6 1.17-1.44(5H, m), 1.53-2.02 (10H, m), 2.80-2.96 (6H, m), 3.04 (2H, dd, J=5.0, 5.4Hz), 3.50 (2H, s), 3.57 (1H, br), 6.94 (1H, s), 7.18-7.35 (6H, m).
Elemental analysis for C₂₆H₃₃N₃O·2HCl·2H₂O
Calcd.: C, 60.93; H, 7.67; N,8.20
Found: C, 60.82; H, 7.66; N,8.53

### Test Example 1A

Measurement of the CAMP concentration increasing activity in fat cells using mouse-derived pre-fat cell strain (3T3-L1)

The cAMP concentration increasing activity of Compound (IA) in fat cells was examined using a mouse-derived pre-fat cell strain (3T3-L1). That is, 3T3-L1 cells were seeded on a 96-well microtiter plate (10,000 cells/well), and cultured to confluence for 5 to 6 days. After cultured for 72 hours after confluence, the above Compound (IA) (10⁻⁶M, 10⁻⁷M, 10⁻⁸M and 10⁻⁹M) was added and allowed to stand at 37°C for 40 minutes (100 µl/well). After the cells were washed three times with a phosphate buffer at 4°C, 0.1N hydrochloric acid was added, which was boiled at 95°C for 10 minutes. 25 µl was taken from each well, which was dissolved in 75 µl of an assay buffer attached to Cyclic AMP Enzyme Immunoassay Kit (manufactured by Keiman Chemical Company, USA) and 50 µl of the mixture was quantitated as a sample using the above kit. That is, the above sample (50 µl), Cyclic AMP Tracer (50 µl) attached to the above kit and Cyclic AMP rabbit antibody (50 µl) attached to the above kit were added to an anti-rabbit IgG mouse antibody-solid phased 96-well microtiter plate, which was allowed to stand at room temperature for 18 hours. After each well was aspirated, the mixture was washed with a washing solution four times (400 µl/well). Then, 200 µl of a chromogenic reagent attached to the kit was added to each well, which was incubated at room temperature for 60 minutes while shaking. After completion of the reaction, the amount of cAMP was quantitated by measuring the absorbance at the wavelength of 405 nm.

The CAMP amounts at respective concentrations when a subject compound was added at 10⁻⁶M, 10⁻⁷M, 10⁻⁸M and 10⁻⁹M, are shown in [Table 2]. The values are an average of four experiments. In addition, significance test relative to the cAMP concentration value in a control experiment (without addition of a subject compound) was performed by the known ANOVA method.
(*p<0.05 vs control)

**[Table 2]**

| cAMP concentration in fat cells (pmol/ml) | | | | | |
|---|---|---|---|---|---|
| Compound No. (Example No.) | Compound concentration | | | | |
| | 10⁻⁶M | 10⁻⁷M | 10⁻⁸M | 10⁻⁹M | Control |
| 2A | 440.6* | 49.0* | 16.3* | 11.0* | 2.4 |

From [Table 2], it can be seen that Compound (IA) or a salt thereof has an excellent cAMP concentration increasing activity in fat cells.

When the cAMP concentration in fat cells is increased, since the thermal production is increased (Newest Medical, vol. 52, No.6, pp.1093-1100, 1997), it can be said that Compound (IA) or a salt thereof has an excellent thermal production promoting activity.

### Preparation 1A

| | |
|---|---|
| (1) 3-[3-[1-(phenylmethyl)-4-piperidinyl]propyl]-7-(phenylmethyl)-6,7,8,9-tetrahydro-5H-isoxazolo[4,5-h][3]benzazepine dihydrochloride (compound of Example 2A) | 1 g |
| (2) Lactose | 197 g |
| (3) Corn starch | 50 g |
| (4) Magnesium stearate | 2 g |

One g of the above (1), 197 g of the above (2) and 20 g of corn starch were kneaded, which was granulated together with a paste made from 15 g of corn starch and 25 ml of water, to this were added 15 g of corn starch and 2 g of the above (4), the mixture was compressed with a compression tabletting machine to prepare 2000 tablets having a diameter of 3 mm containing 0.5 mg of the above (1) per one tablet.

### Preparation 2A

| | |
|---|---|
| (1) 3-[3-[1-(phenylmethyl)-4-piperidinyl]propyl]-7-(phenylmethyl)-6,7,8,9-tetrahydro-5H-isoxazolo[4,5-h][3]benzazepine dihydrochloride (compound of Example 2A) | 2 g |
| (2) Lactose | 197 g |
| (3) Corn starch | 50 g |
| (4) Magnesium stearate | 2 g |

2 g of the above (1), 197 g of the above (2) and 20 g of corn starch were kneaded, which was granulated together with a paste made from 15 g of corn starch and 25 ml of water, to this were added 15 g of corn starch and 2 g of the above (4), the mixture was compressed with a compression tabletting machine to prepare 2000 tablets having a diameter of 3 mm containing 1.0 mg of the above (1) per one tablet.

### Preparation 3A

| | |
|---|---|
| (1) 3-[3-[1-(phenylmethyl)-4-piperidinyl]propyl]-7-(phenylmethyl)-6,7,8,9-tetrahydro-5H-isoxazolo[4,5-h][3]benzazepine dihydrochloride (compound of Example 2A) | 25 g |
| (2) Lactose | 80 g |
| (3) Corn starch | 42 g |
| (4) Talc powder | 3 g |
| (5) Magnesium stearate | 0.5 g |

25 g of the above (1), 80 g of the above (2) and 21 g of corn starch were kneaded, which was granulated together with a paste made from 10 g of corn starch and 9 ml of water, to this were added 11 g of corn starch, 3 g of the above (4) and 0.5 g of the above (5), the mixture was compressed with a compression tabletting machine to prepare 1000 tablets having a diameter of 3 mm containing 25 mg of the above (1) per one tablet.

### Preparation 4A

| | |
|---|---|
| (1) 3-[3-[1-(phenylmethyl)-4-piperidinyl]propyl]-7-(phenylmethyl)-6,7,8,9-tetrahydro-5H-isoxazolo[4,5-h][3]benzazepine dihydrochloride (compound of Example 2A) | 0.5 mg |
| (2) Lactose | 60.0 mg |
| (3) Corn starch | 35.0 mg |
| (4) Gelatin | 3.0 mg |
| (5) Magnesium stearate | 2.0 mg |

After a mixture of 10.0 mg of the above (1), 60 mg of the above (2) and 35 mg of the above (3) was granulated using 0.03 ml of a 10% aqueous gelatin solution (3.0 mg in terms of gelatin) and 1 mm mesh sieve, the granules were dried at 40°C and passed again through a sieve. The resulting granules were mixed with 2.0 mg of the above (5), and compressed. The resulting core tablets were coated with a sugar-coating by an aqueous suspension of sucrose, titanium dioxide, talc and gum arabic. The coated tablets were polished with a beewax to obtain coated tablets.

### INDUSTRIAL APPLICABILITY

Compound (1), (I'), (IA) or a salt thereof has a lipolysis promoting activity, a thermal production promoting activity, a body weight decreasing activity (more strictly, a body fat percentage lowering activity) and activity of inhibiting a body weight increase, and is useful as a novel agent for preventing or treating obesity and obesity-based diseases.

## Claims

1. A compound represented by the formula: 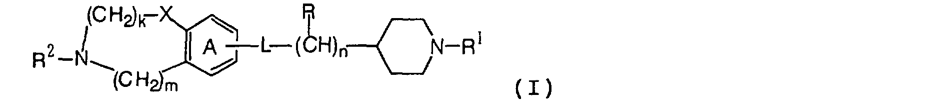 wherein ring A represents benzene ring optionally having a further substituent, -L- represents -O-, -NR^{3a}-, -S-, -SO-, -SO₂-, -SO₂NR^{3a}-, -SO₂NHCONR^{3a}-, -SO₂NHC(=NH)NR^{3a}-, -C(=S)-, 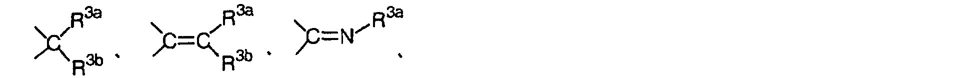 or -CONR^{3a}- (wherein R^{3a} and R^{3b} represent independently hydrogen atom, cyano group, hydroxy group, amino group, a C₁₋₆ alkyl group or a C₁₋₆ alkoxy group), n represents an integer of 0 to 6, R is hydrogen atom or a hydrocarbon group optionally having a substituent, and may be different in repetition of n, R¹ represents a hydrocarbon group optionally having a substituent or a group represented by the formula: 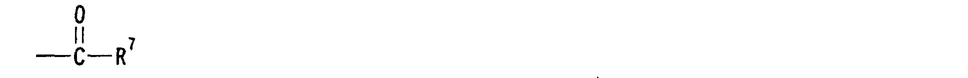 (wherein R⁷ represents a hydrocarbon group optionally having a substituent, R² represents hydrogen atom, an acyl group, a hydrocarbon group optionally having a substituent or a heterocyclic group optionally having a substituent, X represents a bond, O, S, SO, SO₂ or NR⁴ (wherein R⁴ represents hydrogen atom, an acyl group or a hydrocarbon group optionally having a substituent), k and m represent independently an integer of 0 to 5, and 1<k+m<5, or a salt thereof.

2. The compound according to claim 1, wherein n is an integer of 1 to 6.

3. The compound according to claim 1, wherein -L- is -O-, -S-, -SO-, -SO₂-, -CH₂-, -CHOH-,

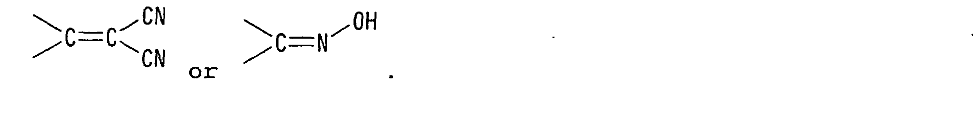

4. The compound according to claim 1, wherein X is a bond and k=m=2.

5. The compound according to claim 1, wherein X is a bond, k=3 and m=1.

6. The compound according to claim 1, wherein X is O, k=2 and m=1.

7. The compound according to claim 1, wherein R is hydrogen atom.

8. The compound according to claim 1, wherein n is an integer of 2 to 4.

9. The compound according to claim 1, wherein R¹ is a C₇₋₁₆ aralkyl group optionally having a substituent.

10. The compound according to claim 1, wherein R² is a C₇₋₁₆ aralkyl group optionally having a substituent.

11. The compound according to claim 1, wherein R is hydrogen atom, n is an integer of 2 to 4, and R¹ and R² are benzyl group optionally having a substituent.

12. The compound according to claim 1, which is (i) 2-[(2-methylphenyl)methyl]-7-[2-[1-[[2-(trifluoromethyl)phenyl]methyl]-4-piperidinyl]ethoxy]-2,3,4,5-tetrahydro-lH-2-benzazepine, (ii) 2-[(2-methylphenyl)methyl]-8-[2-[1-[(4-chlorophenyl)methyl)-4-piperidinyl]ethoxy]-2,3,4,5-tetrahydro-1H-2-benzazepine, (iii) 1-(4-pyridyl)-5-[1-hydroxy-3-[1-(phenylmethyl)-4-piperidinyl]propyl]-2,3-dihydroindole, (iv) 3-[1-(phenylmethyl)-4-piperidinyl]-1-[3-(phenylmethyl)-2,3,4,5-tetrahydro-1H-3-benzazepin-7-yl]-1-propanone oxime, (v) 2-[1-[3-(phenylmethyl)-2,3,4,5-tetrahydro-1H-3-benzazepin-7-yl]-3-[1-(phenylmethyl)-4-piperidinyl]propylidene]malononitrile, (vi) 3-(phenylmethyl)-7-[[2-[1-(phenylmethyl)-4-piperidinyl]ethyl]sulfanyl]-2,3,4,5-tetrahydro-1H-3-benzazepine, (vii) 7-[[2-[1-[(2-chlorophenyl)methyl]-4-piperidinyl]ethyl]sulfinyl]-3-(phenylmethyl)-2,3,4,5-tetrahydro-1H-3-benzazepine, (viii) 7-[[2-[1-[(4-chlorophenyl)methyl]-4-piperidinyl]ethyl]sulfinyl]-3-(phenylmethyl)-2,3,4.5-tetrahydro-1H-3-benzazepine, (ix) 7-[[2-[1-[(3-chlorophenyl)methyl]-4-piperidinyl]ethyl]sulfonyl]-3-(phenylmethyl)-2,3,4,5-tetrahydro-1H-3-benzazepine, (x) 8-[3-[1-[[3-(4,5-dihydro-1H-2-imidazolyl)phenyl]methyl]-4-piperidinyl]propoxy]-2-[(4-fluorophenyl)methyl]-2,3,4,5-tetrahydro-1H-2-benzazepine, (xi) 4-[[4-[2-[[2-[(2-methylphenyl)methyl]-2,3,4,5-tetrahydro-1H-2-benzazepin-8-yl]oxy]ethyl]-1-piperidinyl]methyl]-1-benzenecarboxyimidamide, (xii) 8-[2-[1-[[4-(4,5-dihydro-1H-2-imidazolyl)phenyl)methyl]-4-piperidinyl]ethoxy]-2-[(2-methylphenyl)methyl]-2,3,4,5-tetrahydro-1H-2-benzazepine, (xiii) 2-(phenylmethyl)-8-[2-[1-[[4-(N,N-diethylaminomethyl)phenyl]methyl]-4-piperidinyl]ethoxy]-2,3,4,5-tetrahydro-1H-2-benzazepine, (xiv) 2-[(2-methylphenyl)methyl]-8-[2-[1-[[3-(4,5-dihydro-1H-2-imidazolyl)phenyl]methyl]-4-piperidinyl]ethoxy]-2,3,4,5-tetrahydro-1H-2-benzazepine, (xv) 2-[(2-methylphenyl)methyl]-8-[2-[1-[4-(4,5-dihydro-1H-2-imidazolyl)benzoyl]-4-piperidinyl]ethoxy]-2,3,4,5-tetrahydro-1H-2-benazepine, (xvi) 2-(phenylmethyl)-7-[[1-[[4-(4,5-dihydro-1H-2-imidazolyl)phenyl]methyl]-4-piperidinyl]methoxy]-2,3,4,5-tetrahydro-1H-2-benazepine, (xvii) 2-(phenylmethyl)-8-[[1-[[4-(4,5-dihydro-1H-2-imidazolyl)phenyl]methyl]-4-piperidinyl]methoxy]-2,3,4,5-tetrahydro-1H-2-benzazepine, (xviii) 2-(phenylmethyl)-8-[2-[1-[[4-(4,5-dihydro-1H-2-imidazolyl)phenyl]methyl]-4-piperidinyl]ethoxy]-2,3,4,5-tetrahydro-1H-2-benzazepine, or (xix) 2-(phenylmethyl)-8-[2-[1-[(4-dimethylaminophenyl)methyl]-4-piperidinyl]ethoxy]-2,3,4,5-tetrahydro-1H-2-benzazepine, or a salt thereof.

13. A prodrug of the compound according to claim 1.

14. A process for producing the compound according to claim 1, which comprises reacting a compound represented by the formula: 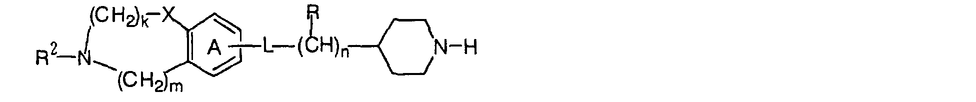 wherein respective symbols represent the same meanings as those for claim 1 or a salt thereof with a compound represented by the formula:
R¹―Z¹
wherein Z¹ represents a leaving group and R¹ represents the same meaning as that for claim 1 or a salt thereof.

15. A compound represented by the formula: 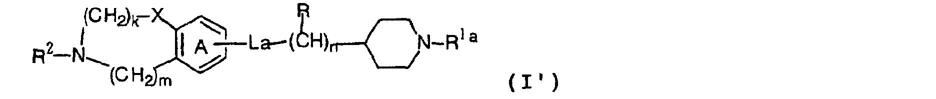 wherein ring A represents benzene ring optionally having a further substituent, -L^{a}- represents -NR^{3a}-, -S-, -SO-, -SO₂-, -SO₂NR^{3a}-, -SO₂NHCONR^{3a}-, -SO₂NHC(=NH)NR^{3a}-, -C(=S)-, 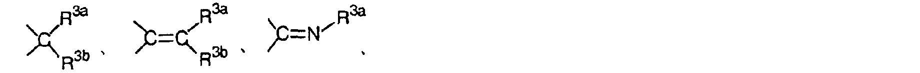 or -CONR^{3a}- (wherein R^{3a} and R^{3b} represent independently hydrogen atom, cyano group, hydroxy group, amino group, a C₁₋₆ alkyl group or a C₁₋₆ alkoxy group), n represents an integer of 0 to 6, R is hydrogen atom or a hydrocarbon group optionally having a substituent, and may be different in repetition of n, R^{1a} represents hydrogen atom or a group represented by the formula: 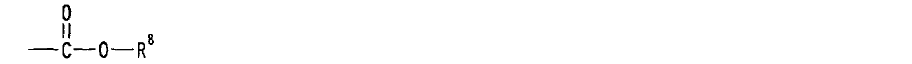 (wherein R⁸ represents a hydrocarbon group optionally having a substituent), R² represents hydrogen atom, an acyl group, a hydrocarbon group optionally having a substituent or a heterocyclic group optionally having a substituent, X represents a bond, O, S, SO, SO₂ or NR⁴ (wherein R⁴ represents hydrogen atom, an acyl group or a hydrocarbon group optionally having a substituent), k and m represent independently an integer of 0 to 5, 1<k+m<5, or a salt thereof.

16. A compound represented by the formula: 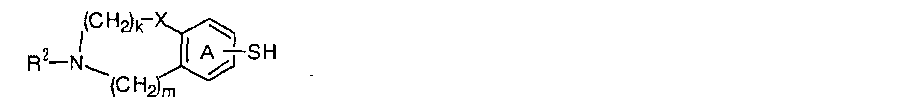 wherein ring A represents benzene ring optionally having a further substituent, R² represents hydrogen atom, an acyl group, a hydrocarbon group optionally having a substituent or a heterocyclic group optionally having a substituent, X represents a bond, O, S, SO, SO₂ or NR⁴ (wherein R⁴ represents hydrogen atom, an acyl group or a hydrocarbon group optionally having a substituent), k and m represent independently an integer of 0 to 5, 1<k+m<5, or a salt thereof.

17. A pharmaceutical composition comprising a compound represented by the formula: 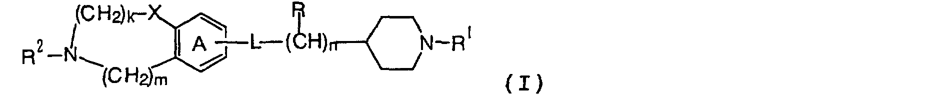 wherein ring A represents benzene ring optionally having a further substituent, -L-represents -O-, -NR^{3a}- , -S-, -SO-, -SO₂-, -SO₂NR^{3a}-, -SO₂NHCONR^{3a}-, -SO₂NHC(=NH)NR^{3a}-, -C(=S)-, 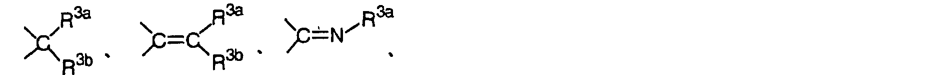 or -CONR^{3a}- (wherein R^{3a} and R^{3b} represent independently hydrogen atom, cyano group, hydroxy group, amino group, a C₁₋₆ alkyl group or a C₁₋₆ alkoxy group), n represents an integer of 0 to 6, R is hydrogen atom or a hydrocarbon group optionally having a substituent, and may be different in repetition of n, R¹ represents a hydrocarbon group optionally having a substituent or a group represented by the formula: 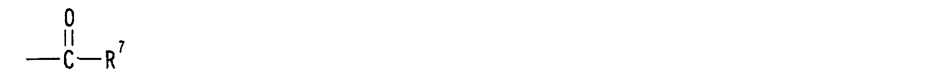 (wherein R⁷ represents a hydrocarbon group optionally having a substituent), R² represents hydrogen atom, an acyl group, a hydrocarbon group optionally having a substituent or a heterocyclic group optionally having a substituent, X represents a bond, O, S, SO, SO₂ or NR⁴ (wherein R⁴ represents hydrogen atom, an acyl group or a hydrocarbon group optionally having a substituent), k and m represent independently an integer of 0 to 5, and 1<k+m<5, or a salt thereof or a prodrug thereof.

18. The composition according to claim 17, which is a thermal production promoting agent.

19. The composition according to claim 18, which is an anti-obesity agent.

20. The composition according to claim 18, which is a lipolysis promoting agent.

21. The composition according to claim 18, which is an agent for preventing or treating obesity-based diseases.

22. A method for treating obesity and obesity-based diseases, which comprises administering an effective amount of the compound according to claim 1 to a mammal.

23. Use of the compound according to claim 1 for producing a thermal production promoting agent.

24. A compound represented by the formula: 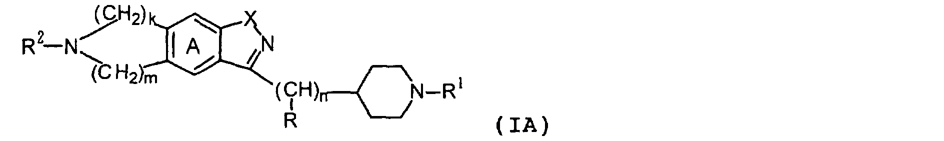 wherein ring A represents benzene ring-optionally having a substituent, k and m represent independently an integer of 0 to 5, 1<k+m<5, n represents an integer of 1 to 6, R represents hydrogen atom or a hydrocarbon group optionally having a substituent, and may be different in repetition of n, R¹ and R² represent independently hydrogen atom, an acyl group or a hydrocarbon group optionally having a substituent, and X represents O or S, or a salt thereof.

25. The compound according to claim 24, wherein k=m=2.

26. The compound according to claim 24, wherein k=3 and m=1.

27. The compound according to claim 24, wherein R is hydrogen atom.

28. The compound according to claim 24, wherein n is an integer of 2 to 4.

29. The compound according to claim 24, wherein R¹ is a C₇₋₁₆ aralkyl group optionally having a substituent.

30. The compound according to claim 24, wherein R² is a C₇₋₁₆ aralkyl group optionally having a substituent.

31. The compound according to claim 24, wherein X is O.

32. The compound according to claim 24, wherein R is hydrogen atom, n is an integer of 2 to 4, R¹ and R² are benzyl group optionally having a substituent.

33. The compound according to claim 24, which is 3-[3-[1-(phenylmethyl)-4-piperidinyl]propyl]-7-(phenylmethyl)-6,7,8,9-tetrahydro-5H-isoxazolo[4,5-h][3]benzazepine; 3-[3-[1-[(2-chlorophenyl)methyl]-4-piperidinyl]propyl]-6-(phenylmethyl)-6,7,8,9-tetrahydro-5H-isoxazolo[5,4-h][2]benzazepine; or 3-[3-[1-(phenylmethyl)-4-piperidinyl]propyl]-6,7,8,9-tetrahydro-5H-isoxazolo[5,4-h][1]benzazepine or a salt thereof.

34. A prodrug of the compound according to claim 24.

35. A process for producing the compound according to claim 24, which comprises (i) ring-closing a compound represented by the formula: 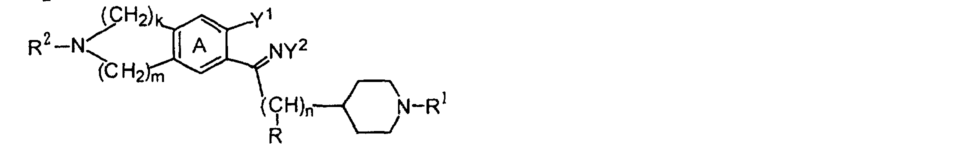 wherein Y¹ represents OZ^{a}, SZ^{a} (wherein Z^{a} represents hydrogen atom, a halogen atom, an alkyl group, or an acyl group), nitro group or a halogen atom, Y² represents hydrogen atom or OZ^{b} (wherein Z^{b} represents hydrogen atom or an acyl group) and other symbols represent the same meanings as those for claim 24 or a salt thereof, or
(ii) reacting a compound represented by the formula: 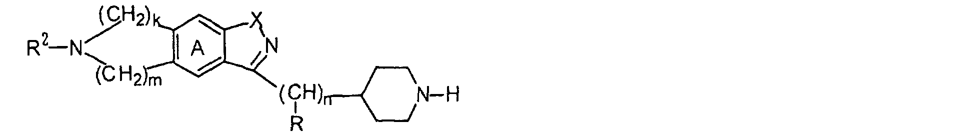 wherein respective symbols represent the same meanings as those for claim 24 or a salt thereof, with a compound represented by the formula:
R¹―Z¹
wherein Z¹ represents a leaving group, and R¹ represents the same meaning as that for claim 24 or a salt thereof, or
(iii) reacting a compound represented by the formula: 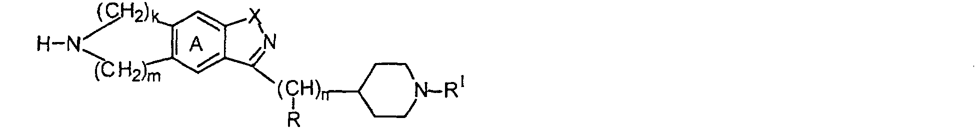 wherein respective symbols represent the same meanings as those for claim 24 or a salt thereof, with a compound represented by the formula:
R²―Z¹
wherein Z¹ represents a leaving group, and R² represents the same meaning as that for claim 24 or a salt thereof, or
(iv) reacting a compound represented by the formula: 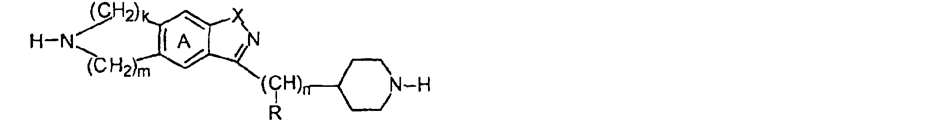 wherein respective symbols represent the same meanings as those for claim 24 or a salt thereof, with a compound represented by the formula:
R¹―Z¹
wherein Z¹ represents a leaving group, and R¹ represents the same meaning as that for claim 24 or a salt thereof.

36. A pharmaceutical composition comprising a compound represented by the formula: 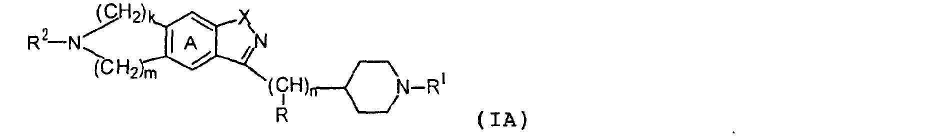 wherein ring A represents benzene ring optionally having a substituent, k and m represent independently an integer of 0 to 5, 1<k+m<5, n represents an integer of 1 to 6, R represents hydrogen atom or a hydrocarbon group optionally having a substituent, and may be different in repetition of n, R¹ and R² represent independently hydrogen, an acyl group or a hydrocarbon group optionally having a substituent, and X represents O or S, or a salt thereof or a prodrug thereof.

37. The composition according to claim 36, which is a thermal production promoting agent.

38. The composition according to claim 37, which is an anti-obesity agent.

39. The composition according to claim 37, which is a lipolysis promoting agent.

40. The composition according to claim 37, which is an agent for preventing or treating obesity-based diseases.

41. A method for treating obesity or obesity-based diseases, which comprises administering an effective amount of the compound according to claim 24 to a mammal.

42. Use of the compound according to claim 24 for producing a thermal production promoting agent.
